# EUROPEAN PATENT APPLICATION

(11) **EP 4 104 867 A2**
(43) Date of publication of application: **21.12.2022**
(21) Application number: 22166699.3
(22) Date of filing: 14.12.2016
(51) Int. Cl.: A61K 48/00, C12N 15/113, C12Q 1/68

(54) **COMPOSITIONS AND METHODS FOR TREATMENT OF CENTRAL NERVOUS SYSTEM DISEASES**

(30) Priority: 14.12.2015 US 201562267256 P; 06.04.2016 US 201662319011 P
(62) Divisional of application: 16876621.0
(71) Applicant: COLD SPRING HARBOR LABORATORY, Cold Spring Harbor, NY 11724 (US); Stoke Therapeutics, Inc., Bedford, Massachusetts 01730 (US)
(72) Inventor: AZNAREZ, Isabel, Cambridge, 02139 (US); NASH, Huw M., Lexington, 02421 (US); KRAINER, Adrian, East Northport, 11731 (US)
(74) Representative: Avidity IP

(57) **Abstract**

Provided herein are methods and compositions for increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B, and for treating a subject in need thereof, e.g., a subject with deficient ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein expression.

## Description

### CROSS-REFERENCE

This application claims the benefit of U.S. Provisional Application No. 62/267,256, filed December 14, 2015, and U.S. Provisional Application No. 62/319,011, filed April 6, 2016.

### SEQUENCE LISTING

The instant application contains a Sequence Listing which has been submitted in computer-readable format in accordance with WIPO Standard ST.25.

### BACKGROUND OF THE INVENTION

Certain diseases affecting central nervous system function are associated with a deficiency in the expression of a gene, and in turn, a deficiency in the gene product. Examples of gene products for which increased expression can provide benefit in central nervous system diseases or conditions include, ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA and STX1B.

### SUMMARY OF THE INVENTION

The invention provides compositions and methods for treating a central nervous system (CNS) disease or conditions, including antisense oligomers (ASOs) that promote constitutive splicing at intron splice sites of a retained-intron-containing pre-mRNA (RIC pre-mRNA) encoding a gene product that is deficient in the central nervous system disease or condition. The invention further provides compositions and methods for increasing production of mature mRNA encoding a gene product, wherein either the gene product is deficient in a CNS disease or condition, or wherein increased expression of the gene product could provide benefit in a subject having a CNS disease or condition. Increasing the production of the mature mRNA results in increased production of the encoded protein in cells of a subject in need thereof, for example, any subject that can benefit from increased production of the gene product. The described methods may be used to treat subjects having a CNS disease or condition caused by a gene mutation(s), including missense, splicing, frameshift and nonsense mutations, as well as whole gene deletions, that result in deficient protein production.

Disclosed herein, in certain embodiments, is a method of treating a CNS disease in a subject in need thereof by increasing the expression of a target protein or functional RNA by cells of the subject, wherein the cells have a retained-intron-containing pre-mRNA (RIC pre-mRNA), the RIC pre-mRNA comprising a retained intron, an exon flanking the 5' splice site, an exon flanking the 3' splice site, and wherein the RIC pre-mRNA encodes the target protein or functional RNA, the method comprising contacting the cells of the subject with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of the target protein or functional RNA in the cells of the subject. In some embodiments, the CNS disease is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder la, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9. In some embodiments, also described herein is a method of increasing expression of a target protein, wherein the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B, by cells having a retained-intron-containing pre-mRNA (RIC pre-mRNA), the RIC pre-mRNA comprising a retained intron, an exon flanking the 5' splice site of the retained intron, an exon flanking the 3' splice site of the retained intron, and wherein the RIC pre-mRNA encodes ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, the method comprising contacting the cells with an antisense oligomer (ASO) complementary to a targeted portion of the RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, whereby the retained intron is constitutively spliced from the RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, thereby increasing the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein in the cells. In some embodiments, the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B. In some embodiments, the target protein is ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B. In some embodiments, the target protein or the functional RNA is a compensating protein or a compensating functional RNA that functionally augments or replaces a target protein or functional RNA that is deficient in amount or activity in the subject. In some embodiments, the cells are in or from a subject having a condition caused by a deficient amount or activity of ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B protein. In some embodiments, the cells are in or from a subject having a condition caused by a deficient amount or activity of ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein. In some embodiments, the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a RIC pre-mRNA transcribed from the first allele. In some embodiments, the subject has a condition caused by a disorder resulting from a deficiency in the amount or function of the target protein, wherein the subject has (a) a first mutant allele from which (i) the target protein is produced at a reduced level compared to production from a wild-type allele, (ii) the target protein is produced in a form having reduced function compared to an equivalent wild-type protein, or (iii) the target protein is not produced, and (b) a second mutant allele from which (i) the target protein is produced at a reduced level compared to production from a wild-type allele, (ii) the target protein is produced in a form having reduced function compared to an equivalent wild-type protein, or (iii) the target protein is not produced, and wherein when the subject has a first mutant allele a(iii), the second mutant allele is b(i) or b(ii), and wherein when the subject has a second mutant allele b(iii), the first mutant allele is a(i) or a(ii), and wherein the RIC pre-mRNA is transcribed from either the first mutant allele that is a(i) or a(ii), and/or the second allele that is b(i) or b(ii). In some embodiments, the target protein is produced in a form having reduced function compared to the equivalent wild-type protein. In some embodiments, the target protein is produced in a form that is fully-functional compared to the equivalent wild-type protein. In some embodiments, the targeted portion of the RIC pre-mRNA is in the retained intron within the region +6 relative to the 5' splice site of the retained intron to -16 relative to the 3' splice site of the retained intron. In some embodiments, the targeted portion of the RIC pre-mRNA is in the retained intron within: (a) the region +6 to +4000, +6 to + 3000, +6 to +2000 or +6 to +100 relative to the 5' splice site of the retained intron; or (b) the region -16 to -4000, -16 to -2000, -16 to -1000, -16 to -500 or -16 to -100 relative to the 3' splice site of the retained intron. In some embodiments, the targeted portion of the RIC pre-mRNA is within: (a) the region +2e to -4e in the exon flanking the 5' splice site of the retained intron; or (b) the region +2e to -4e in the exon flanking the 3' splice site of the retained intron. In some embodiments, the RIC pre-mRNA is encoded by a genetic sequence with at least about 80%, 85%, 90%, 95%, 96%, 97%, 98%, 99% or 100% sequence identity to SEQ ID NOs: 1-19. In some embodiments, the RIC pre-mRNA comprises a sequence with at least about 80%, 85%, 90%, 95%, 96%, 97%, 98%, 99% or 100% sequence identity to any one of SEQ ID NOs: 20-80. In some embodiments, the antisense oligomer does not increase the amount of the target protein or the functional RNA by modulating alternative splicing of pre-mRNA transcribed from a gene encoding the functional RNA or target protein. In some embodiments, the antisense oligomer does not increase the amount of the target protein or the functional RNA by modulating aberrant splicing resulting from mutation of the gene encoding the target protein or the functional RNA. In some embodiments, the RIC pre-mRNA was produced by partial splicing of a full-length pre-mRNA or partial splicing of a wild-type pre-mRNA. In some embodiments, the mRNA encoding the target protein or functional RNA is a full-length mature mRNA, or a wild-type mature mRNA. In some embodiments, the target protein produced is full-length protein, or wild-type protein. In some embodiments, the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell. In some embodiments, the total amount of target protein produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of target protein produced by a control cell. In some embodiments, the antisense oligomer comprises a backbone modification comprising a phosphorothioate linkage or a phosphorodiamidate linkage. In some embodiments, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some embodiments, the antisense oligomer comprises at least one modified sugar moiety. In some embodiments, each sugar moiety is a modified sugar moiety. In some embodiments, the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some embodiments, the antisense oligomer is at least 80%, at least 85%, at least 90%, at least 95%, at least 98%, at least 99%, or 100%, complementary to the targeted portion of the RIC pre-mRNA encoding the protein. In some embodiments, the targeted portion of the RIC pre-mRNA is within a sequence selected from SEQ ID NOs: 24351-24354, 24356-24378, 24380-24386, or 28515-28516. In some embodiments, the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the cell comprises a population of RIC pre-mRNAs transcribed from the gene encoding the target protein or functional RNA, wherein the population of RIC pre-mRNAs comprises two or more retained introns, and wherein the antisense oligomer binds to the most abundant retained intron in the population of RIC pre-mRNAs. In some embodiments, the binding of the antisense oligomer to the most abundant retained intron induces splicing out of the two or more retained introns from the population of RIC pre-mRNAs to produce mRNA encoding the target protein or functional RNA. In some embodiments, the cell comprises a population of RIC pre-mRNAs transcribed from the gene encoding the target protein or functional RNA, wherein the population of RIC pre-mRNAs comprises two or more retained introns, and wherein the antisense oligomer binds to the second most abundant retained intron in the population of RIC pre-mRNAs. In some embodiments, the binding of the antisense oligomer to the second most abundant retained intron induces splicing out of the two or more retained introns from the population of RIC pre-mRNAs to produce mRNA encoding the target protein or functional RNA. In some embodiments, the condition is a disease or disorder. In some embodiments, the disease or disorder is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder la, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9. In some embodiments, the target protein and the RIC pre-mRNA are encoded by the ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B gene. In some embodiments, the method further comprises assessing protein expression. In some embodiments, the antisense oligomer binds to a targeted portion of a ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B RIC pre-mRNA. In some embodiments, the subject is a human. In some embodiments, the subject is a non-human animal. In some embodiments, the subject is a fetus, an embryo, or a child. In some embodiments, the cells are *ex vivo.* In some embodiments, the antisense oligomer is administered by intrathecal injection, intracerebroventricular injection, intraperitoneal injection, intramuscular injection, subcutaneous injection, or intravenous injection of the subject. In some embodiments, the 9 nucleotides at -3e to -1e of the exon flanking the 5' splice site and +1 to +6 of the retained intron, are identical to the corresponding wild-type sequence. In some embodiments, the 16 nucleotides at -15 to -1 of the retained intron and +1e of the exon flanking the 3' splice site are identical to the corresponding wild-type sequence.

Described herein, in certain embodiments, is an antisense oligomer as used in a method described above.

Described herein, in certain embodiments, is an antisense oligomer comprising a sequence with at least about 80%, 85%, 90%, 95%, 97%, or 100% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.

Disclosed herein, in certain embodiments, is a pharmaceutical composition comprising the antisense oligomer described above, and an excipient. In some embodiments, also described herein is a method of treating a subject in need thereof by administering the pharmaceutical composition by intrathecal injection, intracerebroventricular injection, intraperitoneal injection, intramuscular injection, subcutaneous injection, or intravenous injection.

Disclosed herein, in certain embodiments, is a composition comprising an antisense oligomer for use in a method of increasing expression of a target protein or a functional RNA by cells to treat Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder la, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9 in a subject in need thereof associated with a deficient protein or deficient functional RNA, wherein the deficient protein or deficient functional RNA is deficient in amount or activity in the subject, wherein the antisense oligomer enhances constitutive splicing of a retained intron-containing pre-mRNA (RIC pre-mRNA) encoding the target protein or the functional RNA, wherein the target protein is: (a) the deficient protein; or (b) a compensating protein which functionally augments or replaces the deficient protein or in the subject; and wherein the functional RNA is: (a) the deficient RNA; or (b) a compensating functional RNA which functionally augments or replaces the deficient functional RNA in the subject; wherein the RIC pre-mRNA comprises a retained intron, an exon flanking the 5' splice site and an exon flanking the 3' splice site, and wherein the retained intron is spliced from the RIC pre-mRNA encoding the target protein or the functional RNA, thereby increasing production or activity of the target protein or the functional RNA in the subject. In some embodiments, also described herein is a composition comprising an antisense oligomer for use in a method of treating a condition associated with ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in a subject in need thereof, the method comprising the step of increasing expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein by cells of the subject, wherein the cells have a retained-intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, the method comprising contacting the cells with the antisense oligomer, whereby the retained intron is constitutively spliced from the RIC pre-mRNA transcripts encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, thereby increasing the level of mRNA encoding the target protein or functional RNA, and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, in the cells of the subject. In some embodiments, the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B. In some embodiments, the target protein is ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B. In some embodiments, the condition is a disease or disorder. In some embodiments, the disease or disorder is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9. In some embodiments, the target protein and RIC pre-mRNA are encoded by the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B gene. In some embodiments, the target protein and RIC pre-mRNA are encoded by the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene. In some embodiments, the antisense oligomer targets a portion of the RIC pre-mRNA that is in the retained intron within the region +6 relative to the 5' splice site of the retained intron to -16 relative to the 3' splice site of the retained intron. In some embodiments, the antisense oligomer targets a portion of the RIC pre-mRNA that is in the retained intron within: (a) the region +6 to +100 relative to the 5' splice site of the retained intron; or (b) the region -16 to -100 relative to the 3' splice site of the retained intron. In some embodiments, the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron. In some embodiments, the targeted portion of the RIC pre-mRNA is within: (a) the region +2e to -4e in the exon flanking the 5' splice site of the retained intron; or (b) the region +2e to -4e in the exon flanking the 3' splice site of the retained intron. In some embodiments, the RIC pre-mRNA is encoded by a genetic sequence with at least about 80%, 85%, 90%, 95%, 96%, 97%, 98%, 99% or 100% sequence identity to SEQ ID NOs: 1-19. In some embodiments, the RIC pre-mRNA comprises a sequence with at least about 80%, 85%, 90%, 95%, 96%, 97%, 98%, 99% or 100% sequence identity to any one of SEQ ID NOs: 20-80. In some embodiments, the antisense oligomer does not increase the amount of target protein or functional RNA by modulating alternative splicing of the pre-mRNA transcribed from a gene encoding the target protein or functional RNA. In some embodiments, the antisense oligomer does not increase the amount of the functional RNA or functional protein by modulating aberrant splicing resulting from mutation of the gene encoding the target protein or faunctional RNA. In some embodiments, the RIC pre-mRNA was produced by partial splicing from a full-length pre-mRNA or a wild-type pre-mRNA. In some embodiments, the mRNA encoding the target protein or functional RNA is a full-length mature mRNA, or a wild-type mature mRNA. In some embodiments, the target protein produced is full-length protein, or wild-type protein. In some embodiments, the retained intron is a rate-limiting intron. In some embodiments, said retained intron is the most abundant retained intron in said RIC pre-mRNA. In some embodiments, the retained intron is the second most abundant retained intron in said RIC pre-mRNA. In some embodiments, the antisense oligomer comprises a backbone modification comprising a phosphorothioate linkage or a phosphorodiamidate linkage. In some embodiments, said antisense oligomer is an antisense oligonucleotide. In some embodiments, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some embodiments, the antisense oligomer comprises at least one modified sugar moiety. In some embodiments, each sugar moiety is a modified sugar moiety. In some embodiments, the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some embodiments, the antisense oligomer is at least 80%, at least 85%, at least 90%, at least 95%, at least 98%, at least 99%, or is 100% complementary to the targeted portion of the RIC pre-mRNA encoding the protein. In some embodiments, the antisense oligomer binds to a targeted portion of a tuberin RIC pre-mRNA, wherein the targeted portion of the RIC pre-mRNA is within a sequence selected from SEQ ID NOs: 24351-24354, 24356-24378, 24380-24386, or 28515-28516. In some embodiments, the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the antisense oligomer binds to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B RIC pre-mRNA. In some embodiments, the antisense oligomer binds to a targeted portion of a ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B RIC pre-mRNA.

Disclosed herein, in certain embodiments, is a pharmaceutical composition comprising the antisense oligomer of any of the compositions described above, and an excipient. In some embodiments, also described herein is a method of treating a subject in need thereof by administering the pharmaceutical composition by intrathecal injection, intracerebroventricular injection, intraperitoneal injection, intramuscular injection, subcutaneous injection, or intravenous injection.

Disclosed herein, in certain embodiments, is a pharmaceutical composition comprising: an antisense oligomer that hybridizes to a target sequence of a deficient *ATP1A2, CACNA1A*, *SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA,* or *STX1B* mRNA transcript, wherein the deficient *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA,* or *STX1B* mRNA transcript comprises a retained intron, wherein the antisense oligomer induces splicing out of the retained intron from the deficient *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA,* or *STX1B* mRNA transcript; and a pharmaceutical acceptable excipient. In some embodiments, the antisense oligomer hybridizes to a target sequence of a deficient *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2,* or *STX1B* mRNA transcript. In some embodiments, the deficient *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2,* or *STX1B* mRNA transcript is a *ATP1A2, CACNA1A, SETD5, SHANK3, NF2, DNMT1, TCF4, RAI1, PEX1, ARSA, EIF2H5, EIF2B1, EIF2B2, NPC1, ADAR, STXBP1, PRICKLE2, PRRT2,* or *STX1B* RIC pre-mRNA transcript. In some embodiments, the antisense oligomer hybridizes to a target sequence of a deficient *ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2,* or *STX1B* mRNA transcript. In some embodiments, the deficient *ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2,* or *STX1B* mRNA transcript is a *ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2,* or *STX1B* RIC pre-mRNA transcript. In some embodiments, the targeted portion of the RIC pre-mRNA transcript is in the retained intron within the region +4000 relative to the 5' splice site of the retained intron to -2000 relative to the 3' spliced site of the retained intron. In some embodiments, the RIC pre-mRNA transcript is encoded by a genetic sequence with at least about 80%, 85%, 90%, 95%, 96%, 97%, 98%, 99% or 100% sequence identity to SEQ ID NOs: 1-19. In some embodiments, the RIC pre-mRNA transcript comprises a sequence with at least about 80%, 85%, 90%, 95%, 96%, 97%, 98%, 99% or 100% sequence identity to any one of SEQ ID NOs: 20-80. In some embodiments, the antisense oligomer comprises a backbone modification comprising a phosphorothioate linkage or a phosphorodiamidate linkage. In some embodiments, the antisense oligomer is an antisense oligonucleotide. In some embodiments, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some embodiments, the antisense oligomer comprises at least one modified sugar moiety. In some embodiments, the antisense oligomer comprises from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some embodiments, the antisense oligomer is at least 80%, at least 85%, at least 90%, at least 95%, at least 98%, at least 99%, or is 100% complementary to a targeted portion of the RIC pre-mRNA transcript. In some embodiments, the targeted portion of the RIC pre-mRNA transcript is within a sequence selected from SEQ ID NOs: 24351-24354, 24356-24378, 24380-24386, or 28515-28516. In some embodiments, the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350. In some embodiments, the pharmaceutical composition is formulated for intrathecal injection, intracerebroventricular injection, intraperitoneal injection, intramuscular injection, subcutaneous injection, or intravenous injection.

Disclosed herein, in certain embodiments, is a method of inducing processing of a mRNA transcript to facilitate removal of a retained intron to produce a fully processed mRNA transcript that encodes a functional form of a target protein, the method comprising: (a) contacting an antisense oligomer to a target cell of a subject; (b) hybridizing the antisense oligomer to the mRNA transcript, wherein the mRNA transcript is capable of encoding the functional form of the target protein and comprises at least one retained intron; (c) removing the at least one retained intron from the mRNA transcript to produce the fully processed mRNA transcript that encodes the functional form of the target protein; and (d) translating the functional form of the target protein from the fully processed mRNA transcript; wherein the mRNA transcript is selected from *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA,* or *STX1B* mRNA transcript. In some embodiments, the retained intron is an entire retained intron. In some embodiments, the mRNA transcript is a RIC pre-mRNA transcript. In some embodiments, the mRNA transcript is selected from *ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2,* or *STX1B* mRNA transcript. In some embodiments, the mRNA transcript is selected from *ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2,* or *STX1B* mRNA transcript.

Disclosed herein, in certain embodiments, is a method of treating a subject having a condition caused by a deficient amount or activity of a target protein comprising: administering to the subject an antisense oligomer comprising a nucleotide sequence with at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings.

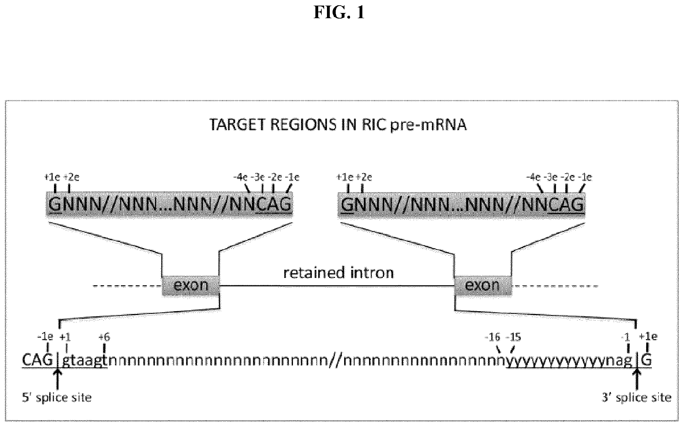

**FIG.** 1 illustrates a schematic representation of an exemplary retained-intron-containing (RIC) pre-mRNA transcript. The 5' splice site consensus sequence is indicated with underlined letters (letters are nucleotides; upper case: exonic portion and lower case: intronic portion) from -3e to -1e and +1 to +6 (numbers labeled "e" are exonic and unlabeled numbers are intronic). The 3' splice site consensus sequence is indicated with underlined letters (letters are nucleotides; upper case: exonic portion and lower case: intronic portion) from -15 to -1 and +1e (numbers labeled "e" are exonic and unlabeled numbers are intronic). Intronic target regions for ASO screening comprise nucleotides +6 relative to the 5' splice site of the retained intron (arrow at left) to -16 relative to the 3' splice site of the retained intron (arrow at right). In embodiments, intronic target regions for ASO screening comprise nucleotides +6 to +100 relative to the 5' splice site of the retained intron and -16 to -100 relative to the 3' splice site of the retained intron. Exonic target regions comprise nucleotides +2e to -4e in the exon flanking the 5' splice site of the retained intron and +2e to -4e in the exon flanking the 3' splice site of the retained intron. "n" or "N" denote any nucleotide, "y" denotes pyrimidine. The sequences shown represent consensus sequences for mammalian splice sites and individual introns and exons need not match the consensus sequences at every position. FIG. 1 discloses SEQ ID NOS 28519-28520, respectively, in order of appearance.

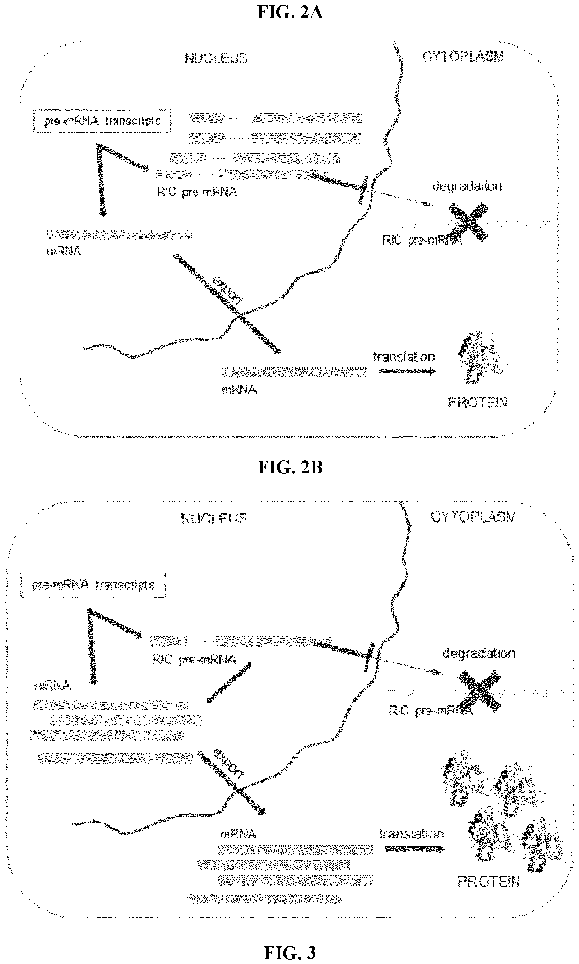

**FIG. 2A****-****FIG. 2B** show schematic representations of the Targeted Augmentation of Nuclear Gene Output (TANGO) approach. FIG. 2A shows a cell divided into nuclear and cytoplasmic compartments. In the nucleus, a pre-mRNA transcript of a target gene consisting of exons (rectangles) and introns (connecting lines) undergoes splicing to generate an mRNA, and this mRNA is exported to the cytoplasm and translated into target protein. For this target gene, the splicing of intron 1 is inefficient and a retained intron-containing (RIC) pre-mRNA accumulates primarily in the nucleus, and if exported to the cytoplasm, is degraded, leading to no target protein production. FIG. 2B shows an example of the same cell divided into nuclear and cytoplasmic compartments. Treatment with an antisense oligomer (ASO) promotes the splicing of intron 1 and results in an increase in mRNA, which is in turn translated into higher levels of target protein.
**FIG. 3** depicts a schematic of the ReSeq Genes for *ADAR* intron 2 corresponding to NM_001111. The Percent Intron Retention (PIR) of the circled intron is shown.
**FIG. 4** depicts a schematic of the ReSeq Genes for *ARSA* intron 3 corresponding to NM_001085425. The Percent Intron Retention (PIR) of the circled intron is shown.
**FIG. 5** depicts a schematic of the ReSeq Genes for *ARSA* intron 4 corresponding to NM_001085425. The Percent Intron Retention (PIR) of the circled intron is shown.
**FIG. 6** depicts a schematic of the ReSeq Genes for *DNMT1* intron 30 corresponding to NM_001130823. The Percent Intron Retention (PIR) of the circled intron is shown.
**FIG. 7** depicts an exemplary graph showing the average (n=3) fold change in expression levels of *DNMT1* mRNA without intron 30 in ARPE-19 cells treated for 24 hrs with 80 nM of the indicated ASOs over mock treated cells. Data is normalized to RPL32 expression.
**FIG. 8** depicts a schematic of the ReSeq Genes for *EIF2B2* intron 1 corresponding to NM_014239. The Percent Intron Retention (PIR) of the circled intron is shown.
**FIG. 9** depicts an exemplary graph showing the average (n=3) fold change in expression levels of *EIF2B2* mRNA without intron 1 in ARPE-19 cells treated for 24 hrs with 80 nM of the indicated ASOs over mock treated cells. Data is normalized to RPL32 expression.
**FIG. 10** depicts a schematic of the ReSeq Genes for *EIF2B5* intron 12 corresponding to NM_003907. The Percent Intron Retention (PIR) of the circled intron is shown.
**FIG. 11** depicts an exemplary graph showing the average (n=3) fold change in expression levels of *EIF2B5* mRNA without intron 12 in ARPE-19 cells treated for 24 hrs with 80 nM of the indicated ASOs over mock treated cells. Data is normalized to RPL32 expression.
**FIG. 12** depicts a schematic of the ReSeq Genes for *EIF2B5* intron 13 corresponding to NM_003907. The Percent Intron Retention (PIR) of the circled intron is shown.
**FIG. 13** depicts an exemplary graph showing the average (n=3) fold change in expression levels of *EIF2B5* mRNA without intron 13 in ARPE-19 cells treated for 24 hrs with 80 nM of the indicated ASOs over mock treated cells. Data is normalized to RPL32 expression.
**FIG. 14** depicts a schematic of the ReSeq Genes for *PEX1* intron 10 corresponding to NM_000466. The Percent Intron Retention (PIR) of the circled intron is shown.
**FIG. 15** depicts an exemplary graph showing the average (n=3) fold change in expression levels of *PEX1* mRNA without intron 10 in ARPE-19 cells treated for 24 hrs with 80 nM of the indicated ASOs over mock treated cells. Data is normalized to RPL32 expression.
**FIG. 16** depicts a schematic of the ReSeq Genes for *PEX1* intron 14 corresponding to NM_000466. The Percent Intron Retention (PIR) of the circled intron is shown.
**FIG. 17** depicts an exemplary graph showing the average (n=3) fold change in expression levels of *PEX1* mRNA without intron 14 in ARPE-19 cells treated for 24 hrs with 80 nM of the indicated ASOs over mock treated cells. Data is normalized to RPL32 expression.
**FIG. 18** depicts a schematic of the ReSeq Genes for *PRICKLE2* intron 4 corresponding to NM_198859. The Percent Intron Retention (PIR) of the circled intron is shown.
**FIG. 19** depicts an exemplary graph showing the average (n=3) fold change in expression levels of *PRICKLE2* mRNA without intron 4 in ARPE-19 cells treated for 24 hrs with 80 nM of the indicated ASOs over mock treated cells. Data is normalized to RPL32 expression.
**FIG. 20** depicts a schematic of the ReSeq Genes for *PRRT2* intron 1 corresponding to NM_145239. The Percent Intron Retention (PIR) of the circled intron is shown.
**FIG. 21** depicts a schematic of the ReSeq Genes for *RAI1* intron 4 corresponding to NM_030665. The Percent Intron Retention (PIR) of the circled intron is shown.
**FIG. 22** depicts an exemplary graph showing the average (n=3) fold change in expression levels of *RAI1* mRNA without intron 4 in ARPE-19 cells treated for 24 hrs with 80 nM of the indicated ASOs over mock treated cells. Data is normalized to RPL32 expression.
**FIG. 23** depicts a schematic of the ReSeq Genes for *SHANK3* intron 16 corresponding to NM_033517. The Percent Intron Retention (PIR) of the circled intron is shown.
**FIG. 24** depicts an exemplary graph showing the average (n=3) fold change in expression levels of *SHANK3* mRNA without intron 16 in ARPE-19 cells treated for 24 hrs with 80 nM of the indicated ASOs over mock treated cells. Data is normalized to RPL32 expression.
**FIG. 25** depicts a schematic of the ReSeq Genes for *STXBP1* intron 18 corresponding to NM_001032221. The Percent Intron Retention (PIR) of the circled intron is shown.
**FIG. 26** depicts an exemplary graph showing the average (n=3) fold change in expression levels of *STXBP1* mRNA without intron 18 in ARPE-19 cells treated for 24 hrs with 80 nM of the indicated ASOs over mock treated cells. Data is normalized to RPL32 expression.
**FIG. 27** depicts a schematic of the ReSeq Genes for *SETD5* intron 4 corresponding to NM_001080517. The Percent Intron Retention (PIR) of the circled intron is shown.
**FIG. 28** depicts a schematic of the ReSeq Genes for *ATP1A2* intron 22 corresponding to NM_000702. The Percent Intron Retention (PIR) of the circled intron is shown.
**FIG. 29** depicts a schematic of the ReSeq Genes for *CACNA1A* intron 36 corresponding to NM_023035. The Percent Intron Retention (PIR) of the circled intron is shown.
**FIG. 30** depicts a schematic of the ReSeq Genes for *CACNA1A* intron 37 corresponding to NM_023035. The Percent Intron Retention (PIR) of the circled intron is shown.
**FIG. 31** depicts a schematic of the ReSeq Genes for *NF2* intron 16 corresponding to NM_181832. The Percent Intron Retention (PIR) of the circled intron is shown.
**FIG. 32** depicts a schematic of the ReSeq Genes for *PEX1* intron 19 corresponding to NM_000466. The Percent Intron Retention (PIR) of the circled intron is shown.
**FIG. 33** depicts a schematic of the ReSeq Genes for *PEX1* intron 21 corresponding to NM_000466. The Percent Intron Retention (PIR) of the circled intron is shown.
**FIG. 34** depicts a schematic of the ReSeq Genes for *STX1B* intron 6 corresponding to NM_052874. The Percent Intron Retention (PIR) of the circled intron is shown.
**FIG. 35** depicts a schematic of the ReSeq Genes for *STX1B* intron 7 corresponding to NM_052874. The Percent Intron Retention (PIR) of the circled intron is shown.
**FIG. 36** depicts a schematic of the ReSeq Genes for *NF2* intron 16 corresponding to NM_181832. The Percent Intron Retention (PIR) of the circled intron is shown.
**FIG. 37** depicts an exemplary graph showing the average (n=3) fold change in expression levels of *NF2* mRNA without intron 16 in ARPE-19 cells treated for 24 hrs with 80 nM of the indicated ASOs over mock treated cells. Data is normalized to RPL32 expression.
**FIG. 38** depicts a schematic of the ReSeq Genes for *NPC1* intron 15 corresponding to NM_000271. The Percent Intron Retention (PIR) of the circled intron is shown.
**FIG. 39** depicts an exemplary graph showing the average (n=3) fold change in expression levels of *NPC1* mRNA without intron 15 in ARPE-19 cells treated for 24 hrs with 80 nM of the indicated ASOs over mock treated cells. Data is normalized to RPL32 expression.
**FIG. 40** depicts a schematic of the ReSeq Genes for *ATP1A2* intron 22 corresponding to NM_000702. The Percent Intron Retention (PIR) of the circled intron is shown.
**FIG. 41** depicts an exemplary graph showing the average (n=3) fold change in expression levels of *ATP1A2* mRNA without intron 22 in ARPE-19 cells treated for 24 hrs with 80 nM of the indicated ASOs over mock treated cells. Data is normalized to RPL32 expression.
**FIG. 42** depicts a schematic of the ReSeq Genes for *TCF4* intron 18 corresponding to NM_001243226. The Percent Intron Retention (PIR) of the circled intron is shown.

### DETAILED DESCRIPTION OF THE INVENTION

Individual introns in primary transcripts of protein-coding genes having more than one intron are spliced from the primary transcript with different efficiencies. In most cases only the fully spliced mRNA is exported through nuclear pores for subsequent translation in the cytoplasm. Unspliced and partially spliced transcripts are detectable in the nucleus. It is generally thought that nuclear accumulation of transcripts that are not fully spliced is a mechanism to prevent the accumulation of potentially deleterious mRNAs in the cytoplasm that may be translated to protein. For some genes, splicing of the least efficient intron is a rate-limiting post-transcriptional step in gene expression, prior to translation in the cytoplasm.

Substantial levels of partially-spliced transcripts encoding the: ATP1A2 protein, deficient in Familial hemiplegic migraine-2, Familial Basilar migraine, and Alternating hemiplegia of childhood; CACNA1A protein, deficient in Episodic ataxia type 2 (EA2), Familial hemiplegic migraine, and Spinocerebellar ataxia type 6; SETD5 protein, deficient in Mental retardation-23 (MRD23), and 3p25 microdeletion syndrome; SHANK3 protein, deficient in Phelan-McDermid syndrome (PHMDS), and Schizophrenia-15 (SCZD15); NF2 protein, deficient in Neurofibromatosis, type 2, Meningioma, NF2-related, and Schwannomatosis 1; DNMT1 protein, deficient in Hereditary sensory neuropathy type IE (HSN1E), Autosomal dominant cerebellar ataxia, deafness, and narcolepsy (ADCADN); TCF4 protein, deficient in Pitt-hopkins syndrome; RAI1 protein, deficient in Smith-magenis syndrome; PEX1 protein, deficient in PEROXISOME BIOGENESIS DISORDER 1A (Zellweger syndrome)(PBD1A), and Heimler syndrome-1 (HMLR1); ARSA protein, deficient in Metachromatic Leukodystrophy; EIF2B5 protein/EIF2B 1 protein/EIF2B2, deficient in Leukoencephalopathy with vanishing white matter (VWM); NPC1 protein, deficient in Niemann-Pick disease type C1 and Niemann-Pick disease type D; ADAR protein, deficient in Aicardi-Goutieres syndrome-6 (AGS6); MFSD8 protein, deficient in Neuronal ceroid lipofuscinosis-7; STXBP1 protein, deficient in early infantile epileptic encephalopathy-4; PRICKLE2 protein, deficient in progressive myoclonic epilepsy 5; PRRT2 protein, deficient in familial infantile convulsion with paroxysmal choreoathetosis, episodic kinesigenic dyskinesia 1, or benign familial infantile seizuers-2; IDUA protein, deficient in attenuated MPS-1 (Hurler-scheie syndrome); and STX1B protein, deficient in generalized Epilepsy with febrile seizure plus type 9 have been discovered in the nucleus of human cells. These ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, and STX1B pre-mRNA species comprise at least one retained intron. The present invention provides compositions and methods for upregulating splicing of one or more retained ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B introns that are rate-limiting for the nuclear stages of gene expression to increase steady-state production of fully-spliced, mature mRNA, and thus, translated ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein levels. These compositions and methods can utilize antisense oligomers (ASOs) that promote constitutive splicing at intron splice sites of a retained-intron-containing ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA (RIC pre-mRNA) that accumulates in the nucleus. Thus, in embodiments, ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein can be increased using the methods of the invention to treat a condition caused by ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B deficiency.

In some embodiments, disclosed herein are compositions and methods for upregulating splicing of one or more retained ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B introns that are rate-limiting for the nuclear stages of gene expression to increase steady-state production of fully-spliced, mature mRNA, and thus, translated ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein levels. These compositions and methods can utilize antisense oligomers (ASOs) that promote constitutive splicing at intron splice sites of a retained-intron-containing ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B pre-mRNA (RIC pre-mRNA) that accumulates in the nucleus. Thus, in embodiments, ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein can be increased using the methods of the invention to treat a condition caused by ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B deficiency.

In some embodiments, disclosed herein are compositions and methods for upregulating splicing of one or more retained ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B introns that are rate-limiting for the nuclear stages of gene expression to increase steady-state production of fully-spliced, mature mRNA, and thus, translated ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein levels. These compositions and methods can utilize antisense oligomers (ASOs) that promote constitutive splicing at intron splice sites of a retained-intron-containing ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B pre-mRNA (RIC pre-mRNA) that accumulates in the nucleus. Thus, in embodiments, ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein can be increased using the methods of the invention to treat a condition caused by ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B deficiency.

In other embodiments, the methods of the invention can be used to increase ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B production to treat a condition in a subject in need thereof. In embodiments, the subject has a condition in which ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B is not necessarily deficient relative to wild-type, but where an increase in ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B mitigates the condition nonetheless. In embodiments, the condition can be caused by a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B haploinsufficiency.

In other embodiments, the methods of the invention can be used to increase ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B production to treat a condition in a subject in need thereof. In embodiments, the subject has a condition in which ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B is not necessarily deficient relative to wild-type, but where an increase in ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B mitigates the condition nonetheless. In embodiments, the condition can be caused by a ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B haploinsufficiency.

In additional embodiments, the methods of the invention can be used to increase ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B production to treat a condition in a subject in need thereof. In embodiments, the subject has a condition in which ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B is not necessarily deficient relative to wild-type, but where an increase in ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B mitigates the condition nonetheless. In embodiments, the condition can be caused by a ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B haploinsufficiency.

In embodiments, the described compositions and methods are used to treat a subject or patient having a CNS disease or condition that is caused by a deficiency in the target protein. In embodiments the described compositions and methods are used to treat a subject or patient having a CNS disease or condition that is not caused by a deficiency in the target protein. In embodiments, the subject or patient having a CNS disease or condition can benefit from increased production of the target protein by supplementing normal production of the target protein. In embodiments, the target protein acts on a secondary target to ameliorate or treat the CNS disease or condition in the subject. In embodiments, the secondary target protein is deficient in the subject. In embodiments, the secondary target protein is not deficient in the subject.

### ATP1A2

ATP1A2, encodes the α2 isoform of Na+K+-ATPase (sodium potassium ATPase). The α2 isoform belongs to the family of P-type cation transport ATPases, and to the subfamily of Na+/K+ -ATPases. Na+/K+ -ATPase is an integral membrane protein responsible for establishing and maintaining the electrochemical gradients of Na and K ions across the plasma membrane. These gradients are essential for osmoregulation, for sodium-coupled transport of a variety of organic and inorganic molecules, and for electrical excitability of nerve and muscle.

Na+/K+ -ATPase transfers Na+ out and K+ into the cell, using ATP as energy source. Na+/K+ ATPases are present in all human cells; however, the α2 isoform is mainly restricted to the brain and muscle. Na+/K+ -ATPases that include the α -2 subunit are primarily found in glia cells, particularly in astrocytes, where they contribute to the glial clearance of high extracellular K+ to prevent further depolarization. Through its action in glia, the Na+/K+ -ATPase plays a critical role in the normal function of neurons. Communication between neurons depend neurotransmitters. Briefly, neurons release neurotransmitters, which bind to receptor proteins on neighboring neurons. After the neurotransmitters have had their effect, neurotransmitters detach from their receptors and are removed from the spaces between neurons by glia. The Na+/K+ ATPase helps regulate this process by stimulating glia to clear neurotransmitters from the spaces between neurons. Na+/K+-ATPases also removes excess potassium ions from these spaces. In humans, mutations in ATP1A2 are associated with genetic diseases, for example familial hemiplegic migraine-2 (FHM2), familial basilar migraine and alternating hemiplegia of childhood.

### Familial hemiplegic migraine-2

Familial hemiplegic migraine-2 (FHM2) and familial basilar migraine are caused by heterozygous mutation in *ATP1A2.* FHM2 is a monogenic variant of migraine with aura. Migraines can cause intense, throbbing pain in one area of the head, often accompanied by nausea, vomiting, and extreme sensitivity to light and sound. These recurrent headaches can begin in childhood or adolescence and can be triggered by certain foods, emotional stress, and minor head trauma. Each headache may last from a few hours to a few days. In some types of migraine, including familial hemiplegic migraine, a pattern of neurological symptoms called an aura precedes the headache. The most common symptoms associated with an aura are temporary visual changes such as blind spots (scotomas), flashing lights, zig-zagging lines, and double vision. In people with familial hemiplegic migraine, auras are also characterized by temporary numbness or weakness, often affecting one side of the body (hemiparesis). Additional features of an aura can include difficulty with speech, confusion, and drowsiness. An aura can develops gradually over a few minutes and lasts about an hour. Familial hemiplegic migraine episodes includes fever, seizures, prolonged weakness, coma, and, rarely, death. Although most people with familial hemiplegic migraine recover completely between episodes, neurological symptoms such as memory loss and problems with attention can last for weeks or months. About 20 percent of people with this condition develop mild but permanent difficulty coordinating movements (ataxia), which may worsen with time, and rapid, involuntary eye movements called nystagmus.

FHM 2 is caused by mutations present in ATP1A2. The mutations and deficiencies in ATP1A2 that cause FHM2 are responsible for approximately 20% of FHM in families. There are over 50 mutations in ATP1A2 that have been identified in association with FHM2. Almost all FHM2 mutations are non-synonymous SNPs, but there are also small deletions and a mutation affecting the stop codon, causing an extension of the ATP1A2 protein by 27 amino acid residues. Most of the mutations are associated with pure FHM without additional clinical symptoms. ATP1A2 is expressed primarily in astrocytes in the adult, where it appears functionally coupled to various transporters (glutamate transporter and Na⁺/Ca²⁺ exchanger), and is essential in the clearance of released glutamate and potassium from the extracellular space during neuronal activity. FHM2 mutations that cause loss of function of ATP1A2 may lead to decreased glutamate clearance and an increase of potassium in the synaptic cleft during neuronal activity, which could lead to prolonged recovery time after neuronal excitation, and may render the brain to be more susceptible to cortical spreading depression. Cortical spreading depression is a wave of continual strong neuronal depolarization that slowly progresses across the cortex, generating a brief intense spike of activity that is followed by long-lasting neural suppression. Cortical spreading depression has been shown to activate the trigeminovascular system which is responsible for the headache associated with migraines.

There are two hypothesized mechanisms for the effects of ATP1A2 mutations. First, the mutations cause an increase in extracellular potassium, which can result in the impaired clearance of potassium ions and therefore induce Cortical spreading depression. Second, since the distribution of ATP1A2 is co-localized with the Na⁺/Ca²⁺ exchanger, the mutations to ATP1A2 would cause intracellular sodium to increase, which increases intracellular calcium levels through the Na⁺/Ca²⁺ exchanger, resulting in glutamate release and a decrease in glutamate clearance which can also lead to Cortical spreading depression. Both hypotheses result in making the brain more susceptible to Cortical spreading depression and therefore migraines with aura.

Recently, a number of ATP1A2 mutations were reported to be associated with FHM and cerebellar problems, specifically motor problems, childhood convulsions, epilepsy, and mental retardation. Some ATP1A2 mutations have been shown to be associated with non-hemiplegic migraine phenotypes, such as basilar migraine and the common migraine.

### Familial Basilar migraine

Basilar migraine is a subtype of migraine with aura in which the aura symptoms originate from the brainstem or reflect the simultaneous involvement of both hemispheres.

### Alternating hemiplegia of childhood

Alternating hemiplegia of childhood is an autosomal dominant condition. Alternating hemiplegia of childhood can result from new mutations in the gene and occur in people with no history of the disorder in their family. The primary feature of this condition is recurrent episodes of temporary paralysis, often affecting one side of the body (hemiplegia). During some episodes, the paralysis alternates from one side to the other or affects both sides of the body at the same time. The known ATP1A2 gene mutation associated with this condition replaces a single amino acid in Na+/K+ ATPase: the amino acid threonine is replaced with the amino acid asparagine at protein position 378. This genetic change can impair the protein's ability to transport ions. In addition to paralysis, affected individuals can have sudden attacks of uncontrollable muscle activity; these can cause involuntary limb movements (choreoathetosis), muscle tensing (dystonia), movement of the eyes (nystagmus), or shortness of breath (dyspnea). Individuals with alternating hemiplegia of childhood may also experience sudden redness and warmth (flushing) or unusual paleness (pallor) of the skin. These attacks can occur during or separately from episodes of hemiplegia. The episodes of hemiplegia or uncontrolled movements can be triggered by certain factors, such as stress, extreme tiredness, cold temperatures, or bathing, although the trigger is not always known. The number and length of the episodes initially worsen throughout childhood but then begin to decrease over time. The uncontrollable muscle movements may disappear entirely, but the episodes of hemiplegia occur throughout life. Alternating hemiplegia of childhood also causes mild to severe cognitive problems. Almost all affected individuals have some level of developmental delay and intellectual disability. Their cognitive functioning typically declines over time.

### CACNA1A

The CACNA1A gene encodes the alpha-1 subunit of the calcium channel CaV2.1. Voltage-dependent Ca(2+) channels not only mediate the entry of Ca(2+) ions into excitable cells but are also involved in a variety of Ca(2+)-dependent processes, including muscle contraction, hormone or neurotransmitter release, and gene expression. Diriong et al. (1995) noted that calcium channels are multisubunit complexes and that the channel activity is directed by a pore-forming alpha-1 subunit, which is often sufficient to generate voltage-sensitive Ca(2+) channel activity. This subunit forms the pore through which calcium ions can flow. CaV2.1 channels play an essential role in neuronal communication. These channels help control the release of neurotransmitters, and are involved in neuronal plasticity. Different mutations in the alpha-1 subunit the CaV2.1 calcium channel are responsible for familial hemiplegic migraine (FHM), episodic ataxia type 2 (EA2), and spinocerebellar ataxia type 6 (SCA6). Missense and splice site mutations have been found in FHM and episodic ataxia type 2 (EA2), respectively, whereas a CAG repeat in the CACNA1A gene was found expanded in patients with spinocerebellar ataxia type 6 (SCA6).

### Episodic ataxia type 2 (EA2)

Episodic ataxia is a group of related conditions that affect the nervous system and cause problems with movement. People with episodic ataxia have recurrent episodes of poor coordination and balance (ataxia). During these episodes, many affected individuals also experience dizziness (vertigo), nausea and vomiting, migraine headaches, blurred or double vision, slurred speech, and ringing in the ears (tinnitus). Seizures, muscle weakness, and paralysis affecting one side of the body (hemiplegia) may also occur during attacks. Additionally, some affected individuals have a muscle abnormality called myokymia during or between episodes. This abnormality can cause muscle cramping, stiffness, and continuous, fine muscle twitching that appears as rippling under the skin.

Episodes of ataxia and other symptoms can begin anytime from early childhood to adulthood. They can be triggered by environmental factors such as emotional stress, caffeine, alcohol, certain medications, physical activity, and illness. The frequency of attacks ranges from several per day to one or two per year. Between episodes, some affected individuals continue to experience ataxia, which may worsen over time, as well as involuntary eye movements called nystagmus.

Researchers have identified at least seven types of episodic ataxia, designated type 1 through type 7. The types are distinguished by their pattern of signs and symptoms, age of onset, length of attacks, and, when known, genetic cause.

More than 50 mutations in the CACNA1A gene have been found to cause episodic ataxia type 2 (EA2), the most common form of episodic ataxia. In addition to problems with coordination and balance (ataxia), EA2 is associated with involuntary eye movements called nystagmus. The CACNA1A mutations responsible for EA2 reduce the production of functional CaV2.1 channels or prevent these channels from reaching the cell membrane, where they are needed to transport calcium ions. A decrease in the number of these channels reduces the total flow of calcium ions into neurons, which disrupts the release of neurotransmitters in the brain. Although changes in signaling between neurons underlie the episodes of uncoordinated movement seen in people with episodic ataxia, it is unclear how altered calcium ion transport causes the specific features of the condition.

### Familial hemiplegic migraine

At least 20 mutations in the CACNA1A gene have been identified in people with familial hemiplegic migraine type 1 (FHM1). FHM is characterized by an aura of hemiplegia that is always associated with at least one other aura symptom (e.g., hemianopsia, hemisensory deficit, aphasia). The aura is followed by a moderate to severe headache. In FHM1, the aura includes temporary numbness or weakness on one side of the body (hemiparesis). Like EA2, FHM1 is commonly associated with ataxia and nystagmus. Most of the mutations that cause FHM1 change single amino acids in the CaV2.1 channel. The most common mutation, which has been found in more than a dozen affected families, replaces the amino acid threonine with the amino acid methionine at protein position 666.

The CACNA1A mutations responsible for familial hemiplegic migraine change the structure of the CaV2.1 channel. The altered channels open more easily than usual, which increases the inward flow of calcium ions. A greater influx of calcium ions through CaV2.1 channels increases the cell's release of neurotransmitters. The resulting changes in signaling between neurons lead to development of these severe headaches in people with familial hemiplegic migraine. Approximately 50% of families with FHM, including all those with permanent cerebellar symptoms, have missense mutations in CACNA1A [Battistini et al 1999, Ducros et al 1999, Friend et al 1999].

### Spinocerebellar ataxia type 6

Spinocerebellar ataxia type 6 is a late-onset, autosomal dominant disorder. Spinocerebellar ataxia type 6 (SCA6) is another disorder caused by CACNA1A gene mutations. The major features of this condition include progressive ataxia, nystagmus, and impaired speech (dysarthria), most often beginning in a person's forties or fifties. SCA6 results from an increased number of copies (expansion) of the CAG trinucleotide repeat in the CACNA1A gene. Most cases of SCA6 are a result of CAG repeat expansion beyond the normal range, i.e., more than 19 repeats. In people with this condition, the CAG segment is repeated from 20 to more than 30 times. An increase in the length of the CAG segment leads to the production of an abnormally long version of the alpha-1 subunit. The abnormal subunit is found in the cell membrane as well as in the cytoplasm, where it clusters together and forms aggregates. The effect these aggregates have on cell functioning is unknown. The lack of normal calcium channels impairs the cells' ability to transport calcium ions. These changes alter the release of neurotransmitters in the brain and eventually lead to the death of neurons. Certain neurons called Purkinje cells seem to be particularly sensitive to a disruption in calcium transport. Purkinje cells are located in the part of the brain that coordinates movement (cerebellum). Over time, the loss of Purkinje cells and other cells of the cerebellum causes the movement problems characteristic of SCA6.

### SETD5

SETD5 expression in adult brain, followed by spinal cord, most isolated adult brain regions, and ovary. Much lower expression was detected in other adult peripheral tissues and in fetal brain and liver. The SETD5 gene encodes a 1,442-residue protein that is a putative methyltransferase. Mutations in this gene have been associated with autosomal dominant mental retardation-23.

### Mental retardation-23 (MRD23)

Autosomal dominant mental retardation-23 (MRD23) is caused by heterozygous mutation in the SETD5 gene on chromosome 3p25. Mental retardation, autosomal dominant 23 (MRD23) is a disorder characterized by significantly below average general intellectual functioning associated with impairments in adaptive behavior and manifested during the developmental period. MRD23 patients manifest moderate to severe intellectual disability with additional variable features of brachycephaly, a low hairline, depressed nasal bridge, prominent high nasal root, tubular nose, upslanting palpebral fissures, long and smooth philtrum, micrognathia, thin upper lip, and crowded teeth. Behavioral problems, including obsessive-compulsive disorder, hand flapping with ritualized behavior, and autism, are prominent features. The disease is caused by mutations affecting the gene represented in this entry.

### 3p25 microdeletion syndrome

De novo loss-of-function mutations in SETD5, encoding a methyltransferase in a 3p25 microdeletion syndrome critical region, causes intellectual disability. Characteristic features of the distal 3p- syndrome include low birth weight, microcephaly, trigonocephaly, hypotonia, psychomotor and growth retardation, ptosis, telecanthus, downslanting palpebral fissures, and micrognathia. Postaxial polydactyly, renal anomalies, cleft palate, congenital heart defects (especially atrioventricular septal defects), preauricular pits, sacral dimple, and gastrointestinal anomalies are variable features. Although intellectual deficits are almost invariably associated with cytogenetically visible 3p deletions, rare patients with a 3p26-p25 deletion and normal intelligence or only mild abnormalities have been described.

### SHANK3

In humans, SHANK3 encodes SH3 and multiple ankyrin repeat domains 3 (SHANK3), also known as proline-rich synapse-associated protein 2 (ProSAP2). Northern blot analysis indicated that human PSAP2 is expressed primarily in brain as 7- and 8-kb transcripts. The SHANK3 gene spans 60 kb and contains 22 exons. In rats and humans, PSAP2 is expressed preferentially in cerebral cortex and cerebellum. This gene is a member of the Shank gene family. Shank proteins are multidomain scaffold proteins of the postsynaptic density that connect neurotransmitter receptors, ion channels, and other membrane proteins to the actin cytoskeleton and G-protein-coupled signaling pathways. Shank proteins also play a role in synapse formation and dendritic spine maturation. SHANK3 is one of the genes disrupted in patients with the 22q13.3 deletion syndrome, also known as Phelan-McDermid syndrome.

### Phelan-McDermid syndrome (PHMDS)

Phelan-McDermid syndrome (PHMDS) can be caused by a heterozygous contiguous gene deletion at chromosome 22q13 or by mutation in the SHANK3 gene. Phelan-McDermid syndrome is a developmental disorder with variable features. Common features include neonatal hypotonia, global developmental delay, normal to accelerated growth, absent to severely delayed speech, autistic behavior, and minor dysmorphic features. Other less common features associated with this syndrome included increased tolerance to pain, dysplastic toenails, chewing behavior, fleshy hands, dysplastic ears, pointed chin, dolichocephaly, ptosis, tendency to overheat, and epicanthic folds. Researchers have showed that Phelan-McDermid syndrome neurons have reduced SHANK3 expression and major defects in excitatory, but not inhibitory, synaptic transmission. Excitatory synaptic transmission in Phelan-McDermid syndrome neurons could be corrected by restoring SHANK3 expression or by treating neurons with insulin-like growth factor-1. IGF1 treatment promoted formation of mature excitatory synapses that lack SHANK3 but contained PSD95 and NMDA receptors with fast deactivation kinetics.

### Schizophrenia-15 (SCZD15)

Susceptibility to schizophrenia-15 (SCZD15) has been associated with mutation in the SH3 and multiple ankyrin repeat domains-3 gene (SHANK3). Schizophrenia-15 is a complex, multifactorial psychotic disorder or group of disorders characterized by disturbances in the form and content of thought (e.g. delusions, hallucinations), in mood (e.g. inappropriate affect), in sense of self and relationship to the external world (e.g. loss of ego boundaries, withdrawal), and in behavior (e.g bizarre or apparently purposeless behavior). Although it affects emotions, it is distinguished from mood disorders in which such disturbances are primary. Similarly, there may be mild impairment of cognitive function, and it is distinguished from the dementias in which disturbed cognitive function is considered primary. Some patients manifest schizophrenic as well as bipolar disorder symptoms and are often given the diagnosis of schizoaffective disorder.

### NF2

The NF2 gene encodes the protein merlin, also known as schwannomin. Merlin is a membrane-cytoskeleton scaffolding protein, i.e. linking actin filaments to cell membrane or membrane glycoproteins. Human merlin is predominantly found in nervous tissue, but also in several other fetal tissues, and is mainly located in adherens junctions. NF2 belongs to the tumor suppressor group of genes. The phosphorylation of serine 518 is known to alter the functional state of merlin. The signaling pathway of merlin is proposed to include several salient cell growth controlling molecules, including eIF3c, CD44, protein kinase A, and p21 activated kinases. Mutations of the NF2 gene cause a human autosomal dominant disease called neurofibromatosis type 2. It is characterized by the development of tumors of the nervous system, most commonly of bilateral vestibular schwannomas (also called acoustic neuromas).

### Neurofibromatosis, type 2

Neurofibromatosis type II is caused by mutation in the gene encoding neurofibromin-2, which is also called merlin, on chromosome 22q12.2. Neurofibromatosis type II is an inheritable disorder with an autosomal dominant mode of transmission. Incidence of the disease is about 1 in 60,000. Through statistics, it is suspected that one-half of cases are inherited, and one-half are the result of new, *de novo* mutations.

More than 200 mutations in the *NF2* gene have been identified in people with neurofibromatosis type 2. About 90 percent of *NF2* mutations result in an abnormally shortened version of the merlin protein. This short protein cannot perform its normal tumor suppressor function in cells. Research suggests that the loss of merlin allows cells, especially Schwann cells, to multiply too frequently and form noncancerous tumors. The most common tumors in neurofibromatosis type 2 are vestibular schwannomas, which develop along the nerve that carries information from the inner ear to the brain. Other tumors affecting the nervous system also occur in people with this condition. Somatic mutations in the *NF2* gene are involved in the development of several types of tumors, both noncancerous (benign) and cancerous (malignant).

It is known that merlin's deficiency can result in unmediated progression through the cell cycle due to the lack of contact-mediated tumor suppression, sufficient to result in the tumors characteristic of Neurofibromatosis type II. Mutations of NF II is presumed to result in either a failure to synthesize merlin or the production of a defective peptide that lacks the normal tumor-suppressive effect.

### Meningioma, NF2-related

Somatic mutations in the gene encoding merlin NF2 on chromosome 22q12 have also been found in tumor tissues of a subset of patients with meningiomas without other features of neurofibromatosis-2. Meningioma, is a relatively common neoplasm of the central nervous system that arises from arachnoidal cells. The majority are well differentiated vascular tumors which grow slowly and have a low potential to be invasive, although malignant subtypes occur. Meningiomas have a predilection to arise from the parasagittal region, cerebral convexity, sphenoidal ridge, olfactory groove, and spinal canal. Meningiomas tend to present in the fourth to sixth decades of life with signs indicative of a slowly progressive mass lesion. Specific clinical manifestations depend on the location of the tumor, but may include intracranial hypertension, cranial neuropathies, ataxia, and other focal neurologic signs. Most meningiomas are benign; only a very small percentage of meningiomas become malignant. Many meningiomas are asymptomatic, producing no symptoms throughout a person's life, and if discovered, require no treatment other than periodic observation. Typically, symptomatic meningiomas are treated with either radiosurgery or conventional surgery. Historical evidence of meningiomas has been found going back hundreds of years, with some successful surgeries for their removal beginning in the 1800s.

### Schwannomatosis 1

Individual schwannoma tumors from patients with schwannomatosis have been found to harbor somatic mutations in the neurofibromin-2 gene (NF2). Schwannomas are benign tumors of the peripheral nerve sheath that usually occur singly in otherwise normal individuals. Multiple schwannomas in the same individual suggest an underlying tumor predisposition syndrome. The most common such syndrome is neurofibromatosis II. The hallmark of NF2 is the development of bilateral vestibular nerve schwannomas, but two-thirds or more of all NF2-affected individuals develop schwannomas in other locations, and dermal schwannomas (or neurilemmomas) may precede vestibular tumors in NF2-affected children.

### DNMT1

The DNMT1 gene encodes the enzyme DNA (cytosine-5)-methyltransferase 1. This enzyme is involved in DNA methylation. In particular, the enzyme helps add methyl groups to cytosines. DNA methylation is important in many cellular functions. These functions include gene silencing, regulating reactions involving proteins and lipids, and controlling the processing of neurotransmitters. DNA (cytosine-5)-methyltransferase 1 is active in the adult nervous system. Although its specific function is not well understood, the enzyme may help regulate neuron maturation and differentiation, the ability of neurons to migrate where needed and connect with each other, and neuron survival. In vitro functional expression studies in E. coli and HeLa cells showed that the mutations affected proper folding of DNMT1 and resulted in premature degradation, reduced methyltransferase activity, and impaired heterochromatin binding during the G2 cell cycle phase, leading to global hypomethylation and site-specific hypermethylation. These changes indicated epigenetic dysregulation.

### Hereditary sensory neuropathy type IE (HSN1E)

Hereditary sensory neuropathy type IE (HSN1E) is caused by heterozygous mutation in the DNMT1 gene on chromosome 19p13. Hereditary sensory neuropathy type IE is an autosomal dominant neurodegenerative disorder characterized by adult onset of progressive peripheral sensory loss associated with progressive hearing impairment and early-onset dementia. At least three DNMT1 gene mutations have been identified in people with hereditary sensory and autonomic neuropathy (HSAN IE), a disorder characterized by a gradual dementia, deafness, and sensory problems in the feet. HSAN IE is further characterized by impaired function of sensory neurons, which transmit information about sensations such as pain, temperature, and touch. Sensations in the feet and legs are particularly affected in people with HSAN IE. Gradual loss of sensation in the feet (peripheral neuropathy), which usually begins in adolescence or early adulthood, can lead to difficulty walking. Affected individuals may not be aware of injuries to their feet, which can lead to open sores and infections. If these complications are severe, amputation of the affected areas may be required. HSAN IE is also characterized by a loss of the ability to sweat (sudomotor function), especially on the hands and feet. Sweating is a function of the autonomic nervous system, which also controls involuntary body functions such as heart rate, digestion, and breathing. These other autonomic functions are unaffected in people with HSAN IE. The severity of the signs and symptoms of HSAN IE and their age of onset are variable, even within the same family. The mutations in exon 20, reduce or eliminate the DNA (cytosine-5)-methyltransferase 1 enzyme's methylation function. As a result, maintenance of the neurons that make up the nervous system is impaired. However, it is not known how the mutations cause the specific signs and symptoms of HSAN IE.

### Autosomal dominant cerebellar ataxia, deafness, and narcolepsy (ADCADN)

Autosomal dominant cerebellar ataxia, deafness, and narcolepsy (ADCADN) is caused by heterozygous mutation in the DNMT1 gene on chromosome 19p13. ADCADN is an autosomal dominant neurologic disorder characterized by adult onset of progressive cerebellar ataxia, narcolepsy/cataplexy, sensorineural deafness, and dementia. More variable features include optic atrophy, sensory neuropathy, psychosis, and depression.

At least three DNMT1 gene mutations have been identified in people with autosomal dominant cerebellar ataxia, deafness, and narcolepsy. The mutations associated with this disorder are in exon 2 the DNMT1 gene. Mutations in different locations within the gene may affect the DNA (cytosine-5)-methyltransferase 1 enzyme differently, which can lead to particular combinations of signs and symptoms.

### TCF4

TCF4 encodes transcription factor 4, a basic helix-loop-helix transcription factor. The encoded protein recognizes an Ephrussi-box ('E-box') binding site ('CANNTG') - a motif first identified in immunoglobulin enhancers. This gene is broadly expressed, and may play an important role in nervous system development. The TCF4 protein is found in the brain, muscles, lungs, and heart. This protein also appears to be active in various tissues before birth. The TCF4 protein plays a role cell differentiation and apoptosis.

### Pitt-hopkins syndrome

Pitt-Hopkins syndrome is characterized by mental retardation, wide mouth and distinctive facial features, and intermittent hyperventilation followed by apnea. Pitt-Hopkins syndrome is linked to haploinsufficiency of the TCF4 transcription factor gene. At least 50 mutations in the TCF4 gene have been found to cause Pitt-Hopkins syndrome. Some mutations delete a nucleotide within the TCF4 gene, while other mutations delete the *TCF4* gene as well as a number of genes that surround it. Still other TCF4 gene mutations replace single nucleotides. The size of the mutation does not appear to affect the severity of the condition; individuals with large deletions and those with single nucleotide changes seem to have similar signs and symptoms. TCF4 gene mutations disrupt the protein's ability to bind to DNA and control the activity of certain genes. These gene mutations typically do not affect the TCF4 protein's ability to bind to other proteins. The TCF4 protein's inability to bind to DNA and control the activity of certain genes, particularly those genes involved in nervous system development and function, contributes to the signs and symptoms of Pitt-Hopkins syndrome. It is also likely that the loss of the normal proteins that are attached to the nonfunctional TCF4 proteins contribute to the features of this condition.

### RAI1

The RAI1 gene encodes Retinoic acid-induced protein 1. Retinoic acid-induced protein 1 is an important transcriptional regulator of the circadian clock components: CLOCK, ARNTL/BMAL1, ARNTL2/BMAL2, PER1/3, CRY1/2, NR1D1/2 and RORA/C. Retinoic acid-induced protein 1 positively regulates the transcriptional activity of CLOCK a core component of the circadian clock. Retinoic acid-induced protein 1 regulates transcription through chromatin remodeling by interacting with other proteins in chromatin as well as proteins in the basic transcriptional machinery. Retinoic acid-induced protein 1 may be important for embryonic and postnatal development. Retinoic acid-induced protein 1 may be involved in neuronal differentiation. Mutations in one copy of this gene lead to the production of a nonfunctional version of the RAI1 protein or reduce the amount of this protein that is produced.

### Smith-magenis syndrome

Smith-Magenis syndrome (SMS) is caused in most cases (90%) by a 3.7-Mb interstitial deletion in chromosome 17p11.2. The disorder can also be caused by mutations in the RAI1 gene, which is within the Smith-Magenis chromosome region. Smith-Magenis syndrome is a developmental disorder that affects many parts of the body. The major features of this condition include mild to moderate intellectual disability, delayed speech and language skills, distinctive facial features, sleep disturbances, and behavioral problems. Most people with Smith-Magenis syndrome have a broad, square-shaped face with deep-set eyes, full cheeks, and a prominent lower jaw. The middle of the face and the bridge of the nose often appear flattened. The mouth tends to turn downward with a full, outward-curving upper lip. These facial differences can be subtle in early childhood, but they usually become more distinctive in later childhood and adulthood. Dental abnormalities are also common in affected individuals. Disrupted sleep patterns are characteristic of Smith-Magenis syndrome, typically beginning early in life. Affected individuals may be very sleepy during the day, but they have trouble falling asleep and awaken several times each night. Individuals with Smith-Magenis syndrome have affectionate, engaging personalities, but most also have behavioral problems. These include frequent temper tantrums and outbursts, aggression, anxiety, impulsiveness, and difficulty paying attention. Self-injury, including biting, hitting, head banging, and skin picking, is very common. Repetitive self-hugging is a behavioral trait that may be unique to Smith-Magenis syndrome. Individuals with this condition also compulsively lick their fingers and flip pages of books and magazines (a behavior known as "lick and flip"). Other signs and symptoms of Smith-Magenis syndrome include short stature, abnormal curvature of the spine (scoliosis), reduced sensitivity to pain and temperature, and a hoarse voice. Some people with this disorder have ear abnormalities that lead to hearing loss. Affected individuals may have eye abnormalities that cause nearsightedness (myopia) and other vision problems. Although less common, heart and kidney defects also have been reported in people with Smith-Magenis syndrome. A small percentage of individuals with Smith-Magenis syndrome have a mutation in the RAI1 gene instead of a chromosomal deletion. Although these individuals have many of the major features of the condition, they are less likely than people with a chromosomal deletion to have short stature, hearing loss, and heart or kidney abnormalities.

### PEX1

The PEX1 gene encodes peroxisomal biogenesis factor 1 (Pex1p), which is part of a group of proteins called peroxins. Peroxins are essential for the formation and normal functioning of cell structures called peroxisomes. Peroxisomes are sac-like compartments that contain enzymes needed to break down many different substances, including fatty acids and certain toxic compounds. They are also important for the production of lipids used in digestion and in the nervous system. Peroxins assist in the biogenesis of peroxisomes by producing the membrane that separates the peroxisome from the rest of the cell and by importing enzymes into the peroxisome. Pex1p enables other peroxins to bring enzymes into the peroxisome.

### Peroxisome Biogenesis Disorder 1a (Zellweger syndrome)(PBD1A)

Zellweger syndrome (PBD1A) is caused by homozygous or compound heterozygous mutation in the PEX1 gene on chromosome 7q21-q22. Zellweger syndrome is an autosomal recessive systemic disorder characterized clinically by severe neurologic dysfunction, craniofacial abnormalities, and liver dysfunction, and biochemically by the absence of peroxisomes. Most severely affected individuals with classic Zellweger syndrome phenotype die within the first year of life.

At least 114 mutations in the PEX1 gene have been identified in people with Zellweger spectrum disorder. The conditions' features, which vary in severity, can include weak muscle tone (hypotonia), developmental delay, and vision and hearing problems. Mutations in the PEX1 gene are the most common cause of Zellweger spectrum disorder and are found in nearly 70 percent of affected individuals. There are two common PEX1 gene mutations found in people with Zellweger spectrum disorder. One mutation replaces the amino acid glycine with the amino acid aspartic acid at position 843 in Pex1p (written as Gly843Asp or G843D). This mutation leads to reduced levels of the protein. Individuals who have the G843D mutation tend to have signs and symptoms that are at the less-severe end of the condition spectrum. The other common mutation, which is known as the 1700fs mutation, leads to the production of an abnormally short, nonfunctional Pex1p. Individuals who have the 1700fs mutation often have signs and symptoms that are at the severe end of the condition spectrum. Mutations in the *PEX1* gene that cause Zellweger spectrum disorder reduce or eliminate the activity of the Pex1p protein. Without enough functional Pex1p, enzymes are not properly imported into peroxisomes. As a result, cells contain empty peroxisomes that cannot carry out their usual functions. The severe end of the condition spectrum is caused by the absence of functional peroxisomes within cells. The less severe end of the condition spectrum results from mutations that allow some peroxisomes to form.

### Heimler syndrome-1 (HMLR1)

Heimler syndrome-1 (HMLR1) is caused by homozygous or compound heterozygous mutations in the PEX1 gene on chromosome 7q21. Heimler syndrome-1 (HMLR1), represents the mildest end of the peroxisomal biogenesis disorder spectrum. Heimler syndrome-1 is a rare autosomal recessive disorder characterized by sensorineural hearing loss, enamel hyoplasia of the secondary dentition, and nail abnormalities.

### ARSA

The ARSA encodes enzyme arylsulfatase A. This enzyme is localized in lysosomes. Arylsulfatase A aids the processing of sulfatides. For example, Arylsulfatase A hydrolyzes cerebroside sulfate to cerebroside and sulfate. Sulfatides are a subgroup of sphingolipids, a category of fats that are important components of cell membranes. Sulfatides are abundant in the nervous system's white matter, consisting of nerve fibers covered by myelin.

### Metachromatic Leukodystrophy

Metachromatic leukodystrophy is caused by mutation in the arylsulfatase A gene (ARSA) on chromosome 22q13. Metachromatic leukodystrophy is an inherited disorder characterized by the accumulation of sulfatides in cells. This accumulation especially affects cells in the nervous system that produce myelin. Sulfatide accumulation in myelin-producing cells causes progressive destruction of white matter (leukodystrophy) throughout the nervous system, including in the central nervous system and the nerves connecting the brain and spinal cord to muscles and sensory cells that detect sensations such as touch, pain, heat, and sound (the peripheral nervous system). In individuals with metachromatic leukodystrophy, white matter damage causes progressive deterioration of intellectual functions and motor skills, such as the ability to walk. Affected individuals also develop loss of sensation in the extremities (peripheral neuropathy), incontinence, seizures, paralysis, an inability to speak, blindness, and hearing loss. Eventually they lose awareness of their surroundings and become unresponsive. While neurological problems are the primary feature of metachromatic leukodystrophy, effects of sulfatide accumulation on other organs and tissues have been reported, most often involving the gallbladder.

The most common form of metachromatic leukodystrophy, affecting about 50 to 60 percent of all individuals with this disorder, is called the late infantile form. This form of the disorder usually appears in the second year of life. Affected children lose any speech they have developed, become weak, and develop problems with walking (gait disturbance). As the disorder worsens, muscle tone generally first decreases, and then increases to the point of rigidity. Individuals with the late infantile form of metachromatic leukodystrophy typically do not survive past childhood. In 20 to 30 percent of individuals with metachromatic leukodystrophy, onset occurs between the age of 4 and adolescence. In this juvenile form, the first signs of the disorder may be behavioral problems and increasing difficulty with schoolwork. Progression of the disorder is slower than in the late infantile form, and affected individuals may survive for about 20 years after diagnosis.

The adult form of metachromatic leukodystrophy affects approximately 15 to 20 percent of individuals with the disorder. In this form, the first symptoms appear during the teenage years or later. Often behavioral problems such as alcoholism, drug abuse, or difficulties at school or work are the first symptoms to appear. The affected individual may experience psychiatric symptoms such as delusions or hallucinations. People with the adult form of metachromatic leukodystrophy may survive for 20 to 30 years after diagnosis. During this time there may be some periods of relative stability and other periods of more rapid decline.

### EIF2b5/EIF2B1/EIF2B2

The EIF2B5, EIF2B1 and EIF2B2 genes encodes a subunit of eukaryotic translation initiation factor 2B (eIF2B), a heteropentameric guanine nucleotide exchange factor necessary for the proper function of the translation initiation factor eIF2, and an essential regulator for protein synthesis. eIF2B catalyzes the exchange of GDP for GTP. Under some conditions, eIF2B increases protein synthesis under other conditions, eLF2B slows protein synthesis. Proper regulation of protein synthesis is vital for ensuring that the correct levels of protein are available for the cell to cope with changing conditions. Mutations in eIF2B can cause partial loss of eIF2B function. Impairment of eIF2B function makes it more difficult for cells to regulate protein synthesis and deal with changing conditions and stress.

### Leukoencephalopathy with vanishing white matter (VWM)

Leukoencephalopathy with vanishing white matter (VWM) can be caused by homozygous or compound heterozygous mutation in any of the 5 genes encoding subunits of the translation initiation factor EIF2B: EIF2B 1 on chromosome 12q24, EIF2B2 on chromosome 14q24, EIF2B3 on chromosome 1p34, EIF2B4 on chromosome 2p23, or EIF2B5 on chromosome 3q27. Vanishing white matter leukodystrophy is an autosomal recessive neurologic disorder characterized by variable neurologic features, including progressive cerebellar ataxia, spasticity, and cognitive impairment associated with white matter lesions on brain imaging. The age at onset can range from early infancy to adulthood. Rapid neurologic deterioration can occur following minor head trauma. Female mutation carriers may develop ovarian failure, manifest as primary amenorrhea or as secondary amenorrhea lasting more than 6 months, associated with elevated gonadotropin levels at age less than 40 years.

Neurologic deterioration usually begins in late infancy or early childhood, but juvenile- and adult-onset cases have been described. Mild and severe cases have been observed, even within families. The neurologic signs include progressive cerebellar ataxia, spasticity, inconstant optic atrophy, and relatively preserved mental abilities. Disease is chronic-progressive with, in most individuals, additional episodes of rapid deterioration following febrile infections or minor head trauma. Head trauma leads only to motor deterioration, whereas infections with fever may end in coma. Death occurs after a variable period of several years to a few decades, usually following an episode of fever and coma. MRI is diagnostic and shows a diffuse abnormality of the cerebral white matter beginning in the presymptomatic stage. Both MRI and magnetic resonance spectroscopy indicate that, with time, an increasing amount of the abnormal white matter vanishes and is replaced by cerebrospinal fluid. The mode of inheritance is autosomal recessive.

### NPC1

NPC1 encodes Niemann-Pick disease, type C1 (NPC1), a large protein that resides in the limiting membrane of endosomes and lysosomes and mediates intracellular cholesterol trafficking via binding of cholesterol to its N-terminal domain. It is predicted to have a cytoplasmic C-terminus, 13 transmembrane domains, and 3 large loops in the lumen of the endosome - the last loop being at the N-terminus. This protein transports low-density lipoproteins to late endosomal/lysosomal compartments where they are hydrolized and released as free cholesterol.

Mutations in NPC1 leads to a shortage of NPC1, which prevents movement of cholesterol and other lipids, leading to their accumulation in cells. Because these lipids are not in their proper location in cells, many normal cell functions that require lipids (such as cell membrane formation) are impaired. The accumulation of lipids as well as the cell dysfunction eventually leads to cell death, causing the tissue and organ damage seen in Niemann-Pick disease types C1.

### Niemann-Pick disease type C1 and Niemann-Pick disease type D

Niemann-Pick disease type C1 and Niemann-Pick disease type D, also known as the Nova Scotian type, are caused by mutation in the NPC1 gene. Niemann-Pick type C (NPC) disease is an autosomal recessive lipid storage disorder characterized by progressive neurodegeneration. Approximately 95% of cases are caused by mutations in the NPC1 gene, referred to as type C1; 5% are caused by mutations in the NPC2 gene, referred to as type C2. The clinical manifestations of types C1 and C2 are similar because the respective genes are both involved in egress of lipids, particularly cholesterol, from late endosomes or lysosomes. Niemann-Pick disease types C1 and C2 usually become apparent in childhood, although signs and symptoms can develop at any time. People with these types usually develop difficulty coordinating movements (ataxia), an inability to move the eyes vertically (vertical supranuclear gaze palsy), poor muscle tone (dystonia), severe liver disease, and interstitial lung disease. Individuals with Niemann-Pick disease types C1 and C2 have problems with speech and swallowing that worsen over time, eventually interfering with feeding. Affected individuals often experience progressive decline in intellectual function and about one-third have seizures.

### ADAR

ADAR( adenosine deaminase acting on RNA) encodes Double-stranded RNA-specific adenosine deaminase. ADAR catalyzes the deamination of adenosine to inosine in dsRNA substrates, induces translation within the nucleus, possibly at the surface of the nucleolus. ADAR catalyzes the hydrolytic deamination of adenosine to inosine in double-stranded RNA (dsRNA) referred to as A-to-I RNA editing. This may affect gene expression and function in a number of ways that include mRNA translation by changing codons and hence the amino acid sequence of proteins; pre-mRNA splicing by altering splice site recognition sequences; RNA stability by changing sequences involved in nuclease recognition; genetic stability in the case of RNA virus genomes by changing sequences during viral RNA replication; and RNA structure-dependent activities such as microRNA production or targeting or protein-RNA interactions. ADAR can edit both viral and cellular RNAs and can edit RNAs at multiple sites (hyper-editing) or at specific sites (site-specific editing). ADAR cellular RNA substrates include: bladder cancer-associated protein (BLCAP), neurotransmitter receptors for glutamate (GRIA2) and serotonin (HTR2C) and GABA receptor (GABRA3). Site-specific RNA editing of transcripts encoding these proteins results in amino acid substitutions which consequently alters their functional activities. Exhibits low-level editing at the GRIA2 Q/R site, but edits efficiently at the R/G site and HOTSPOT1. Its viral RNA substrates include: hepatitis C virus (HCV), vesicular stomatitis virus (VSV), measles virus (MV), hepatitis delta virus (HDV), and human immunodeficiency virus type 1 (HIV-1). ADAR exhibits either a proviral (HDV, MV, VSV and HIV-1) or an antiviral effect (HCV) and this can be editing-dependent (HDV and HCV), editing-independent (VSV and MV) or both (HIV-1). ADAR impairs HCV replication via RNA editing at multiple sites. ADAR enhances the replication of MV, VSV and HIV-1 through an editing-independent mechanism via suppression of EIF2AK2/PKR activation and function. ADAR stimulates both the release and infectivity of HIV-1 viral particles by an editing-dependent mechanism where it associates with viral RNAs and edits adenosines in the 5'UTR and the Rev and Tat coding sequence. ADAR can enhance viral replication of HDV via A-to-I editing at a site designated as amber/W, thereby changing an UAG amber stop codon to an UIG tryptophan (W) codon that permits synthesis of the large delta antigen (L-HDAg) which has a key role in the assembly of viral particles. However, high levels of ADAR1 inhibit HDV replication.

### Aicardi-Goutieres syndrome-6 (AGS6)

Aicardi-Goutieres syndrome-6 (AGS6) can be caused by homozygous, compound heterozygous, or heterozygous mutation in the ADAR gene on chromosome 1q21.3. Aicardi-Goutières syndrome (AGS) manifests as an early-onset encephalopathy that usually, but not always, results in severe intellectual and physical handicap. A subgroup of infants with AGS present at birth with abnormal neurologic findings, hepatosplenomegaly, elevated liver enzymes, and thrombocytopenia, a picture highly suggestive of congenital infection. Otherwise, most affected infants present at variable times after the first few weeks of life, frequently after a period of apparently normal development. Typically, they demonstrate the subacute onset of a severe encephalopathy characterized by extreme irritability, intermittent sterile pyrexias, loss of skills, and slowing of head growth. Over time, as many as 40% develop chilblain skin lesions on the fingers, toes, and ears. It is becoming apparent that atypical, sometimes milder, cases of AGS exist, and thus the true extent of the phenotype associated with mutation of the AGS-related genes is not yet known. For example, mutation of ADAR has recently been associated with a clinical presentation of acute bilateral striatal necrosis.

### MFSD8

MFSD8 gene, encodes a putative lysosomal transporter, on chromosome 4q28.The MFSD8 protein is found in lysosomes. The MFSD8 protein belongs to a large group of related proteins called the major facilitator superfamily of secondary active transporter proteins. Proteins in this family move certain molecules between structures in cells or in and out of cells. While it is likely that the MFSD8 protein transports molecules, the specific molecules it moves are unknown. The MFSD8 protein probably transports substances across the membranes of lysosomes.

### Neuronal ceroid lipofuscinosis-7

Neuronal ceroid lipofuscinosis-7 (CLN7) is caused by homozygous or compound heterozygous mutation in the MFSD8 gene, which encodes a putative lysosomal transporter, on chromosome 4q28. The neuronal ceroid-lipofuscinoses (NCLs) are a group of inherited, neurodegenerative, lysosomal storage disorders characterized by progressive intellectual and motor deterioration, seizures, and early death. Visual gvhmloss is a feature of most forms. Clinical phenotypes have been characterized traditionally according to the age of onset and order of appearance of clinical features into infantile, late-infantile, juvenile, adult, and Northern epilepsy (also known as progressive epilepsy with mental retardation).

### STXBP1

STXBP1 gene, encodes a syntaxin-binding protein. The encoded protein can play a role in release of neurotransmitters via regulation of syntaxin, a transmembrane attachment protein receptor. Mutations in STXBP1 gene is associated with infantile epileptic encephalopathy-4. STXBP1 can participate in the regulation of synaptic vesicle docking and fusion, through interaction with GTP-binding proteins. STXBP1 can interact with syntaxins 1, 2, and 3 but not syntaxin 4. STXBP1 can play a role in determining the specificity of intracellular fusion reactions.

In humans, STXBP1 is expressed as a 4-kb transcript in various tissues. The highest levels of expression can be observed in the retina and cerebellum.

### Early Infantile Epileptic Encephalopathy-4

Epileptic encephalopathy, early infantile, 4 (EIEE4) is a severe form of epilepsy characterized by frequent tonic seizures or spasms beginning in infancy with a specific EEG finding of suppression-burst patterns, characterized by high-voltage bursts alternating with almost flat suppression phases. Affected individuals can have neonatal or infantile onset of seizures, profound mental retardation, and MRI evidence of brain hypomyelination.

In some cases, in a patient with early infantile epileptic encephalopathy-4, a heterozygous 1631G-A transition in the STXBP1 gene, resulting in a gly544-to-asp (G544D) substitution can be identified. In some cases, a patient with early infantile epileptic encephalopathy-4 can develop seizures by age 10 days with a suppression-burst pattern on EEG. In some cases, a patient with early infantile epileptic encephalopathy-4 can have profound mental retardation and spastic paraplegia at about age 37 years.

In some cases, in a patient with early infantile epileptic encephalopathy-4, a heterozygous 539G-A transition in the STXBP1 gene, resulting in a cys1SO-to-tyr (C180Y) substitution can be identified. In some cases, a patient with early infantile epileptic encephalopathy-4 can have infantile onset of tonic and myoclonic seizures with suppression-burst pattern and hypsarrhythmia, delayed brain myelination, and spastic quadriplegia. In embodiments, mutations in the STXBP1 gene can impair structural stability, lower thermostability, and decrease binding to several functional synaptic proteins.

In some cases, in a patient with early infantile epileptic encephalopathy-4, a heterozygous 1328T-G transversion in the STXBP1 gene, resulting in a met443-to-arg (M443R) substitution can be identified. In some cases, a patient with early infantile epileptic encephalopathy-4 can develop tonic seizures at age 6 weeks and can later have profoundly delayed development. In some cases, a patient with early infantile epileptic encephalopathy-4 can have delayed brain myelination.

In some cases, in a patient with early infantile epileptic encephalopathy-4, a heterozygous 251T-A transversion in the STXBP1 gene, resulting in a val84-to-asp (V84D) substitution can be identified. In some cases, a patient with early infantile epileptic encephalopathy-4 can develop tonic seizures at age 2 months with suppression-burst pattern and hypsarrhythmia, and can later show profound mental retardation.

In some cases, in a patient with Early infantile epileptic encephalopathy-4, a de novo heterozygous 1162C-T transition in exon 14 of the STXBP1 gene, resulting in an arg388-to-ter (R388X) substitution, predicted to truncate the domain-3 region, which together with domain-1 provides a binding surface for syntaxin-1 can be identified. In some cases, a patient with early infantile epileptic encephalopathy-4 can have severe mental retardation, with hypotonia, abnormal gait, tremor, and/or seizures.

In some cases, in a patient with early infantile epileptic encephalopathy-4, a de novo heterozygous G-to-A transition in intron 3 of the STXBP1 gene, resulting in the creation of a stop codon downstream of exon 3 can be identified. In some cases, a patient with early infantile epileptic encephalopathy-4 can have severe mental retardation, with hypotonia, abnormal gait, tremor, and/or seizures.

In some cases, in a patient with Early infantile epileptic encephalopathy-4, a de novo heterozygous 847G-A transition in the STXBP1 gene, resulting in a glu283-to-lys (E283K) substitution at a highly conserved residue can be identified.

### PRICKLE2

The PRICKLE2 gene encodes a postsynaptic protein involved in neuronal architecture and function, prickle planar cell polarity protein 2. Mutations in this gene are associated with progressive myoclonic epilepsy type 5.

### Progressive Myoclonic Epilepsy 5

Epilepsy, progressive myoclonic 5 (EPM5) is a neurodegenerative disorder characterized by myoclonic seizures and variable neurologic symptoms including cognitive decline and persistent movement abnormalities.

In some cases, a heterozygosity for a complex mutation in the PRICKLE2 gene, a 443G-A transition, resulting in an arg148-to-his (R148H) substitution, and a 457G-A transition, resulting in a val153-to-ile (V153I) substitution can be identified in a progressive myoclonic epilepsy patient. In some cases, a heterozygous 1813G-T transversion in the PRICKLE2 gene, resulting in a val605-to-phe (V605F) substitution can be identified in a progressive myoclonic epilepsy patient.

### PRRT2

The PRRT2 gene provides instructions for making the proline-rich transmembrane protein 2 (PRRT2). The function of this protein is thought to be involved in signaling in the brain. Studies show that PRRT2 protein interacts with SNAP25, which is involved in signaling between nerve cells in the brain. SNAP25 helps control the release of neurotransmitters.

### Familial Infantile Convulsion with Paroxysmal Choreoathetosis

Infantile Convulsions and paroxysmal ChoreoAthetosis (ICCA) syndrome is a neurological condition characterized by the occurrence of seizures during the first year of life (Benign familial infantile epilepsy) and choreoathetotic dyskinetic attacks during childhood or adolescence. Benign familial infantile epilepsy can begin at 3 to 12 months of age with a family history of the same type of seizures. Seizures are afebrile, partial or sometimes generalized, and normally disappear after the first year of life. During childhood or adolescence, affected individuals present with paroxysmal kinesigenic dyskinesia with frequent and recurrent episodic choreathetotic or dystonic movements that last less than 1 minute. The attacks can be triggered by the initiation of voluntary movements or startle. The association with other paroxysmal disorders such as migraine, with or without aura, hemiplegic migraine, episodic ataxia and tics has also been described. The genetic loci of ICCA syndrome have been described on chromosomes 16p11.2-q12.1, 16q13-q22.1 and 3q29-29. Mutations in the Proline-rich transmembrane protein 2 (PRRT2) gene, located on 16p11.2, have recently been found in families affected by ICCA syndrome. This gene encodes a membrane protein that interacts with the presynaptic protein SNAP-25 but the mechanism leading to the disease remains unknown. The diagnosis is mainly clinical, based on the appearance of infantile convulsions with benign evolution followed by kinesigenic dyskinesia attacks later on. Genetic testing can confirm the diagnosis. Differential diagnosis can include other paroxysmal dystonias such as paroxysmal exertion-induced dyskinesia and paroxysmal non-kinesigenic dyskinesia triggered by drugs or food intake (such as caffeine and alcohol). ICCA syndrome can present as sporadic or familial; in the latter case, it is transmitted as an autosomal dominant trait that can be variably expressed within the same family. Antiepileptic drugs, mainly phenytoin or carbamazepine, can be effective in controlling seizures and dyskinesia during the active phase of the disorder.

In some cases, in patients familial infantile convulsions with paroxysmal choreoathetosis, a heterozygous 950G-A transition in the PRRT2 gene, resulting in a ser317-to-asn (S317N) substitution in a highly conserved residue can be identified.

In some cases, in patients with familial infantile convulsions with paroxysmal choreoathetosis, a heterozygous C-to-T transition in the PRRT2 gene, resulting in an arg240-to-ter (R240X) substitution can be identified. In some cases, in patients with familial infantile convulsions with paroxysmal choreoathetosis, a heterozygous 1-bp insertion (516insT) in the PRRT2 gene, resulting in premature termination at residue 173 can be identified. In some cases, in patients with familial infantile convulsions with paroxysmal choreoathetosis, a heterozygous 562C-T transition in the PRRT2 gene, resulting in a gln188-to-ter (Q188X) substitution can be identified.

### Episodic Kinesigenic Dyskinesia 1

Epidsodic kinesigenic dyskinesia 1 can be referred to as familial paroxysmal kinesiginc dyskinesia. Familial paroxysmal kinesigenic dyskinesia is a disorder characterized by episodes of abnormal movement that range from mild to severe. In the condition name, paroxysmal indicates that the abnormal movements come and go over time, kinesigenic indicates that episodes are triggered by movement, and dyskinesia refers to involuntary movement of the body.

People with familial paroxysmal kinesigenic dyskinesia experience episodes of irregular jerking or shaking movements that are induced by sudden motion, such as standing up quickly or being startled. An episode may involve slow, prolonged muscle contractions (dystonia); small, fast, "dance-like" motions (chorea); writhing movements of the limbs (athetosis); or, rarely, flailing movements of the limbs (ballismus). Familial paroxysmal kinesigenic dyskinesia may affect one or both sides of the body. The type of abnormal movement varies among affected individuals, even among members of the same family. In many people with familial paroxysmal kinesigenic dyskinesia, a pattern of symptoms called an aura immediately precedes the episode. The aura is often described as a crawling or tingling sensation in the affected body part. Individuals with this condition do not lose consciousness during an episode and do not experience any symptoms between episodes.

Individuals with familial paroxysmal kinesigenic dyskinesia usually begin to show signs and symptoms of the disorder during childhood or adolescence. Episodes typically last less than five minutes, and the frequency of episodes ranges from one per month to 100 per day. In most affected individuals, episodes occur less often with age.

In some people with familial paroxysmal kinesigenic dyskinesia the disorder begins in infancy with recurring seizures called benign infantile convulsions. These seizures usually develop in the first year of life and stop by age 3. When benign infantile convulsions are associated with familial paroxysmal kinesigenic dyskinesia, the condition is known as infantile convulsions and choreoathetosis (ICCA). In families with ICCA, some individuals develop only benign infantile convulsions, some have only familial paroxysmal kinesigenic dyskinesia, and others develop both.
In some cases, in a patient with episodic kinesigenic dyskinesia 1, a heterozygous 1-bp duplication (649dupC) in exon 2 of the PRRT2 gene in the proline-rich domain, resulting in a frameshift and introduction of a stop codon 7 amino acids downstream of the insertion (Arg217ProfsTer8) can be identified. In some cases, in a patient with episodic kinesigenic dyskinesia 1, a heterozygous 4-bp deletion (514delTCTG) in exon 2 of the PRRT2 gene in the proline-rich domain, resulting in a frameshift and premature termination can be identified. In some cases, in a patient with episodic kinesigenic dyskinesia 1, a heterozygous 1-bp deletion (972de1A) in exon 3 of the PRRT2 gene, resulting in a frameshift and premature termination in the second transmembrane motif can be identified. In some cases, in a patient with episodic kinesigenic dyskinesia 1, identified a heterozygous 487C-T transition in exon 2 of the PRRT2 gene can be identified. In some cases, in a patient with episodic kinesigenic dyskinesia 1, a heterozygous 796C-T transition in exon 2 of the PRRT2 gene, resulting in an arg266-to-trp (R266W) substitution in a highly conserved residue can be identified. In some cases, in a patient with Episodic kinesigenic dyskinesia 1, a heterozygous 1-bp deletion (649delC) in the PRRT2 gene, resulting in a frameshift and premature termination (Arg217GlufsTer12) can be identified. In some cases, in a patient with Episodic kinesigenic dyskinesia 1, a heterozygous 748C-T transition in the PRRT2 gene, resulting in a gln250-to-ter (Q250X) substitution in the N-terminal extracellular domain can be identified.

### Benign Familial Infantile Seizuers-2

Benign familial infantile epilepsy (BFIE) is a genetic epileptic syndrome characterized by the occurrence of afebrile repeated seizures in healthy infants, between the third and eighth month of life. Although BFIE cases have been reported worldwide, prevalence and incidence remain unknown. In an Argentinian case series, BFIE have been listed as the third most common type of epilepsy in the first two years of life. Seizures usually occur between 3 to 8 months of life, with clusters (8-10 a day) of repeated and brief episodes (2-5 minutes) over a few days. They are usually focal but can sometimes become generalized. Patients present with motor arrest, unresponsiveness, head and/or eye deviation to one side, staring, fluttering of eyelids, grunting, cyanosis, diffuse hypertonia and unilateral or bilateral clonic jerks of the limbs. During the interictal period, patients regain full consciousness and activity. Psychomotor development is normal. A family history of the same epilepsy is a constant finding. A syndrome called familial infantile convulsions and choreoathetosis has been observed in which BFIE patients present in childhood and/or adolescence with choreoathetotic dyskinetic attacks occurring spontaneously or following diverse stimuli (e.g. exercise, stress). In some rare cases, BFIE has been associated with familial or sporadic hemiplegic migraine.

BFIE is a genetically heterogeneous disease. In the majority of cases, mutations in the proline-rich transmembrane protein 2 (*PRRT2*) gene located at 16p11.2 have been found. This gene encodes a membrane protein that interacts with the presynaptic protein SNAP-25. Mutations have also been found in the *SCN2A* gene (2q24.3) encoding the brain sodium channel NaV1.2 and rarely in the *KCNQ2* (20q13.33) and *KCNQ3* (8q24) genes both encoding potassium channels. Additionally, three other chromosomal loci have been identified that are mapped to chromosome 19q, 16p and 1p.

Family history can orient the diagnosis which can be based on electroencephalography (EEG) and video recordings. EEG can show that partial seizures originate from the parietal-occipital region and that the side of the hemisphere involved can vary between episodes. Seizures can sometimes spread and involve the entire brain. During a cluster of seizures, postictal EEG shows lateralized occipito-parietal delta waves and spikes. Outside the cluster, waking and sleeping interictal EEG is normal. Interictal neurological examination and brain imaging (brain CT and/or MRI) are normal. Genetic testing can confirm the diagnosis.

Differential diagnosis can include benign familial neonatal-infantile seizures, an epileptic syndrome with an intermediate onset between the neonatal and infantile age that shares overlapping clinical characteristics with BFIE and that is mainly due to mutations in the *SCN2A* gene. Other differential diagnoses are benign non-familial infantile seizures, benign infantile seizures associated with mild gastroenteritis and benign infantile focal epilepsy with midline spikes and waves during sleep (BIMSE)

BFIE is transmitted as an autosomal dominant trait with incomplete penetrance. With anti-epileptic treatment (e.g. carbamazepine, valproate, phenobarbital), symptoms quickly disappear and no other type of epilepsy has been reported to reappear.

In some cases, in patients with benign familial infantile seizuers-2, a heterozygous 1-bp deletion (629delC) in the PRRT2 gene, resulting in a frameshift and premature termination (Pro210GlnfsTer19) can be identified. In some cases, in patients with benign familial infantile seizuers-2, a heterozygous 1-bp deletion (291delC) in the PRRT2 gene, resulting in a frameshift and premature termination (Asn98ThrfsTer17) can be identified. In some cases, in patients with Benign familial infantile seizuers-2, a heterozygous 1-bp deletion (c.650delG) in the PRRT2 gene, resulting in a frameshift and premature termination (Arg217GlnfsTer12) can be identified.

### STX1B

The protein encoded by this gene belongs to a family of proteins thought to play a role in the exocytosis of synaptic vesicles. Vesicle exocytosis releases vesicular contents and is important to various cellular functions. For instance, the secretion of transmitters from neurons plays an important role in synaptic transmission. After exocytosis, the membrane and proteins from the vesicle are retrieved from the plasma membrane through the process of endocytosis. Mutations in this gene have been identified as one cause of fever-associated epilepsy syndromes. A possible link between this gene and Parkinson's disease has also been suggested.

Syntaxins are cellular receptors for transport vesicles. One of these proteins, designated syntaxin 1B (STX1B), is directly implicated in the process of calcium-dependent synaptic transmission in rat brain. The expression of this protein is transiently induced by long-term potentiation of synaptic responses in the rat hippocampus. The protein may play an important role in the excitatory pathway of synaptic transmission, which is known to be implicated in several neurologic diseases.

### Generalized Epilepsy with Febrile Seizure Plus Type 9

Generalized epilepsy with febrile seizures plus-9 is an autosomal dominant neurologic disorder characterized by onset of febrile and/or afebrile seizures in early childhood, usually before age 3 years. Seizure types are variable and include generalized tonic-clonic, atonic, myoclonic, complex partial, and absence. Most patients have remission of seizures later in childhood with no residual neurologic deficits, but rare patients may show mild developmental delay or mild intellectual disabilities.

In some cases, in a patient with generalized epilepsy with febrile seizure plus type 9, a heterozygous c.166C-T transition in the STX1B gene, resulting in a gln56-to-ter (Q56X) substitution can be identified.

In some cases, in a patient with generalized epilepsy with febrile seizure plus type 9, a heterozygous complex insertion/deletion mutation in the STX1B gene c.133_134insGGATGTGCATTG ("GGATGTGCATTG" disclosed as SEQ ID NO: 28517), resulting in Lys45delinsArgMetCysIleGlu ("ArgMetCysIleGlu" disclosed as SEQ ID NO: 28518), and c.135_136AC-GA, resulting in a leu46-to-met (L46M) substitution) can be identified. In some cases, in a patient with generalized epilepsy with febrile seizure plus type 9, a heterozygous c.140C-A transversion in the STX1B gene, resulting in a ser47-to-ter (S47X) substitution can be identified In some cases, in a patient with generalized epilepsy with febrile seizure plus type 9, ) identified a heterozygous c.657T-A transversion in the STX1B gene, resulting in a val216-to-glu (V216E) substitution at a highly conserved residue in the SNARE motif can be identified. In some cases, in a patient with generalized epilepsy with febrile seizure plus type 9, a de novo heterozygous c.676G-C transversion in the STX1B gene, resulting in a gly226-to-arg (G226R) substitution at a highly conserved residue in the SNARE motif can be identified.

### IDUA

The *IDUA* gene provides instructions for producing the enzyme alpha-L-iduronidase, which is essential for the breakdown of large sugar molecules, for example glycosaminoglycans (GAGs). Through hydrolysis, alpha-L-iduronidase uses water molecules to break down unsulfated alpha-L-iduronic acid, which is present in heparan sulfate and dermatan sulfate. Alpha-L-iduronidase can be located in lysosomes. More than 100 mutations in the *IDUA* gene have been found to cause mucopolysaccharidosis type I (MPS I). Many mutations that cause MPS I reduce or completely eliminate the function of alpha-L-iduronidase. It usually cannot be determined whether a certain mutation will cause severe or attenuated MPS I; however, people who do not produce alpha-L-iduronidase have the severe form of this disorder.

The lack of alpha-L-iduronidase enzyme activity leads to the accumulation of heparan sulfate and dermatan sulfate within the lysosomes. The buildup of GAGs increases the size of the lysosomes. The accumulated GAGs may also interfere with the functions of other proteins inside the lysosomes and disrupt the movement of molecules inside the cell.

### Hurler-Scheie Syndrome

Hurler-Scheie syndrome is the intermediate form of mucopolysaccharidosis type 1 (MPS1) between the two extremes Hurler syndrome and Scheie syndrome, it is a rare lysosomal storage disease, characterized by skeletal deformities and a delay in motor development. The prevalence of MPS I has been estimated at 1/100,000, with Hurler-Scheie syndrome accounting for 23% of cases or a prevalence of approximately 1/435,000. Patients with Hurler-Scheie syndrome have normal or almost normal intelligence but exhibit various degrees of physical impairment. Patients present in the first years of life with musculoskeletal alterations to different degrees including short stature, multiple dysostosis, thoracic-lumbar kyphosis, progressive coarsening of the facial features to different degrees, cardiomyopathy and valvular abnormalities, neurosensorial hearing loss, enlarged tonsils and adenoids, and nasal secretion. Hydrocephaly can occur after the age of two. Corneal opacity is seen between two and four years of age and requires keratoplasty to restore sight. Other manifestations may include organomegaly, hernias and hirsutism.

Hurler-Scheie syndrome is caused by mutations in the *IDUA* gene (4p16.3) leading to partial deficiency in the alpha-L-iduronidase enzyme and lysosomal accumulation of dermatan sulfate and heparan sulfate. Early diagnosis is difficult because the first clinical signs are not specific. Diagnosis can be based on detection of increased urinary secretion of heparan and dermatan sulfate through 1,9-dimethylmethylene blue (DMB) test and glycosaminoglycan (GAG) electrophoresis, and demonstration of enzymatic deficiency in leukocytes or fibroblasts. Genetic testing is available. Differential diagnoses can include the milder and more severe forms of mucopolysaccharidosis type 1 (Scheie syndrome and Hurler syndrome respectively), mucopolysaccharidosis typeVI and mucopolysaccharidosis type II. Antenatal diagnosis is possible by measurement of enzymatic activity in cultivated chorionic villus or amniocytes and by genetic testing if the disease-causing mutation is known. Transmission is autosomal recessive. Bone marrow or umbilical cord blood transplant has been successful and can preserve neurocognition, improve some aspects of the somatic disease and increase survival. However, it is associated with many risks and most of the positive effects occur only if the procedure is performed in the first two years of life.

The enzyme substitute (laronidase) obtained EU marketing authorization as an orphan drug in 2003. Given through weekly infusions it leads to improvement of lung function and joint mobility. Enzyme replacement therapy (ERT) can be started at diagnosis and may be beneficial in patients awaiting hematopoietic stem cell transplantation (HSCT). Early treatment can slow the progression of the disease. In individual patients with MPS1 of intermediate severity, HSCT may be considered if there is a suitable donor. There are however no data on the efficacy of HSCT in patients with this form of the disease.

Life expectancy for Hurler-Scheie syndrome may be reduced, with death occurring before adolescence due to serious cardiovascular and respiratory complications.

In some cases, in a patient with hurler scheie syndrome, homozygosity for an arg619-to-gly (R619G) mutation due to a C-to-G transversion at nucleotide 1943 can be identified. In some cases, in a patient with hurler scheie syndrome, homozygosity for a thr364-to-met (T364M) mutation in the IDUA gene can be identified.

### Retained Intron Containing Pre-mRNA (RIC Pre-mRNA)

In embodiments, the methods of the present invention can exploit the presence of retained-intron-containing pre-mRNA (RIC pre-mRNA) transcribed from the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B gene and encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, in the cell nucleus. Splicing of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA species to produce mature, fully-spliced, ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B mRNA, can be induced using ASOs that stimulate splicing out of the retained introns. The resulting mature ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B mRNA can be exported to the cytoplasm and translated, thereby increasing the amount of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in the patient's cells and alleviating symptoms of the CNS disease or conditions caused by deficiency in ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B. This method, described further below, is known as Targeted Augmentation of Nuclear Gene Output (TANGO).

In some cases, the methods of the present invention can exploit the presence of retained-intron-containing pre-mRNA (RIC pre-mRNA) transcribed from the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B gene and encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein, in the cell nucleus. Splicing of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B RIC pre-mRNA species to produce mature, fully-spliced, ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B mRNA, can be induced using ASOs that stimulate splicing out of the retained introns. The resulting mature ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B mRNA can be exported to the cytoplasm and translated, thereby increasing the amount of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein in the patient's cells and alleviating symptoms of the CNS disease or conditions caused by deficiency in ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B. This method, described further below, is known as Targeted Augmentation of Nuclear Gene Output (TANGO).

In some instances, the methods of the present invention can exploit the presence of retained-intron-containing pre-mRNA (RIC pre-mRNA) transcribed from the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene and encoding ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein, in the cell nucleus. Splicing of ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B RIC pre-mRNA species to produce mature, fully-spliced, ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B mRNA, can be induced using ASOs that stimulate splicing out of the retained introns. The resulting mature ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B mRNA can be exported to the cytoplasm and translated, thereby increasing the amount of ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein in the patient's cells and alleviating symptoms of the CNS disease or conditions caused by deficiency in ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B. This method, described further below, is known as Targeted Augmentation of Nuclear Gene Output (TANGO).

### Nuclear Transcripts

ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B gene can be analyzed for intron-retention events. In some cases, ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B gene can be analyzed for intron-retention events. In some cases, ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene can be analyzed for intron-retention events. RNA sequencing (RNAseq), can be visualized in the UCSC genome browser, and can show ATP1A2, CACNA1A, SETD5, SHANK3, NF2, DNMT1, TCF4, RAI1, PEX1, ARSA, MFSD8, EIF2B5, EIF2B1, EIF2B2, NPC1, ADAR, STXBP1, PRICKLE2, PRRT2, or STX1B transcripts expressed in human cortical neurons (HCN) or (AST) and localized in either the cytoplasmic or nuclear fraction. The retained-intron containing pre-mRNA transcripts are retained in the nucleus and are not exported out to the cytoplasm.

In embodiments, a retained intron is an intron that is identified as a retained intron based on a determination of at least about 5%, at least about 10%, at least about 15%, at least about 20%, at least about 25%, at least about 30%, at least about 35%, at least about 40%, at least about 45%, or at least about 50%, retention. In embodiments, a retained intron is an intron that is identified as a retained intron based on a determination of about 5% to about 100%, about 5% to about 95%, about 5% to about 90%, about 5% to about 85%, about 5% to about 80%, about 5% to about 75%, about 5% to about 70%, about 5% to about 65%, about 5% to about 60%, about 5% to about 65%, about 5% to about 60%, about 5% to about 55%, about 5% to about 50%, about 5% to about 45%, about 5% to about 40%, about 5% to about 35%, about 5% to about 30%, about 5% to about 25%, about 5% to about 20%, about 5% to about 15%, about 10% to about 100%, about 10% to about 95%, about 10% to about 90%, about 10% to about 85%, about 10% to about 80%, about 10% to about 75%, about 10% to about 70%, about 10% to about 65%, about 10% to about 60%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 15% to about 100%, about 15% to about 95%, about 15% to about 90%, about 15% to about 85%, about 15% to about 80%, about 15% to about 75%, about 15% to about 70%, about 15% to about 65%, about 15% to about 60%, about 15% to about 65%, about 15% to about 60%, about 15% to about 55%, about 15% to about 50%, about 15% to about 45%, about 15% to about 40%, about 15% to about 35%, about 15% to about 30%, about 15% to about 25%, about 20% to about 100%, about 20% to about 95%, about 20% to about 90%, about 20% to about 85%, about 20% to about 80%, about 20% to about 75%, about 20% to about 70%, about 20% to about 65%, about 20% to about 60%, about 20% to about 65%, about 20% to about 60%, about 20% to about 55%, about 20% to about 50%, about 20% to about 45%, about 20% to about 40%, about 20% to about 35%, about 20% to about 30%, about 25% to about 100%, about 25% to about 95%, about 25% to about 90%, about 25% to about 85%, about 25% to about 80%, about 25% to about 75%, about 25% to about 70%, about 25% to about 65%, about 25% to about 60%, about 25% to about 65%, about 25% to about 60%, about 25% to about 55%, about 25% to about 50%, about 25% to about 45%, about 25% to about 40%, or about 25% to about 35%, retention. In embodiments, other ASOs useful for this purpose are identified, using, e.g., methods described herein.

Table 1 provides a non-limiting list of target sequences of RIC pre-mRNA transcript of *ATP1A2*, *CACNA1A*, *SETD5, SHANK3*, *NF2, DNMT1*, *TCF4, RAI1*, *PEX1*, *ARSA, EIF2B5, EIF2B1*, *EIF2B2, NPC1*, *ADAR, STXBP1*, *PRICKLE2*, *PRRT2,* and *STX1B* by sequence ID, and ASOs by sequence ID, useful for increasing production of a protein by targeting a region of a RIC pre-mRNA of *ATP1A2, CACNA1A*, *SETD5, SHANK3*, *NF2, DNMT1*, *TCF4, RAI1*, *PEX1*, *ARSA, EIF2B5, EIF2B1*, *EIF2B2, NPC1*, *ADAR, STXBP1*, *PRICKLE2*, *PRRT2,* or *STX1B.* In embodiments, other ASOs useful for these purposes are identified, using, e.g., methods described herein.

**Table 1. List of targets and ASOs that targeting a gene disclosed herein**

| **Gene SEQ ID NO:** | **Pre-mRNA SEQ ID NO:** | **ASOs** | **Retained Intron** | **Target Sequence SEQ ID NO:** |
|---|---|---|---|---|
| ADAR SEQ ID NO: 1 | ADAR:NM_001193495 SEQ ID NO: 20 | SEQ ID NOs: 81-599 | 2 | 24383 |
| | ADAR:NM 015841 SEQ ID NO: 21 | SEQ ID NOs: 600-1118 | 2 | 24383 |
| | ADAR:NM_015840 SEQ ID NO: 22 | SEQ ID NOs: 1119-1637 | 2 | 24383 |
| | ADAR:NM_001111 SEQ ID NO: 23 | SEQ ID NOs: 1638-2156 | 2 | 24383 |
| | ADAR:NM_001025107 SEQ ID NO: 24 | SEQ ID NOs: 2157-2675 | 2 | 24383 |
| ATP1A2 SEQ ID NO: 2 | ATP1A2:NM_000702 SEQ ID NO: 25 | SEQ ID NOs: 2676-3302 | 22 | 24359 |
| PRICKLE2 SEQ ID NO: 3 | PRICKLE2:NM_198859 SEQ ID NO: 26 | SEQ ID NOs: 3303-3552 | 4 | 24372 |
| SETD5 SEQ ID NO: 4 | SETD5:NM_001080517 SEQ ID NO: 27 | SEQ ID NOs: 3553-3673 | 4 | 24386 |
| | SETD5:NM_001292043 SEQ ID NO: 28 | SEQ ID NOs: 3674-3794 | 5 | 24386 |
| EIF2B5 SEQ ID NO: 5 | EIF2B5:NM_003907 SEQ ID NO: 29 | SEQ ID NOs: 3795-3886 | 12 | 24376 |
| | | SEQ ID NOs: 3887-4032 | 13 | 24364 |
| PEX1 SEQ ID NO: 6 | PEX1:NM_000466 SEQ ID NO: 30 | SEQ ID NOs: 4033-4203 | 10 | 24361 |
| | | SEQ ID NOs: 4204-4445 | 19 | 24370 |
| | | SEQ ID NOs: 4446-4715 | 21 | 24380 |
| | | SEQ ID NOs: 4716-4825 | 14 | 24356 |
| | PEX1:NM_001282677 SEQ ID NO: 31 | SEQ ID NOs: 4826-4996 | 10 | 24361 |
| | | SEQ ID NOs: 4997-5238 | 18 | 24370 |
| | | SEQ ID NOs: 5239-5508 | 20 | 24380 |
| | | SEQ ID NOs: 5509-5618 | 13 | 24356 |
| | PEX1:NM_001282678 SEQ ID NO: 32 | SEQ ID NOs: 5619-5789 | 10 | 24361 |
| | | SEQ ID NOs: 5790-6031 | 19 | 24370 |
| | | SEQ ID NOs: 6032-6301 | 21 | 24380 |
| | | SEQ ID NOs: 6302-6411 | 14 | 24356 |
| STXBP1 SEQ ID NO: 7 | STXBP1:NM_003165 SEQ ID NO: 33 | SEQ ID NOs: 6412-6645 | 18 | 24354 |
| | | SEQ ID NOs: 6646-7196 | 19 | 24351 |
| | STXBP1:NM_001032221 SEQ ID NO: 34 | SEQ ID NOs: 7197-7753 | 18 | 24360 |
| EIF2B 1 SEQ ID NO: 8 | EIF2B1:NM_001414 SEQ ID NO: 35 | SEQ ID NOs: 7754-7964 | 6 | 24353 |
| EIF2B2 SEQ ID NO: 9 | EIF2B2:NM_014239 SEQ ID NO: 36 | SEQ ID NOs: 7965-8053 | 1 | 24358 |
| PRRT2 SEQ ID NO: 10 | PRRT2:NM_001256443 SEQ ID NO: 37 | SEQ ID NOs: 8054-8710 | 1 | 24369 |
| | PRRT2:NM_145239 SEQ ID NO: 38 | SEQ ID NOs: 8711-9021 | 1 | 24365 |
| | PRRT2:NM_001256442 SEQ ID NO: 39 | SEQ ID NOs: 9022-9332 | 1 | 24365 |
| STX1B SEQ ID NO: 11 | STX1B:NM_052874 SEQ ID NO: 40 | SEQ ID NOs: 9333-9379 | 6 | 24385 |
| | | SEQ ID NOs: 9380-9600 | 7 | 24375 |
| RAI1 SEQ ID NO: 12 | RAI1:NM_030665 SEQ ID NO: 41 | SEQ ID NOs: 9601-9803 | 4 | 24382 |
| TCF4 SEQ ID NO: 13 | TCF4:NM_001243236 SEQ ID NO: 42 | SEQ ID NOs: 24387-24643 | 10 | 28516 |
| | TCF4:NM_001243235 SEQ ID NO: 43 | SEQ ID NOs: 24644-24900 | 10 | 28516 |
| | TCF4:NM_001243234 SEQ ID NO: 44 | SEQ ID NOs: 24901-25159 | 10 | 28515 |
| | TCF4:NM_001243233 SEQ ID NO: 45 | SEQ ID NOs: 25160-25416 | 13 | 28516 |
| | TCF4:NM_001243232 SEQ ID NO: 46 | SEQ ID NOs: 25417-25675 | 13 | 28515 |
| | TCF4:NM_001243231 SEQ ID NO: 47 | SEQ ID NOs: 25676-25932 | 15 | 28516 |
| | TCF4:NM_003199 SEQ ID NO: 48 | SEQ ID NOs: 25933-26189 | 17 | 28516 |
| | TCF4:NM_001306207 SEQ ID NO: 49 | SEQ ID NOs: 26190-26446 | 16 | 28516 |
| | TCF4:NM_001306208 SEQ ID NO: 50 | SEQ ID NOs: 26447-26703 | 13 | 28516 |
| | TCF4:NM_001243227 SEQ ID NO: 51 | SEQ ID NOs: 26704-26962 | 16 | 28515 |
| | TCF4:NM_001243228 SEQ ID NO: 52 | SEQ ID NOs: 26963-27221 | 17 | 28515 |
| | TCF4:NM_001243230 SEQ ID NO: 53 | SEQ ID NOs: 27222-27478 | 16 | 28516 |
| | TCF4:NM_001243226 SEQ ID NO: 54 | SEQ ID NOs: 27479-27737 | 18 | 28515 |
| | TCF4:NM_001083962 SEQ ID NO: 55 | SEQ ID NOs: 27738-27996 | 17 | 28515 |
| | TCF4:NM_001330605 SEQ ID NO: 56 | SEQ ID NOs: 27997-28255 | 12 | 28515 |
| | TCF4:NM_001330604 SEQ ID NO: 57 | SEQ ID NOs: 28256-28514 | 17 | 28515 |
| NPC1 SEQ ID NO: 14 | NPC1:NM_000271 SEQ ID NO: 58 | SEQ ID NOs: 13692-13760 | 15 | 24382 |
| CACNA1A SEQ ID NO: 15 | CACNA1A:NM_000068 SEQ ID NO: 59 | SEQ ID NOs: 13761-13980 | 36 | 24384 |
| | | SEQ ID NOs: 13981-14196 | 37 | 24377 |
| | CACNA1A:NM_023035 SEQ ID NO: 60 | SEQ ID NOs: 14197-14416 | 36 | 24384 |
| | | SEQ ID NOs: 14417-14632 | 37 | 24377 |
| | CACNA1A:NM_001174080 SEQ ID NO: 61 | SEQ ID NOs: 14633-14852 | 36 | 24384 |
| | | SEQ ID NOs: 14853-15068 | 37 | 24377 |
| | CACNA1A:NM_001127222 SEQ ID NO: 62 | SEQ ID NOs: 15069-15288 | 36 | 24384 |
| | | SEQ ID NOs: 15289-15504 | 37 | 24377 |
| | CACNA1A:NM_001127221 SEQ ID NO: 63 | SEQ ID NOs: 15505-15710 | 36 | 24374 |
| | | SEQ ID NOs: 15711-15914 | 37 | 24366 |
| DNMT1 SEQ ID NO: 16 | DNMT1:NM_001379 SEQ ID NO: 64 | SEQ ID NOs: 15915-16127 | 29 | 24371 |
| | | SEQ ID NOs: 16128-16168 | 14 | 24362 |
| | DNMT1:NM_001130823 SEQ ID NO: 65 | SEQ ID NOs: 16169-16381 | 30 | 24371 |
| | | SEQ ID NOs: 16382-16422 | 15 | 24362 |
| | DNMT1:NM_001318730 SEQ ID NO: 66 | SEQ ID NOs: 16423-16635 | 29 | 24371 |
| | | SEQ ID NOs: 16636-16676 | 14 | 24362 |
| | DNMT1:NM_001318731 SEQ ID NO: 67 | SEQ ID NOs: 16677-16889 | 30 | 24371 |
| | | SEQ ID NOs: 16890-16930 | 15 | 24362 |
| NF2 SEQ ID NO: 17 | NF2:NM_181830 SEQ ID NO: 68 | SEQ ID NOs: 16931-17842 | 14 | 24373 |
| | NF2:NM_181829 SEQ ID NO: 69 | SEQ ID NOs: 17843-18754 | 15 | 24373 |
| | NF2:NM_181833 SEQ ID NO: 70 | SEQ ID NOs: 18755-19672 | 4 | 24367 |
| | NF2:NM_181832 SEQ ID NO: 71 | SEQ ID NOs: 19673-20587 | 16 | 24352 |
| | NF2:NM_181828 SEQ ID NO: 72 | SEQ ID NOs: 20588-21499 | 15 | 24373 |
| | NF2:NM_016418 SEQ ID NO: 73 | SEQ ID NOs: 21500-22411 | 16 | 24373 |
| | NF2:NM_000268 SEQ ID NO: 74 | SEQ ID NOs: 22412-23347 | 15 | 24368 |
| SHANK3 SEQ ID NO: 18 | SHANK3:NM_033517 SEQ ID NO: 75 | SEQ ID NOs: 23348-23535 | 16 | 24357 |
| ARSA SEQ ID NO: 19 | ARSA:NM_000487 SEQ ID NO: 76 | SEQ ID NOs: 23536-23622 | 2 | 24378 |
| | | SEQ ID NOs: 23623-23698 | 3 | 24363 |
| | ARSA:NM_001085425 SEQ ID NO: 77 | SEQ ID NOs: 23699-23785 | 3 | 24378 |
| | | SEQ ID NOs: 23786-23861 | 4 | 24363 |
| | ARSA:NM_001085426 SEQ ID NO: 78 | SEQ ID NOs: 23862-23948 | 3 | 24378 |
| | | SEQ ID NOs: 23949-24024 | 4 | 24363 |
| | ARSA:NM_001085427 SEQ ID NO: 79 | SEQ ID NOs: 24025-24111 | 3 | 24378 |
| | | SEQ ID NOs: 24112-24187 | 4 | 24363 |
| | ARSA:NM_001085428 SEQ ID NO: 80 | SEQ ID NOs: 24188-24274 | 2 | 24378 |
| | | SEQ ID NOs: 24275-24350 | 3 | 24363 |

In some embodiments, the ASOs disclosed herein target a RIC pre-mRNA transcribed from a *ADAR* genomic sequence. In some embodiments, the ASO targets a RIC pre-mRNA transcript from a *ADAR* genomic sequence comprising a retained intron. In some embodiments, the ASO targets a RIC pre-mRNA transcript of SEQ ID NO: 1. In some embodiments, the ASO targets a RIC pre-mRNA transcript of SEQ ID NO: 1 comprising a retained intron. In some embodiments, the ASOs disclosed herein target a *ADAR* RIC pre-mRNA sequence. In some embodiments, the ASO targets a *ADAR* RIC pre-mRNA transcriptcomprising a retained intron at 2, wherein the intron numbering correspond to the mRNA sequence at NM_001193495. In some embodiments, the ASO targets a *ADAR* RIC pre-mRNA transcriptcomprising a retained intron at 2, wherein the intron numbering correspond to the mRNA sequence at NM_015841. In some embodiments, the ASO targets a *ADAR* RIC pre-mRNA transcriptcomprising a retained intron at 2, wherein the intron numbering correspond to the mRNA sequence at NM_015840. In some embodiments, the ASO targets a *ADAR* RIC pre-mRNA transcriptcomprising a retained intron at 2, wherein the intron numbering correspond to the mRNA sequence at NM _001111. In some embodiments, the ASO targets a *ADAR* RIC pre-mRNA transcriptcomprising a retained intron at 2, wherein the intron numbering correspond to the mRNA sequence at NM_001025107.

In some embodiments, the ASO targets *a ADAR* RIC pre-mRNA sequence according to SEQ ID NO: 20. In some embodiments, the ASO targets a *ADAR* RIC pre-mRNA sequence according to SEQ ID NO: 20 comprising a retained intron 2. In some embodiments, the ASO targets *a ADAR* RIC pre-mRNA sequence according to SEQ ID NO: 21. In some embodiments, the ASO targets a *ADAR* RIC pre-mRNA sequence according to SEQ ID NO: 21 comprising a retained intron 2. In some embodiments, the ASO targets *a ADAR* RIC pre-mRNA sequence according to SEQ ID NO: 22. In some embodiments, the ASO targets a *ADAR* RIC pre-mRNA sequence according to SEQ ID NO: 22 comprising a retained intron 2. In some embodiments, the ASO targets a *ADAR* RIC pre-mRNA sequence according to SEQ ID NO: 23. In some embodiments, the ASO targets a *ADAR* RIC pre-mRNA sequence according to SEQ ID NO: 23 comprising a retained intron 2. In some embodiments, the ASO targets *a ADAR* RIC pre-mRNA sequence according to SEQ ID NO: 24. In some embodiments, the ASO targets a *ADAR* RIC pre-mRNA sequence according to SEQ ID NO: 24 comprising a retained intron 2. In some embodiments, the ASOs disclosed herein target SEQ ID NO: 24383. In some embodiments, the ASO has a sequence according to any one of SEQ ID NOs: 81-2675.

In some embodiments, the ASO targets exon 2 or exon 3 of a *ADAR* RIC pre-mRNA comprising a retained intron 2, wherein the intron numbering correspond to the mRNA sequence at NM_001193495. In some embodiments, the ASO targets an exon 2 sequence upstream (or 5') from the 5' splice site *of a ADAR* RIC pre-mRNA comprising the retained intron 2. In some embodiments, the ASO targets an exon 2 sequence about 4 to about 1566 or about 14 to about 1566 nucleotides upstream (or 5') from the 5' splice site *of a ADAR* RIC pre-mRNA comprising the retained intron 2. In some embodiments, the ASO targets an exon 3 sequence downstream (or 3') from the 3' splice site of a *ADAR* RIC pre-mRNA comprising the retained intron 2. In some embodiments, the ASO targets an exon 3 sequence about 2 to about 165 nucleotides downstream (or 3') from the 3' splice site of a *ADAR* RIC pre-mRNA comprising the retained intron 2.

In some embodiments, the ASO targets intron 2 in *a ADAR* RIC pre-mRNA comprising a retained intron 2, wherein the intron numbering correspond to the mRNA sequence at NM_001193495. In some embodiments, the ASO targets an intron 2 sequence downstream (or 3') from the 5' splice site *of a ADAR* RIC pre-mRNA comprising the retained intron 2. In some embodiments, the ASO targets an intron 2 sequence about 6 to about 500 nucleotides downstream (or 3') from the 5' splice site of a *ADAR* RIC pre-mRNA comprising the retained intron 2. In some embodiments, the ASO targets an intron 2 sequence upstream (or 5') from the 3' splice site *of a ADAR* RIC pre-mRNA comprising the retained intron 2. In some embodiments, the ASO targets an intron 2 sequence about 16 to about 499 nucleotides upstream (or 5') from the 3' splice site of a *ADAR* RIC pre-mRNA comprising the retained intron 2.

In some embodiments, the ASO targets exon 2 or exon 3 of a *ADAR* RIC pre-mRNA comprising a retained intron 2, wherein the intron numbering correspond to the mRNA sequence at NM_015841. In some embodiments, the ASO targets an exon 2 sequence upstream (or 5') from the 5' splice site *of a ADAR* RIC pre-mRNA comprising the retained intron 2. In some embodiments, the ASO targets an exon 2 sequence about 4 to about 1566 or about 14 to about 1566 nucleotides upstream (or 5') from the 5' splice site *of a ADAR* RIC pre-mRNA comprising the retained intron 2. In some embodiments, the ASO targets an exon 3 sequence downstream (or 3') from the 3' splice site of a *ADAR* RIC pre-mRNA comprising the retained intron 2. In some embodiments, the ASO targets an exon 3 sequence about 2 to about 165 nucleotides downstream (or 3') from the 3' splice site of a *ADAR* RIC pre-mRNA comprising the retained intron 2.

In some embodiments, the ASO targets intron 2 in *a ADAR* RIC pre-mRNA comprising a retained intron 2, wherein the intron numbering correspond to the mRNA sequence at NM_015841. In some embodiments, the ASO targets an intron 2 sequence downstream (or 3') from the 5' splice site *of a ADAR* RIC pre-mRNA comprising the retained intron 2. In some embodiments, the ASO targets an intron 2 sequence about 6 to about 500 nucleotides downstream (or 3') from the 5' splice site of a *ADAR* RIC pre-mRNA comprising the retained intron 2. In some embodiments, the ASO targets an intron 2 sequence upstream (or 5') from the 3' splice site *of a ADAR* RIC pre-mRNA comprising the retained intron 2. In some embodiments, the ASO targets an intron 2 sequence about 16 to about 499 nucleotides upstream (or 5') from the 3' splice site of a *ADAR* RIC pre-mRNA comprising the retained intron 2.

In some embodiments, the ASO targets exon 2 or exon 3 *of a ADAR* RIC pre-mRNA comprising a retained intron 2, wherein the intron numbering correspond to the mRNA sequence at NM_015840. In some embodiments, the ASO targets an exon 2 sequence upstream (or 5') from the 5' splice site *of a ADAR* RIC pre-mRNA comprising the retained intron 2. In some embodiments, the ASO targets an exon 2 sequence about 4 to about 1566 or about 14 to about 1566 nucleotides upstream (or 5') from the 5' splice site *of a ADAR* RIC pre-mRNA comprising the retained intron 2. In some embodiments, the ASO targets an exon 3 sequence downstream (or 3') from the 3' splice site of a *ADAR* RIC pre-mRNA comprising the retained intron 2. In some embodiments, the ASO targets an exon 3 sequence about 2 to about 165 nucleotides downstream (or 3') from the 3' splice site of a *ADAR* RIC pre-mRNA comprising the retained intron 2.

In some embodiments, the ASO targets intron 2 in *a ADAR* RIC pre-mRNA comprising a retained intron 2, wherein the intron numbering correspond to the mRNA sequence at NM_015840. In some embodiments, the ASO targets an intron 2 sequence downstream (or 3') from the 5' splice site *of a ADAR* RIC pre-mRNA comprising the retained intron 2. In some embodiments, the ASO targets an intron 2 sequence about 6 to about 500 nucleotides downstream (or 3') from the 5' splice site of a *ADAR* RIC pre-mRNA comprising the retained intron 2. In some embodiments, the ASO targets an intron 2 sequence upstream (or 5') from the 3' splice site *of a ADAR* RIC pre-mRNA comprising the retained intron 2. In some embodiments, the ASO targets an intron 2 sequence about 16 to about 499 nucleotides upstream (or 5') from the 3' splice site of a *ADAR* RIC pre-mRNA comprising the retained intron 2.

In some embodiments, the ASO targets exon 2 or exon 3 of *a ADAR* RIC pre-mRNA comprising a retained intron 2, wherein the intron numbering correspond to the mRNA sequence at NM_001111. In some embodiments, the ASO targets an exon 2 sequence upstream (or 5') from the 5' splice site *of a ADAR* RIC pre-mRNA comprising the retained intron 2. In some embodiments, the ASO targets an exon 2 sequence about 4 to about 1566 or about 14 to about 1566 nucleotides upstream (or 5') from the 5' splice site *of a ADAR* RIC pre-mRNA comprising the retained intron 2. In some embodiments, the ASO targets an exon 3 sequence downstream (or 3') from the 3' splice site of a *ADAR* RIC pre-mRNA comprising the retained intron 2. In some embodiments, the ASO targets an exon 3 sequence about 2 to about 165 nucleotides downstream (or 3') from the 3' splice site of a *ADAR* RIC pre-mRNA comprising the retained intron 2.

In some embodiments, the ASO targets intron 2 in *a ADAR* RIC pre-mRNA comprising a retained intron 2, wherein the intron numbering correspond to the mRNA sequence at NM_001111. In some embodiments, the ASO targets an intron 2 sequence downstream (or 3') from the 5' splice site *of a ADAR* RIC pre-mRNA comprising the retained intron 2. In some embodiments, the ASO targets an intron 2 sequence about 6 to about 500 nucleotides downstream (or 3') from the 5' splice site of a *ADAR* RIC pre-mRNA comprising the retained intron 2. In some embodiments, the ASO targets an intron 2 sequence upstream (or 5') from the 3' splice site *of a ADAR* RIC pre-mRNA comprising the retained intron 2. In some embodiments, the ASO targets an intron 2 sequence about 16 to about 499 nucleotides upstream (or 5') from the 3' splice site of a *ADAR* RIC pre-mRNA comprising the retained intron 2.

In some embodiments, the ASO targets exon 2 or exon 3 *of a ADAR* RIC pre-mRNA comprising a retained intron 2, wherein the intron numbering correspond to the mRNA sequence at NM_001025107. In some embodiments, the ASO targets an exon 2 sequence upstream (or 5') from the 5' splice site *of a ADAR* RIC pre-mRNA comprising the retained intron 2. In some embodiments, the ASO targets an exon 2 sequence about 4 to about 1566 or about 14 to about 1566 nucleotides upstream (or 5') from the 5' splice site *of a ADAR* RIC pre-mRNA comprising the retained intron 2. In some embodiments, the ASO targets an exon 3 sequence downstream (or 3') from the 3' splice site of a *ADAR* RIC pre-mRNA comprising the retained intron 2. In some embodiments, the ASO targets an exon 3 sequence about 2 to about 165 nucleotides downstream (or 3') from the 3' splice site of a *ADAR* RIC pre-mRNA comprising the retained intron 2.

In some embodiments, the ASO targets intron 2 in *a ADAR* RIC pre-mRNA comprising a retained intron 2, wherein the intron numbering correspond to the mRNA sequence at NM_001025107. In some embodiments, the ASO targets an intron 2 sequence downstream (or 3') from the 5' splice site *of a ADAR* RIC pre-mRNA comprising the retained intron 2. In some embodiments, the ASO targets an intron 2 sequence about 6 to about 500 nucleotides downstream (or 3') from the 5' splice site of a *ADAR* RIC pre-mRNA comprising the retained intron 2. In some embodiments, the ASO targets an intron 2 sequence upstream (or 5') from the 3' splice site *of a ADAR* RIC pre-mRNA comprising the retained intron 2. In some embodiments, the ASO targets an intron 2 sequence about 16 to about 499 nucleotides upstream (or 5') from the 3' splice site of a *ADAR* RIC pre-mRNA comprising the retained intron 2.

In some embodiments, the ASOs disclosed herein target a RIC pre-mRNA transcribed from a *ATP1A2* genomic sequence. In some embodiments, the ASO targets a RIC pre-mRNA transcript from a *ATP1A2* genomic sequence comprising a retained intron. In some embodiments, the ASO targets a RIC pre-mRNA transcript of SEQ ID NO: 2. In some embodiments, the ASO targets a RIC pre-mRNA transcript of SEQ ID NO: 2 comprising a retained intron. In some embodiments, the ASOs disclosed herein target a *ATP1A2* RIC pre-mRNA sequence. In some embodiments, the ASO targets a *ATP1A2* RIC pre-mRNA transcript comprising a retained intron at 22, wherein the intron numbering correspond to the mRNA sequence at NM_000702.

In some embodiments, the ASO targets *a ATP1A2* RIC pre-mRNA sequence according to SEQ ID NO: 25. In some embodiments, the ASO targets a *ATP1A2* RIC pre-mRNA sequence according to SEQ ID NO: 25 comprising a retained intron 22. In some embodiments, the ASOs disclosed herein target SEQ ID NO: 24359. In some embodiments, the ASO has a sequence according to any one of SEQ ID NOs: 2676-3302.

In some embodiments, the ASO targets exon 22 or exon 23 of a *ATP1A2* RIC pre-mRNA comprising a retained intron 22, wherein the intron numbering correspond to the mRNA sequence at NM_000702. In some embodiments, the ASO targets an exon 22 sequence upstream (or 5') from the 5' splice site of a *ATP1A2* RIC pre-mRNA comprising the retained intron 22. In some embodiments, the ASO targets an exon 22 sequence about 4 to about 71 nucleotides upstream (or 5') from the 5' splice site of *a ATP1A2* RIC pre-mRNA comprising the retained intron 22. In some embodiments, the ASO targets an exon 23 sequence downstream (or 3') from the 3' splice site of a *ATP1A2* RIC pre-mRNA comprising the retained intron 22. In some embodiments, the ASO targets an exon 23 sequence about 2 to about 2271 nucleotides downstream (or 3') from the 3' splice site of a *ATP1A2* RIC pre-mRNA comprising the retained intron 22.

In some embodiments, the ASO targets intron 22 in a *ATP1A2* RIC pre-mRNA comprising a retained intron 22, wherein the intron numbering correspond to the mRNA sequence at NM_000702. In some embodiments, the ASO targets an intron 22 sequence downstream (or 3') from the 5' splice site of a *ATP1A2* RIC pre-mRNA comprising the retained intron 22. In some embodiments, the ASO targets an intron 22 sequence about 6 to about 498 or about 21 to about 498 nucleotides downstream (or 3') from the 5' splice site of a *ATP1A2* RIC pre-mRNA comprising the retained intron 22. In some embodiments, the ASO targets an intron 22 sequence upstream (or 5') from the 3' splice site of a *ATP1A2* RIC pre-mRNA comprising the retained intron 22. In some embodiments, the ASO targets an intron 22 sequence about 16 to about 500 nucleotides upstream (or 5') from the 3' splice site of a *ATP1A2* RIC pre-mRNA comprising the retained intron 22.

In some embodiments, the ASOs disclosed herein target a RIC pre-mRNA transcribed from a *PRICKLE2* genomic sequence. In some embodiments, the ASO targets a RIC pre-mRNA transcript from a *PRICKLE2* genomic sequence comprising a retained intron. In some embodiments, the ASO targets a RIC pre-mRNA transcript of SEQ ID NO: 3. In some embodiments, the ASO targets a RIC pre-mRNA transcript of SEQ ID NO: 3 comprising a retained intron. In some embodiments, the ASOs disclosed herein target a *PRICKLE2* RIC pre-mRNA sequence. In some embodiments, the ASO targets a *PRICKLE2* RIC pre-mRNA transcript comprising a retained intron at 4, wherein the intron numbering correspond to the mRNA sequence at NM_198859.

In some embodiments, the ASO targets a *PRICKLE2* RIC pre-mRNA sequence according to SEQ ID NO: 26. In some embodiments, the ASO targets a *PRICKLE2* RIC pre-mRNA sequence according to SEQ ID NO: 26 comprising a retained intron 4. In some embodiments, the ASOs disclosed herein target SEQ ID NO: 24372. In some embodiments, the ASO has a sequence according to any one of SEQ ID NOs: 3303-3552.

In some embodiments, the ASO targets exon 4 or exon 5 of a *PRICKLE2* RIC pre-mRNA comprising a retained intron 4, wherein the intron numbering correspond to the mRNA sequence at NM_198859. In some embodiments, the ASO targets an exon 4 sequence upstream (or 5') from the 5' splice site of a *PRICKLE2* RIC pre-mRNA comprising the retained intron 4. In some embodiments, the ASO targets an exon 4 sequence about 4 to about 119 nucleotides upstream (or 5') from the 5' splice site of a *PRICKLE2* RIC pre-mRNA comprising the retained intron 4. In some embodiments, the ASO targets an exon 5 sequence downstream (or 3') from the 3' splice site of a *PRICKLE2* RIC pre-mRNA comprising the retained intron 4. In some embodiments, the ASO targets an exon 5 sequence about 2 to about 187 nucleotides downstream (or 3') from the 3' splice site of a *PRICKLE2* RIC pre-mRNA comprising the retained intron 4.

In some embodiments, the ASO targets intron 4 in a *PRICKLE2* RIC pre-mRNA comprising a retained intron 4, wherein the intron numbering correspond to the mRNA sequence at NM_198859. In some embodiments, the ASO targets an intron 4 sequence downstream (or 3') from the 5' splice site of a *PRICKLE2* RIC pre-mRNA comprising the retained intron 4. In some embodiments, the ASO targets an intron 4 sequence about 6 to about 496 nucleotides downstream (or 3') from the 5' splice site of a *PRICKLE2* RIC pre-mRNA comprising the retained intron 4. In some embodiments, the ASO targets an intron 4 sequence upstream (or 5') from the 3' splice site of a *PRICKLE2* RIC pre-mRNA comprising the retained intron 4. In some embodiments, the ASO targets an intron 4 sequence about 16 to about 499 nucleotides upstream (or 5') from the 3' splice site of a *PRICKLE2* RIC pre-mRNA comprising the retained intron 4.

In some embodiments, the ASOs disclosed herein target a RIC pre-mRNA transcribed from a *SETD5* genomic sequence. In some embodiments, the ASO targets a RIC pre-mRNA transcript from a *SETD5* genomic sequence comprising a retained intron. In some embodiments, the ASO targets a RIC pre-mRNA transcript of SEQ ID NO: 4. In some embodiments, the ASO targets a RIC pre-mRNA transcript of SEQ ID NO: 4 comprising a retained intron. In some embodiments, the ASOs disclosed herein target a *SETD5* RIC pre-mRNA sequence. In some embodiments, the ASO targets a *SETD5* RIC pre-mRNA transcript comprising a retained intron at 4, wherein the intron numbering correspond to the mRNA sequence at NM_001080517. In some embodiments, the ASO targets a *SETD5* RIC pre-mRNA transcript comprising a retained intron at 5, wherein the intron numbering correspond to the mRNA sequence at NM_001292043.

In some embodiments, the ASO targets a *SETD5* RIC pre-mRNA sequence according to SEQ ID NO: 27. In some embodiments, the ASO targets a *SETD5* RIC pre-mRNA sequence according to SEQ ID NO: 27 comprising a retained intron 4. In some embodiments, the ASO targets a *SETD5* RIC pre-mRNA sequence according to SEQ ID NO: 28. In some embodiments, the ASO targets a *SETD5* RIC pre-mRNA sequence according to SEQ ID NO: 28 comprising a retained intron 5. In some embodiments, the ASOs disclosed herein target SEQ ID NO: 24386. In some embodiments, the ASO has a sequence according to any one of SEQ ID NOs: 3553-3794.

In some embodiments, the ASO targets exon 4 or exon 5 of a *SETD5* RIC pre-mRNA comprising a retained intron 4, wherein the intron numbering correspond to the mRNA sequence at NM_001080517. In some embodiments, the ASO targets an exon 4 sequence upstream (or 5') from the 5' splice site of a *SETD5* RIC pre-mRNA comprising the retained intron 4. In some embodiments, the ASO targets an exon 4 sequence about 4 to about 89 nucleotides upstream (or 5') from the 5' splice site of a *SETD5* RIC pre-mRNA comprising the retained intron 4. In some embodiments, the ASO targets an exon 5 sequence downstream (or 3') from the 3' splice site of a *SETD5* RIC pre-mRNA comprising the retained intron 4. In some embodiments, the ASO targets an exon 5 sequence about 2 to about 132 nucleotides downstream (or 3') from the 3' splice site of a *SETD5* RIC pre-mRNA comprising the retained intron 4.

In some embodiments, the ASO targets intron 4 in a *SETD5* RIC pre-mRNA comprising a retained intron 4, wherein the intron numbering correspond to the mRNA sequence at NM_001080517. In some embodiments, the ASO targets an intron 4 sequence downstream (or 3') from the 5' splice site of a *SETD5* RIC pre-mRNA comprising the retained intron 4. In some embodiments, the ASO targets an intron 4 sequence about 6 to about 204 nucleotides downstream (or 3') from the 5' splice site of a *SETD5* RIC pre-mRNA comprising the retained intron 4. In some embodiments, the ASO targets an intron 4 sequence upstream (or 5') from the 3' splice site of a *SETD5* RIC pre-mRNA comprising the retained intron 4. In some embodiments, the ASO targets an intron 4 sequence about 16 to about 204 nucleotides upstream (or 5') from the 3' splice site of a *SETD5* RIC pre-mRNA comprising the retained intron 4.

In some embodiments, the ASO targets exon 5 or exon 6 of a *SETD5* RIC pre-mRNA comprising a retained intron 5, wherein the intron numbering correspond to the mRNA sequence at NM_001292043. In some embodiments, the ASO targets an exon 5 sequence upstream (or 5') from the 5' splice site of a *SETD5* RIC pre-mRNA comprising the retained intron 5. In some embodiments, the ASO targets an exon 5 sequence about 4 to about 89 nucleotides upstream (or 5') from the 5' splice site of a *SETD5* RIC pre-mRNA comprising the retained intron 5. In some embodiments, the ASO targets an exon 6 sequence downstream (or 3') from the 3' splice site of a *SETD5* RIC pre-mRNA comprising the retained intron 5. In some embodiments, the ASO targets an exon 6 sequence about 2 to about 132 nucleotides downstream (or 3') from the 3' splice site of a *SETD5* RIC pre-mRNA comprising the retained intron 5.

In some embodiments, the ASO targets intron 5 in a *SETD5* RIC pre-mRNA comprising a retained intron 5, wherein the intron numbering correspond to the mRNA sequence at NM_001292043. In some embodiments, the ASO targets an intron 5 sequence downstream (or 3') from the 5' splice site of a *SETD5* RIC pre-mRNA comprising the retained intron 5. In some embodiments, the ASO targets an intron 5 sequence about 6 to about 204 nucleotides downstream (or 3') from the 5' splice site of a *SETD5* RIC pre-mRNA comprising the retained intron 5. In some embodiments, the ASO targets an intron 5 sequence upstream (or 5') from the 3' splice site of a *SETD5* RIC pre-mRNA comprising the retained intron 5. In some embodiments, the ASO targets an intron 5 sequence about 16 to about 204 nucleotides upstream (or 5') from the 3' splice site of a *SETD5* RIC pre-mRNA comprising the retained intron 5.

In some embodiments, the ASOs disclosed herein target a RIC pre-mRNA transcribed from a *EIF2B5* genomic sequence. In some embodiments, the ASO targets a RIC pre-mRNA transcript from a *EIF2B5* genomic sequence comprising a retained intron. In some embodiments, the ASO targets a RIC pre-mRNA transcript of SEQ ID NO: 5. In some embodiments, the ASO targets a RIC pre-mRNA transcript of SEQ ID NO: 5 comprising a retained intron. In some embodiments, the ASOs disclosed herein target a *EIF2B5* RIC pre-mRNA sequence. In some embodiments, the ASO targets a *EIF2B5* RIC pre-mRNA transcript comprising a retained intron at 12, 13 or a combination thereof, wherein the intron numbering correspond to the mRNA sequence at NM_003907.

In some embodiments, the ASO targets a *EIF2B5* RIC pre-mRNA sequence according to SEQ ID NO: 29. In some embodiments, the ASO targets a *EIF2B5* RIC pre-mRNA sequence according to SEQ ID NO: 29 comprising a retained intron 12, a retained intron 13, or a combination thereof. In some embodiments, the ASOs disclosed herein target SEQ ID NOs: 24364 or 24376. In some embodiments, the ASO has a sequence according to any one of SEQ ID NOs: 3795-4032.

In some embodiments, the ASO targets exon 12 or exon 13 of a *EIF2B5* RIC pre-mRNA comprising a retained intron 12, wherein the intron numbering correspond to the mRNA sequence at NM_003907. In some embodiments, the ASO targets an exon 12 sequence upstream (or 5') from the 5' splice site of a *EIF2B5* RIC pre-mRNA comprising the retained intron 12. In some embodiments, the ASO targets an exon 12 sequence about 4 to about 74 nucleotides upstream (or 5') from the 5' splice site of a *EIF2B5* RIC pre-mRNA comprising the retained intron 12. In some embodiments, the ASO targets an exon 13 sequence downstream (or 3') from the 3' splice site of a *EIF2B5* RIC pre-mRNA comprising the retained intron 12. In some embodiments, the ASO targets an exon 13 sequence about 2 to about 105 nucleotides downstream (or 3') from the 3' splice site of a *EIF2B5* RIC pre-mRNA comprising the retained intron 12.

In some embodiments, the ASO targets intron 12 in a *EIF2B5* RIC pre-mRNA comprising a retained intron 12, wherein the intron numbering correspond to the mRNA sequence at NM_003907. In some embodiments, the ASO targets an intron 12 sequence downstream (or 3') from the 5' splice site of a *EIF2B5* RIC pre-mRNA comprising the retained intron 12. In some embodiments, the ASO targets an intron 12 sequence about 6 to about 161 nucleotides downstream (or 3') from the 5' splice site of a *EIF2B5* RIC pre-mRNA comprising the retained intron 12. In some embodiments, the ASO targets an intron 12 sequence upstream (or 5') from the 3' splice site of a *EIF2B5* RIC pre-mRNA comprising the retained intron 12. In some embodiments, the ASO targets an intron 12 sequence about 16 to about 164 nucleotides upstream (or 5') from the 3' splice site of a *EIF2B5* RIC pre-mRNA comprising the retained intron 12.

In some embodiments, the ASO targets exon 13 or exon 14 of a *EIF2B5* RIC pre-mRNA comprising a retained intron 13, wherein the intron numbering correspond to the mRNA sequence at NM_003907. In some embodiments, the ASO targets an exon 13 sequence upstream (or 5') from the 5' splice site of a *EIF2B5* RIC pre-mRNA comprising the retained intron 13. In some embodiments, the ASO targets an exon 13 sequence about 4 to about 104 nucleotides upstream (or 5') from the 5' splice site of a *EIF2B5* RIC pre-mRNA comprising the retained intron 13. In some embodiments, the ASO targets an exon 14 sequence downstream (or 3') from the 3' splice site of a *EIF2B5* RIC pre-mRNA comprising the retained intron 13. In some embodiments, the ASO targets an exon 14 sequence about 2 to about 107 nucleotides downstream (or 3') from the 3' splice site of a *EIF2B5* RIC pre-mRNA comprising the retained intron 13.

In some embodiments, the ASO targets intron 13 in a *EIF2B5* RIC pre-mRNA comprising a retained intron 13, wherein the intron numbering correspond to the mRNA sequence at NM_003907. In some embodiments, the ASO targets an intron 13 sequence downstream (or 3') from the 5' splice site of a *EIF2B5* RIC pre-mRNA comprising the retained intron 13. In some embodiments, the ASO targets an intron 13 sequence about 6 to about 276 nucleotides downstream (or 3') from the 5' splice site of a *EIF2B5* RIC pre-mRNA comprising the retained intron 13. In some embodiments, the ASO targets an intron 13 sequence upstream (or 5') from the 3' splice site of a *EIF2B5* RIC pre-mRNA comprising the retained intron 13. In some embodiments, the ASO targets an intron 13 sequence about 16 to about 281 nucleotides upstream (or 5') from the 3' splice site of a *EIF2B5* RIC pre-mRNA comprising the retained intron 13.

In some embodiments, the ASOs disclosed herein target a RIC pre-mRNA transcribed from a *PEX1* genomic sequence. In some embodiments, the ASO targets a RIC pre-mRNA transcript from a *PEX1* genomic sequence comprising a retained intron. In some embodiments, the ASO targets a RIC pre-mRNA transcript of SEQ ID NO: 6. In some embodiments, the ASO targets a RIC pre-mRNA transcript of SEQ ID NO: 6 comprising a retained intron. In some embodiments, the ASOs disclosed herein target a *PEX1* RIC pre-mRNA sequence. In some embodiments, the ASO targets a *PEX1* RIC pre-mRNA transcript comprising a retained intron at 10, 19, 21, 14 or a combination thereof, wherein the intron numbering correspond to the mRNA sequence at NM_000466. In some embodiments, the ASO targets a *PEX1* RIC pre-mRNA transcript comprising a retained intron at 10, 18, 20, 13 or a combination thereof, wherein the intron numbering correspond to the mRNA sequence at NM_001282677. In some embodiments, the ASO targets a *PEX1* RIC pre-mRNA transcript comprising a retained intron at 10, 19, 21, 14 or a combination thereof, wherein the intron numbering correspond to the mRNA sequence at NM_001282678.

In some embodiments, the ASO targets a *PEX1* RIC pre-mRNA sequence according to SEQ ID NO: 30. In some embodiments, the ASO targets a *PEX1* RIC pre-mRNA sequence according to SEQ ID NO: 30 comprising a retained intron 10, a retained intron 19, a retained intron 21, a retained intron 14, or a combination thereof. In some embodiments, the ASO targets a *PEX1* RIC pre-mRNA sequence according to SEQ ID NO: 31. In some embodiments, the ASO targets a *PEX1* RIC pre-mRNA sequence according to SEQ ID NO: 31 comprising a retained intron 10, a retained intron 18, a retained intron 20, a retained intron 13, or a combination thereof. In some embodiments, the ASO targets a *PEX1* RIC pre-mRNA sequence according to SEQ ID NO: 32. In some embodiments, the ASO targets a *PEX1* RIC pre-mRNA sequence according to SEQ ID NO: 32 comprising a retained intron 10, a retained intron 19, a retained intron 21, a retained intron 14, or a combination thereof. In some embodiments, the ASOs disclosed herein target SEQ ID NOs: 24356, 24380, 24370, or 24361. In some embodiments, the ASO has a sequence according to any one of SEQ ID NOs: 4033-6411.

In some embodiments, the ASO targets exon 10 or exon 11 of a *PEX1* RIC pre-mRNA comprising a retained intron 10, wherein the intron numbering correspond to the mRNA sequence at NM_000466. In some embodiments, the ASO targets an exon 10 sequence upstream (or 5') from the 5' splice site of a *PEX1* RIC pre-mRNA comprising the retained intron 10. In some embodiments, the ASO targets an exon 10 sequence about 4 to about 114 nucleotides upstream (or 5') from the 5' splice site of a *PEX1* RIC pre-mRNA comprising the retained intron 10. In some embodiments, the ASO targets an exon 11 sequence downstream (or 3') from the 3' splice site of a *PEX1* RIC pre-mRNA comprising the retained intron 10. In some embodiments, the ASO targets an exon 11 sequence about 2 to about 77 nucleotides downstream (or 3') from the 3' splice site of a *PEX1* RIC pre-mRNA comprising the retained intron 10.

In some embodiments, the ASO targets intron 10 in a *PEX1* RIC pre-mRNA comprising a retained intron 10, wherein the intron numbering correspond to the mRNA sequence at NM_000466. In some embodiments, the ASO targets an intron 10 sequence downstream (or 3') from the 5' splice site of a *PEX1* RIC pre-mRNA comprising the retained intron 10. In some embodiments, the ASO targets an intron 10 sequence about 6 to about 336 nucleotides downstream (or 3') from the 5' splice site of a *PEX1* RIC pre-mRNA comprising the retained intron 10. In some embodiments, the ASO targets an intron 10 sequence upstream (or 5') from the 3' splice site of a *PEX1* RIC pre-mRNA comprising the retained intron 10. In some embodiments, the ASO targets an intron 10 sequence about 16 to about 336 nucleotides upstream (or 5') from the 3' splice site of a *PEX1* RIC pre-mRNA comprising the retained intron 10.

In some embodiments, the ASO targets exon 19 or exon 20 of a *PEX1* RIC pre-mRNA comprising a retained intron 19, wherein the intron numbering correspond to the mRNA sequence at NM_000466. In some embodiments, the ASO targets an exon 19 sequence upstream (or 5') from the 5' splice site of a *PEX1* RIC pre-mRNA comprising the retained intron 19. In some embodiments, the ASO targets an exon 19 sequence about 4 to about 84 nucleotides upstream (or 5') from the 5' splice site of a *PEX1* RIC pre-mRNA comprising the retained intron 19. In some embodiments, the ASO targets an exon 20 sequence downstream (or 3') from the 3' splice site of a *PEX1* RIC pre-mRNA comprising the retained intron 19. In some embodiments, the ASO targets an exon 20 sequence about 2 to about 157 nucleotides downstream (or 3') from the 3' splice site of a *PEX1* RIC pre-mRNA comprising the retained intron 19.

In some embodiments, the ASO targets intron 19 in a *PEX1* RIC pre-mRNA comprising a retained intron 19, wherein the intron numbering correspond to the mRNA sequence at NM_000466. In some embodiments, the ASO targets an intron 19 sequence downstream (or 3') from the 5' splice site of a *PEX1* RIC pre-mRNA comprising the retained intron 19. In some embodiments, the ASO targets an intron 19 sequence about 6 to about 496 nucleotides downstream (or 3') from the 5' splice site of a *PEX1* RIC pre-mRNA comprising the retained intron 19. In some embodiments, the ASO targets an intron 19 sequence upstream (or 5') from the 3' splice site of a *PEX1* RIC pre-mRNA comprising the retained intron 19. In some embodiments, the ASO targets an intron 19 sequence about 16 to about 496 nucleotides upstream (or 5') from the 3' splice site of a *PEX1* RIC pre-mRNA comprising the retained intron 19.

In some embodiments, the ASO targets exon 21 or exon 22 of a *PEX1* RIC pre-mRNA comprising a retained intron 21, wherein the intron numbering correspond to the mRNA sequence at NM_000466. In some embodiments, the ASO targets an exon 21 sequence upstream (or 5') from the 5' splice site of a *PEX1* RIC pre-mRNA comprising the retained intron 21. In some embodiments, the ASO targets an exon 21 sequence about 4 to about 214 nucleotides upstream (or 5') from the 5' splice site of a *PEX1* RIC pre-mRNA comprising the retained intron 21. In some embodiments, the ASO targets an exon 22 sequence downstream (or 3') from the 3' splice site of a *PEX1* RIC pre-mRNA comprising the retained intron 21. In some embodiments, the ASO targets an exon 22 sequence about 2 to about 177 nucleotides downstream (or 3') from the 3' splice site of a *PEX1* RIC pre-mRNA comprising the retained intron 21.

In some embodiments, the ASO targets intron 21 in a *PEX1* RIC pre-mRNA comprising a retained intron 21, wherein the intron numbering correspond to the mRNA sequence at NM_000466. In some embodiments, the ASO targets an intron 21 sequence downstream (or 3') from the 5' splice site of a *PEX1* RIC pre-mRNA comprising the retained intron 21. In some embodiments, the ASO targets an intron 21 sequence about 6 to about 496 nucleotides downstream (or 3') from the 5' splice site of a *PEX1* RIC pre-mRNA comprising the retained intron 21. In some embodiments, the ASO targets an intron 21 sequence upstream (or 5') from the 3' splice site of a *PEX1* RIC pre-mRNA comprising the retained intron 21. In some embodiments, the ASO targets an intron 21 sequence about 16 to about 497 nucleotides upstream (or 5') from the 3' splice site of a *PEX1* RIC pre-mRNA comprising the retained intron 21.

In some embodiments, the ASO targets exon 14 or exon 15 of a *PEX1* RIC pre-mRNA comprising a retained intron 14, wherein the intron numbering correspond to the mRNA sequence at NM_000466. In some embodiments, the ASO targets an exon 14 sequence upstream (or 5') from the 5' splice site of a *PEX1* RIC pre-mRNA comprising the retained intron 14. In some embodiments, the ASO targets an exon 14 sequence about 4 to about 169 nucleotides upstream (or 5') from the 5' splice site of a *PEX1* RIC pre-mRNA comprising the retained intron 14. In some embodiments, the ASO targets an exon 15 sequence downstream (or 3') from the 3' splice site of a *PEX1* RIC pre-mRNA comprising the retained intron 14. In some embodiments, the ASO targets an exon 15 sequence about 2 to about 147 nucleotides downstream (or 3') from the 3' splice site of a *PEX1* RIC pre-mRNA comprising the retained intron 14.

In some embodiments, the ASO targets intron 14 in a *PEX1* RIC pre-mRNA comprising a retained intron 14, wherein the intron numbering correspond to the mRNA sequence at NM_000466. In some embodiments, the ASO targets an intron 14 sequence downstream (or 3') from the 5' splice site of a *PEX1* RIC pre-mRNA comprising the retained intron 14. In some embodiments, the ASO targets an intron 14 sequence about 6 to about 121 nucleotides downstream (or 3') from the 5' splice site of a *PEX1* RIC pre-mRNA comprising the retained intron 14. In some embodiments, the ASO targets an intron 14 sequence upstream (or 5') from the 3' splice site of a *PEX1* RIC pre-mRNA comprising the retained intron 14. In some embodiments, the ASO targets an intron 14 sequence about 16 to about 121 nucleotides upstream (or 5') from the 3' splice site of a *PEX1* RIC pre-mRNA comprising the retained intron 14.

In some embodiments, the ASO targets exon 10 or exon 11 of a *PEX1* RIC pre-mRNA comprising a retained intron 10, wherein the intron numbering correspond to the mRNA sequence at NM_001282677. In some embodiments, the ASO targets an exon 10 sequence upstream (or 5') from the 5' splice site of a *PEX1* RIC pre-mRNA comprising the retained intron 10. In some embodiments, the ASO targets an exon 10 sequence about 4 to about 114 nucleotides upstream (or 5') from the 5' splice site of a *PEX1* RIC pre-mRNA comprising the retained intron 10. In some embodiments, the ASO targets an exon 11 sequence downstream (or 3') from the 3' splice site of a *PEX1* RIC pre-mRNA comprising the retained intron 10. In some embodiments, the ASO targets an exon 11 sequence about 2 to about 77 nucleotides downstream (or 3') from the 3' splice site of a *PEX1* RIC pre-mRNA comprising the retained intron 10.

In some embodiments, the ASO targets intron 10 in a *PEX1* RIC pre-mRNA comprising a retained intron 10, wherein the intron numbering correspond to the mRNA sequence at NM_001282677. In some embodiments, the ASO targets an intron 10 sequence downstream (or 3') from the 5' splice site of a *PEX1* RIC pre-mRNA comprising the retained intron 10. In some embodiments, the ASO targets an intron 10 sequence about 6 to about 336 nucleotides downstream (or 3') from the 5' splice site of a *PEX1* RIC pre-mRNA comprising the retained intron 10. In some embodiments, the ASO targets an intron 10 sequence upstream (or 5') from the 3' splice site of a *PEX1* RIC pre-mRNA comprising the retained intron 10. In some embodiments, the ASO targets an intron 10 sequence about 16 to about 336 nucleotides upstream (or 5') from the 3' splice site of a *PEX1* RIC pre-mRNA comprising the retained intron 10.

In some embodiments, the ASO targets exon 18 or exon 19 of a *PEX1* RIC pre-mRNA comprising a retained intron 18, wherein the intron numbering correspond to the mRNA sequence at NM_001282677. In some embodiments, the ASO targets an exon 18 sequence upstream (or 5') from the 5' splice site of a *PEX1* RIC pre-mRNA comprising the retained intron 18. In some embodiments, the ASO targets an exon 18 sequence about 4 to about 84 nucleotides upstream (or 5') from the 5' splice site of a *PEX1* RIC pre-mRNA comprising the retained intron 18. In some embodiments, the ASO targets an exon 19 sequence downstream (or 3') from the 3' splice site of a *PEX1* RIC pre-mRNA comprising the retained intron 18. In some embodiments, the ASO targets an exon 19 sequence about 2 to about 157 nucleotides downstream (or 3') from the 3' splice site of a *PEX1* RIC pre-mRNA comprising the retained intron 18.

In some embodiments, the ASO targets intron 18 in a *PEX1* RIC pre-mRNA comprising a retained intron 18, wherein the intron numbering correspond to the mRNA sequence at NM_001282677. In some embodiments, the ASO targets an intron 18 sequence downstream (or 3') from the 5' splice site of a *PEX1* RIC pre-mRNA comprising the retained intron 18. In some embodiments, the ASO targets an intron 18 sequence about 6 to about 496 nucleotides downstream (or 3') from the 5' splice site of a *PEX1* RIC pre-mRNA comprising the retained intron 18. In some embodiments, the ASO targets an intron 18 sequence upstream (or 5') from the 3' splice site of a *PEX1* RIC pre-mRNA comprising the retained intron 18. In some embodiments, the ASO targets an intron 18 sequence about 16 to about 496 nucleotides upstream (or 5') from the 3' splice site of a *PEX1* RIC pre-mRNA comprising the retained intron 18.

In some embodiments, the ASO targets exon 20 or exon 21 of a *PEX1* RIC pre-mRNA comprising a retained intron 20, wherein the intron numbering correspond to the mRNA sequence at NM_001282677. In some embodiments, the ASO targets an exon 20 sequence upstream (or 5') from the 5' splice site of a *PEX1* RIC pre-mRNA comprising the retained intron 20. In some embodiments, the ASO targets an exon 20 sequence about 4 to about 214 nucleotides upstream (or 5') from the 5' splice site of a *PEX1* RIC pre-mRNA comprising the retained intron 20. In some embodiments, the ASO targets an exon 21 sequence downstream (or 3') from the 3' splice site of a *PEX1* RIC pre-mRNA comprising the retained intron 20. In some embodiments, the ASO targets an exon 21 sequence about 2 to about 177 nucleotides downstream (or 3') from the 3' splice site of a *PEX1* RIC pre-mRNA comprising the retained intron 20.

In some embodiments, the ASO targets intron 20 in a *PEX1* RIC pre-mRNA comprising a retained intron 20, wherein the intron numbering correspond to the mRNA sequence at NM_001282677. In some embodiments, the ASO targets an intron 20 sequence downstream (or 3') from the 5' splice site of a *PEX1* RIC pre-mRNA comprising the retained intron 20. In some embodiments, the ASO targets an intron 20 sequence about 6 to about 496 nucleotides downstream (or 3') from the 5' splice site of a *PEX1* RIC pre-mRNA comprising the retained intron 20. In some embodiments, the ASO targets an intron 20 sequence upstream (or 5') from the 3' splice site of a *PEX1* RIC pre-mRNA comprising the retained intron 20. In some embodiments, the ASO targets an intron 20 sequence about 16 to about 497 nucleotides upstream (or 5') from the 3' splice site of a *PEX1* RIC pre-mRNA comprising the retained intron 20.

In some embodiments, the ASO targets exon 13 or exon 14 of a *PEX1* RIC pre-mRNA comprising a retained intron 13, wherein the intron numbering correspond to the mRNA sequence at NM_001282677. In some embodiments, the ASO targets an exon 13 sequence upstream (or 5') from the 5' splice site of a *PEX1* RIC pre-mRNA comprising the retained intron 13. In some embodiments, the ASO targets an exon 13 sequence about 4 to about 169 nucleotides upstream (or 5') from the 5' splice site of a *PEX1* RIC pre-mRNA comprising the retained intron 13. In some embodiments, the ASO targets an exon 14 sequence downstream (or 3') from the 3' splice site of a *PEX1* RIC pre-mRNA comprising the retained intron 13. In some embodiments, the ASO targets an exon 14 sequence about 2 to about 147 nucleotides downstream (or 3') from the 3' splice site of a *PEX1* RIC pre-mRNA comprising the retained intron 13.

In some embodiments, the ASO targets intron 13 in a *PEX1* RIC pre-mRNA comprising a retained intron 13, wherein the intron numbering correspond to the mRNA sequence at NM_001282677. In some embodiments, the ASO targets an intron 13 sequence downstream (or 3') from the 5' splice site of a *PEX1* RIC pre-mRNA comprising the retained intron 13. In some embodiments, the ASO targets an intron 13 sequence about 6 to about 121 nucleotides downstream (or 3') from the 5' splice site of a *PEX1* RIC pre-mRNA comprising the retained intron 13. In some embodiments, the ASO targets an intron 13 sequence upstream (or 5') from the 3' splice site of a *PEX1* RIC pre-mRNA comprising the retained intron 13. In some embodiments, the ASO targets an intron 13 sequence about 16 to about 121 nucleotides upstream (or 5') from the 3' splice site of a *PEX1* RIC pre-mRNA comprising the retained intron 13.

In some embodiments, the ASO targets exon 10 or exon 11 of a *PEX1* RIC pre-mRNA comprising a retained intron 10, wherein the intron numbering correspond to the mRNA sequence at NM_001282678. In some embodiments, the ASO targets an exon 10 sequence upstream (or 5') from the 5' splice site of a *PEX1* RIC pre-mRNA comprising the retained intron 10. In some embodiments, the ASO targets an exon 10 sequence about 4 to about 114 nucleotides upstream (or 5') from the 5' splice site of a *PEX1* RIC pre-mRNA comprising the retained intron 10. In some embodiments, the ASO targets an exon 11 sequence downstream (or 3') from the 3' splice site of a *PEX1* RIC pre-mRNA comprising the retained intron 10. In some embodiments, the ASO targets an exon 11 sequence about 2 to about 77 nucleotides downstream (or 3') from the 3' splice site of a *PEX1* RIC pre-mRNA comprising the retained intron 10.

In some embodiments, the ASO targets intron 10 in a *PEX1* RIC pre-mRNA comprising a retained intron 10, wherein the intron numbering correspond to the mRNA sequence at NM_001282678. In some embodiments, the ASO targets an intron 10 sequence downstream (or 3') from the 5' splice site of a *PEX1* RIC pre-mRNA comprising the retained intron 10. In some embodiments, the ASO targets an intron 10 sequence about 6 to about 336 nucleotides downstream (or 3') from the 5' splice site of a *PEX1* RIC pre-mRNA comprising the retained intron 10. In some embodiments, the ASO targets an intron 10 sequence upstream (or 5') from the 3' splice site of a *PEX1* RIC pre-mRNA comprising the retained intron 10. In some embodiments, the ASO targets an intron 10 sequence about 16 to about 336 nucleotides upstream (or 5') from the 3' splice site of a *PEX1* RIC pre-mRNA comprising the retained intron 10.

In some embodiments, the ASO targets exon 19 or exon 20 of a *PEX1* RIC pre-mRNA comprising a retained intron 19, wherein the intron numbering correspond to the mRNA sequence at NM_001282678. In some embodiments, the ASO targets an exon 19 sequence upstream (or 5') from the 5' splice site of a *PEX1* RIC pre-mRNA comprising the retained intron 19. In some embodiments, the ASO targets an exon 19 sequence about 4 to about 84 nucleotides upstream (or 5') from the 5' splice site of a *PEX1* RIC pre-mRNA comprising the retained intron 19. In some embodiments, the ASO targets an exon 20 sequence downstream (or 3') from the 3' splice site of a *PEX1* RIC pre-mRNA comprising the retained intron 19. In some embodiments, the ASO targets an exon 20 sequence about 2 to about 157 nucleotides downstream (or 3') from the 3' splice site of a *PEX1* RIC pre-mRNA comprising the retained intron 19.

In some embodiments, the ASO targets intron 19 in a *PEX1* RIC pre-mRNA comprising a retained intron 19, wherein the intron numbering correspond to the mRNA sequence at NM_001282678. In some embodiments, the ASO targets an intron 19 sequence downstream (or 3') from the 5' splice site of a *PEX1* RIC pre-mRNA comprising the retained intron 19. In some embodiments, the ASO targets an intron 19 sequence about 6 to about 496 nucleotides downstream (or 3') from the 5' splice site of a *PEX1* RIC pre-mRNA comprising the retained intron 19. In some embodiments, the ASO targets an intron 19 sequence upstream (or 5') from the 3' splice site of a *PEX1* RIC pre-mRNA comprising the retained intron 19. In some embodiments, the ASO targets an intron 19 sequence about 16 to about 496 nucleotides upstream (or 5') from the 3' splice site of a *PEX1* RIC pre-mRNA comprising the retained intron 19.

In some embodiments, the ASO targets exon 21 or exon 22 of a *PEX1* RIC pre-mRNA comprising a retained intron 21, wherein the intron numbering correspond to the mRNA sequence at NM_001282678. In some embodiments, the ASO targets an exon 21 sequence upstream (or 5') from the 5' splice site of a *PEX1* RIC pre-mRNA comprising the retained intron 21. In some embodiments, the ASO targets an exon 21 sequence about 4 to about 214 nucleotides upstream (or 5') from the 5' splice site of a *PEX1* RIC pre-mRNA comprising the retained intron 21. In some embodiments, the ASO targets an exon 22 sequence downstream (or 3') from the 3' splice site of a *PEX1* RIC pre-mRNA comprising the retained intron 21. In some embodiments, the ASO targets an exon 22 sequence about 2 to about 177 nucleotides downstream (or 3') from the 3' splice site of a *PEX1* RIC pre-mRNA comprising the retained intron 21.

In some embodiments, the ASO targets intron 21 in a *PEX1* RIC pre-mRNA comprising a retained intron 21, wherein the intron numbering correspond to the mRNA sequence at NM_001282678. In some embodiments, the ASO targets an intron 21 sequence downstream (or 3') from the 5' splice site of a *PEX1* RIC pre-mRNA comprising the retained intron 21. In some embodiments, the ASO targets an intron 21 sequence about 6 to about 496 nucleotides downstream (or 3') from the 5' splice site of a *PEX1* RIC pre-mRNA comprising the retained intron 21. In some embodiments, the ASO targets an intron 21 sequence upstream (or 5') from the 3' splice site of a *PEX1* RIC pre-mRNA comprising the retained intron 21. In some embodiments, the ASO targets an intron 21 sequence about 16 to about 497 nucleotides upstream (or 5') from the 3' splice site of a *PEX1* RIC pre-mRNA comprising the retained intron 21.

In some embodiments, the ASO targets exon 14 or exon 15 of a *PEX1* RIC pre-mRNA comprising a retained intron 14, wherein the intron numbering correspond to the mRNA sequence at NM_001282678. In some embodiments, the ASO targets an exon 14 sequence upstream (or 5') from the 5' splice site of a *PEX1* RIC pre-mRNA comprising the retained intron 14. In some embodiments, the ASO targets an exon 14 sequence about 4 to about 169 nucleotides upstream (or 5') from the 5' splice site of a *PEX1* RIC pre-mRNA comprising the retained intron 14. In some embodiments, the ASO targets an exon 15 sequence downstream (or 3') from the 3' splice site of a *PEX1* RIC pre-mRNA comprising the retained intron 14. In some embodiments, the ASO targets an exon 15 sequence about 2 to about 147 nucleotides downstream (or 3') from the 3' splice site of a *PEX1* RIC pre-mRNA comprising the retained intron 14.

In some embodiments, the ASO targets intron 14 in a *PEX1* RIC pre-mRNA comprising a retained intron 14, wherein the intron numbering correspond to the mRNA sequence at NM_001282678. In some embodiments, the ASO targets an intron 14 sequence downstream (or 3') from the 5' splice site of a *PEX1* RIC pre-mRNA comprising the retained intron 14. In some embodiments, the ASO targets an intron 14 sequence about 6 to about 121 nucleotides downstream (or 3') from the 5' splice site of a *PEX1* RIC pre-mRNA comprising the retained intron 14. In some embodiments, the ASO targets an intron 14 sequence upstream (or 5') from the 3' splice site of a *PEX1* RIC pre-mRNA comprising the retained intron 14. In some embodiments, the ASO targets an intron 14 sequence about 16 to about 121 nucleotides upstream (or 5') from the 3' splice site of a *PEX1* RIC pre-mRNA comprising the retained intron 14.

In some embodiments, the ASOs disclosed herein target a RIC pre-mRNA transcribed from a *STXBP1* genomic sequence. In some embodiments, the ASO targets a RIC pre-mRNA transcript from a *STXBP1* genomic sequence comprising a retained intron. In some embodiments, the ASO targets a RIC pre-mRNA transcript of SEQ ID NO: 7. In some embodiments, the ASO targets a RIC pre-mRNA transcript of SEQ ID NO: 7 comprising a retained intron. In some embodiments, the ASOs disclosed herein target a *STXBP1* RIC pre-mRNA sequence. In some embodiments, the ASO targets a *STXBP1* RIC pre-mRNA transcript comprising a retained intron at 18, 19 or a combination thereof, wherein the intron numbering correspond to the mRNA sequence at NM_003165. In some embodiments, the ASO targets a *STXBP1* RIC pre-mRNA transcript comprising a retained intron at 18, wherein the intron numbering correspond to the mRNA sequence at NM_001032221.

In some embodiments, the ASO targets a *STXBP1* RIC pre-mRNA sequence according to SEQ ID NO: 33. In some embodiments, the ASO targets a *STXBP1* RIC pre-mRNA sequence according to SEQ ID NO: 33 comprising a retained intron 18, a retained intron 19, or a combination thereof. In some embodiments, the ASO targets a *STXBP1* RIC pre-mRNA sequence according to SEQ ID NO: 34. In some embodiments, the ASO targets a *STXBP1* RIC pre-mRNA sequence according to SEQ ID NO: 34 comprising a retained intron 18. In some embodiments, the ASOs disclosed herein target SEQ ID NOs: 24354, 24351, or 24360. In some embodiments, the ASO has a sequence according to any one of SEQ ID NOs: 6412-7753.

In some embodiments, the ASO targets exon 18 or exon 19 of a *STXBP1* RIC pre-mRNA comprising a retained intron 18, wherein the intron numbering correspond to the mRNA sequence at NM_003165. In some embodiments, the ASO targets an exon 18 sequence upstream (or 5') from the 5' splice site of a *STXBP1* RIC pre-mRNA comprising the retained intron 18. In some embodiments, the ASO targets an exon 18 sequence about 4 to about 136 nucleotides upstream (or 5') from the 5' splice site of a *STXBP1* RIC pre-mRNA comprising the retained intron 18. In some embodiments, the ASO targets an exon 19 sequence downstream (or 3') from the 3' splice site of a *STXBP1* RIC pre-mRNA comprising the retained intron 18. In some embodiments, the ASO targets an exon 19 sequence about 2 to about 106 nucleotides downstream (or 3') from the 3' splice site of a *STXBP1* RIC pre-mRNA comprising the retained intron 18.

In some embodiments, the ASO targets intron 18 in a *STXBP1* RIC pre-mRNA comprising a retained intron 18, wherein the intron numbering correspond to the mRNA sequence at NM_003165. In some embodiments, the ASO targets an intron 18 sequence downstream (or 3') from the 5' splice site of a *STXBP1* RIC pre-mRNA comprising the retained intron 18. In some embodiments, the ASO targets an intron 18 sequence about 6 to about 496 nucleotides downstream (or 3') from the 5' splice site of a *STXBP1* RIC pre-mRNA comprising the retained intron 18. In some embodiments, the ASO targets an intron 18 sequence upstream (or 5') from the 3' splice site of a *STXBP1* RIC pre-mRNA comprising the retained intron 18. In some embodiments, the ASO targets an intron 18 sequence about 16 to about 498 nucleotides upstream (or 5') from the 3' splice site of a *STXBP1* RIC pre-mRNA comprising the retained intron 18.

In some embodiments, the ASO targets exon 19 or exon 20 of a *STXBP1* RIC pre-mRNA comprising a retained intron 19, wherein the intron numbering correspond to the mRNA sequence at NM_003165. In some embodiments, the ASO targets an exon 19 sequence upstream (or 5') from the 5' splice site of a *STXBP1* RIC pre-mRNA comprising the retained intron 19. In some embodiments, the ASO targets an exon 19 sequence about 4 to about 109 nucleotides upstream (or 5') from the 5' splice site of a *STXBP1* RIC pre-mRNA comprising the retained intron 19. In some embodiments, the ASO targets an exon 20 sequence downstream (or 3') from the 3' splice site of a *STXBP1* RIC pre-mRNA comprising the retained intron 19. In some embodiments, the ASO targets an exon 20 sequence about 2 to about 1922 nucleotides downstream (or 3') from the 3' splice site of a *STXBP1* RIC pre-mRNA comprising the retained intron 19.

In some embodiments, the ASO targets intron 19 in a *STXBP1* RIC pre-mRNA comprising a retained intron 19, wherein the intron numbering correspond to the mRNA sequence at NM_003165. In some embodiments, the ASO targets an intron 19 sequence downstream (or 3') from the 5' splice site of a *STXBP1* RIC pre-mRNA comprising the retained intron 19. In some embodiments, the ASO targets an intron 19 sequence about 6 to about 499 nucleotides downstream (or 3') from the 5' splice site of a *STXBP1* RIC pre-mRNA comprising the retained intron 19. In some embodiments, the ASO targets an intron 19 sequence upstream (or 5') from the 3' splice site of a *STXBP1* RIC pre-mRNA comprising the retained intron 19. In some embodiments, the ASO targets an intron 19 sequence about 16 to about 500 nucleotides upstream (or 5') from the 3' splice site of a *STXBP1* RIC pre-mRNA comprising the retained intron 19.

In some embodiments, the ASO targets exon 18 or exon 19 of a *STXBP1* RIC pre-mRNA comprising a retained intron 18, wherein the intron numbering correspond to the mRNA sequence at NM_001032221. In some embodiments, the ASO targets an exon 18 sequence upstream (or 5') from the 5' splice site of a *STXBP1* RIC pre-mRNA comprising the retained intron 18. In some embodiments, the ASO targets an exon 18 sequence about 4 to about 136 nucleotides upstream (or 5') from the 5' splice site of a *STXBP1* RIC pre-mRNA comprising the retained intron 18. In some embodiments, the ASO targets an exon 19 sequence downstream (or 3') from the 3' splice site of a *STXBP1* RIC pre-mRNA comprising the retained intron 18. In some embodiments, the ASO targets an exon 19 sequence about 2 to about 1922 nucleotides downstream (or 3') from the 3' splice site of a *STXBP1* RIC pre-mRNA comprising the retained intron 18.

In some embodiments, the ASO targets intron 18 in a *STXBP1* RIC pre-mRNA comprising a retained intron 18, wherein the intron numbering correspond to the mRNA sequence at NM_001032221. In some embodiments, the ASO targets an intron 18 sequence downstream (or 3') from the 5' splice site of a *STXBP1* RIC pre-mRNA comprising the retained intron 18. In some embodiments, the ASO targets an intron 18 sequence about 6 to about 496 nucleotides downstream (or 3') from the 5' splice site of a *STXBP1* RIC pre-mRNA comprising the retained intron 18. In some embodiments, the ASO targets an intron 18 sequence upstream (or 5') from the 3' splice site of a *STXBP1* RIC pre-mRNA comprising the retained intron 18. In some embodiments, the ASO targets an intron 18 sequence about 16 to about 500 nucleotides upstream (or 5') from the 3' splice site of a *STXBP1* RIC pre-mRNA comprising the retained intron 18.

In some embodiments, the ASOs disclosed herein target a RIC pre-mRNA transcribed from a *EIF2B1* genomic sequence. In some embodiments, the ASO targets a RIC pre-mRNA transcript from a *EIF2B1* genomic sequence comprising a retained intron. In some embodiments, the ASO targets a RIC pre-mRNA transcript of SEQ ID NO: 8. In some embodiments, the ASO targets a RIC pre-mRNA transcript of SEQ ID NO: 8 comprising a retained intron. In some embodiments, the ASOs disclosed herein target a *EIF2B1* RIC pre-mRNA sequence. In some embodiments, the ASO targets a *EIF2B1* RIC pre-mRNA transcript comprising a retained intron at 6, wherein the intron numbering correspond to the mRNA sequence at NM_001414.

In some embodiments, the ASO targets a *EIF2B1* RIC pre-mRNA sequence according to SEQ ID NO: 35. In some embodiments, the ASO targets a *EIF2B1* RIC pre-mRNA sequence according to SEQ ID NO: 35 comprising a retained intron 6. In some embodiments, the ASOs disclosed herein target SEQ ID NO: 24353. In some embodiments, the ASO has a sequence according to any one of SEQ ID NOs: 7754-7964.

In some embodiments, the ASO targets exon 6 or exon 7 of a *EIF2B1* RIC pre-mRNA comprising a retained intron 6, wherein the intron numbering correspond to the mRNA sequence at NM_001414. In some embodiments, the ASO targets an exon 6 sequence upstream (or 5') from the 5' splice site of a *EIF2B1* RIC pre-mRNA comprising the retained intron 6. In some embodiments, the ASO targets an exon 6 sequence about 4 to about 49 nucleotides upstream (or 5') from the 5' splice site of a *EIF2B1* RIC pre-mRNA comprising the retained intron 6. In some embodiments, the ASO targets an exon 7 sequence downstream (or 3') from the 3' splice site of a *EIF2B1* RIC pre-mRNA comprising the retained intron 6. In some embodiments, the ASO targets an exon 7 sequence about 2 to about 57 nucleotides downstream (or 3') from the 3' splice site of a *EIF2B1* RIC pre-mRNA comprising the retained intron 6.

In some embodiments, the ASO targets intron 6 in a *EIF2B1* RIC pre-mRNA comprising a retained intron 6, wherein the intron numbering correspond to the mRNA sequence at NM_001414. In some embodiments, the ASO targets an intron 6 sequence downstream (or 3') from the 5' splice site of a *EIF2B1* RIC pre-mRNA comprising the retained intron 6. In some embodiments, the ASO targets an intron 6 sequence about 19 to about 496 nucleotides downstream (or 3') from the 5' splice site of a *EIF2B1* RIC pre-mRNA comprising the retained intron 6. In some embodiments, the ASO targets an intron 6 sequence upstream (or 5') from the 3' splice site of a *EIF2B1* RIC pre-mRNA comprising the retained intron 6. In some embodiments, the ASO targets an intron 6 sequence about 16 to about 498 nucleotides upstream (or 5') from the 3' splice site of a *EIF2B1* RIC pre-mRNA comprising the retained intron 6.

In some embodiments, the ASOs disclosed herein target a RIC pre-mRNA transcribed from a *EIF2B2* genomic sequence. In some embodiments, the ASO targets a RIC pre-mRNA transcript from a *EIF2B2* genomic sequence comprising a retained intron. In some embodiments, the ASO targets a RIC pre-mRNA transcript of SEQ ID NO: 9. In some embodiments, the ASO targets a RIC pre-mRNA transcript of SEQ ID NO: 9 comprising a retained intron. In some embodiments, the ASOs disclosed herein target a *EIF2B2* RIC pre-mRNA sequence. In some embodiments, the ASO targets a *EIF2B2* RIC pre-mRNA transcript comprising a retained intron at 1, wherein the intron numbering correspond to the mRNA sequence at NM_014239.

In some embodiments, the ASO targets a *EIF2B2* RIC pre-mRNA sequence according to SEQ ID NO: 36. In some embodiments, the ASO targets a *EIF2B2* RIC pre-mRNA sequence according to SEQ ID NO: 36 comprising a retained intron 1. In some embodiments, the ASOs disclosed herein target SEQ ID NO: 24358. In some embodiments, the ASO has a sequence according to any one of SEQ ID NOs: 7965-8053.

In some embodiments, the ASO targets exon 1 or exon 2 of a *EIF2B2* RIC pre-mRNA comprising a retained intron 1, wherein the intron numbering correspond to the mRNA sequence at NM_014239. In some embodiments, the ASO targets an exon 1 sequence upstream (or 5') from the 5' splice site of a *EIF2B2* RIC pre-mRNA comprising the retained intron 1. In some embodiments, the ASO targets an exon 1 sequence about 4 to about 219 nucleotides upstream (or 5') from the 5' splice site of a *EIF2B2* RIC pre-mRNA comprising the retained intron 1. In some embodiments, the ASO targets an exon 2 sequence downstream (or 3') from the 3' splice site of a *EIF2B2* RIC pre-mRNA comprising the retained intron 1. In some embodiments, the ASO targets an exon 2 sequence about 2 to about 102 nucleotides downstream (or 3') from the 3' splice site of a *EIF2B2* RIC pre-mRNA comprising the retained intron 1.

In some embodiments, the ASO targets intron 1 in a *EIF2B2* RIC pre-mRNA comprising a retained intron 1, wherein the intron numbering correspond to the mRNA sequence at NM_014239. In some embodiments, the ASO targets an intron 1 sequence downstream (or 3') from the 5' splice site of a *EIF2B2* RIC pre-mRNA comprising the retained intron 1. In some embodiments, the ASO targets an intron 1 sequence about 6 to about 70 nucleotides downstream (or 3') from the 5' splice site of a *EIF2B2* RIC pre-mRNA comprising the retained intron 1. In some embodiments, the ASO targets an intron 1 sequence upstream (or 5') from the 3' splice site of a *EIF2B2* RIC pre-mRNA comprising the retained intron 1. In some embodiments, the ASO targets an intron 1 sequence about 16 to about 70 nucleotides upstream (or 5') from the 3' splice site of a *EIF2B2* RIC pre-mRNA comprising the retained intron 1.

In some embodiments, the ASOs disclosed herein target a RIC pre-mRNA transcribed from a *PRRT2* genomic sequence. In some embodiments, the ASO targets a RIC pre-mRNA transcript from a *PRRT2* genomic sequence comprising a retained intron. In some embodiments, the ASO targets a RIC pre-mRNA transcript of SEQ ID NO: 10. In some embodiments, the ASO targets a RIC pre-mRNA transcript of SEQ ID NO: 10 comprising a retained intron. In some embodiments, the ASOs disclosed herein target a *PRRT2* RIC pre-mRNA sequence. In some embodiments, the ASO targets a *PRRT2* RIC pre-mRNA transcript comprising a retained intron at 1, wherein the intron numbering correspond to the mRNA sequence at NM_001256443. In some embodiments, the ASO targets a *PRRT2* RIC pre-mRNA transcript comprising a retained intron at 1, wherein the intron numbering correspond to the mRNA sequence at NM_145239. In some embodiments, the ASO targets a *PRRT2* RIC pre-mRNA transcript comprising a retained intron at 1, wherein the intron numbering correspond to the mRNA sequence at NM_001256442.

In some embodiments, the ASO targets a *PRRT2* RIC pre-mRNA sequence according to SEQ ID NO: 37. In some embodiments, the ASO targets a *PRRT2* RIC pre-mRNA sequence according to SEQ ID NO: 37 comprising a retained intron 1. In some embodiments, the ASO targets a *PRRT2* RIC pre-mRNA sequence according to SEQ ID NO: 38. In some embodiments, the ASO targets a *PRRT2* RIC pre-mRNA sequence according to SEQ ID NO: 38 comprising a retained intron 1. In some embodiments, the ASO targets a *PRRT2* RIC pre-mRNA sequence according to SEQ ID NO: 39. In some embodiments, the ASO targets a *PRRT2* RIC pre-mRNA sequence according to SEQ ID NO: 39 comprising a retained intron 1. In some embodiments, the ASOs disclosed herein target SEQ ID NOs: 24369 or 24365. In some embodiments, the ASO has a sequence according to any one of SEQ ID NOs: 8054-9332.

In some embodiments, the ASO targets exon 1 or exon 2 of a *PRRT2* RIC pre-mRNA comprising a retained intron 1, wherein the intron numbering correspond to the mRNA sequence at NM_001256443. In some embodiments, the ASO targets an exon 1 sequence upstream (or 5') from the 5' splice site of a *PRRT2* RIC pre-mRNA comprising the retained intron 1. In some embodiments, the ASO targets an exon 1 sequence about 4 to about 244 nucleotides upstream (or 5') from the 5' splice site of a *PRRT2* RIC pre-mRNA comprising the retained intron 1. In some embodiments, the ASO targets an exon 2 sequence downstream (or 3') from the 3' splice site of a *PRRT2* RIC pre-mRNA comprising the retained intron 1. In some embodiments, the ASO targets an exon 2 sequence about 2 to about 2875 nucleotides downstream (or 3') from the 3' splice site of a *PRRT2* RIC pre-mRNA comprising the retained intron 1.

In some embodiments, the ASO targets intron 1 in a *PRRT2* RIC pre-mRNA comprising a retained intron 1, wherein the intron numbering correspond to the mRNA sequence at NM_001256443. In some embodiments, the ASO targets an intron 1 sequence downstream (or 3') from the 5' splice site of a *PRRT2* RIC pre-mRNA comprising the retained intron 1. In some embodiments, the ASO targets an intron 1 sequence about 6 to about 333 nucleotides downstream (or 3') from the 5' splice site of a *PRRT2* RIC pre-mRNA comprising the retained intron 1. In some embodiments, the ASO targets an intron 1 sequence upstream (or 5') from the 3' splice site of a *PRRT2* RIC pre-mRNA comprising the retained intron 1. In some embodiments, the ASO targets an intron 1 sequence about 16 to about 330 or about 26 to about 330 nucleotides upstream (or 5') from the 3' splice site of a *PRRT2* RIC pre-mRNA comprising the retained intron 1.

In some embodiments, the ASO targets exon 1 or exon 2 of a *PRRT2* RIC pre-mRNA comprising a retained intron 1, wherein the intron numbering correspond to the mRNA sequence at NM_145239. In some embodiments, the ASO targets an exon 1 sequence upstream (or 5') from the 5' splice site of a *PRRT2* RIC pre-mRNA comprising the retained intron 1. In some embodiments, the ASO targets an exon 1 sequence about 4 to about 219 nucleotides upstream (or 5') from the 5' splice site of a *PRRT2* RIC pre-mRNA comprising the retained intron 1. In some embodiments, the ASO targets an exon 2 sequence downstream (or 3') from the 3' splice site of a *PRRT2* RIC pre-mRNA comprising the retained intron 1. In some embodiments, the ASO targets an exon 2 sequence about 2 to about 924 nucleotides downstream (or 3') from the 3' splice site of a *PRRT2* RIC pre-mRNA comprising the retained intron 1.

In some embodiments, the ASO targets intron 1 in a *PRRT2* RIC pre-mRNA comprising a retained intron 1, wherein the intron numbering correspond to the mRNA sequence at NM_145239. In some embodiments, the ASO targets an intron 1 sequence downstream (or 3') from the 5' splice site of a *PRRT2* RIC pre-mRNA comprising the retained intron 1. In some embodiments, the ASO targets an intron 1 sequence about 6 to about 346 nucleotides downstream (or 3') from the 5' splice site of a *PRRT2* RIC pre-mRNA comprising the retained intron 1. In some embodiments, the ASO targets an intron 1 sequence upstream (or 5') from the 3' splice site of a *PRRT2* RIC pre-mRNA comprising the retained intron 1. In some embodiments, the ASO targets an intron 1 sequence about 16 to about 345 nucleotides upstream (or 5') from the 3' splice site of a *PRRT2* RIC pre-mRNA comprising the retained intron 1.

In some embodiments, the ASO targets exon 1 or exon 2 of a *PRRT2* RIC pre-mRNA comprising a retained intron 1, wherein the intron numbering correspond to the mRNA sequence at NM_001256442. In some embodiments, the ASO targets an exon 1 sequence upstream (or 5') from the 5' splice site of a *PRRT2* RIC pre-mRNA comprising the retained intron 1. In some embodiments, the ASO targets an exon 1 sequence about 4 to about 219 nucleotides upstream (or 5') from the 5' splice site of a *PRRT2* RIC pre-mRNA comprising the retained intron 1. In some embodiments, the ASO targets an exon 2 sequence downstream (or 3') from the 3' splice site of a *PRRT2* RIC pre-mRNA comprising the retained intron 1. In some embodiments, the ASO targets an exon 2 sequence about 2 to about 924 nucleotides downstream (or 3') from the 3' splice site of a *PRRT2* RIC pre-mRNA comprising the retained intron 1.

In some embodiments, the ASO targets intron 1 in a *PRRT2* RIC pre-mRNA comprising a retained intron 1, wherein the intron numbering correspond to the mRNA sequence at NM_001256442. In some embodiments, the ASO targets an intron 1 sequence downstream (or 3') from the 5' splice site of a *PRRT2* RIC pre-mRNA comprising the retained intron 1. In some embodiments, the ASO targets an intron 1 sequence about 6 to about 346 nucleotides downstream (or 3') from the 5' splice site of a *PRRT2* RIC pre-mRNA comprising the retained intron 1. In some embodiments, the ASO targets an intron 1 sequence upstream (or 5') from the 3' splice site of a *PRRT2* RIC pre-mRNA comprising the retained intron 1. In some embodiments, the ASO targets an intron 1 sequence about 16 to about 345 or about 26 to about 345 nucleotides upstream (or 5') from the 3' splice site of a *PRRT2* RIC pre-mRNA comprising the retained intron 1.

In some embodiments, the ASOs disclosed herein target a RIC pre-mRNA transcribed from a *STX1B* genomic sequence. In some embodiments, the ASO targets a RIC pre-mRNA transcript from a *STX1B* genomic sequence comprising a retained intron. In some embodiments, the ASO targets a RIC pre-mRNA transcript of SEQ ID NO: 11. In some embodiments, the ASO targets a RIC pre-mRNA transcript of SEQ ID NO: 11 comprising a retained intron. In some embodiments, the ASOs disclosed herein target a *STX1B* RIC pre-mRNA sequence. In some embodiments, the ASO targets a *STX1B* RIC pre-mRNA transcript comprising a retained intron at 6, 7 or a combination thereof, wherein the intron numbering correspond to the mRNA sequence at NM_052874.

In some embodiments, the ASO targets a *STX1B* RIC pre-mRNA sequence according to SEQ ID NO: 40. In some embodiments, the ASO targets a *STX1B* RIC pre-mRNA sequence according to SEQ ID NO: 40 comprising a retained intron 6, a retained intron 7, or a combination thereof. In some embodiments, the ASOs disclosed herein target SEQ ID NOs: 24385 or 24375. In some embodiments, the ASO has a sequence according to any one of SEQ ID NOs: 9333-9600.

In some embodiments, the ASO targets exon 6 or exon 7 of a *STX1B* RIC pre-mRNA comprising a retained intron 6, wherein the intron numbering correspond to the mRNA sequence at NM_052874. In some embodiments, the ASO targets an exon 6 sequence upstream (or 5') from the 5' splice site of a *STX1B* RIC pre-mRNA comprising the retained intron 6. In some embodiments, the ASO targets an exon 6 sequence about 4 to about 89 nucleotides upstream (or 5') from the 5' splice site of a *STX1B* RIC pre-mRNA comprising the retained intron 6. In some embodiments, the ASO targets an exon 7 sequence downstream (or 3') from the 3' splice site of a *STX1B* RIC pre-mRNA comprising the retained intron 6. In some embodiments, the ASO targets an exon 7 sequence about 2 to about 57 nucleotides downstream (or 3') from the 3' splice site of a *STX1B* RIC pre-mRNA comprising the retained intron 6.

In some embodiments, the ASO targets intron 6 in a *STX1B* RIC pre-mRNA comprising a retained intron 6, wherein the intron numbering correspond to the mRNA sequence at NM_052874. In some embodiments, the ASO targets an intron 6 sequence downstream (or 3') from the 5' splice site of a *STX1B* RIC pre-mRNA comprising the retained intron 6. In some embodiments, the ASO targets an intron 6 sequence about 6 to about 99 nucleotides downstream (or 3') from the 5' splice site of a *STX1B* RIC pre-mRNA comprising the retained intron 6. In some embodiments, the ASO targets an intron 6 sequence upstream (or 5') from the 3' splice site of a *STX1B* RIC pre-mRNA comprising the retained intron 6. In some embodiments, the ASO targets an intron 6 sequence about 16 to about 94 nucleotides upstream (or 5') from the 3' splice site of a *STX1B* RIC pre-mRNA comprising the retained intron 6.

In some embodiments, the ASO targets exon 7 or exon 8 of a *STX1B* RIC pre-mRNA comprising a retained intron 7, wherein the intron numbering correspond to the mRNA sequence at NM_052874. In some embodiments, the ASO targets an exon 7 sequence upstream (or 5') from the 5' splice site of a *STX1B* RIC pre-mRNA comprising the retained intron 7. In some embodiments, the ASO targets an exon 7 sequence about 4 to about 54 nucleotides upstream (or 5') from the 5' splice site of a *STX1B* RIC pre-mRNA comprising the retained intron 7. In some embodiments, the ASO targets an exon 8 sequence downstream (or 3') from the 3' splice site of a *STX1B* RIC pre-mRNA comprising the retained intron 7. In some embodiments, the ASO targets an exon 8 sequence about 2 to about 117 nucleotides downstream (or 3') from the 3' splice site of a *STX1B* RIC pre-mRNA comprising the retained intron 7.

In some embodiments, the ASO targets intron 7 in a *STX1B* RIC pre-mRNA comprising a retained intron 7, wherein the intron numbering correspond to the mRNA sequence at NM_052874. In some embodiments, the ASO targets an intron 7 sequence downstream (or 3') from the 5' splice site of a *STX1B* RIC pre-mRNA comprising the retained intron 7. In some embodiments, the ASO targets an intron 7 sequence about 6 to about 500 nucleotides downstream (or 3') from the 5' splice site of a *STX1B* RIC pre-mRNA comprising the retained intron 7. In some embodiments, the ASO targets an intron 7 sequence upstream (or 5') from the 3' splice site of a *STX1B* RIC pre-mRNA comprising the retained intron 7. In some embodiments, the ASO targets an intron 7 sequence about 16 to about 496 nucleotides upstream (or 5') from the 3' splice site of a *STX1B* RIC pre-mRNA comprising the retained intron 7.

In some embodiments, the ASOs disclosed herein target a RIC pre-mRNA transcribed from a *RAI1* genomic sequence. In some embodiments, the ASO targets a RIC pre-mRNA transcript from a *RAIl* genomic sequence comprising a retained intron. In some embodiments, the ASO targets a RIC pre-mRNA transcript of SEQ ID NO: 12. In some embodiments, the ASO targets a RIC pre-mRNA transcript of SEQ ID NO: 12 comprising a retained intron. In some embodiments, the ASOs disclosed herein target a *RAI1* RIC pre-mRNA sequence. In some embodiments, the ASO targets a *RAI1* RIC pre-mRNA transcript comprising a retained intron at 4, wherein the intron numbering correspond to the mRNA sequence at NM_030665.

In some embodiments, the ASO targets a *RAI1* RIC pre-mRNA sequence according to SEQ ID NO: 41. In some embodiments, the ASO targets a *RAI1* RIC pre-mRNA sequence according to SEQ ID NO: 41 comprising a retained intron 4. In some embodiments, the ASOs disclosed herein target SEQ ID NO: 24382. In some embodiments, the ASO has a sequence according to any one of SEQ ID NOs: 9601-9803.

In some embodiments, the ASO targets exon 4 or exon 5 of a *RAI1* RIC pre-mRNA comprising a retained intron 4, wherein the intron numbering correspond to the mRNA sequence at NM_030665. In some embodiments, the ASO targets an exon 4 sequence upstream (or 5') from the 5' splice site of a *RAI1* RIC pre-mRNA comprising the retained intron 4. In some embodiments, the ASO targets an exon 4 sequence about 4 to about 74 nucleotides upstream (or 5') from the 5' splice site of a *RAI1* RIC pre-mRNA comprising the retained intron 4. In some embodiments, the ASO targets an exon 5 sequence downstream (or 3') from the 3' splice site of a *RAI1* RIC pre-mRNA comprising the retained intron 4. In some embodiments, the ASO targets an exon 5 sequence about 2 to about 32 nucleotides downstream (or 3') from the 3' splice site of a *RAI1* RIC pre-mRNA comprising the retained intron 4.

In some embodiments, the ASO targets intron 4 in a *RAI1* RIC pre-mRNA comprising a retained intron 4, wherein the intron numbering correspond to the mRNA sequence at NM_030665. In some embodiments, the ASO targets an intron 4 sequence downstream (or 3') from the 5' splice site of a *RAI1* RIC pre-mRNA comprising the retained intron 4. In some embodiments, the ASO targets an intron 4 sequence about 6 to about 500 nucleotides downstream (or 3') from the 5' splice site of a *RAI1* RIC pre-mRNA comprising the retained intron 4. In some embodiments, the ASO targets an intron 4 sequence upstream (or 5') from the 3' splice site of a *RAI1* RIC pre-mRNA comprising the retained intron 4. In some embodiments, the ASO targets an intron 4 sequence about 16 to about 499 nucleotides upstream (or 5') from the 3' splice site of a *RAI1* RIC pre-mRNA comprising the retained intron 4.

In some embodiments, the ASOs disclosed herein target a RIC pre-mRNA transcribed from a *TCF4* genomic sequence. In some embodiments, the ASO targets a RIC pre-mRNA transcript from a *TCF4* genomic sequence comprising a retained intron. In some embodiments, the ASO targets a RIC pre-mRNA transcript of SEQ ID NO: 13. In some embodiments, the ASO targets a RIC pre-mRNA transcript of SEQ ID NO: 13 comprising a retained intron. In some embodiments, the ASOs disclosed herein target a *TCF4* RIC pre-mRNA sequence. In some embodiments, the ASO targets a *TCF4* RIC pre-mRNA transcript comprising a retained intron at 10, wherein the intron numbering correspond to the mRNA sequence at NM_001243236. In some embodiments, the ASO targets a *TCF4* RIC pre-mRNA transcript comprising a retained intron at 10, wherein the intron numbering correspond to the mRNA sequence at NM_001243235. In some embodiments, the ASO targets a *TCF4* RIC pre-mRNA transcript comprising a retained intron at 10, wherein the intron numbering correspond to the mRNA sequence at NM_001243234. In some embodiments, the ASO targets a *TCF4* RIC pre-mRNA transcript comprising a retained intron at 13, wherein the intron numbering correspond to the mRNA sequence at NM_001243233. In some embodiments, the ASO targets a *TCF4* RIC pre-mRNA transcript comprising a retained intron at 13, wherein the intron numbering correspond to the mRNA sequence at NM_001243232. In some embodiments, the ASO targets a *TCF4* RIC pre-mRNA transcript comprising a retained intron at 15, wherein the intron numbering correspond to the mRNA sequence at NM_001243231. In some embodiments, the ASO targets a *TCF4* RIC pre-mRNA transcript comprising a retained intron at 17, wherein the intron numbering correspond to the mRNA sequence at NM_003199. In some embodiments, the ASO targets a *TCF4* RIC pre-mRNA transcript comprising a retained intron at 16, wherein the intron numbering correspond to the mRNA sequence at NM_001306207. In some embodiments, the ASO targets a *TCF4* RIC pre-mRNA transcript comprising a retained intron at 13, wherein the intron numbering correspond to the mRNA sequence at NM_001306208. In some embodiments, the ASO targets a *TCF4* RIC pre-mRNA transcript comprising a retained intron at 16, wherein the intron numbering correspond to the mRNA sequence at NM_001243227. In some embodiments, the ASO targets a *TCF4* RIC pre-mRNA transcript comprising a retained intron at 17, wherein the intron numbering correspond to the mRNA sequence at NM_001243228. In some embodiments, the ASO targets a *TCF4* RIC pre-mRNA transcript comprising a retained intron at 16, wherein the intron numbering correspond to the mRNA sequence at NM_001243230. In some embodiments, the ASO targets a *TCF4* RIC pre-mRNA transcript comprising a retained intron at 18, wherein the intron numbering correspond to the mRNA sequence at NM_001243226. In some embodiments, the ASO targets a *TCF4* RIC pre-mRNA transcript comprising a retained intron at 17, wherein the intron numbering correspond to the mRNA sequence at NM_001083962. In some embodiments, the ASO targets a *TCF4* RIC pre-mRNA transcript comprising a retained intron at 12, wherein the intron numbering correspond to the mRNA sequence at NM_001330605. In some embodiments, the ASO targets a *TCF4* RIC pre-mRNA transcript comprising a retained intron at 17, wherein the intron numbering correspond to the mRNA sequence at NM_001330604.

In some embodiments, the ASO targets a *TCF4* RIC pre-mRNA sequence according to SEQ ID NO: 42. In some embodiments, the ASO targets a *TCF4* RIC pre-mRNA sequence according to SEQ ID NO: 42 comprising a retained intron 10. In some embodiments, the ASO targets a *TCF4* RIC pre-mRNA sequence according to SEQ ID NO: 43. In some embodiments, the ASO targets a *TCF4* RIC pre-mRNA sequence according to SEQ ID NO: 43 comprising a retained intron 10. In some embodiments, the ASO targets a *TCF4* RIC pre-mRNA sequence according to SEQ ID NO: 44. In some embodiments, the ASO targets a *TCF4* RIC pre-mRNA sequence according to SEQ ID NO: 44 comprising a retained intron 10. In some embodiments, the ASO targets a *TCF4* RIC pre-mRNA sequence according to SEQ ID NO: 45. In some embodiments, the ASO targets a *TCF4* RIC pre-mRNA sequence according to SEQ ID NO: 45 comprising a retained intron 13. In some embodiments, the ASO targets a *TCF4* RIC pre-mRNA sequence according to SEQ ID NO: 46. In some embodiments, the ASO targets a *TCF4* RIC pre-mRNA sequence according to SEQ ID NO: 46 comprising a retained intron 13. In some embodiments, the ASO targets a *TCF4* RIC pre-mRNA sequence according to SEQ ID NO: 47. In some embodiments, the ASO targets a *TCF4* RIC pre-mRNA sequence according to SEQ ID NO: 47 comprising a retained intron 15. In some embodiments, the ASO targets a *TCF4* RIC pre-mRNA sequence according to SEQ ID NO: 48. In some embodiments, the ASO targets a *TCF4* RIC pre-mRNA sequence according to SEQ ID NO: 48 comprising a retained intron 17. In some embodiments, the ASO targets a *TCF4* RIC pre-mRNA sequence according to SEQ ID NO: 49. In some embodiments, the ASO targets a *TCF4* RIC pre-mRNA sequence according to SEQ ID NO: 49 comprising a retained intron 16. In some embodiments, the ASO targets a *TCF4* RIC pre-mRNA sequence according to SEQ ID NO: 50. In some embodiments, the ASO targets a *TCF4* RIC pre-mRNA sequence according to SEQ ID NO: 50 comprising a retained intron 13. In some embodiments, the ASO targets a *TCF4* RIC pre-mRNA sequence according to SEQ ID NO: 51. In some embodiments, the ASO targets a *TCF4* RIC pre-mRNA sequence according to SEQ ID NO: 51 comprising a retained intron 16. In some embodiments, the ASO targets a *TCF4* RIC pre-mRNA sequence according to SEQ ID NO: 52. In some embodiments, the ASO targets a *TCF4* RIC pre-mRNA sequence according to SEQ ID NO: 52 comprising a retained intron 17. In some embodiments, the ASO targets a *TCF4* RIC pre-mRNA sequence according to SEQ ID NO: 53. In some embodiments, the ASO targets a *TCF4* RIC pre-mRNA sequence according to SEQ ID NO: 53 comprising a retained intron 16. In some embodiments, the ASO targets a *TCF4* RIC pre-mRNA sequence according to SEQ ID NO: 54. In some embodiments, the ASO targets a *TCF4* RIC pre-mRNA sequence according to SEQ ID NO: 54 comprising a retained intron 18. In some embodiments, the ASO targets a *TCF4* RIC pre-mRNA sequence according to SEQ ID NO: 55. In some embodiments, the ASO targets a *TCF4* RIC pre-mRNA sequence according to SEQ ID NO: 55 comprising a retained intron 17. In some embodiments, the ASO targets a *TCF4* RIC pre-mRNA sequence according to SEQ ID NO: 56. In some embodiments, the ASO targets a *TCF4* RIC pre-mRNA sequence according to SEQ ID NO: 56 comprising a retained intron 12. In some embodiments, the ASO targets a *TCF4* RIC pre-mRNA sequence according to SEQ ID NO: 57. In some embodiments, the ASO targets a *TCF4* RIC pre-mRNA sequence according to SEQ ID NO: 57 comprising a retained intron 17. In some embodiments, the ASOs disclosed herein target SEQ ID NOs: 28515 or 28516. In some embodiments, the ASO has a sequence according to any one of SEQ ID NOs: 24387-24643.

In some embodiments, the ASO targets exon 10 or exon 11 of a *TCF4* RIC pre-mRNA comprising a retained intron 10, wherein the intron numbering correspond to the mRNA sequence at NM_001243236. In some embodiments, the ASO targets an exon 10 sequence upstream (or 5') from the 5' splice site of a *TCF4* RIC pre-mRNA comprising the retained intron 10. In some embodiments, the ASO targets an exon 10 sequence about 4 to about 134 nucleotides upstream (or 5') from the 5' splice site of a *TCF4* RIC pre-mRNA comprising the retained intron 10. In some embodiments, the ASO targets an exon 11 sequence downstream (or 3') from the 3' splice site of a *TCF4* RIC pre-mRNA comprising the retained intron 10. In some embodiments, the ASO targets an exon 11 sequence about 2 to about 212 nucleotides downstream (or 3') from the 3' splice site of a *TCF4* RIC pre-mRNA comprising the retained intron 10.

In some embodiments, the ASO targets intron 10 in a *TCF4* RIC pre-mRNA comprising a retained intron 10, wherein the intron numbering correspond to the mRNA sequence at NM_001243236. In some embodiments, the ASO targets an intron 10 sequence downstream (or 3') from the 5' splice site of a *TCF4* RIC pre-mRNA comprising the retained intron 10. In some embodiments, the ASO targets an intron 10 sequence about 6 to about 496 nucleotides downstream (or 3') from the 5' splice site of a *TCF4* RIC pre-mRNA comprising the retained intron 10. In some embodiments, the ASO targets an intron 10 sequence upstream (or 5') from the 3' splice site of a *TCF4* RIC pre-mRNA comprising the retained intron 10. In some embodiments, the ASO targets an intron 10 sequence about 16 to about 496 nucleotides upstream (or 5') from the 3' splice site of a *TCF4* RIC pre-mRNA comprising the retained intron 10.

In some embodiments, the ASO targets exon 10 or exon 11 of a *TCF4* RIC pre-mRNA comprising a retained intron 10, wherein the intron numbering correspond to the mRNA sequence at NM_001243235. In some embodiments, the ASO targets an exon 10 sequence upstream (or 5') from the 5' splice site of a *TCF4* RIC pre-mRNA comprising the retained intron 10. In some embodiments, the ASO targets an exon 10 sequence about 4 to about 134 nucleotides upstream (or 5') from the 5' splice site of a *TCF4* RIC pre-mRNA comprising the retained intron 10. In some embodiments, the ASO targets an exon 11 sequence downstream (or 3') from the 3' splice site of a *TCF4* RIC pre-mRNA comprising the retained intron 10. In some embodiments, the ASO targets an exon 11 sequence about 2 to about 212 nucleotides downstream (or 3') from the 3' splice site of a *TCF4* RIC pre-mRNA comprising the retained intron 10.

In some embodiments, the ASO targets intron 10 in a *TCF4* RIC pre-mRNA comprising a retained intron 10, wherein the intron numbering correspond to the mRNA sequence at NM_001243235. In some embodiments, the ASO targets an intron 10 sequence downstream (or 3') from the 5' splice site of a *TCF4* RIC pre-mRNA comprising the retained intron 10. In some embodiments, the ASO targets an intron 10 sequence about 6 to about 496 nucleotides downstream (or 3') from the 5' splice site of a *TCF4* RIC pre-mRNA comprising the retained intron 10. In some embodiments, the ASO targets an intron 10 sequence upstream (or 5') from the 3' splice site of a *TCF4* RIC pre-mRNA comprising the retained intron 10. In some embodiments, the ASO targets an intron 10 sequence about 16 to about 496 nucleotides upstream (or 5') from the 3' splice site of a *TCF4* RIC pre-mRNA comprising the retained intron 10.

In some embodiments, the ASO targets exon 10 or exon 11 of a *TCF4* RIC pre-mRNA comprising a retained intron 10, wherein the intron numbering correspond to the mRNA sequence at NM_001243234. In some embodiments, the ASO targets an exon 10 sequence upstream (or 5') from the 5' splice site of a *TCF4* RIC pre-mRNA comprising the retained intron 10. In some embodiments, the ASO targets an exon 10 sequence about 4 to about 144 nucleotides upstream (or 5') from the 5' splice site of a *TCF4* RIC pre-mRNA comprising the retained intron 10. In some embodiments, the ASO targets an exon 11 sequence downstream (or 3') from the 3' splice site of a *TCF4* RIC pre-mRNA comprising the retained intron 10. In some embodiments, the ASO targets an exon 11 sequence about 2 to about 212 nucleotides downstream (or 3') from the 3' splice site of a *TCF4* RIC pre-mRNA comprising the retained intron 10.

In some embodiments, the ASO targets intron 10 in a *TCF4* RIC pre-mRNA comprising a retained intron 10, wherein the intron numbering correspond to the mRNA sequence at NM_001243234. In some embodiments, the ASO targets an intron 10 sequence downstream (or 3') from the 5' splice site of a *TCF4* RIC pre-mRNA comprising the retained intron 10. In some embodiments, the ASO targets an intron 10 sequence about 6 to about 496 nucleotides downstream (or 3') from the 5' splice site of a *TCF4* RIC pre-mRNA comprising the retained intron 10. In some embodiments, the ASO targets an intron 10 sequence upstream (or 5') from the 3' splice site of a *TCF4* RIC pre-mRNA comprising the retained intron 10. In some embodiments, the ASO targets an intron 10 sequence about 16 to about 496 nucleotides upstream (or 5') from the 3' splice site of a *TCF4* RIC pre-mRNA comprising the retained intron 10.

In some embodiments, the ASO targets exon 13 or exon 14 of a *TCF4* RIC pre-mRNA comprising a retained intron 13, wherein the intron numbering correspond to the mRNA sequence at NM_001243233. In some embodiments, the ASO targets an exon 13 sequence upstream (or 5') from the 5' splice site of a *TCF4* RIC pre-mRNA comprising the retained intron 13. In some embodiments, the ASO targets an exon 13 sequence about 4 to about 134 nucleotides upstream (or 5') from the 5' splice site of a *TCF4* RIC pre-mRNA comprising the retained intron 13. In some embodiments, the ASO targets an exon 14 sequence downstream (or 3') from the 3' splice site of a *TCF4* RIC pre-mRNA comprising the retained intron 13. In some embodiments, the ASO targets an exon 14 sequence about 2 to about 212 nucleotides downstream (or 3') from the 3' splice site of a *TCF4* RIC pre-mRNA comprising the retained intron 13.

In some embodiments, the ASO targets intron 13 in a *TCF4* RIC pre-mRNA comprising a retained intron 13, wherein the intron numbering correspond to the mRNA sequence at NM_001243233. In some embodiments, the ASO targets an intron 13 sequence downstream (or 3') from the 5' splice site of a *TCF4* RIC pre-mRNA comprising the retained intron 13. In some embodiments, the ASO targets an intron 13 sequence about 6 to about 496 nucleotides downstream (or 3') from the 5' splice site of a *TCF4* RIC pre-mRNA comprising the retained intron 13. In some embodiments, the ASO targets an intron 13 sequence upstream (or 5') from the 3' splice site of a *TCF4* RIC pre-mRNA comprising the retained intron 13. In some embodiments, the ASO targets an intron 13 sequence about 16 to about 496 nucleotides upstream (or 5') from the 3' splice site of a *TCF4* RIC pre-mRNA comprising the retained intron 13.

In some embodiments, the ASO targets exon 13 or exon 14 of a *TCF4* RIC pre-mRNA comprising a retained intron 13, wherein the intron numbering correspond to the mRNA sequence at NM_001243232. In some embodiments, the ASO targets an exon 13 sequence upstream (or 5') from the 5' splice site of a *TCF4* RIC pre-mRNA comprising the retained intron 13. In some embodiments, the ASO targets an exon 13 sequence about 4 to about 144 nucleotides upstream (or 5') from the 5' splice site of a *TCF4* RIC pre-mRNA comprising the retained intron 13. In some embodiments, the ASO targets an exon 14 sequence downstream (or 3') from the 3' splice site of a *TCF4* RIC pre-mRNA comprising the retained intron 13. In some embodiments, the ASO targets an exon 14 sequence about 2 to about 212 nucleotides downstream (or 3') from the 3' splice site of a *TCF4* RIC pre-mRNA comprising the retained intron 13.

In some embodiments, the ASO targets intron 13 in a *TCF4* RIC pre-mRNA comprising a retained intron 13, wherein the intron numbering correspond to the mRNA sequence at NM_001243232. In some embodiments, the ASO targets an intron 13 sequence downstream (or 3') from the 5' splice site of a *TCF4* RIC pre-mRNA comprising the retained intron 13. In some embodiments, the ASO targets an intron 13 sequence about 6 to about 496 nucleotides downstream (or 3') from the 5' splice site of a *TCF4* RIC pre-mRNA comprising the retained intron 13. In some embodiments, the ASO targets an intron 13 sequence upstream (or 5') from the 3' splice site of a *TCF4* RIC pre-mRNA comprising the retained intron 13. In some embodiments, the ASO targets an intron 13 sequence about 16 to about 496 nucleotides upstream (or 5') from the 3' splice site of a *TCF4* RIC pre-mRNA comprising the retained intron 13.

In some embodiments, the ASO targets exon 15 or exon 16 of a *TCF4* RIC pre-mRNA comprising a retained intron 15, wherein the intron numbering correspond to the mRNA sequence at NM_001243231. In some embodiments, the ASO targets an exon 15 sequence upstream (or 5') from the 5' splice site of a *TCF4* RIC pre-mRNA comprising the retained intron 15. In some embodiments, the ASO targets an exon 15 sequence about 4 to about 134 nucleotides upstream (or 5') from the 5' splice site of a *TCF4* RIC pre-mRNA comprising the retained intron 15. In some embodiments, the ASO targets an exon 16 sequence downstream (or 3') from the 3' splice site of a *TCF4* RIC pre-mRNA comprising the retained intron 15. In some embodiments, the ASO targets an exon 16 sequence about 2 to about 212 nucleotides downstream (or 3') from the 3' splice site of a *TCF4* RIC pre-mRNA comprising the retained intron 15.

In some embodiments, the ASO targets intron 15 in a *TCF4* RIC pre-mRNA comprising a retained intron 15, wherein the intron numbering correspond to the mRNA sequence at NM_001243231. In some embodiments, the ASO targets an intron 15 sequence downstream (or 3') from the 5' splice site of a *TCF4* RIC pre-mRNA comprising the retained intron 15. In some embodiments, the ASO targets an intron 15 sequence about 6 to about 496 nucleotides downstream (or 3') from the 5' splice site of a *TCF4* RIC pre-mRNA comprising the retained intron 15. In some embodiments, the ASO targets an intron 15 sequence upstream (or 5') from the 3' splice site of a *TCF4* RIC pre-mRNA comprising the retained intron 15. In some embodiments, the ASO targets an intron 15 sequence about 16 to about 496 nucleotides upstream (or 5') from the 3' splice site of a *TCF4* RIC pre-mRNA comprising the retained intron 15.

In some embodiments, the ASO targets exon 17 or exon 18 of a *TCF4* RIC pre-mRNA comprising a retained intron 17, wherein the intron numbering correspond to the mRNA sequence at NM_003199. In some embodiments, the ASO targets an exon 17 sequence upstream (or 5') from the 5' splice site of a *TCF4* RIC pre-mRNA comprising the retained intron 17. In some embodiments, the ASO targets an exon 17 sequence about 4 to about 134 nucleotides upstream (or 5') from the 5' splice site of a *TCF4* RIC pre-mRNA comprising the retained intron 17. In some embodiments, the ASO targets an exon 18 sequence downstream (or 3') from the 3' splice site of a *TCF4* RIC pre-mRNA comprising the retained intron 17. In some embodiments, the ASO targets an exon 18 sequence about 2 to about 212 nucleotides downstream (or 3') from the 3' splice site of a *TCF4* RIC pre-mRNA comprising the retained intron 17.

In some embodiments, the ASO targets intron 17 in a *TCF4* RIC pre-mRNA comprising a retained intron 17, wherein the intron numbering correspond to the mRNA sequence at NM_003199. In some embodiments, the ASO targets an intron 17 sequence downstream (or 3') from the 5' splice site of a *TCF4* RIC pre-mRNA comprising the retained intron 17. In some embodiments, the ASO targets an intron 17 sequence about 6 to about 496 nucleotides downstream (or 3') from the 5' splice site of a *TCF4* RIC pre-mRNA comprising the retained intron 17. In some embodiments, the ASO targets an intron 17 sequence upstream (or 5') from the 3' splice site of a *TCF4* RIC pre-mRNA comprising the retained intron 17. In some embodiments, the ASO targets an intron 17 sequence about 16 to about 496 nucleotides upstream (or 5') from the 3' splice site of a *TCF4* RIC pre-mRNA comprising the retained intron 17.

In some embodiments, the ASO targets exon 16 or exon 17 of a *TCF4* RIC pre-mRNA comprising a retained intron 16, wherein the intron numbering correspond to the mRNA sequence at NM_001306207. In some embodiments, the ASO targets an exon 16 sequence upstream (or 5') from the 5' splice site of a *TCF4* RIC pre-mRNA comprising the retained intron 16. In some embodiments, the ASO targets an exon 16 sequence about 4 to about 134 nucleotides upstream (or 5') from the 5' splice site of a *TCF4* RIC pre-mRNA comprising the retained intron 16. In some embodiments, the ASO targets an exon 17 sequence downstream (or 3') from the 3' splice site of a *TCF4* RIC pre-mRNA comprising the retained intron 16. In some embodiments, the ASO targets an exon 17 sequence about 2 to about 212 nucleotides downstream (or 3') from the 3' splice site of a *TCF4* RIC pre-mRNA comprising the retained intron 16.

In some embodiments, the ASO targets intron 16 in a *TCF4* RIC pre-mRNA comprising a retained intron 16, wherein the intron numbering correspond to the mRNA sequence at NM_001306207. In some embodiments, the ASO targets an intron 16 sequence downstream (or 3') from the 5' splice site of a *TCF4* RIC pre-mRNA comprising the retained intron 16. In some embodiments, the ASO targets an intron 16 sequence about 6 to about 496 nucleotides downstream (or 3') from the 5' splice site of a *TCF4* RIC pre-mRNA comprising the retained intron 16. In some embodiments, the ASO targets an intron 16 sequence upstream (or 5') from the 3' splice site of a *TCF4* RIC pre-mRNA comprising the retained intron 16. In some embodiments, the ASO targets an intron 16 sequence about 16 to about 496 nucleotides upstream (or 5') from the 3' splice site of a *TCF4* RIC pre-mRNA comprising the retained intron 16.

In some embodiments, the ASO targets exon 13 or exon 14 of a *TCF4* RIC pre-mRNA comprising a retained intron 13, wherein the intron numbering correspond to the mRNA sequence at NM_001306208. In some embodiments, the ASO targets an exon 13 sequence upstream (or 5') from the 5' splice site of a *TCF4* RIC pre-mRNA comprising the retained intron 13. In some embodiments, the ASO targets an exon 13 sequence about 4 to about 134 nucleotides upstream (or 5') from the 5' splice site of a *TCF4* RIC pre-mRNA comprising the retained intron 13. In some embodiments, the ASO targets an exon 14 sequence downstream (or 3') from the 3' splice site of a *TCF4* RIC pre-mRNA comprising the retained intron 13. In some embodiments, the ASO targets an exon 14 sequence about 2 to about 212 nucleotides downstream (or 3') from the 3' splice site of a *TCF4* RIC pre-mRNA comprising the retained intron 13.

In some embodiments, the ASO targets intron 13 in a *TCF4* RIC pre-mRNA comprising a retained intron 13, wherein the intron numbering correspond to the mRNA sequence at NM_001306208. In some embodiments, the ASO targets an intron 13 sequence downstream (or 3') from the 5' splice site of a *TCF4* RIC pre-mRNA comprising the retained intron 13. In some embodiments, the ASO targets an intron 13 sequence about 6 to about 496 nucleotides downstream (or 3') from the 5' splice site of a *TCF4* RIC pre-mRNA comprising the retained intron 13. In some embodiments, the ASO targets an intron 13 sequence upstream (or 5') from the 3' splice site of a *TCF4* RIC pre-mRNA comprising the retained intron 13. In some embodiments, the ASO targets an intron 13 sequence about 16 to about 496 nucleotides upstream (or 5') from the 3' splice site of a *TCF4* RIC pre-mRNA comprising the retained intron 13.

In some embodiments, the ASO targets exon 16 or exon 17 of a *TCF4* RIC pre-mRNA comprising a retained intron 16, wherein the intron numbering correspond to the mRNA sequence at NM_001243227. In some embodiments, the ASO targets an exon 16 sequence upstream (or 5') from the 5' splice site of a *TCF4* RIC pre-mRNA comprising the retained intron 16. In some embodiments, the ASO targets an exon 16 sequence about 4 to about 144 nucleotides upstream (or 5') from the 5' splice site of a *TCF4* RIC pre-mRNA comprising the retained intron 16. In some embodiments, the ASO targets an exon 17 sequence downstream (or 3') from the 3' splice site of a *TCF4* RIC pre-mRNA comprising the retained intron 16. In some embodiments, the ASO targets an exon 17 sequence about 2 to about 212 nucleotides downstream (or 3') from the 3' splice site of a *TCF4* RIC pre-mRNA comprising the retained intron 16.

In some embodiments, the ASO targets intron 16 in a *TCF4* RIC pre-mRNA comprising a retained intron 16, wherein the intron numbering correspond to the mRNA sequence at NM_001243227. In some embodiments, the ASO targets an intron 16 sequence downstream (or 3') from the 5' splice site of a *TCF4* RIC pre-mRNA comprising the retained intron 16. In some embodiments, the ASO targets an intron 16 sequence about 6 to about 496 nucleotides downstream (or 3') from the 5' splice site of a *TCF4* RIC pre-mRNA comprising the retained intron 16. In some embodiments, the ASO targets an intron 16 sequence upstream (or 5') from the 3' splice site of a *TCF4* RIC pre-mRNA comprising the retained intron 16. In some embodiments, the ASO targets an intron 16 sequence about 16 to about 496 nucleotides upstream (or 5') from the 3' splice site of a *TCF4* RIC pre-mRNA comprising the retained intron 16.

In some embodiments, the ASO targets exon 17 or exon 18 of a *TCF4* RIC pre-mRNA comprising a retained intron 17, wherein the intron numbering correspond to the mRNA sequence at NM_001243228. In some embodiments, the ASO targets an exon 17 sequence upstream (or 5') from the 5' splice site of a *TCF4* RIC pre-mRNA comprising the retained intron 17. In some embodiments, the ASO targets an exon 17 sequence about 4 to about 144 nucleotides upstream (or 5') from the 5' splice site of a *TCF4* RIC pre-mRNA comprising the retained intron 17. In some embodiments, the ASO targets an exon 18 sequence downstream (or 3') from the 3' splice site of a *TCF4* RIC pre-mRNA comprising the retained intron 17. In some embodiments, the ASO targets an exon 18 sequence about 2 to about 212 nucleotides downstream (or 3') from the 3' splice site of a *TCF4* RIC pre-mRNA comprising the retained intron 17.

In some embodiments, the ASO targets intron 17 in a *TCF4* RIC pre-mRNA comprising a retained intron 17, wherein the intron numbering correspond to the mRNA sequence at NM_001243228. In some embodiments, the ASO targets an intron 17 sequence downstream (or 3') from the 5' splice site of a *TCF4* RIC pre-mRNA comprising the retained intron 17. In some embodiments, the ASO targets an intron 17 sequence about 6 to about 496 nucleotides downstream (or 3') from the 5' splice site of a *TCF4* RIC pre-mRNA comprising the retained intron 17. In some embodiments, the ASO targets an intron 17 sequence upstream (or 5') from the 3' splice site of a *TCF4* RIC pre-mRNA comprising the retained intron 17. In some embodiments, the ASO targets an intron 17 sequence about 16 to about 496 nucleotides upstream (or 5') from the 3' splice site of a *TCF4* RIC pre-mRNA comprising the retained intron 17.

In some embodiments, the ASO targets exon 16 or exon 17 of a *TCF4* RIC pre-mRNA comprising a retained intron 16, wherein the intron numbering correspond to the mRNA sequence at NM_001243230. In some embodiments, the ASO targets an exon 16 sequence upstream (or 5') from the 5' splice site of a *TCF4* RIC pre-mRNA comprising the retained intron 16. In some embodiments, the ASO targets an exon 16 sequence about 4 to about 134 nucleotides upstream (or 5') from the 5' splice site of a *TCF4* RIC pre-mRNA comprising the retained intron 16. In some embodiments, the ASO targets an exon 17 sequence downstream (or 3') from the 3' splice site of a *TCF4* RIC pre-mRNA comprising the retained intron 16. In some embodiments, the ASO targets an exon 17 sequence about 2 to about 212 nucleotides downstream (or 3') from the 3' splice site of a *TCF4* RIC pre-mRNA comprising the retained intron 16.

In some embodiments, the ASO targets intron 16 in a *TCF4* RIC pre-mRNA comprising a retained intron 16, wherein the intron numbering correspond to the mRNA sequence at NM_001243230. In some embodiments, the ASO targets an intron 16 sequence downstream (or 3') from the 5' splice site of a *TCF4* RIC pre-mRNA comprising the retained intron 16. In some embodiments, the ASO targets an intron 16 sequence about 6 to about 496 nucleotides downstream (or 3') from the 5' splice site of a *TCF4* RIC pre-mRNA comprising the retained intron 16. In some embodiments, the ASO targets an intron 16 sequence upstream (or 5') from the 3' splice site of a *TCF4* RIC pre-mRNA comprising the retained intron 16. In some embodiments, the ASO targets an intron 16 sequence about 16 to about 496 nucleotides upstream (or 5') from the 3' splice site of a *TCF4* RIC pre-mRNA comprising the retained intron 16.

In some embodiments, the ASO targets exon 18 or exon 19 of a *TCF4* RIC pre-mRNA comprising a retained intron 18, wherein the intron numbering correspond to the mRNA sequence at NM_001243226. In some embodiments, the ASO targets an exon 18 sequence upstream (or 5') from the 5' splice site of a *TCF4* RIC pre-mRNA comprising the retained intron 18. In some embodiments, the ASO targets an exon 18 sequence about 4 to about 144 nucleotides upstream (or 5') from the 5' splice site of a *TCF4* RIC pre-mRNA comprising the retained intron 18. In some embodiments, the ASO targets an exon 19 sequence downstream (or 3') from the 3' splice site of a *TCF4* RIC pre-mRNA comprising the retained intron 18. In some embodiments, the ASO targets an exon 19 sequence about 2 to about 212 nucleotides downstream (or 3') from the 3' splice site of a *TCF4* RIC pre-mRNA comprising the retained intron 18.

In some embodiments, the ASO targets intron 18 in a *TCF4* RIC pre-mRNA comprising a retained intron 18, wherein the intron numbering correspond to the mRNA sequence at NM_001243226. In some embodiments, the ASO targets an intron 18 sequence downstream (or 3') from the 5' splice site of a *TCF4* RIC pre-mRNA comprising the retained intron 18. In some embodiments, the ASO targets an intron 18 sequence about 6 to about 496 nucleotides downstream (or 3') from the 5' splice site of a *TCF4* RIC pre-mRNA comprising the retained intron 18. In some embodiments, the ASO targets an intron 18 sequence upstream (or 5') from the 3' splice site of a *TCF4* RIC pre-mRNA comprising the retained intron 18. In some embodiments, the ASO targets an intron 18 sequence about 16 to about 496 nucleotides upstream (or 5') from the 3' splice site of a *TCF4* RIC pre-mRNA comprising the retained intron 18.

In some embodiments, the ASO targets exon 17 or exon 18 of a *TCF4* RIC pre-mRNA comprising a retained intron 17, wherein the intron numbering correspond to the mRNA sequence at NM_001083962. In some embodiments, the ASO targets an exon 17 sequence upstream (or 5') from the 5' splice site of a *TCF4* RIC pre-mRNA comprising the retained intron 17. In some embodiments, the ASO targets an exon 17 sequence about 4 to about 144 nucleotides upstream (or 5') from the 5' splice site of a *TCF4* RIC pre-mRNA comprising the retained intron 17. In some embodiments, the ASO targets an exon 18 sequence downstream (or 3') from the 3' splice site of a *TCF4* RIC pre-mRNA comprising the retained intron 17. In some embodiments, the ASO targets an exon 18 sequence about 2 to about 212 nucleotides downstream (or 3') from the 3' splice site of a *TCF4* RIC pre-mRNA comprising the retained intron 17.

In some embodiments, the ASO targets intron 17 in a *TCF4* RIC pre-mRNA comprising a retained intron 17, wherein the intron numbering correspond to the mRNA sequence at NM_001083962. In some embodiments, the ASO targets an intron 17 sequence downstream (or 3') from the 5' splice site of a *TCF4* RIC pre-mRNA comprising the retained intron 17. In some embodiments, the ASO targets an intron 17 sequence about 6 to about 496 nucleotides downstream (or 3') from the 5' splice site of a *TCF4* RIC pre-mRNA comprising the retained intron 17. In some embodiments, the ASO targets an intron 17 sequence upstream (or 5') from the 3' splice site of a *TCF4* RIC pre-mRNA comprising the retained intron 17. In some embodiments, the ASO targets an intron 17 sequence about 16 to about 496 nucleotides upstream (or 5') from the 3' splice site of a *TCF4* RIC pre-mRNA comprising the retained intron 17.

In some embodiments, the ASO targets exon 12 or exon 13 of a *TCF4* RIC pre-mRNA comprising a retained intron 12, wherein the intron numbering correspond to the mRNA sequence at NM_001330605. In some embodiments, the ASO targets an exon 12 sequence upstream (or 5') from the 5' splice site of a *TCF4* RIC pre-mRNA comprising the retained intron 12. In some embodiments, the ASO targets an exon 12 sequence about 4 to about 144 nucleotides upstream (or 5') from the 5' splice site of a *TCF4* RIC pre-mRNA comprising the retained intron 12. In some embodiments, the ASO targets an exon 13 sequence downstream (or 3') from the 3' splice site of a *TCF4* RIC pre-mRNA comprising the retained intron 12. In some embodiments, the ASO targets an exon 13 sequence about 2 to about 212 nucleotides downstream (or 3') from the 3' splice site of a *TCF4* RIC pre-mRNA comprising the retained intron 12.

In some embodiments, the ASO targets intron 12 in a *TCF4* RIC pre-mRNA comprising a retained intron 12, wherein the intron numbering correspond to the mRNA sequence at NM_001330605. In some embodiments, the ASO targets an intron 12 sequence downstream (or 3') from the 5' splice site of a *TCF4* RIC pre-mRNA comprising the retained intron 12. In some embodiments, the ASO targets an intron 12 sequence about 6 to about 496 nucleotides downstream (or 3') from the 5' splice site of a *TCF4* RIC pre-mRNA comprising the retained intron 12. In some embodiments, the ASO targets an intron 12 sequence upstream (or 5') from the 3' splice site of a *TCF4* RIC pre-mRNA comprising the retained intron 12. In some embodiments, the ASO targets an intron 12 sequence about 16 to about 496 nucleotides upstream (or 5') from the 3' splice site of a *TCF4* RIC pre-mRNA comprising the retained intron 12.

In some embodiments, the ASO targets exon 17 or exon 18 of a *TCF4* RIC pre-mRNA comprising a retained intron 17, wherein the intron numbering correspond to the mRNA sequence at NM_001330604. In some embodiments, the ASO targets an exon 17 sequence upstream (or 5') from the 5' splice site of a *TCF4* RIC pre-mRNA comprising the retained intron 17. In some embodiments, the ASO targets an exon 17 sequence about 4 to about 144 nucleotides upstream (or 5') from the 5' splice site of a *TCF4* RIC pre-mRNA comprising the retained intron 17. In some embodiments, the ASO targets an exon 18 sequence downstream (or 3') from the 3' splice site of a *TCF4* RIC pre-mRNA comprising the retained intron 17. In some embodiments, the ASO targets an exon 18 sequence about 2 to about 212 nucleotides downstream (or 3') from the 3' splice site of a *TCF4* RIC pre-mRNA comprising the retained intron 17.

In some embodiments, the ASO targets intron 17 in a *TCF4* RIC pre-mRNA comprising a retained intron 17, wherein the intron numbering correspond to the mRNA sequence at NM_001330604. In some embodiments, the ASO targets an intron 17 sequence downstream (or 3') from the 5' splice site of a *TCF4* RIC pre-mRNA comprising the retained intron 17. In some embodiments, the ASO targets an intron 17 sequence about 6 to about 496 nucleotides downstream (or 3') from the 5' splice site of a *TCF4* RIC pre-mRNA comprising the retained intron 17. In some embodiments, the ASO targets an intron 17 sequence upstream (or 5') from the 3' splice site of a *TCF4* RIC pre-mRNA comprising the retained intron 17. In some embodiments, the ASO targets an intron 17 sequence about 16 to about 496 nucleotides upstream (or 5') from the 3' splice site of a *TCF4* RIC pre-mRNA comprising the retained intron 17.

In some embodiments, the ASOs disclosed herein target a RIC pre-mRNA transcribed from a *NPC1* genomic sequence. In some embodiments, the ASO targets a RIC pre-mRNA transcript from a *NPC1* genomic sequence comprising a retained intron. In some embodiments, the ASO targets a RIC pre-mRNA transcript of SEQ ID NO: 14. In some embodiments, the ASO targets a RIC pre-mRNA transcript of SEQ ID NO: 14 comprising a retained intron. In some embodiments, the ASOs disclosed herein target a *NPC1* RIC pre-mRNA sequence. In some embodiments, the ASO targets a *NPC1* RIC pre-mRNA transcript comprising a retained intron at 15, wherein the intron numbering correspond to the mRNA sequence at NM_000271.

In some embodiments, the ASO targets a *NPC1* RIC pre-mRNA sequence according to SEQ ID NO: 58. In some embodiments, the ASO targets a *NPC1* RIC pre-mRNA sequence according to SEQ ID NO: 58 comprising a retained intron 15. In some embodiments, the ASOs disclosed herein target SEQ ID NO: 24382. In some embodiments, the ASO has a sequence according to any one of SEQ ID NOs: 13692-13760.

In some embodiments, the ASO targets exon 15 or exon 16 of a *NPC1* RIC pre-mRNA comprising a retained intron 15, wherein the intron numbering correspond to the mRNA sequence at NM_000271. In some embodiments, the ASO targets an exon 15 sequence upstream (or 5') from the 5' splice site of a *NPC1* RIC pre-mRNA comprising the retained intron 15. In some embodiments, the ASO targets an exon 15 sequence about 4 to about 109 nucleotides upstream (or 5') from the 5' splice site of a *NPC1* RIC pre-mRNA comprising the retained intron 15. In some embodiments, the ASO targets an exon 16 sequence downstream (or 3') from the 3' splice site of a *NPC1* RIC pre-mRNA comprising the retained intron 15. In some embodiments, the ASO targets an exon 16 sequence about 2 to about 122 nucleotides downstream (or 3') from the 3' splice site of a *NPC1* RIC pre-mRNA comprising the retained intron 15.

In some embodiments, the ASO targets intron 15 in a *NPC1* RIC pre-mRNA comprising a retained intron 15, wherein the intron numbering correspond to the mRNA sequence at NM_000271. In some embodiments, the ASO targets an intron 15 sequence downstream (or 3') from the 5' splice site of a *NPC1* RIC pre-mRNA comprising the retained intron 15. In some embodiments, the ASO targets an intron 15 sequence about 6 to about 61 nucleotides downstream (or 3') from the 5' splice site of a *NPC1* RIC pre-mRNA comprising the retained intron 15. In some embodiments, the ASO targets an intron 15 sequence upstream (or 5') from the 3' splice site of a *NPC1* RIC pre-mRNA comprising the retained intron 15. In some embodiments, the ASO targets an intron 15 sequence about 16 to about 61 nucleotides upstream (or 5') from the 3' splice site of a *NPC1* RIC pre-mRNA comprising the retained intron 15.

In some embodiments, the ASOs disclosed herein target a RIC pre-mRNA transcribed from a *CACNA1A* genomic sequence. In some embodiments, the ASO targets a RIC pre-mRNA transcript from a *CACNA1A* genomic sequence comprising a retained intron. In some embodiments, the ASO targets a RIC pre-mRNA transcript of SEQ ID NO: 15. In some embodiments, the ASO targets a RIC pre-mRNA transcript of SEQ ID NO: 15 comprising a retained intron. In some embodiments, the ASOs disclosed herein target a *CACNA1A* RIC pre-mRNA sequence. In some embodiments, the ASO targets a *CACNA1A* RIC pre-mRNA transcript comprising a retained intron at 36, 37 or a combination thereof, wherein the intron numbering correspond to the mRNA sequence at NM_000068. In some embodiments, the ASO targets a *CACNA1A* RIC pre-mRNA transcript comprising a retained intron at 36, 37 or a combination thereof, wherein the intron numbering correspond to the mRNA sequence at NM_023035. In some embodiments, the ASO targets a *CACNA1A* RIC pre-mRNA transcript comprising a retained intron at 36, 37 or a combination thereof, wherein the intron numbering correspond to the mRNA sequence at NM_001174080. In some embodiments, the ASO targets a *CACNA1A* RIC pre-mRNA transcript comprising a retained intron at 36, 37 or a combination thereof, wherein the intron numbering correspond to the mRNA sequence at NM_001127222. In some embodiments, the ASO targets a *CACNA1A* RIC pre-mRNA transcript comprising a retained intron at 36, 37 or a combination thereof, wherein the intron numbering correspond to the mRNA sequence at NM_001127221.

In some embodiments, the ASO targets a *CACNA1A* RIC pre-mRNA sequence according to SEQ ID NO: 59. In some embodiments, the ASO targets a *CACNA1A* RIC pre-mRNA sequence according to SEQ ID NO: 59 comprising a retained intron 36, a retained intron 37, or a combination thereof. In some embodiments, the ASO targets a *CACNA1A* RIC pre-mRNA sequence according to SEQ ID NO: 60. In some embodiments, the ASO targets a *CACNA1A* RIC pre-mRNA sequence according to SEQ ID NO: 69 comprising a retained intron 36, a retained intron 37, or a combination thereof. In some embodiments, the ASO targets a *CACNA1A* RIC pre-mRNA sequence according to SEQ ID NO: 61. In some embodiments, the ASO targets a *CACNA1A* RIC pre-mRNA sequence according to SEQ ID NO: 61 comprising a retained intron 36, a retained intron 37, or a combination thereof. In some embodiments, the ASO targets a *CACNA1A* RIC pre-mRNA sequence according to SEQ ID NO: 62. In some embodiments, the ASO targets a *CACNA1A* RIC pre-mRNA sequence according to SEQ ID NO: 62 comprising a retained intron 36, a retained intron 37, or a combination thereof. In some embodiments, the ASO targets a *CACNA1A* RIC pre-mRNA sequence according to SEQ ID NO: 63. In some embodiments, the ASO targets a *CACNA1A* RIC pre-mRNA sequence according to SEQ ID NO: 63 comprising a retained intron 36, a retained intron 37, or a combination thereof. In some embodiments, the ASOs disclosed herein target SEQ ID NOs: 24366, 24374, 24377, or 24384. In some embodiments, the ASO has a sequence according to any one of SEQ ID NOs: 13761-15914.

In some embodiments, the ASO targets exon 36 or exon 37 of a *CACNA1A* RIC pre-mRNA comprising a retained intron 36, wherein the intron numbering correspond to the mRNA sequence at NM_000068. In some embodiments, the ASO targets an exon 36 sequence upstream (or 5') from the 5' splice site of a *CACNA1A* RIC pre-mRNA comprising the retained intron 36. In some embodiments, the ASO targets an exon 36 sequence about 4 to about 110 nucleotides upstream (or 5') from the 5' splice site of a *CACNA1A* RIC pre-mRNA comprising the retained intron 36. In some embodiments, the ASO targets an exon 37 sequence downstream (or 3') from the 3' splice site of a *CACNA1A* RIC pre-mRNA comprising the retained intron 36. In some embodiments, the ASO targets an exon 37 sequence about 2 to about 79 nucleotides downstream (or 3') from the 3' splice site of a *CACNA1A* RIC pre-mRNA comprising the retained intron 36.

In some embodiments, the ASO targets intron 36 in a *CACNA1A* RIC pre-mRNA comprising a retained intron 36, wherein the intron numbering correspond to the mRNA sequence at NM_000068. In some embodiments, the ASO targets an intron 36 sequence downstream (or 3') from the 5' splice site of a *CACNA1A* RIC pre-mRNA comprising the retained intron 36. In some embodiments, the ASO targets an intron 36 sequence about 6 to about 499 nucleotides downstream (or 3') from the 5' splice site of a *CACNA1A* RIC pre-mRNA comprising the retained intron 36. In some embodiments, the ASO targets an intron 36 sequence upstream (or 5') from the 3' splice site of a *CACNA1A* RIC pre-mRNA comprising the retained intron 36. In some embodiments, the ASO targets an intron 36 sequence about 16 to about 498 nucleotides upstream (or 5') from the 3' splice site of a *CACNA1A* RIC pre-mRNA comprising the retained intron 36.

In some embodiments, the ASO targets exon 37 or exon 38 of a *CACNA1A* RIC pre-mRNA comprising a retained intron 37, wherein the intron numbering correspond to the mRNA sequence at NM_000068. In some embodiments, the ASO targets an exon 37 sequence upstream (or 5') from the 5' splice site of a *CACNA1A* RIC pre-mRNA comprising the retained intron 37. In some embodiments, the ASO targets an exon 37 sequence about 4 to about 78 nucleotides upstream (or 5') from the 5' splice site of a *CACNA1A* RIC pre-mRNA comprising the retained intron 37. In some embodiments, the ASO targets an exon 38 sequence downstream (or 3') from the 3' splice site of a *CACNA1A* RIC pre-mRNA comprising the retained intron 37. In some embodiments, the ASO targets an exon 38 sequence about 2 to about 87 nucleotides downstream (or 3') from the 3' splice site of a *CACNA1A* RIC pre-mRNA comprising the retained intron 37.

In some embodiments, the ASO targets intron 37 in a *CACNA1A* RIC pre-mRNA comprising a retained intron 37, wherein the intron numbering correspond to the mRNA sequence at NM_000068. In some embodiments, the ASO targets an intron 37 sequence downstream (or 3') from the 5' splice site of a *CACNA1A* RIC pre-mRNA comprising the retained intron 37. In some embodiments, the ASO targets an intron 37 sequence about 6 to about 500 nucleotides downstream (or 3') from the 5' splice site of a *CACNA1A* RIC pre-mRNA comprising the retained intron 37. In some embodiments, the ASO targets an intron 37 sequence upstream (or 5') from the 3' splice site of a *CACNA1A* RIC pre-mRNA comprising the retained intron 37. In some embodiments, the ASO targets an intron 37 sequence about 16 to about 498 nucleotides upstream (or 5') from the 3' splice site of a *CACNA1A* RIC pre-mRNA comprising the retained intron 37.

In some embodiments, the ASO targets exon 36 or exon 37 of a *CACNA1A* RIC pre-mRNA comprising a retained intron 36, wherein the intron numbering correspond to the mRNA sequence at NM_023035. In some embodiments, the ASO targets an exon 36 sequence upstream (or 5') from the 5' splice site of a *CACNA1A* RIC pre-mRNA comprising the retained intron 36. In some embodiments, the ASO targets an exon 36 sequence about 4 to about 110 nucleotides upstream (or 5') from the 5' splice site of a *CACNA1A* RIC pre-mRNA comprising the retained intron 36. In some embodiments, the ASO targets an exon 37 sequence downstream (or 3') from the 3' splice site of a *CACNA1A* RIC pre-mRNA comprising the retained intron 36. In some embodiments, the ASO targets an exon 37 sequence about 2 to about 79 nucleotides downstream (or 3') from the 3' splice site of a *CACNA1A* RIC pre-mRNA comprising the retained intron 36.

In some embodiments, the ASO targets intron 36 in a *CACNA1A* RIC pre-mRNA comprising a retained intron 36, wherein the intron numbering correspond to the mRNA sequence at NM_023035. In some embodiments, the ASO targets an intron 36 sequence downstream (or 3') from the 5' splice site of a *CACNA1A* RIC pre-mRNA comprising the retained intron 36. In some embodiments, the ASO targets an intron 36 sequence about 6 to about 499 nucleotides downstream (or 3') from the 5' splice site of a *CACNA1A* RIC pre-mRNA comprising the retained intron 36. In some embodiments, the ASO targets an intron 36 sequence upstream (or 5') from the 3' splice site of a *CACNA1A* RIC pre-mRNA comprising the retained intron 36. In some embodiments, the ASO targets an intron 36 sequence about 16 to about 498 nucleotides upstream (or 5') from the 3' splice site of a *CACNA1A* RIC pre-mRNA comprising the retained intron 36.

In some embodiments, the ASO targets exon 37 or exon 38 of a *CACNA1A* RIC pre-mRNA comprising a retained intron 37, wherein the intron numbering correspond to the mRNA sequence at NM_023035. In some embodiments, the ASO targets an exon 37 sequence upstream (or 5') from the 5' splice site of a *CACNA1A* RIC pre-mRNA comprising the retained intron 37. In some embodiments, the ASO targets an exon 37 sequence about 4 to about 78 nucleotides upstream (or 5') from the 5' splice site of a *CACNA1A* RIC pre-mRNA comprising the retained intron 37. In some embodiments, the ASO targets an exon 38 sequence downstream (or 3') from the 3' splice site of a *CACNA1A* RIC pre-mRNA comprising the retained intron 37. In some embodiments, the ASO targets an exon 38 sequence about 2 to about 87 nucleotides downstream (or 3') from the 3' splice site of a *CACNA1A* RIC pre-mRNA comprising the retained intron 37.

In some embodiments, the ASO targets intron 37 in a *CACNA1A* RIC pre-mRNA comprising a retained intron 37, wherein the intron numbering correspond to the mRNA sequence at NM_023035. In some embodiments, the ASO targets an intron 37 sequence downstream (or 3') from the 5' splice site of a *CACNA1A* RIC pre-mRNA comprising the retained intron 37. In some embodiments, the ASO targets an intron 37 sequence about 6 to about 500 nucleotides downstream (or 3') from the 5' splice site of a *CACNA1A* RIC pre-mRNA comprising the retained intron 37. In some embodiments, the ASO targets an intron 37 sequence upstream (or 5') from the 3' splice site of a *CACNA1A* RIC pre-mRNA comprising the retained intron 37. In some embodiments, the ASO targets an intron 37 sequence about 16 to about 498 nucleotides upstream (or 5') from the 3' splice site of a *CACNA1A* RIC pre-mRNA comprising the retained intron 37.

In some embodiments, the ASO targets exon 36 or exon 37 of a *CACNA1A* RIC pre-mRNA comprising a retained intron 36, wherein the intron numbering correspond to the mRNA sequence at NM_001174080. In some embodiments, the ASO targets an exon 36 sequence upstream (or 5') from the 5' splice site of a *CACNA1A* RIC pre-mRNA comprising the retained intron 36. In some embodiments, the ASO targets an exon 36 sequence about 4 to about 110 nucleotides upstream (or 5') from the 5' splice site of a *CACNA1A* RIC pre-mRNA comprising the retained intron 36. In some embodiments, the ASO targets an exon 37 sequence downstream (or 3') from the 3' splice site of a *CACNA1A* RIC pre-mRNA comprising the retained intron 36. In some embodiments, the ASO targets an exon 37 sequence about 2 to about 79 nucleotides downstream (or 3') from the 3' splice site of a *CACNA1A* RIC pre-mRNA comprising the retained intron 36.

In some embodiments, the ASO targets intron 36 in a *CACNA1A* RIC pre-mRNA comprising a retained intron 36, wherein the intron numbering correspond to the mRNA sequence at NM_001174080. In some embodiments, the ASO targets an intron 36 sequence downstream (or 3') from the 5' splice site of a *CACNA1A* RIC pre-mRNA comprising the retained intron 36. In some embodiments, the ASO targets an intron 36 sequence about 6 to about 499 or about 16 to about 499 nucleotides downstream (or 3') from the 5' splice site of a *CACNA1A* RIC pre-mRNA comprising the retained intron 36. In some embodiments, the ASO targets an intron 36 sequence upstream (or 5') from the 3' splice site of a *CACNA1A* RIC pre-mRNA comprising the retained intron 36. In some embodiments, the ASO targets an intron 36 sequence about 16 to about 498 nucleotides upstream (or 5') from the 3' splice site of a *CACNA1A* RIC pre-mRNA comprising the retained intron 36.

In some embodiments, the ASO targets exon 37 or exon 38 of a *CACNA1A* RIC pre-mRNA comprising a retained intron 37, wherein the intron numbering correspond to the mRNA sequence at NM_001174080. In some embodiments, the ASO targets an exon 37 sequence upstream (or 5') from the 5' splice site of a *CACNA1A* RIC pre-mRNA comprising the retained intron 37. In some embodiments, the ASO targets an exon 37 sequence about 4 to about 78 nucleotides upstream (or 5') from the 5' splice site of a *CACNA1A* RIC pre-mRNA comprising the retained intron 37. In some embodiments, the ASO targets an exon 38 sequence downstream (or 3') from the 3' splice site of a *CACNA1A* RIC pre-mRNA comprising the retained intron 37. In some embodiments, the ASO targets an exon 38 sequence about 2 to about 87 nucleotides downstream (or 3') from the 3' splice site of a *CACNA1A* RIC pre-mRNA comprising the retained intron 37.

In some embodiments, the ASO targets intron 37 in a *CACNA1A* RIC pre-mRNA comprising a retained intron 37, wherein the intron numbering correspond to the mRNA sequence at NM_001174080. In some embodiments, the ASO targets an intron 37 sequence downstream (or 3') from the 5' splice site of a *CACNA1A* RIC pre-mRNA comprising the retained intron 37. In some embodiments, the ASO targets an intron 37 sequence about 6 to about 500 nucleotides downstream (or 3') from the 5' splice site of a *CACNA1A* RIC pre-mRNA comprising the retained intron 37. In some embodiments, the ASO targets an intron 37 sequence upstream (or 5') from the 3' splice site of a *CACNA1A* RIC pre-mRNA comprising the retained intron 37. In some embodiments, the ASO targets an intron 37 sequence about 16 to about 498 nucleotides upstream (or 5') from the 3' splice site of a *CACNA1A* RIC pre-mRNA comprising the retained intron 37.

In some embodiments, the ASO targets exon 36 or exon 37 of a *CACNA1A* RIC pre-mRNA comprising a retained intron 36, wherein the intron numbering correspond to the mRNA sequence at NM_001127222. In some embodiments, the ASO targets an exon 36 sequence upstream (or 5') from the 5' splice site of a *CACNA1A* RIC pre-mRNA comprising the retained intron 36. In some embodiments, the ASO targets an exon 36 sequence about 4 to about 110 or about 14 to about 110 nucleotides upstream (or 5') from the 5' splice site of a *CACNA1A* RIC pre-mRNA comprising the retained intron 36. In some embodiments, the ASO targets an exon 37 sequence downstream (or 3') from the 3' splice site of a *CACNA1A* RIC pre-mRNA comprising the retained intron 36. In some embodiments, the ASO targets an exon 37 sequence about 2 to about 79 nucleotides downstream (or 3') from the 3' splice site of a *CACNA1A* RIC pre-mRNA comprising the retained intron 36.

In some embodiments, the ASO targets intron 36 in a *CACNA1A* RIC pre-mRNA comprising a retained intron 36, wherein the intron numbering correspond to the mRNA sequence at NM_001127222. In some embodiments, the ASO targets an intron 36 sequence downstream (or 3') from the 5' splice site of a *CACNA1A* RIC pre-mRNA comprising the retained intron 36. In some embodiments, the ASO targets an intron 36 sequence about 6 to about 499 or about 16 to about 499 nucleotides downstream (or 3') from the 5' splice site of a *CACNA1A* RIC pre-mRNA comprising the retained intron 36. In some embodiments, the ASO targets an intron 36 sequence upstream (or 5') from the 3' splice site of a *CACNA1A* RIC pre-mRNA comprising the retained intron 36. In some embodiments, the ASO targets an intron 36 sequence about 16 to about 498 nucleotides upstream (or 5') from the 3' splice site of a *CACNA1A* RIC pre-mRNA comprising the retained intron 36.

In some embodiments, the ASO targets exon 37 or exon 38 of a *CACNA1A* RIC pre-mRNA comprising a retained intron 37, wherein the intron numbering correspond to the mRNA sequence at NM_001127222. In some embodiments, the ASO targets an exon 37 sequence upstream (or 5') from the 5' splice site of a *CACNA1A* RIC pre-mRNA comprising the retained intron 37. In some embodiments, the ASO targets an exon 37 sequence about 4 to about 78 nucleotides upstream (or 5') from the 5' splice site of a *CACNA1A* RIC pre-mRNA comprising the retained intron 37. In some embodiments, the ASO targets an exon 38 sequence downstream (or 3') from the 3' splice site of a *CACNA1A* RIC pre-mRNA comprising the retained intron 37. In some embodiments, the ASO targets an exon 38 sequence about 2 to about 87 nucleotides downstream (or 3') from the 3' splice site of a *CACNA1A* RIC pre-mRNA comprising the retained intron 37.

In some embodiments, the ASO targets intron 37 in a *CACNA1A* RIC pre-mRNA comprising a retained intron 37, wherein the intron numbering correspond to the mRNA sequence at NM_001127222. In some embodiments, the ASO targets an intron 37 sequence downstream (or 3') from the 5' splice site of a *CACNA1A* RIC pre-mRNA comprising the retained intron 37. In some embodiments, the ASO targets an intron 37 sequence about 6 to about 500 nucleotides downstream (or 3') from the 5' splice site of a *CACNA1A* RIC pre-mRNA comprising the retained intron 37. In some embodiments, the ASO targets an intron 37 sequence upstream (or 5') from the 3' splice site of a *CACNA1A* RIC pre-mRNA comprising the retained intron 37. In some embodiments, the ASO targets an intron 37 sequence about 16 to about 498 nucleotides upstream (or 5') from the 3' splice site of a *CACNA1A* RIC pre-mRNA comprising the retained intron 37.

In some embodiments, the ASO targets exon 36 or exon 37 of a *CACNA1A* RIC pre-mRNA comprising a retained intron 36, wherein the intron numbering correspond to the mRNA sequence at NM_001127221. In some embodiments, the ASO targets an exon 36 sequence upstream (or 5') from the 5' splice site of a *CACNA1A* RIC pre-mRNA comprising the retained intron 36. In some embodiments, the ASO targets an exon 36 sequence about 14 to about 110 nucleotides upstream (or 5') from the 5' splice site of a *CACNA1A* RIC pre-mRNA comprising the retained intron 36. In some embodiments, the ASO targets an exon 37 sequence downstream (or 3') from the 3' splice site of a *CACNA1A* RIC pre-mRNA comprising the retained intron 36. In some embodiments, the ASO targets an exon 37 sequence about 2 to about 77 nucleotides downstream (or 3') from the 3' splice site of a *CACNA1A* RIC pre-mRNA comprising the retained intron 36.

In some embodiments, the ASO targets intron 36 in a *CACNA1A* RIC pre-mRNA comprising a retained intron 36, wherein the intron numbering correspond to the mRNA sequence at NM_001127221. In some embodiments, the ASO targets an intron 36 sequence downstream (or 3') from the 5' splice site of a *CACNA1A* RIC pre-mRNA comprising the retained intron 36. In some embodiments, the ASO targets an intron 36 sequence about 6 to about 499 or about 16 to about 499 nucleotides downstream (or 3') from the 5' splice site of a *CACNA1A* RIC pre-mRNA comprising the retained intron 36. In some embodiments, the ASO targets an intron 36 sequence upstream (or 5') from the 3' splice site of a *CACNA1A* RIC pre-mRNA comprising the retained intron 36. In some embodiments, the ASO targets an intron 36 sequence about 16 to about 499 or about 21 to about 499 nucleotides upstream (or 5') from the 3' splice site of a *CACNA1A* RIC pre-mRNA comprising the retained intron 36.

In some embodiments, the ASO targets exon 37 or exon 38 of a *CACNA1A* RIC pre-mRNA comprising a retained intron 37, wherein the intron numbering correspond to the mRNA sequence at NM_001127221. In some embodiments, the ASO targets an exon 37 sequence upstream (or 5') from the 5' splice site of a *CACNA1A* RIC pre-mRNA comprising the retained intron 37. In some embodiments, the ASO targets an exon 37 sequence about 4 to about 79 nucleotides upstream (or 5') from the 5' splice site of a *CACNA1A* RIC pre-mRNA comprising the retained intron 37. In some embodiments, the ASO targets an exon 38 sequence downstream (or 3') from the 3' splice site of a *CACNA1A* RIC pre-mRNA comprising the retained intron 37. In some embodiments, the ASO targets an exon 38 sequence about 2 to about 87 nucleotides downstream (or 3') from the 3' splice site of a *CACNA1A* RIC pre-mRNA comprising the retained intron 37.

In some embodiments, the ASO targets intron 37 in a *CACNA1A* RIC pre-mRNA comprising a retained intron 37, wherein the intron numbering correspond to the mRNA sequence at NM_001127221. In some embodiments, the ASO targets an intron 37 sequence downstream (or 3') from the 5' splice site of a *CACNA1A* RIC pre-mRNA comprising the retained intron 37. In some embodiments, the ASO targets an intron 37 sequence about 6 to about 500 nucleotides downstream (or 3') from the 5' splice site of a *CACNA1A* RIC pre-mRNA comprising the retained intron 37. In some embodiments, the ASO targets an intron 37 sequence upstream (or 5') from the 3' splice site of a *CACNA1A* RIC pre-mRNA comprising the retained intron 37. In some embodiments, the ASO targets an intron 37 sequence about 16 to about 498 nucleotides upstream (or 5') from the 3' splice site of a *CACNA1A* RIC pre-mRNA comprising the retained intron 37.

In some embodiments, the ASOs disclosed herein target a RIC pre-mRNA transcribed from a *DNMT1* genomic sequence. In some embodiments, the ASO targets a RIC pre-mRNA transcript from a *DNMT1* genomic sequence comprising a retained intron. In some embodiments, the ASO targets a RIC pre-mRNA transcript of SEQ ID NO: 16. In some embodiments, the ASO targets a RIC pre-mRNA transcript of SEQ ID NO: 16 comprising a retained intron. In some embodiments, the ASOs disclosed herein target a *DNMT1* RIC pre-mRNA sequence. In some embodiments, the ASO targets a *DNMT1* RIC pre-mRNA transcript comprising a retained intron at 14, 29 or a combination thereof, wherein the intron numbering correspond to the mRNA sequence at NM_001379. In some embodiments, the ASO targets a *DNMT1* RIC pre-mRNA transcript comprising a retained intron at 15, 30 or a combination thereof, wherein the intron numbering correspond to the mRNA sequence at NM_001130823. In some embodiments, the ASO targets a *DNMT1* RIC pre-mRNA transcript comprising a retained intron at 14, 29 or a combination thereof, wherein the intron numbering correspond to the mRNA sequence at NM_001318730. In some embodiments, the ASO targets a *DNMT1* RIC pre-mRNA transcript comprising a retained intron at 15, 30 or a combination thereof, wherein the intron numbering correspond to the mRNA sequence at NM_001318731.

In some embodiments, the ASO targets a *DNMT1* RIC pre-mRNA sequence according to SEQ ID NO: 64. In some embodiments, the ASO targets a *DNMT1* RIC pre-mRNA sequence according to SEQ ID NO: 64 comprising a retained intron 14, a retained intron 29, or a combination thereof. In some embodiments, the ASO targets a *DNMT1* RIC pre-mRNA sequence according to SEQ ID NO: 65. In some embodiments, the ASO targets a *DNMT1* RIC pre-mRNA sequence according to SEQ ID NO: 65 comprising a retained intron 15, a retained intron 30, or a combination thereof. In some embodiments, the ASO targets a *DNMT1* RIC pre-mRNA sequence according to SEQ ID NO: 66. In some embodiments, the ASO targets a *DNMT1* RIC pre-mRNA sequence according to SEQ ID NO: 66 comprising a retained intron 14, a retained intron 29, or a combination thereof. In some embodiments, the ASO targets a *DNMT1* RIC pre-mRNA sequence according to SEQ ID NO: 67. In some embodiments, the ASO targets a *DNMT1* RIC pre-mRNA sequence according to SEQ ID NO: 67 comprising a retained intron 15, a retained intron 30, or a combination thereof. In some embodiments, the ASOs disclosed herein target SEQ ID NOs: 24362 or 24371. In some embodiments, the ASO has a sequence according to any one of SEQ ID NOs: 15915-16930.

In some embodiments, the ASO targets exon 29 or exon 30 of a *DNMT1* RIC pre-mRNA comprising a retained intron 29, wherein the intron numbering correspond to the mRNA sequence at NM_001379. In some embodiments, the ASO targets an exon 29 sequence upstream (or 5') from the 5' splice site of a *DNMT1* RIC pre-mRNA comprising the retained intron 29. In some embodiments, the ASO targets an exon 29 sequence about 4 to about 173 nucleotides upstream (or 5') from the 5' splice site of a *DNMT1* RIC pre-mRNA comprising the retained intron 29. In some embodiments, the ASO targets an exon 30 sequence downstream (or 3') from the 3' splice site of a *DNMT1* RIC pre-mRNA comprising the retained intron 29. In some embodiments, the ASO targets an exon 30 sequence about 2 to about 67 nucleotides downstream (or 3') from the 3' splice site of a *DNMT1* RIC pre-mRNA comprising the retained intron 29.

In some embodiments, the ASO targets intron 29 in a *DNMT1* RIC pre-mRNA comprising a retained intron 29, wherein the intron numbering correspond to the mRNA sequence at NM_001379. In some embodiments, the ASO targets an intron 29 sequence downstream (or 3') from the 5' splice site of a *DNMT1* RIC pre-mRNA comprising the retained intron 29. In some embodiments, the ASO targets an intron 29 sequence about 6 to about 429 nucleotides downstream (or 3') from the 5' splice site of a *DNMT1* RIC pre-mRNA comprising the retained intron 29. In some embodiments, the ASO targets an intron 29 sequence upstream (or 5') from the 3' splice site of a *DNMT1* RIC pre-mRNA comprising the retained intron 29. In some embodiments, the ASO targets an intron 29 sequence about 16 to about 433 nucleotides upstream (or 5') from the 3' splice site of a *DNMT1* RIC pre-mRNA comprising the retained intron 29.

In some embodiments, the ASO targets exon 14 or exon 15 of a *DNMT1* RIC pre-mRNA comprising a retained intron 14, wherein the intron numbering correspond to the mRNA sequence at NM_001379. In some embodiments, the ASO targets an exon 14 sequence upstream (or 5') from the 5' splice site of a *DNMT1* RIC pre-mRNA comprising the retained intron 14. In some embodiments, the ASO targets an exon 14 sequence about 4 to about 29 nucleotides upstream (or 5') from the 5' splice site of a *DNMT1* RIC pre-mRNA comprising the retained intron 14. In some embodiments, the ASO targets an exon 15 sequence downstream (or 3') from the 3' splice site of a *DNMT1* RIC pre-mRNA comprising the retained intron 14. In some embodiments, the ASO targets an exon 15 sequence about 2 to about 62 nucleotides downstream (or 3') from the 3' splice site of a *DNMT1* RIC pre-mRNA comprising the retained intron 14.

In some embodiments, the ASO targets intron 14 in a *DNMT1* RIC pre-mRNA comprising a retained intron 14, wherein the intron numbering correspond to the mRNA sequence at NM_001379. In some embodiments, the ASO targets an intron 14 sequence downstream (or 3') from the 5' splice site of a *DNMT1* RIC pre-mRNA comprising the retained intron 14. In some embodiments, the ASO targets an intron 14 sequence about 6 to about 61 nucleotides downstream (or 3') from the 5' splice site of a *DNMT1* RIC pre-mRNA comprising the retained intron 14. In some embodiments, the ASO targets an intron 14 sequence upstream (or 5') from the 3' splice site of a *DNMT1* RIC pre-mRNA comprising the retained intron 14. In some embodiments, the ASO targets an intron 14 sequence about 16 to about 61 nucleotides upstream (or 5') from the 3' splice site of a *DNMT1* RIC pre-mRNA comprising the retained intron 14.

In some embodiments, the ASO targets exon 30 or exon 31 of a *DNMT1* RIC pre-mRNA comprising a retained intron 30, wherein the intron numbering correspond to the mRNA sequence at NM_001130823. In some embodiments, the ASO targets an exon 30 sequence upstream (or 5') from the 5' splice site of a *DNMT1* RIC pre-mRNA comprising the retained intron 30. In some embodiments, the ASO targets an exon 30 sequence about 4 to about 173 nucleotides upstream (or 5') from the 5' splice site of a *DNMT1* RIC pre-mRNA comprising the retained intron 30. In some embodiments, the ASO targets an exon 31 sequence downstream (or 3') from the 3' splice site of a *DNMT1* RIC pre-mRNA comprising the retained intron 30. In some embodiments, the ASO targets an exon 31 sequence about 2 to about 67 nucleotides downstream (or 3') from the 3' splice site of a *DNMT1* RIC pre-mRNA comprising the retained intron 30.

In some embodiments, the ASO targets intron 30 in a *DNMT1* RIC pre-mRNA comprising a retained intron 30, wherein the intron numbering correspond to the mRNA sequence at NM_001130823. In some embodiments, the ASO targets an intron 30 sequence downstream (or 3') from the 5' splice site of a *DNMT1* RIC pre-mRNA comprising the retained intron 30. In some embodiments, the ASO targets an intron 30 sequence about 6 to about 429 or about 11 to about 429 nucleotides downstream (or 3') from the 5' splice site of a *DNMT1* RIC pre-mRNA comprising the retained intron 30. In some embodiments, the ASO targets an intron 30 sequence upstream (or 5') from the 3' splice site of a *DNMT1* RIC pre-mRNA comprising the retained intron 30. In some embodiments, the ASO targets an intron 30 sequence about 16 to about 433 nucleotides upstream (or 5') from the 3' splice site of a *DNMT1* RIC pre-mRNA comprising the retained intron 30.

In some embodiments, the ASO targets exon 15 or exon 16 of a *DNMT1* RIC pre-mRNA comprising a retained intron 15, wherein the intron numbering correspond to the mRNA sequence at NM_001130823. In some embodiments, the ASO targets an exon 15 sequence upstream (or 5') from the 5' splice site of a *DNMT1* RIC pre-mRNA comprising the retained intron 15. In some embodiments, the ASO targets an exon 15 sequence about 4 to about 29 nucleotides upstream (or 5') from the 5' splice site of a *DNMT1* RIC pre-mRNA comprising the retained intron 15. In some embodiments, the ASO targets an exon 16 sequence downstream (or 3') from the 3' splice site of a *DNMT1* RIC pre-mRNA comprising the retained intron 15. In some embodiments, the ASO targets an exon 16 sequence about 2 to about 62 nucleotides downstream (or 3') from the 3' splice site of a *DNMT1* RIC pre-mRNA comprising the retained intron 15.

In some embodiments, the ASO targets intron 15 in a *DNMT1* RIC pre-mRNA comprising a retained intron 15, wherein the intron numbering correspond to the mRNA sequence at NM_001130823. In some embodiments, the ASO targets an intron 15 sequence downstream (or 3') from the 5' splice site of a *DNMT1* RIC pre-mRNA comprising the retained intron 15. In some embodiments, the ASO targets an intron 15 sequence about 6 to about 61 nucleotides downstream (or 3') from the 5' splice site of a *DNMT1* RIC pre-mRNA comprising the retained intron 15. In some embodiments, the ASO targets an intron 15 sequence upstream (or 5') from the 3' splice site of a *DNMT1* RIC pre-mRNA comprising the retained intron 15. In some embodiments, the ASO targets an intron 15 sequence about 16 to about 61 nucleotides upstream (or 5') from the 3' splice site of a *DNMT1* RIC pre-mRNA comprising the retained intron 15.

In some embodiments, the ASO targets exon 29 or exon 30 of a *DNMT1* RIC pre-mRNA comprising a retained intron 29, wherein the intron numbering correspond to the mRNA sequence at NM_001318730. In some embodiments, the ASO targets an exon 29 sequence upstream (or 5') from the 5' splice site of a *DNMT1* RIC pre-mRNA comprising the retained intron 29. In some embodiments, the ASO targets an exon 29 sequence about 4 to about 173 nucleotides upstream (or 5') from the 5' splice site of a *DNMT1* RIC pre-mRNA comprising the retained intron 29. In some embodiments, the ASO targets an exon 30 sequence downstream (or 3') from the 3' splice site of a *DNMT1* RIC pre-mRNA comprising the retained intron 29. In some embodiments, the ASO targets an exon 30 sequence about 2 to about 67 nucleotides downstream (or 3') from the 3' splice site of a *DNMT1* RIC pre-mRNA comprising the retained intron 29.

In some embodiments, the ASO targets intron 29 in a *DNMT1* RIC pre-mRNA comprising a retained intron 29, wherein the intron numbering correspond to the mRNA sequence at NM_001318730. In some embodiments, the ASO targets an intron 29 sequence downstream (or 3') from the 5' splice site of a *DNMT1* RIC pre-mRNA comprising the retained intron 29. In some embodiments, the ASO targets an intron 29 sequence about 6 to about 429 or about 11 to about 429 nucleotides downstream (or 3') from the 5' splice site of a *DNMT1* RIC pre-mRNA comprising the retained intron 29. In some embodiments, the ASO targets an intron 29 sequence upstream (or 5') from the 3' splice site of a *DNMT1* RIC pre-mRNA comprising the retained intron 29. In some embodiments, the ASO targets an intron 29 sequence about 16 to about 433 nucleotides upstream (or 5') from the 3' splice site of a *DNMT1* RIC pre-mRNA comprising the retained intron 29.

In some embodiments, the ASO targets exon 14 or exon 15 of a *DNMT1* RIC pre-mRNA comprising a retained intron 14, wherein the intron numbering correspond to the mRNA sequence at NM_001318730. In some embodiments, the ASO targets an exon 14 sequence upstream (or 5') from the 5' splice site of a *DNMT1* RIC pre-mRNA comprising the retained intron 14. In some embodiments, the ASO targets an exon 14 sequence about 4 to about 29 nucleotides upstream (or 5') from the 5' splice site of a *DNMT1* RIC pre-mRNA comprising the retained intron 14. In some embodiments, the ASO targets an exon 15 sequence downstream (or 3') from the 3' splice site of a *DNMT1* RIC pre-mRNA comprising the retained intron 14. In some embodiments, the ASO targets an exon 15 sequence about 2 to about 62 nucleotides downstream (or 3') from the 3' splice site of a *DNMT1* RIC pre-mRNA comprising the retained intron 14.

In some embodiments, the ASO targets intron 14 in a *DNMT1* RIC pre-mRNA comprising a retained intron 14, wherein the intron numbering correspond to the mRNA sequence at NM_001318730. In some embodiments, the ASO targets an intron 14 sequence downstream (or 3') from the 5' splice site of a *DNMT1* RIC pre-mRNA comprising the retained intron 14. In some embodiments, the ASO targets an intron 14 sequence about 6 to about 61 nucleotides downstream (or 3') from the 5' splice site of a *DNMT1* RIC pre-mRNA comprising the retained intron 14. In some embodiments, the ASO targets an intron 14 sequence upstream (or 5') from the 3' splice site of a *DNMT1* RIC pre-mRNA comprising the retained intron 14. In some embodiments, the ASO targets an intron 14 sequence about 16 to about 61 nucleotides upstream (or 5') from the 3' splice site of a *DNMT1* RIC pre-mRNA comprising the retained intron 14.

In some embodiments, the ASO targets exon 30 or exon 31 of a *DNMT1* RIC pre-mRNA comprising a retained intron 30, wherein the intron numbering correspond to the mRNA sequence at NM_001318731. In some embodiments, the ASO targets an exon 30 sequence upstream (or 5') from the 5' splice site of a *DNMT1* RIC pre-mRNA comprising the retained intron 30. In some embodiments, the ASO targets an exon 30 sequence about 4 to about 173 nucleotides upstream (or 5') from the 5' splice site of a *DNMT1* RIC pre-mRNA comprising the retained intron 30. In some embodiments, the ASO targets an exon 31 sequence downstream (or 3') from the 3' splice site of a *DNMT1* RIC pre-mRNA comprising the retained intron 30. In some embodiments, the ASO targets an exon 31 sequence about 2 to about 67 nucleotides downstream (or 3') from the 3' splice site of a *DNMT1* RIC pre-mRNA comprising the retained intron 30.

In some embodiments, the ASO targets intron 30 in a *DNMT1* RIC pre-mRNA comprising a retained intron 30, wherein the intron numbering correspond to the mRNA sequence at NM_001318731. In some embodiments, the ASO targets an intron 30 sequence downstream (or 3') from the 5' splice site of a *DNMT1* RIC pre-mRNA comprising the retained intron 30. In some embodiments, the ASO targets an intron 30 sequence about 6 to about 429 or about 11 to about 429 nucleotides downstream (or 3') from the 5' splice site of a *DNMT1* RIC pre-mRNA comprising the retained intron 30. In some embodiments, the ASO targets an intron 30 sequence upstream (or 5') from the 3' splice site of a *DNMT1* RIC pre-mRNA comprising the retained intron 30. In some embodiments, the ASO targets an intron 30 sequence about 16 to about 433 nucleotides upstream (or 5') from the 3' splice site of a *DNMT1* RIC pre-mRNA comprising the retained intron 30.

In some embodiments, the ASO targets exon 15 or exon 16 of a *DNMT1* RIC pre-mRNA comprising a retained intron 15, wherein the intron numbering correspond to the mRNA sequence at NM_001318731. In some embodiments, the ASO targets an exon 15 sequence upstream (or 5') from the 5' splice site of a *DNMT1* RIC pre-mRNA comprising the retained intron 15. In some embodiments, the ASO targets an exon 15 sequence about 4 to about 29 nucleotides upstream (or 5') from the 5' splice site of a *DNMT1* RIC pre-mRNA comprising the retained intron 15. In some embodiments, the ASO targets an exon 16 sequence downstream (or 3') from the 3' splice site of a *DNMT1* RIC pre-mRNA comprising the retained intron 15. In some embodiments, the ASO targets an exon 16 sequence about 2 to about 62 nucleotides downstream (or 3') from the 3' splice site of a *DNMT1* RIC pre-mRNA comprising the retained intron 15.

In some embodiments, the ASO targets intron 15 in a *DNMT1* RIC pre-mRNA comprising a retained intron 15, wherein the intron numbering correspond to the mRNA sequence at NM_001318731. In some embodiments, the ASO targets an intron 15 sequence downstream (or 3') from the 5' splice site of a *DNMT1* RIC pre-mRNA comprising the retained intron 15. In some embodiments, the ASO targets an intron 15 sequence about 6 to about 61 nucleotides downstream (or 3') from the 5' splice site of a *DNMT1* RIC pre-mRNA comprising the retained intron 15. In some embodiments, the ASO targets an intron 15 sequence upstream (or 5') from the 3' splice site of a *DNMT1* RIC pre-mRNA comprising the retained intron 15. In some embodiments, the ASO targets an intron 15 sequence about 16 to about 61 nucleotides upstream (or 5') from the 3' splice site of a *DNMT1* RIC pre-mRNA comprising the retained intron 15.

In some embodiments, the ASOs disclosed herein target a RIC pre-mRNA transcribed from a *NF2* genomic sequence. In some embodiments, the ASO targets a RIC pre-mRNA transcript from a *NF2* genomic sequence comprising a retained intron. In some embodiments, the ASO targets a RIC pre-mRNA transcript of SEQ ID NO: 17. In some embodiments, the ASO targets a RIC pre-mRNA transcript of SEQ ID NO: 17 comprising a retained intron. In some embodiments, the ASOs disclosed herein target a *NF2* RIC pre-mRNA sequence. In some embodiments, the ASO targets a *NF2* RIC pre-mRNA transcript comprising a retained intron at 14, wherein the intron numbering correspond to the mRNA sequence at NM_181830. In some embodiments, the ASO targets a *NF2* RIC pre-mRNA transcript comprising a retained intron at 15, wherein the intron numbering correspond to the mRNA sequence at NM_181829. In some embodiments, the ASO targets a *NF2* RIC pre-mRNA transcript comprising a retained intron at 4, wherein the intron numbering correspond to the mRNA sequence at NM_181833. In some embodiments, the ASO targets a *NF2* RIC pre-mRNA transcript comprising a retained intron at 16, wherein the intron numbering correspond to the mRNA sequence at NM_181832. In some embodiments, the ASO targets a *NF2* RIC pre-mRNA transcript comprising a retained intron at 15, wherein the intron numbering correspond to the mRNA sequence at NM_181828. In some embodiments, the ASO targets a *NF2* RIC pre-mRNA transcript comprising a retained intron at 16, wherein the intron numbering correspond to the mRNA sequence at NM_016418. In some embodiments, the ASO targets a *NF2* RIC pre-mRNA transcript comprising a retained intron at 15, wherein the intron numbering correspond to the mRNA sequence at NM_000268.

In some embodiments, the ASO targets a *NF2* RIC pre-mRNA sequence according to SEQ ID NO: 68. In some embodiments, the ASO targets a *NF2* RIC pre-mRNA sequence according to SEQ ID NO: 68 comprising a retained intron 14. In some embodiments, the ASO targets a *NF2* RIC pre-mRNA sequence according to SEQ ID NO: 69. In some embodiments, the ASO targets a *NF2* RIC pre-mRNA sequence according to SEQ ID NO: 69 comprising a retained intron 15. In some embodiments, the ASO targets a *NF2* RIC pre-mRNA sequence according to SEQ ID NO: 70. In some embodiments, the ASO targets a *NF2* RIC pre-mRNA sequence according to SEQ ID NO: 70 comprising a retained intron 4. In some embodiments, the ASO targets a *NF2* RIC pre-mRNA sequence according to SEQ ID NO: 71. In some embodiments, the ASO targets a *NF2* RIC pre-mRNA sequence according to SEQ ID NO: 71 comprising a retained intron 16. In some embodiments, the ASO targets *a NF2* RIC pre-mRNA sequence according to SEQ ID NO: 72. In some embodiments, the ASO targets a *NF2* RIC pre-mRNA sequence according to SEQ ID NO: 72 comprising a retained intron 15. In some embodiments, the ASO targets *a NF2* RIC pre-mRNA sequence according to SEQ ID NO: 73. In some embodiments, the ASO targets a *NF2* RIC pre-mRNA sequence according to SEQ ID NO: 73 comprising a retained intron 16. In some embodiments, the ASO targets *a NF2* RIC pre-mRNA sequence according to SEQ ID NO: 74. In some embodiments, the ASO targets a *NF2* RIC pre-mRNA sequence according to SEQ ID NO: 74 comprising a retained intron 15. In some embodiments, the ASOs disclosed herein target SEQ ID NOs: 24373, 24367, 24352 or 24368. In some embodiments, the ASO has a sequence according to any one of SEQ ID NOs: 16931-23347.

In some embodiments, the ASO targets exon 14 or exon 15 of a *NF2* RIC pre-mRNA comprising a retained intron 14, wherein the intron numbering correspond to the mRNA sequence at NM_181830. In some embodiments, the ASO targets an exon 14 sequence upstream (or 5') from the 5' splice site of a *NF2* RIC pre-mRNA comprising the retained intron 14. In some embodiments, the ASO targets an exon 14 sequence about 4 to about 24 nucleotides upstream (or 5') from the 5' splice site of a *NF2* RIC pre-mRNA comprising the retained intron 14. In some embodiments, the ASO targets an exon 15 sequence downstream (or 3') from the 3' splice site of a *NF2* RIC pre-mRNA comprising the retained intron 14. In some embodiments, the ASO targets an exon 15 sequence about 2 to about 3828 nucleotides downstream (or 3') from the 3' splice site of a *NF2* RIC pre-mRNA comprising the retained intron 14.

In some embodiments, the ASO targets intron 14 in *a NF2* RIC pre-mRNA comprising a retained intron 14, wherein the intron numbering correspond to the mRNA sequence at NM_181830. In some embodiments, the ASO targets an intron 14 sequence downstream (or 3') from the 5' splice site of a *NF2* RIC pre-mRNA comprising the retained intron 14. In some embodiments, the ASO targets an intron 14 sequence about 6 to about 499 nucleotides downstream (or 3') from the 5' splice site of a *NF2* RIC pre-mRNA comprising the retained intron 14. In some embodiments, the ASO targets an intron 14 sequence upstream (or 5') from the 3' splice site of a *NF2* RIC pre-mRNA comprising the retained intron 14. In some embodiments, the ASO targets an intron 14 sequence about 16 to about 500 nucleotides upstream (or 5') from the 3' splice site of a *NF2* RIC pre-mRNA comprising the retained intron 14.

In some embodiments, the ASO targets exon 15 or exon 16 of a *NF2* RIC pre-mRNA comprising a retained intron 15, wherein the intron numbering correspond to the mRNA sequence at NM_181829. In some embodiments, the ASO targets an exon 15 sequence upstream (or 5') from the 5' splice site of a *NF2* RIC pre-mRNA comprising the retained intron 15. In some embodiments, the ASO targets an exon 15 sequence about 4 to about 24 nucleotides upstream (or 5') from the 5' splice site of a *NF2* RIC pre-mRNA comprising the retained intron 15. In some embodiments, the ASO targets an exon 16 sequence downstream (or 3') from the 3' splice site of a *NF2* RIC pre-mRNA comprising the retained intron 15. In some embodiments, the ASO targets an exon 16 sequence about 2 to about 3828 nucleotides downstream (or 3') from the 3' splice site of a *NF2* RIC pre-mRNA comprising the retained intron 15.

In some embodiments, the ASO targets intron 15 in a *NF2* RIC pre-mRNA comprising a retained intron 15, wherein the intron numbering correspond to the mRNA sequence at NM_181829. In some embodiments, the ASO targets an intron 15 sequence downstream (or 3') from the 5' splice site of a *NF2* RIC pre-mRNA comprising the retained intron 15. In some embodiments, the ASO targets an intron 15 sequence about 6 to about 499 nucleotides downstream (or 3') from the 5' splice site of a *NF2* RIC pre-mRNA comprising the retained intron 15. In some embodiments, the ASO targets an intron 15 sequence upstream (or 5') from the 3' splice site of a *NF2* RIC pre-mRNA comprising the retained intron 15. In some embodiments, the ASO targets an intron 15 sequence about 16 to about 500 nucleotides upstream (or 5') from the 3' splice site of a *NF2* RIC pre-mRNA comprising the retained intron 15.

In some embodiments, the ASO targets exon 4 or exon 5 of a *NF2* RIC pre-mRNA comprising a retained intron 4, wherein the intron numbering correspond to the mRNA sequence at NM_181833. In some embodiments, the ASO targets an exon 4 sequence upstream (or 5') from the 5' splice site of a *NF2* RIC pre-mRNA comprising the retained intron 4. In some embodiments, the ASO targets an exon 4 sequence about 4 to about 64 nucleotides upstream (or 5') from the 5' splice site of a *NF2* RIC pre-mRNA comprising the retained intron 4. In some embodiments, the ASO targets an exon 5 sequence downstream (or 3') from the 3' splice site of a *NF2* RIC pre-mRNA comprising the retained intron 4. In some embodiments, the ASO targets an exon 5 sequence about 2 to about 3828 nucleotides downstream (or 3') from the 3' splice site of a *NF2* RIC pre-mRNA comprising the retained intron 4.

In some embodiments, the ASO targets intron 4 in a *NF2* RIC pre-mRNA comprising a retained intron 4, wherein the intron numbering correspond to the mRNA sequence at NM_181833. In some embodiments, the ASO targets an intron 4 sequence downstream (or 3') from the 5' splice site of a *NF2* RIC pre-mRNA comprising the retained intron 4. In some embodiments, the ASO targets an intron 4 sequence about 6 to about 496 nucleotides downstream (or 3') from the 5' splice site of a *NF2* RIC pre-mRNA comprising the retained intron 4. In some embodiments, the ASO targets an intron 4 sequence upstream (or 5') from the 3' splice site of a *NF2* RIC pre-mRNA comprising the retained intron 4. In some embodiments, the ASO targets an intron 4 sequence about 16 to about 500 nucleotides upstream (or 5') from the 3' splice site of a *NF2* RIC pre-mRNA comprising the retained intron 4.

In some embodiments, the ASO targets exon 16 or exon 17 of a *NF2* RIC pre-mRNA comprising a retained intron 16, wherein the intron numbering correspond to the mRNA sequence at NM_181832. In some embodiments, the ASO targets an exon 16 sequence upstream (or 5') from the 5' splice site of a *NF2* RIC pre-mRNA comprising the retained intron 16. In some embodiments, the ASO targets an exon 16 sequence about 4 to about 39 nucleotides upstream (or 5') from the 5' splice site of a *NF2* RIC pre-mRNA comprising the retained intron 16. In some embodiments, the ASO targets an exon 17 sequence downstream (or 3') from the 3' splice site of a *NF2* RIC pre-mRNA comprising the retained intron 16. In some embodiments, the ASO targets an exon 17 sequence about 2 to about 3828 nucleotides downstream (or 3') from the 3' splice site of a *NF2* RIC pre-mRNA comprising the retained intron 16.

In some embodiments, the ASO targets intron 16 in *a NF2* RIC pre-mRNA comprising a retained intron 16, wherein the intron numbering correspond to the mRNA sequence at NM_181832. In some embodiments, the ASO targets an intron 16 sequence downstream (or 3') from the 5' splice site of a *NF2* RIC pre-mRNA comprising the retained intron 16. In some embodiments, the ASO targets an intron 16 sequence about 6 to about 499 nucleotides downstream (or 3') from the 5' splice site of a *NF2* RIC pre-mRNA comprising the retained intron 16. In some embodiments, the ASO targets an intron 16 sequence upstream (or 5') from the 3' splice site of a *NF2* RIC pre-mRNA comprising the retained intron 16. In some embodiments, the ASO targets an intron 16 sequence about 16 to about 500 nucleotides upstream (or 5') from the 3' splice site of a *NF2* RIC pre-mRNA comprising the retained intron 16.

In some embodiments, the ASO targets exon 15 or exon 16 of a *NF2* RIC pre-mRNA comprising a retained intron 15, wherein the intron numbering correspond to the mRNA sequence at NM_181828. In some embodiments, the ASO targets an exon 15 sequence upstream (or 5') from the 5' splice site of a *NF2* RIC pre-mRNA comprising the retained intron 15. In some embodiments, the ASO targets an exon 15 sequence about 4 to about 24 nucleotides upstream (or 5') from the 5' splice site of a *NF2* RIC pre-mRNA comprising the retained intron 15. In some embodiments, the ASO targets an exon 16 sequence downstream (or 3') from the 3' splice site of a *NF2* RIC pre-mRNA comprising the retained intron 15. In some embodiments, the ASO targets an exon 16 sequence about 2 to about 3828 nucleotides downstream (or 3') from the 3' splice site of a *NF2* RIC pre-mRNA comprising the retained intron 15.

In some embodiments, the ASO targets intron 15 in *a NF2* RIC pre-mRNA comprising a retained intron 15, wherein the intron numbering correspond to the mRNA sequence at NM_181828. In some embodiments, the ASO targets an intron 15 sequence downstream (or 3') from the 5' splice site of a *NF2* RIC pre-mRNA comprising the retained intron 15. In some embodiments, the ASO targets an intron 15 sequence about 6 to about 499 nucleotides downstream (or 3') from the 5' splice site of a *NF2* RIC pre-mRNA comprising the retained intron 15. In some embodiments, the ASO targets an intron 15 sequence upstream (or 5') from the 3' splice site of a *NF2* RIC pre-mRNA comprising the retained intron 15. In some embodiments, the ASO targets an intron 15 sequence about 16 to about 500 nucleotides upstream (or 5') from the 3' splice site of a *NF2* RIC pre-mRNA comprising the retained intron 15.

In some embodiments, the ASO targets exon 16 or exon 17 of a *NF2* RIC pre-mRNA comprising a retained intron 16, wherein the intron numbering correspond to the mRNA sequence at NM_016418. In some embodiments, the ASO targets an exon 16 sequence upstream (or 5') from the 5' splice site of a *NF2* RIC pre-mRNA comprising the retained intron 16. In some embodiments, the ASO targets an exon 16 sequence about 4 to about 24 nucleotides upstream (or 5') from the 5' splice site of a *NF2* RIC pre-mRNA comprising the retained intron 16. In some embodiments, the ASO targets an exon 17 sequence downstream (or 3') from the 3' splice site of a *NF2* RIC pre-mRNA comprising the retained intron 16. In some embodiments, the ASO targets an exon 17 sequence about 2 to about 3828 nucleotides downstream (or 3') from the 3' splice site of a *NF2* RIC pre-mRNA comprising the retained intron 16.

In some embodiments, the ASO targets intron 16 in *a NF2* RIC pre-mRNA comprising a retained intron 16, wherein the intron numbering correspond to the mRNA sequence at NM_016418. In some embodiments, the ASO targets an intron 16 sequence downstream (or 3') from the 5' splice site of a *NF2* RIC pre-mRNA comprising the retained intron 16. In some embodiments, the ASO targets an intron 16 sequence about 6 to about 499 nucleotides downstream (or 3') from the 5' splice site of a *NF2* RIC pre-mRNA comprising the retained intron 16. In some embodiments, the ASO targets an intron 16 sequence upstream (or 5') from the 3' splice site of a *NF2* RIC pre-mRNA comprising the retained intron 16. In some embodiments, the ASO targets an intron 16 sequence about 16 to about 500 nucleotides upstream (or 5') from the 3' splice site of a *NF2* RIC pre-mRNA comprising the retained intron 16.

In some embodiments, the ASO targets exon 15 or exon 16 of a *NF2* RIC pre-mRNA comprising a retained intron 15, wherein the intron numbering correspond to the mRNA sequence at NM_000268. In some embodiments, the ASO targets an exon 15 sequence upstream (or 5') from the 5' splice site of a *NF2* RIC pre-mRNA comprising the retained intron 15. In some embodiments, the ASO targets an exon 15 sequence about 4 to about 144 nucleotides upstream (or 5') from the 5' splice site of a *NF2* RIC pre-mRNA comprising the retained intron 15. In some embodiments, the ASO targets an exon 16 sequence downstream (or 3') from the 3' splice site of a *NF2* RIC pre-mRNA comprising the retained intron 15. In some embodiments, the ASO targets an exon 16 sequence about 2 to about 3828 nucleotides downstream (or 3') from the 3' splice site of a *NF2* RIC pre-mRNA comprising the retained intron 15.

In some embodiments, the ASO targets intron 15 in a *NF2* RIC pre-mRNA comprising a retained intron 15, wherein the intron numbering correspond to the mRNA sequence at NM_000268. In some embodiments, the ASO targets an intron 15 sequence downstream (or 3') from the 5' splice site of a *NF2* RIC pre-mRNA comprising the retained intron 15. In some embodiments, the ASO targets an intron 15 sequence about 6 to about 500 nucleotides downstream (or 3') from the 5' splice site of a *NF2* RIC pre-mRNA comprising the retained intron 15. In some embodiments, the ASO targets an intron 15 sequence upstream (or 5') from the 3' splice site of a *NF2* RIC pre-mRNA comprising the retained intron 15. In some embodiments, the ASO targets an intron 15 sequence about 16 to about 500 nucleotides upstream (or 5') from the 3' splice site of a *NF2* RIC pre-mRNA comprising the retained intron 15.

In some embodiments, the ASOs disclosed herein target a RIC pre-mRNA transcribed from a *SHANK3* genomic sequence. In some embodiments, the ASO targets a RIC pre-mRNA transcript from a *SHANK3* genomic sequence comprising a retained intron. In some embodiments, the ASO targets a RIC pre-mRNA transcript of SEQ ID NO: 18. In some embodiments, the ASO targets a RIC pre-mRNA transcript of SEQ ID NO: 18 comprising a retained intron. In some embodiments, the ASOs disclosed herein target a *SHANK3* RIC pre-mRNA sequence. In some embodiments, the ASO targets a *SHANK3* RIC pre-mRNA transcript comprising a retained intron at 16, wherein the intron numbering correspond to the mRNA sequence at NM_033517.

In some embodiments, the ASO targets a *SHANK3* RIC pre-mRNA sequence according to SEQ ID NO: 75. In some embodiments, the ASO targets a *SHANK3* RIC pre-mRNA sequence according to SEQ ID NO: 75 comprising a retained intron 16. In some embodiments, the ASOs disclosed herein target SEQ ID NO: 24357. In some embodiments, the ASO has a sequence according to any one of SEQ ID NOs: 23348-23535.

In some embodiments, the ASO targets exon 16 or exon 17 of a *SHANK3* RIC pre-mRNA comprising a retained intron 16, wherein the intron numbering correspond to the mRNA sequence at NM_000214. In some embodiments, the ASO targets an exon 16 sequence upstream (or 5') from the 5' splice site of a *SHANK3* RIC pre-mRNA comprising the retained intron 16. In some embodiments, the ASO targets an exon 16 sequence about 4 to about 114 nucleotides upstream (or 5') from the 5' splice site of a *SHANK3* RIC pre-mRNA comprising the retained intron 16. In some embodiments, the ASO targets an exon 17 sequence downstream (or 3') from the 3' splice site of a *SHANK3* RIC pre-mRNA comprising the retained intron 16. In some embodiments, the ASO targets an exon 17 sequence about 2 to about 62 or about 7 to about 62 nucleotides downstream (or 3') from the 3' splice site of a *SHANK3* RIC pre-mRNA comprising the retained intron 16.

In some embodiments, the ASO targets intron 16 in a *SHANK3* RIC pre-mRNA comprising a retained intron 16, wherein the intron numbering correspond to the mRNA sequence at NM_000214. In some embodiments, the ASO targets an intron 16 sequence downstream (or 3') from the 5' splice site of a *SHANK3* RIC pre-mRNA comprising the retained intron 16. In some embodiments, the ASO targets an intron 16 sequence about 6 to about 499 nucleotides downstream (or 3') from the 5' splice site of a *SHANK3* RIC pre-mRNA comprising the retained intron 16. In some embodiments, the ASO targets an intron 16 sequence upstream (or 5') from the 3' splice site of a *SHANK3* RIC pre-mRNA comprising the retained intron 16. In some embodiments, the ASO targets an intron 16 sequence about 16 to about 498 nucleotides upstream (or 5') from the 3' splice site of a *SHANK3* RIC pre-mRNA comprising the retained intron 16.

In some embodiments, the ASOs disclosed herein target a RIC pre-mRNA transcribed from a *ARSA* genomic sequence. In some embodiments, the ASO targets a RIC pre-mRNA transcript from a *ARSA* genomic sequence comprising a retained intron. In some embodiments, the ASO targets a RIC pre-mRNA transcript of SEQ ID NO: 19. In some embodiments, the ASO targets a RIC pre-mRNA transcript of SEQ ID NO: 19 comprising a retained intron. In some embodiments, the ASOs disclosed herein target *a ARSA* RIC pre-mRNA sequence. In some embodiments, the ASO targets a *ARSA* RIC pre-mRNA transcript comprising a retained intron at 2, 3 or a combination thereof, wherein the intron numbering correspond to the mRNA sequence at NM_000487. In some embodiments, the ASO targets a *ARSA* RIC pre-mRNA transcript comprising a retained intron at 3, 4 or a combination thereof, wherein the intron numbering correspond to the mRNA sequence at NM_001085425. In some embodiments, the ASO targets a *ARSA* RIC pre-mRNA transcript comprising a retained intron at 3, 4 or a combination thereof, wherein the intron numbering correspond to the mRNA sequence at NM_001085426. In some embodiments, the ASO targets *a ARSA* RIC pre-mRNA transcript comprising a retained intron at 3, 4 or a combination thereof, wherein the intron numbering correspond to the mRNA sequence at NM_001085427. In some embodiments, the ASO targets a *ARSA* RIC pre-mRNA transcript comprising a retained intron at 2, 3 or a combination thereof, wherein the intron numbering correspond to the mRNA sequence at NM_001085428.

In some embodiments, the ASO targets a *ARSA* RIC pre-mRNA sequence according to SEQ ID NO: 76. In some embodiments, the ASO targets *a ARSA* RIC pre-mRNA sequence according to SEQ ID NO: 76 comprising a retained intron 2, a retained intron 3, or a combination thereof. In some embodiments, the ASO targets *a ARSA* RIC pre-mRNA sequence according to SEQ ID NO: 77. In some embodiments, the ASO targets a *ARSA* RIC pre-mRNA sequence according to SEQ ID NO: 77 comprising a retained intron 3, a retained intron 4, or a combination thereof. In some embodiments, the ASO targets *a ARSA* RIC pre-mRNA sequence according to SEQ ID NO: 78. In some embodiments, the ASO targets a *ARSA* RIC pre-mRNA sequence according to SEQ ID NO: 78 comprising a retained intron 3, a retained intron 4, or a combination thereof. In some embodiments, the ASO targets *a ARSA* RIC pre-mRNA sequence according to SEQ ID NO: 79. In some embodiments, the ASO targets a *ARSA* RIC pre-mRNA sequence according to SEQ ID NO: 79 comprising a retained intron 3, a retained intron 4, or a combination thereof. In some embodiments, the ASO targets *a ARSA* RIC pre-mRNA sequence according to SEQ ID NO: 80. In some embodiments, the ASO targets a *ARSA* RIC pre-mRNA sequence according to SEQ ID NO: 80 comprising a retained intron 2, a retained intron 3, or a combination thereof. In some embodiments, the ASOs disclosed herein target SEQ ID NOs: 24378 or 24363. In some embodiments, the ASO has a sequence according to any one of SEQ ID NOs: 23536-24350.

In some embodiments, the ASO targets exon 2 or exon 3 of a *ARSA* RIC pre-mRNA comprising a retained intron 2, wherein the intron numbering correspond to the mRNA sequence at NM_000487. In some embodiments, the ASO targets an exon 2 sequence upstream (or 5') from the 5' splice site *of a ARSA* RIC pre-mRNA comprising the retained intron 2. In some embodiments, the ASO targets an exon 2 sequence about 4 to about 222 nucleotides upstream (or 5') from the 5' splice site of a *ARSA* RIC pre-mRNA comprising the retained intron 2. In some embodiments, the ASO targets an exon 3 sequence downstream (or 3') from the 3' splice site of a *ARSA* RIC pre-mRNA comprising the retained intron 2. In some embodiments, the ASO targets an exon 3 sequence about 2 to about 202 or about 7 to about 202 nucleotides downstream (or 3') from the 3' splice site of a *ARSA* RIC pre-mRNA comprising the retained intron 2.

In some embodiments, the ASO targets intron 2 in *a ARSA* RIC pre-mRNA comprising a retained intron 2, wherein the intron numbering correspond to the mRNA sequence at NM_000487. In some embodiments, the ASO targets an intron 2 sequence downstream (or 3') from the 5' splice site *of a ARSA* RIC pre-mRNA comprising the retained intron 2. In some embodiments, the ASO targets an intron 2 sequence about 6 to about 56 nucleotides downstream (or 3') from the 5' splice site of a *ARSA* RIC pre-mRNA comprising the retained intron 2. In some embodiments, the ASO targets an intron 2 sequence upstream (or 5') from the 3' splice site of a *ARSA* RIC pre-mRNA comprising the retained intron 2. In some embodiments, the ASO targets an intron 2 sequence about 16 to about 71 nucleotides upstream (or 5') from the 3' splice site *of a ARSA* RIC pre-mRNA comprising the retained intron 2.

In some embodiments, the ASO targets exon 3 or exon 4 *of a ARSA* RIC pre-mRNA comprising a retained intron 3, wherein the intron numbering correspond to the mRNA sequence at NM_000487. In some embodiments, the ASO targets an exon 3 sequence upstream (or 5') from the 5' splice site *of a ARSA* RIC pre-mRNA comprising the retained intron 3. In some embodiments, the ASO targets an exon 3 sequence about 4 to about 199 nucleotides upstream (or 5') from the 5' splice site of a *ARSA* RIC pre-mRNA comprising the retained intron 3. In some embodiments, the ASO targets an exon 4 sequence downstream (or 3') from the 3' splice site of *a ARSA* RIC pre-mRNA comprising the retained intron 3. In some embodiments, the ASO targets an exon 4 sequence about 2 to about 152 nucleotides downstream (or 3') from the 3' splice site *of a ARSA* RIC pre-mRNA comprising the retained intron 3.

In some embodiments, the ASO targets intron 3 in *a ARSA* RIC pre-mRNA comprising a retained intron 3, wherein the intron numbering correspond to the mRNA sequence at NM_000487. In some embodiments, the ASO targets an intron 3 sequence downstream (or 3') from the 5' splice site *of a ARSA* RIC pre-mRNA comprising the retained intron 3. In some embodiments, the ASO targets an intron 3 sequence about 6 to about 49 nucleotides downstream (or 3') from the 5' splice site of a *ARSA* RIC pre-mRNA comprising the retained intron 3. In some embodiments, the ASO targets an intron 3 sequence upstream (or 5') from the 3' splice site of *a ARSA* RIC pre-mRNA comprising the retained intron 3. In some embodiments, the ASO targets an intron 3 sequence about 16 to about 35 nucleotides upstream (or 5') from the 3' splice site *of a ARSA* RIC pre-mRNA comprising the retained intron 3.

In some embodiments, the ASO targets exon 3 or exon 4 *of a ARSA* RIC pre-mRNA comprising a retained intron 3, wherein the intron numbering correspond to the mRNA sequence at NM_001085425. In some embodiments, the ASO targets an exon 3 sequence upstream (or 5') from the 5' splice site *of a ARSA* RIC pre-mRNA comprising the retained intron 3. In some embodiments, the ASO targets an exon 3 sequence about 4 to about 222 nucleotides upstream (or 5') from the 5' splice site of a *ARSA* RIC pre-mRNA comprising the retained intron 3. In some embodiments, the ASO targets an exon 4 sequence downstream (or 3') from the 3' splice site of a *ARSA* RIC pre-mRNA comprising the retained intron 3. In some embodiments, the ASO targets an exon 4 sequence about 2 to about 202 or about 7 to about 202 nucleotides downstream (or 3') from the 3' splice site of a *ARSA* RIC pre-mRNA comprising the retained intron 3.

In some embodiments, the ASO targets intron 3 in *a ARSA* RIC pre-mRNA comprising a retained intron 3, wherein the intron numbering correspond to the mRNA sequence at NM_001085425. In some embodiments, the ASO targets an intron 3 sequence downstream (or 3') from the 5' splice site *of a ARSA* RIC pre-mRNA comprising the retained intron 3. In some embodiments, the ASO targets an intron 3 sequence about 6 to about 56 nucleotides downstream (or 3') from the 5' splice site of a *ARSA* RIC pre-mRNA comprising the retained intron 3. In some embodiments, the ASO targets an intron 3 sequence upstream (or 5') from the 3' splice site of *a ARSA* RIC pre-mRNA comprising the retained intron 3. In some embodiments, the ASO targets an intron 3 sequence about 16 to about 71 nucleotides upstream (or 5') from the 3' splice site of a *ARSA* RIC pre-mRNA comprising the retained intron 3.

In some embodiments, the ASO targets exon 4 or exon 5 *of a ARSA* RIC pre-mRNA comprising a retained intron 4, wherein the intron numbering correspond to the mRNA sequence at NM_001085425. In some embodiments, the ASO targets an exon 4 sequence upstream (or 5') from the 5' splice site *of a ARSA* RIC pre-mRNA comprising the retained intron 4. In some embodiments, the ASO targets an exon 4 sequence about 4 to about 199 nucleotides upstream (or 5') from the 5' splice site of a *ARSA* RIC pre-mRNA comprising the retained intron 4. In some embodiments, the ASO targets an exon 5 sequence downstream (or 3') from the 3' splice site of a *ARSA* RIC pre-mRNA comprising the retained intron 4. In some embodiments, the ASO targets an exon 5 sequence about 2 to about 152 nucleotides downstream (or 3') from the 3' splice site *of a ARSA* RIC pre-mRNA comprising the retained intron 4.

In some embodiments, the ASO targets intron 4 in *a ARSA* RIC pre-mRNA comprising a retained intron 4, wherein the intron numbering correspond to the mRNA sequence at NM_001085425. In some embodiments, the ASO targets an intron 4 sequence downstream (or 3') from the 5' splice site *of a ARSA* RIC pre-mRNA comprising the retained intron 4. In some embodiments, the ASO targets an intron 4 sequence about 5 to about 49 or about 6 to about 49 nucleotides downstream (or 3') from the 5' splice site of a *ARSA* RIC pre-mRNA comprising the retained intron 4. In some embodiments, the ASO targets an intron 4 sequence upstream (or 5') from the 3' splice site *of a ARSA* RIC pre-mRNA comprising the retained intron 4. In some embodiments, the ASO targets an intron 4 sequence about 16 to about 35 nucleotides upstream (or 5') from the 3' splice site of a *ARSA* RIC pre-mRNA comprising the retained intron 4.

In some embodiments, the ASO targets exon 3 or exon 4 *of a ARSA* RIC pre-mRNA comprising a retained intron 3, wherein the intron numbering correspond to the mRNA sequence at NM_001085426. In some embodiments, the ASO targets an exon 3 sequence upstream (or 5') from the 5' splice site *of a ARSA* RIC pre-mRNA comprising the retained intron 3. In some embodiments, the ASO targets an exon 3 sequence about 4 to about 222 nucleotides upstream (or 5') from the 5' splice site of a *ARSA* RIC pre-mRNA comprising the retained intron 3. In some embodiments, the ASO targets an exon 4 sequence downstream (or 3') from the 3' splice site of *a ARSA* RIC pre-mRNA comprising the retained intron 3. In some embodiments, the ASO targets an exon 4 sequence about 2 to about 202 or about 7 to about 202 nucleotides downstream (or 3') from the 3' splice site of a *ARSA* RIC pre-mRNA comprising the retained intron 3.

In some embodiments, the ASO targets intron 3 in *a ARSA* RIC pre-mRNA comprising a retained intron 3, wherein the intron numbering correspond to the mRNA sequence at NM_001085426. In some embodiments, the ASO targets an intron 3 sequence downstream (or 3') from the 5' splice site *of a ARSA* RIC pre-mRNA comprising the retained intron 3. In some embodiments, the ASO targets an intron 3 sequence about 6 to about 56 nucleotides downstream (or 3') from the 5' splice site of a *ARSA* RIC pre-mRNA comprising the retained intron 3. In some embodiments, the ASO targets an intron 3 sequence upstream (or 5') from the 3' splice site of *a ARSA* RIC pre-mRNA comprising the retained intron 3. In some embodiments, the ASO targets an intron 3 sequence about 16 to about 71 nucleotides upstream (or 5') from the 3' splice site *of a ARSA* RIC pre-mRNA comprising the retained intron 3.

In some embodiments, the ASO targets exon 4 or exon 5 *of a ARSA* RIC pre-mRNA comprising a retained intron 4, wherein the intron numbering correspond to the mRNA sequence at NM_001085426. In some embodiments, the ASO targets an exon 4 sequence upstream (or 5') from the 5' splice site *of a ARSA* RIC pre-mRNA comprising the retained intron 4. In some embodiments, the ASO targets an exon 4 sequence about 4 to about 199 nucleotides upstream (or 5') from the 5' splice site of a *ARSA* RIC pre-mRNA comprising the retained intron 4. In some embodiments, the ASO targets an exon 5 sequence downstream (or 3') from the 3' splice site of a *ARSA* RIC pre-mRNA comprising the retained intron 4. In some embodiments, the ASO targets an exon 5 sequence about 2 to about 152 nucleotides downstream (or 3') from the 3' splice site *of a ARSA* RIC pre-mRNA comprising the retained intron 4.

In some embodiments, the ASO targets intron 4 in *a ARSA* RIC pre-mRNA comprising a retained intron 4, wherein the intron numbering correspond to the mRNA sequence at NM_001085426. In some embodiments, the ASO targets an intron 4 sequence downstream (or 3') from the 5' splice site *of a ARSA* RIC pre-mRNA comprising the retained intron 4. In some embodiments, the ASO targets an intron 4 sequence about 5 toa bout 49 or about 6 to about 49 nucleotides downstream (or 3') from the 5' splice site of a *ARSA* RIC pre-mRNA comprising the retained intron 4. In some embodiments, the ASO targets an intron 4 sequence upstream (or 5') from the 3' splice site *of a ARSA* RIC pre-mRNA comprising the retained intron 4. In some embodiments, the ASO targets an intron 4 sequence about 16 to about 35 nucleotides upstream (or 5') from the 3' splice site of a *ARSA* RIC pre-mRNA comprising the retained intron 4.

In some embodiments, the ASO targets exon 3 or exon 4 *of a ARSA* RIC pre-mRNA comprising a retained intron 3, wherein the intron numbering correspond to the mRNA sequence at NM_001085427. In some embodiments, the ASO targets an exon 3 sequence upstream (or 5') from the 5' splice site *of a ARSA* RIC pre-mRNA comprising the retained intron 3. In some embodiments, the ASO targets an exon 3 sequence about 4 to about 222 nucleotides upstream (or 5') from the 5' splice site of a *ARSA* RIC pre-mRNA comprising the retained intron 3. In some embodiments, the ASO targets an exon 4 sequence downstream (or 3') from the 3' splice site of *a ARSA* RIC pre-mRNA comprising the retained intron 3. In some embodiments, the ASO targets an exon 4 sequence about 2 to about 202 or about 7 to about 202 nucleotides downstream (or 3') from the 3' splice site of a *ARSA* RIC pre-mRNA comprising the retained intron 3.

In some embodiments, the ASO targets intron 3 in *a ARSA* RIC pre-mRNA comprising a retained intron 3, wherein the intron numbering correspond to the mRNA sequence at NM_001085427. In some embodiments, the ASO targets an intron 3 sequence downstream (or 3') from the 5' splice site *of a ARSA* RIC pre-mRNA comprising the retained intron 3. In some embodiments, the ASO targets an intron 3 sequence about 6 to about 56 nucleotides downstream (or 3') from the 5' splice site of a *ARSA* RIC pre-mRNA comprising the retained intron 3. In some embodiments, the ASO targets an intron 3 sequence upstream (or 5') from the 3' splice site of a *ARSA* RIC pre-mRNA comprising the retained intron 3. In some embodiments, the ASO targets an intron 3 sequence about 16 to about 71 nucleotides upstream (or 5') from the 3' splice site *of a ARSA* RIC pre-mRNA comprising the retained intron 3.

In some embodiments, the ASO targets exon 4 or exon 5 *of a ARSA* RIC pre-mRNA comprising a retained intron 4, wherein the intron numbering correspond to the mRNA sequence at NM_001085427. In some embodiments, the ASO targets an exon 4 sequence upstream (or 5') from the 5' splice site *of a ARSA* RIC pre-mRNA comprising the retained intron 4. In some embodiments, the ASO targets an exon 4 sequence about 4 to about 199 nucleotides upstream (or 5') from the 5' splice site of a *ARSA* RIC pre-mRNA comprising the retained intron 4. In some embodiments, the ASO targets an exon 5 sequence downstream (or 3') from the 3' splice site of a *ARSA* RIC pre-mRNA comprising the retained intron 4. In some embodiments, the ASO targets an exon 5 sequence about 2 to about 152 nucleotides downstream (or 3') from the 3' splice site *of a ARSA* RIC pre-mRNA comprising the retained intron 4.

In some embodiments, the ASO targets intron 4 in *a ARSA* RIC pre-mRNA comprising a retained intron 4, wherein the intron numbering correspond to the mRNA sequence at NM_001085427. In some embodiments, the ASO targets an intron 4 sequence downstream (or 3') from the 5' splice site of a *ARSA* RIC pre-mRNA comprising the retained intron 4. In some embodiments, the ASO targets an intron 4 sequence about 5 to about 49 or about 6 to about 49 nucleotides downstream (or 3') from the 5' splice site of a *ARSA* RIC pre-mRNA comprising the retained intron 4. In some embodiments, the ASO targets an intron 4 sequence upstream (or 5') from the 3' splice site of a *ARSA* RIC pre-mRNA comprising the retained intron 4. In some embodiments, the ASO targets an intron 4 sequence about 16 to about 35 nucleotides upstream (or 5') from the 3' splice site of a *ARSA* RIC pre-mRNA comprising the retained intron 4.

In some embodiments, the ASO targets exon 2 or exon 3 of a *ARSA* RIC pre-mRNA comprising a retained intron 2, wherein the intron numbering correspond to the mRNA sequence at NM_001085428. In some embodiments, the ASO targets an exon 2 sequence upstream (or 5') from the 5' splice site of a *ARSA* RIC pre-mRNA comprising the retained intron 2. In some embodiments, the ASO targets an exon 2 sequence about 4 to about 222 nucleotides upstream (or 5') from the 5' splice site of a *ARSA* RIC pre-mRNA comprising the retained intron 2. In some embodiments, the ASO targets an exon 3 sequence downstream (or 3') from the 3' splice site of a *ARSA* RIC pre-mRNA comprising the retained intron 2. In some embodiments, the ASO targets an exon 3 sequence about 2 to about 202 or about 7 to about 202 nucleotides downstream (or 3') from the 3' splice site of a *ARSA* RIC pre-mRNA comprising the retained intron 2.

In some embodiments, the ASO targets intron 2 in a *ARSA* RIC pre-mRNA comprising a retained intron 2, wherein the intron numbering correspond to the mRNA sequence at NM_001085428. In some embodiments, the ASO targets an intron 2 sequence downstream (or 3') from the 5' splice site of a *ARSA* RIC pre-mRNA comprising the retained intron 2. In some embodiments, the ASO targets an intron 2 sequence about 6 to about 56 nucleotides downstream (or 3') from the 5' splice site of a *ARSA* RIC pre-mRNA comprising the retained intron 2. In some embodiments, the ASO targets an intron 2 sequence upstream (or 5') from the 3' splice site of a *ARSA* RIC pre-mRNA comprising the retained intron 2. In some embodiments, the ASO targets an intron 2 sequence about 16 to about 71 nucleotides upstream (or 5') from the 3' splice site *of a ARSA* RIC pre-mRNA comprising the retained intron 2.

In some embodiments, the ASO targets exon 3 or exon 4 of *a ARSA* RIC pre-mRNA comprising a retained intron 3, wherein the intron numbering correspond to the mRNA sequence at NM_001085428. In some embodiments, the ASO targets an exon 3 sequence upstream (or 5') from the 5' splice site *of a ARSA* RIC pre-mRNA comprising the retained intron 3. In some embodiments, the ASO targets an exon 3 sequence about 4 to about 199 nucleotides upstream (or 5') from the 5' splice site of a *ARSA* RIC pre-mRNA comprising the retained intron 3. In some embodiments, the ASO targets an exon 4 sequence downstream (or 3') from the 3' splice site of *a ARSA* RIC pre-mRNA comprising the retained intron 3. In some embodiments, the ASO targets an exon 4 sequence about 2 to about 152 nucleotides downstream (or 3') from the 3' splice site *of a ARSA* RIC pre-mRNA comprising the retained intron 3.

In some embodiments, the ASO targets intron 3 in *a ARSA* RIC pre-mRNA comprising a retained intron 3, wherein the intron numbering correspond to the mRNA sequence at NM_001085428. In some embodiments, the ASO targets an intron 3 sequence downstream (or 3') from the 5' splice site *of a ARSA* RIC pre-mRNA comprising the retained intron 3. In some embodiments, the ASO targets an intron 3 sequence about 5 to about 49 or about 6 to about 49 nucleotides downstream (or 3') from the 5' splice site of a *ARSA* RIC pre-mRNA comprising the retained intron 3. In some embodiments, the ASO targets an intron 3 sequence upstream (or 5') from the 3' splice site *of a ARSA* RIC pre-mRNA comprising the retained intron 3. In some embodiments, the ASO targets an intron 3 sequence about 16 to about 35 nucleotides upstream (or 5') from the 3' splice site of a *ARSA* RIC pre-mRNA comprising the retained intron 3.

In embodiments, the targeted portion of the MFSD8 RIC pre-mRNA is in intron 12. The MFSD8 intron numbering used herein corresponds to the mRNA sequence at NM_152778. In embodiments, the percent retained intron can be 42. In embodiments, hybridization of an ASO to the targeted portion of the RIC pre-mRNA results in enhanced splicing at the splice site (5' splice site or 3' splice site) of retained intron 12 and subsequently increases MFSD8 protein production. It is understood that the intron numbering may change in reference to a different MFSD8 isoform sequence. One of skill in the art can determine the corresponding intron number in any isoform based on an intron sequence provided herein or using the number provided in reference to the mRNA sequence at NM_152778. One of skill in the art also can determine the sequences of flanking exons in any MFSD8 isoform for targeting using the methods of the invention, based on an intron sequence provided herein or using the intron number provided in reference to the mRNA sequence at NM_152778.

In embodiments, the targeted portion of the IDUA RIC pre-mRNA is in intron 3, 4, 5, 6 or 7. The IDUA intron numbering used herein corresponds to the mRNA sequence at NM_000203. In embodiments, the percent retained intron can be 25, 63 or 16. In embodiments, hybridization of an ASO to the targeted portion of the RIC pre-mRNA results in enhanced splicing at the splice site (5' splice site or 3' splice site) of retained intron 3, 4, 5, 6 or 7 and subsequently increases IDUA protein production. It is understood that the intron numbering may change in reference to a different IDUA isoform sequence. One of skill in the art can determine the corresponding intron number in any isoform based on an intron sequence provided herein or using the number provided in reference to the mRNA sequence at NM_000203. One of skill in the art also can determine the sequences of flanking exons in any IDUA isoform for targeting using the methods of the invention, based on an intron sequence provided herein or using the intron number provided in reference to the mRNA sequence at NM_000203.

In embodiments, the targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA is in intron 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38. In embodiments, hybridization of an ASO to the targeted portion of the RIC pre-mRNA results in enhanced splicing at the splice site (5' splice site or 3' splice site) of at least one of retained introns 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38, and subsequently increases ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein production.

In embodiments, the targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B pre-mRNA is in intron 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38. In embodiments, hybridization of an ASO to the targeted portion of the RIC pre-mRNA results in enhanced splicing at the splice site (5' splice site or 3' splice site) of at least one of retained introns 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38, and subsequently increases ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein production.

In some cases, the targeted portion of the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B pre-mRNA is in intron 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38. In embodiments, hybridization of an ASO to the targeted portion of the RIC pre-mRNA results in enhanced splicing at the splice site (5' splice site or 3' splice site) of at least one of retained introns 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37 or 38, and subsequently increases ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein production.

The degree of intron retention can be expressed as percent intron retention (PIR), the percentage of transcripts in which a given intron is retained. In brief, PIR can be calculated as the percentage of the average number of reads mapping to the exon-intron junctions, over the sum of the average of the exon-intron junction reads plus the exon-exon junction reads.

PIR values for SCN1A have been reported, e.g., by Braunschweig, et al., 2014, (see, e.g., Supplemental Table S9), incorporated by reference herein in its entirety.

In embodiments, the methods described herein are used to increase the production of a functional ATP1A2, CACNA1A, SETD5, SHANK3, NF2, DNMT1, TCF4, RAI1, PEX1, ARSA, EIF2B5, , EIF2B2, NPC1, ADAR, MFSD8, STXBP1, PRICKLE2, PRRT2, IDUA, STX1B protein, or any combinations thereof. As used herein, the term "functional" refers to the amount of activity or function of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combination thereof that is necessary to eliminate any one or more symptoms of a treated condition. In embodiments, the methods are used to increase the production of a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combination thereof. As used herein, the term "partially functional" refers to any amount of activity or function of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein that is less than the amount of activity or function that is necessary to eliminate or prevent any one or more symptoms of a disease or condition. In some embodiments, a partially functional protein or RNA will have at least 10%, at least 20%, at least 30%, at least 40%, at least 50%, at least 60%, at least 70%, at least 75%, at least 80%, 85%, at least 90%, or at least 95% less activity relative to the fully functional protein or RNA.

In embodiments, the method is a method of increasing the expression of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combination thereof by cells of a subject having a RIC pre-mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, wherein the subject has a deficient amount of activity of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and wherein the deficient amount of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is caused by haploinsufficiency of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. In such an embodiment, the subject has a first allele encoding a functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and a second allele from which the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is not produced. In another such embodiment, the subject has a first allele encoding a functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and a second allele encoding a nonfunctional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. In another such embodiment, the subject has a first allele encoding a functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and a second allele encoding a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. In any of these embodiments, the antisense oligomer binds to a targeted portion of the RIC pre-mRNA transcribed from the first allele (encoding functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein), thereby inducing constitutive splicing of the retained intron from the RIC pre-mRNA, and causing an increase in the level of mature mRNA encoding functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and an increase in the expression of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in the cells of the subject.

In embodiments, the subject has a first allele encoding a functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and a second allele encoding a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and the antisense oligomer binds to a targeted portion of the RIC pre-mRNA transcribed from the first allele (encoding functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein) or a targeted portion of the RIC pre-mRNA transcribed from the second allele (encoding partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein), thereby inducing constitutive splicing of the retained intron from the RIC pre-mRNA, and causing an increase in the level of mature mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, and an increase in the expression of functional or partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in the cells of the subject.

In some instances, the subject has a first allele encoding a functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein, and a second allele encoding a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein, and the antisense oligomer binds to a targeted portion of the RIC pre-mRNA transcribed from the first allele (encoding functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein) or a targeted portion of the RIC pre-mRNA transcribed from the second allele (encoding partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein), thereby inducing constitutive splicing of the retained intron from the RIC pre-mRNA, and causing an increase in the level of mature mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein, and an increase in the expression of functional or partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein in the cells of the subject.

In related embodiments, the method is a method of using an ASO to increase the expression of a protein or functional RNA. In embodiments, an ASO is used to increase the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in cells of a subject having a RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, wherein the subject has a deficiency in the amount or function of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein.

In related embodiments, the method is a method of using an ASO to increase the expression of a protein or functional RNA. In embodiments, an ASO is used to increase the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein in cells of a subject having a RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein, wherein the subject has a deficiency in the amount or function of a ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein.

In embodiments, the RIC pre-mRNA transcript that encodes the protein that is causative of the disease or condition is targeted by the ASOs described herein. In some embodiments, a RIC pre-mRNA transcript that encodes a protein that is not causative of the disease is targeted by the ASOs. For example, a disease that is the result of a mutation or deficiency of a first protein in a particular pathway may be ameliorated by targeting a RIC pre-mRNA that encodes a second protein, thereby increasing production of the second protein. In some embodiments, the function of the second protein is able to compensate for the mutation or deficiency of the first protein (which is causative of the disease or condition).

In embodiments, the subject has:
a. a first mutant allele from which
   i) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is produced at a reduced level compared to production from a wild-type allele,
   ii) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is produced in a form having reduced function compared to an equivalent wild-type protein, or
   iii) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein or functional RNA is not produced; and
b.a second mutant allele from which
   i) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is produced at a reduced level compared to production from a wild-type allele,
   ii) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is produced in a form having reduced function compared to an equivalent wild-type protein, or
   iii) the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is not produced, and
wherein the RIC pre-mRNA is transcribed from the first allele and/or the second allele. In these embodiments, the ASO binds to a targeted portion of the RIC pre-mRNA transcribed from the first allele or the second allele, thereby inducing constitutive splicing of the retained intron from the RIC pre-mRNA, and causing an increase in the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein and an increase in the expression of the target protein or functional RNA in the cells of the subject. In these embodiments, the target protein or functional RNA having an increase in expression level resulting from the constitutive splicing of the retained intron from the RIC pre-mRNA is either in a form having reduced function compared to the equivalent wild-type protein (partially-functional), or having full function compared to the equivalent wild-type protein (fully-functional).

In embodiments, the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combination thereof is increased 1.1 to 10-fold, when compared to the amount of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B that is produced in a control cell, *e.g.,* one that is not treated with the antisense oligomer or one that is treated with an antisense oligomer that does not bind to the targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA.

In embodiments, the condition caused by a deficient amount or activity of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is not a condition caused by alternative or aberrant splicing of the retained intron to which the ASO is targeted. In embodiments, the condition caused by a deficient amount or activity of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is not a condition caused by alternative or aberrant splicing of any retained intron in a RIC pre-mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. In embodiments, alternative or aberrant splicing may occur in a pre-mRNA transcribed from the gene, however the compositions and methods of the invention do not prevent or correct this alternative or aberrant splicing.

In some cases, the condition caused by a deficient amount or activity of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein is not a condition caused by alternative or aberrant splicing of the retained intron to which the ASO is targeted. In embodiments, the condition caused by a deficient amount or activity of the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein is not a condition caused by alternative or aberrant splicing of any retained intron in a RIC pre-mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein. In embodiments, alternative or aberrant splicing may occur in a pre-mRNA transcribed from the gene, however the compositions and methods of the invention do not prevent or correct this alternative or aberrant splicing.

In some instances, the condition caused by a deficient amount or activity of ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein is not a condition caused by alternative or aberrant splicing of the retained intron to which the ASO is targeted. In embodiments, the condition caused by a deficient amount or activity of the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein is not a condition caused by alternative or aberrant splicing of any retained intron in a RIC pre-mRNA encoding the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein. In embodiments, alternative or aberrant splicing may occur in a pre-mRNA transcribed from the gene, however the compositions and methods of the invention do not prevent or correct this alternative or aberrant splicing.

In embodiments, a subject treated using the methods of the invention expresses a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein from one allele, wherein the partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is caused by a frameshift mutation, a nonsense mutation, a missense mutation, or a partial gene deletion. In embodiments, a subject treated using the methods of the invention expresses a nonfunctional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein from one allele, wherein the nonfunctional ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is caused by a frameshift mutation, a nonsense mutation, a missense mutation, a partial gene deletion, in one allele. In embodiments, a subject treated using the methods of the invention has a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B whole gene deletion, in one allele.

In some cases, a subject treated using the methods of the invention expresses a partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein from one allele, wherein the partially functional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein is caused by a frameshift mutation, a nonsense mutation, a missense mutation, or a partial gene deletion. In embodiments, a subject treated using the methods of the invention expresses a nonfunctional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein from one allele, wherein the nonfunctional ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B protein is caused by a frameshift mutation, a nonsense mutation, a missense mutation, a partial gene deletion, in one allele. In embodiments, a subject treated using the methods of the invention has a ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B whole gene deletion, in one allele.

In some instances, a subject treated using the methods of the invention expresses a partially functional ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein from one allele, wherein the partially functional ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein is caused by a frameshift mutation, a nonsense mutation, a missense mutation, or a partial gene deletion. In embodiments, a subject treated using the methods of the invention expresses a nonfunctional ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein from one allele, wherein the nonfunctional ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein is caused by a frameshift mutation, a nonsense mutation, a missense mutation, a partial gene deletion, in one allele. In embodiments, a subject treated using the methods of the invention has a ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B whole gene deletion, in one allele.

### Use of TANGO for Increasing Protein Expression

As described above, in embodiments, Targeted Augmentation of Nuclear Gene Output (TANGO) can be used in the methods of the invention to increase expression of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combinations thereof. In these embodiments, a retained-intron-containing pre-mRNA (RIC pre-mRNA) encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, STX1B protein or any combination thereof is present in the nucleus of a cell. Cells having a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that comprises a retained intron, an exon flanking the 5' splice site, and an exon flanking the 3' splice site, encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, can be contacted with antisense oligomers (ASOs) that are complementary to a targeted portion of the RIC pre-mRNA. Hybridization of the ASOs to the targeted portion of the RIC pre-mRNA can result in enhanced splicing at the splice site (5' splice site or 3' splice site) of the retained intron and subsequently increases target protein production.

The terms "pre-mRNA," and "pre-mRNA transcript" may be used interchangeably and refer to any pre-mRNA species that contains at least one intron. In embodiments, pre-mRNA or pre-mRNA transcripts comprise a 5'-7-methylguanosine cap and/or a poly-A tail. In embodiments, pre-mRNA or pre-mRNA transcripts comprise both a 5'-7-methylguanosine cap and a poly-A tail. In some embodiments, the pre-mRNA transcript does not comprise a 5'-7-methylguanosine cap and/or a poly-A tail. A pre-mRNA transcript is a non-productive messenger RNA (mRNA) molecule if it is not translated into a protein (or transported into the cytoplasm from the nucleus).

As used herein, a "retained-intron-containing pre-mRNA" ("RIC pre-mRNA") is a pre-mRNA transcript that contains at least one retained intron. The RIC pre-mRNA contains a retained intron, an exon flanking the 5' splice site of the retained intron, an exon flanking the 3' splice site of the retained intron, and encodes the target protein. An "RIC pre-mRNA encoding a target protein" is understood to encode the target protein when fully spliced. A "retained intron" is any intron that is present in a pre-mRNA transcript when one or more other introns, such as an adjacent intron, encoded by the same gene have been spliced out of the same pre-mRNA transcript. In some embodiments, the retained intron is the most abundant intron in RIC pre-mRNA encoding the target protein. In embodiments, the retained intron is the most abundant intron in a population of RIC pre-mRNAs transcribed from the gene encoding the target protein in a cell, wherein the population of RIC pre-mRNAs comprises two or more retained introns. In embodiments, an antisense oligomer targeted to the most abundant intron in the population of RIC pre-mRNAs encoding the target protein induces splicing out of two or more retained introns in the population, including the retained intron to which the antisense oligomer is targeted or binds. In embodiments, a mature mRNA encoding the target protein is thereby produced. The terms "mature mRNA," and "fully-spliced mRNA," are used interchangeably herein to describe a fully processed mRNA encoding a target protein *(e.g.,* mRNA that is exported from the nucleus into the cytoplasm and translated into target protein) or a fully processed functional RNA. The term "productive mRNA," also can be used to describe a fully processed mRNA encoding a target protein. In embodiments, the targeted region is in a retained intron that is the most abundant intron in a RIC pre-mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein.

As used herein, the term "comprise" or variations thereof such as "comprises" or "comprising" are to be read to indicate the inclusion of any recited feature *(e.g.* in the case of an antisense oligomer, a defined nucleobase sequence) but not the exclusion of any other features. Thus, as used herein, the term "comprising" is inclusive and does not exclude additional, unrecited features *(e.g.* in the case of an antisense oligomer, the presence of additional, unrecited nucleobases).

In embodiments of any of the compositions and methods provided herein, "comprising" may be replaced with "consisting essentially of " or "consisting of." The phrase "consisting essentially of" is used herein to require the specified feature(s) (*e.g*. nucleobase sequence) as well as those which do not materially affect the character or function of the claimed invention. As used herein, the term "consisting" is used to indicate the presence of the recited feature *(e.g.* nucleobase sequence) alone (so that in the case of an antisense oligomer consisting of a specified nucleobase sequence, the presence of additional, unrecited nucleobases is excluded).

In embodiments, the targeted region is in a retained intron that is the second most abundant intron in a RIC pre-mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. For example, the second most abundant retained intron may be targeted rather than the most abundant retained intron due to the uniqueness of the nucleotide sequence of the second most abundant retained intron, ease of ASO design to target a particular nucleotide sequence, and/or amount of increase in protein production resulting from targeting the intron with an ASO. In embodiments, the retained intron is the second most abundant intron in a population of RIC pre-mRNAs transcribed from the gene encoding the target protein in a cell, wherein the population of RIC pre-mRNAs comprises two or more retained introns. In embodiments, an antisense oligomer targeted to the second most abundant intron in the population of RIC pre-mRNAs encoding the target protein induces splicing out of two or more retained introns in the population, including the retained intron to which the antisense oligomer is targeted or binds. In embodiments, fully-spliced (mature) RNA encoding the target protein is thereby produced.

In embodiments, an ASO is complementary to a targeted region that is within a non-retained intron in a RIC pre-mRNA. In embodiments, the targeted portion of the RIC pre-mRNA is within: the region +6 to +100 relative to the 5' splice site of the non-retained intron; or the region -16 to -100 relative to the 3' splice site of the non-retained intron. In embodiments, the targeted portion of the RIC pre-mRNA is within the region +100 relative to the 5' splice site of the non-retained intron to -100 relative to the 3' splice site of the non-retained intron. As used to identify the location of a region or sequence, "within" is understood to include the residues at the positions recited. For example, a region +6 to +100 includes the residues at positions +6 and +100. In embodiments, fully-spliced (mature) RNA encoding the target protein is thereby produced.

In embodiments, the retained intron of the RIC pre-mRNA is an inefficiently spliced intron. As used herein, "inefficiently spliced" may refer to a relatively low frequency of splicing at a splice site adjacent to the retained intron (5' splice site or 3' splice site) as compared to the frequency of splicing at another splice site in the RIC pre-mRNA. The term "inefficiently spliced" may also refer to the relative rate or kinetics of splicing at a splice site, in which an "inefficiently spliced" intron may be spliced or removed at a slower rate as compared to another intron in a RIC pre-mRNA.

In embodiments, the 9-nucleotide sequence at -3e to -1e of the exon flanking the 5' splice site and +1 to +6 of the retained intron is identical to the corresponding wild-type sequence. In embodiments, the 16 nucleotide sequence at -15 to -1 of the retained intron and +1e of the exon flanking the 3' splice site is identical to the corresponding wild-type sequence. As used herein, the "wild-type sequence" refers to the nucleotide sequence for the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B gene in the published reference genome deposited in the NCBI repository of biological and scientific information (operated by National Center for Biotechnology Information, National Library of Medicine, 8600 Rockville Pike, Bethesda, MD USA 20894). As used herein, the "wild-type sequence" refers to NCBI Gene ID: (ATP1A2:477; CACNA1A:773; SETD5:55209; SHANK3:85358; NF2:4771; DNMT1:1786; TCF4:6925; RAI1:10743; PEX1:5189; ARSA:410; EIF2B5:8893; EIF2B1:1967; EIF2B2:8892; NPC1:4864; ADAR:103; MFSD8:256471; STXBP1:6812; PRICKLE2:166336; PRRT2:112476; STX1B:112755; IDUA: IDUA). Also used herein, a nucleotide position denoted with an "e" indicates the nucleotide is present in the sequence of an exon *(e.g.,* the exon flanking the 5' splice site or the exon flanking the 3' splice site).

The methods involve contacting cells with an ASO that is complementary to a portion of a pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, resulting in increased expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B. As used herein, "contacting" or administering to cells refers to any method of providing an ASO in immediate proximity with the cells such that the ASO and the cells interact. A cell that is contacted with an ASO will take up or transport the ASO into the cell. The method involves contacting a condition or disease-associated or condition or disease-relevant cell with any of the ASOs described herein. In some embodiments, the ASO may be further modified or attached (*e.g*., covalently attached) to another molecule to target the ASO to a cell type, enhance contact between the ASO and the condition or disease-associated or condition or disease-relevant cell, or enhance uptake of the ASO.

As used herein, the term "increasing protein production" or "increasing expression of a target protein" means enhancing the amount of protein that is translated from an mRNA in a cell. A "target protein" may be any protein for which increased expression/production is desired.

In embodiments, contacting a cell that expresses a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA with an ASO that is complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA transcript results in a measurable increase in the amount of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein *(e.g.,* a target protein) encoded by the pre-mRNA. Methods of measuring or detecting production of a protein will be evident to one of skill in the art and include any known method, for example, Western blotting, flow cytometry, immunofluorescence microscopy, and ELISA.

In embodiments, contacting cells with an ASO that is complementary to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA transcript results in an increase in the amount of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein produced by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In embodiments, the total amount of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein produced by the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the amount of target protein produced by an control compound. A control compound can be, for example, an oligonucleotide that is not complementary to the targeted portion of the RIC pre-mRNA.

In some embodiments, contacting cells with an ASO that is complementary to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA transcript results in an increase in the amount of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B respectively, including the mature mRNA encoding the target protein. In some embodiments, the amount of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, or the mature mRNA encoding the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, is increased by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In embodiments, the total amount of the mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, or the mature mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein produced in the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of mature RNA produced in an untreated cell, *e.g.,* an untreated cell or a cell treated with a control compound. A control compound can be, for example, an oligonucleotide that is not complementary to the targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA.

In some cases, contacting cells with an ASO that is complementary to a targeted portion of a ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B RIC pre-mRNA transcript results in an increase in the amount of mRNA encoding ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B respectively, including the mature mRNA encoding the target protein. In some embodiments, the amount of mRNA encoding ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B protein, or the mature mRNA encoding the ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B protein, is increased by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In embodiments, the total amount of the mRNA encoding ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B protein, or the mature mRNA encoding ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B protein produced in the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of mature RNA produced in an untreated cell, *e.g.,* an untreated cell or a cell treated with a control compound. A control compound can be, for example, an oligonucleotide that is not complementary to the targeted portion of the ATP1A2, CACNA1A, SETD5, SHANK3, NF2, DNMT1, TCF4, RAI1, PEX1, ARSA, EIF2B5, EIF2B2, NPC1, ADAR, MFSD8, STXBP1, PRICKLE2, PRRT2, or STX1B RIC pre-mRNA.

### Constitutive Splicing of a Retained Intron from a RIC pre-mRNA

The methods and antisense oligonucleotide compositions provided herein are useful for increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in cells, for example, in a subject having a deficiency in the amount or activity of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, by increasing the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, or the mature mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein. In particular, the methods and compositions as described herein can induce the constitutive splicing of a retained intron from a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA transcript encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, thereby increasing the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, or the mature mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein.

Constitutive splicing of a retained intron from a RIC pre-mRNA correctly removes the retained intron from the RIC pre-mRNA, wherein the retained intron has wild-type splice sequences. Constitutive splicing, as used herein, does not encompass splicing of a retained intron from a RIC pre-mRNA transcribed from a gene or allele having a mutation that causes alternative splicing or aberrant splicing of a pre-mRNA transcribed from the gene or allele. For example, constitutive splicing of a retained intron, as induced using the methods and antisense oligonucleotides provided herein, does not correct aberrant splicing in or influence alternative splicing of a pre-mRNA to result in an increased expression of a target protein or functional RNA.

In embodiments, constitutive splicing can correctly remove a retained intron from a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA, wherein the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA is transcribed from a wild-type gene or allele, or a polymorphic gene or allele, that encodes a fully-functional target protein or functional RNA, and wherein the gene or allele does not have a mutation that causes alternative splicing or aberrant splicing of the retained intron.

In some embodiments, constitutive splicing of a retained intron from a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein correctly removes a retained intron from a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, wherein the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA is transcribed from a gene or allele from which the target gene or functional RNA is produced at a reduced level compared to production from a wild-type allele, and wherein the gene or allele does not have a mutation that causes alternative splicing or aberrant splicing of the retained intron. In these embodiments, the correct removal of the constitutively spliced retained intron results in production of target protein or functional RNA that is functional when compared to an equivalent wild-type protein or functional RNA.

In other embodiments, constitutive splicing can correctly remove a retained intron from a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA, wherein the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA is transcribed from a gene or allele that encodes a target protein or functional RNA produced in a form having reduced function compared to an equivalent wild-type protein or functional RNA, and wherein the gene or allele does not have a mutation that causes alternative splicing or aberrant splicing of the retained intron. In these embodiments, the correct removal of the constitutively spliced retained intron results in production of partially functional target protein, or functional RNA that is partially functional when compared to an equivalent wild-type protein or functional RNA.

"Correct removal" of the retained intron by constitutive splicing refers to removal of the entire intron, without removal of any part of an exon.

In embodiments, an antisense oligomer as described herein or used in any method described herein does not increase the amount of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein or the amount of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein by modulating alternative splicing or aberrant splicing of a pre-mRNA transcribed from the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B gene. Modulation of alternative splicing or aberrant splicing can be measured using any known method for analyzing the sequence and length of RNA species, e.g., by RT-PCR and using methods described elsewhere herein and in the literature. In embodiments, modulation of alternative or aberrant splicing is determined based on an increase or decrease in the amount of the spliced species of interest of at least 10% or 1.1-fold. In embodiments, modulation is determined based on an increase or decrease at a level that is at least 10% to 100% or 1.1 to 10-fold, as described herein regarding determining an increase in mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in the methods of the invention.

In embodiments, the method is a method wherein the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA can be produce by partial splicing of a wild-type ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA. In embodiments, the method is a method wherein the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA can be produce by partial splicing of a full-length wild-type ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA. In embodiments, the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA can be produce by partial splicing of a full-length ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA. In these embodiments, a full-length ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B pre-mRNA may have a polymorphism in a splice site of the retained intron that does not impair correct splicing of the retained intron as compared to splicing of the retained intron having the wild-type splice site sequence.

In embodiments, the mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein is a full-length mature mRNA, or a wild-type mature mRNA. In these embodiments, a full-length mature mRNA may have a polymorphism that does not affect the activity of the target protein or the functional RNA encoded by the mature mRNA, as compared to the activity of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein encoded by the wild-type mature mRNA.

### Antisense Oligomers

One aspect of the present disclosure is a composition comprising antisense oligomers that enhances splicing by binding to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA. As used herein, the terms "ASO" and "antisense oligomer" are used interchangeably and refer to an oligomer such as a polynucleotide, comprising nucleobases, that hybridizes to a target nucleic acid *(e.g.,* a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA) sequence by Watson-Crick base pairing or wobble base pairing (G-U). The ASO may have exact sequence complementary to the target sequence or near complementarity (*e.g*., sufficient complementarity to bind the target sequence and enhancing splicing at a splice site). ASOs are designed so that they bind (hybridize) to a target nucleic acid (*e.g*., a targeted portion of a pre-mRNA transcript) and remain hybridized under physiological conditions. Typically, if they hybridize to a site other than the intended (targeted) nucleic acid sequence, they hybridize to a limited number of sequences that are not a target nucleic acid (to a few sites other than a target nucleic acid). Design of an ASO can take into consideration the occurrence of the nucleic acid sequence of the targeted portion of the pre-mRNA transcript or a sufficiently similar nucleic acid sequence in other locations in the genome or cellular pre-mRNA or transcriptome, such that the likelihood the ASO will bind other sites and cause "off-target" effects is limited. Any antisense oligomers known in the art, for example in PCT Application No. PCT/US2014/054151, published as WO 2015/035091, titled "Reducing Nonsense-Mediated mRNA Decay," can be used to practice the methods described herein.

In some embodiments, ASOs "specifically hybridize" to or are "specific" to a target nucleic acid or a targeted portion of a RIC pre-mRNA. Typically such hybridization occurs with a Tm substantially greater than 37°C, preferably at least 50°C, and typically between 60°C to approximately 90°C. Such hybridization preferably corresponds to stringent hybridization conditions. At a given ionic strength and pH, the Tm is the temperature at which 50% of a target sequence hybridizes to a complementary oligonucleotide.

Oligomers, such as oligonucleotides, are "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree to which one polynucleotide is complementary with another) is quantifiable in terms of the proportion (e.g., the percentage) of bases in opposing strands that are expected to form hydrogen bonds with each other, according to generally accepted base-pairing rules. The sequence of an antisense oligomer (ASO) need not be 100% complementary to that of its target nucleic acid to hybridize. In certain embodiments, ASOs can comprise at least 70%, at least 75%, at least 80%, at least 85%, at least 90%, at least 95%, at least 96%, at least 97%, at least 98%, or at least 99% sequence complementarity to a target region within the target nucleic acid sequence to which they are targeted. For example, an ASO in which 18 of 20 nucleobases of the oligomeric compound are complementary to a target region, and would therefore specifically hybridize, would represent 90 percent complementarity. In this example, the remaining noncomplementary nucleobases may be clustered together or interspersed with complementary nucleobases and need not be contiguous to each other or to complementary nucleobases. Percent complementarity of an ASO with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

An ASO need not hybridize to all nucleobases in a target sequence and the nucleobases to which it does hybridize may be contiguous or noncontiguous. ASOs may hybridize over one or more segments of a pre-mRNA transcript, such that intervening or adjacent segments are not involved in the hybridization event *(e.g.,* a loop structure or hairpin structure may be formed). In certain embodiments, an ASO hybridizes to noncontiguous nucleobases in a target pre-mRNA transcript. For example, an ASO can hybridize to nucleobases in a pre-mRNA transcript that are separated by one or more nucleobase(s) to which the ASO does not hybridize.

The ASOs described herein comprise nucleobases that are complementary to nucleobases present in a target portion of a RIC pre-mRNA. The term ASO embodies oligonucleotides and any other oligomeric molecule that comprises nucleobases capable of hybridizing to a complementary nucleobase on a target mRNA but does not comprise a sugar moiety, such as a peptide nucleic acid (PNA). The ASOs may comprise naturally-occurring nucleotides, nucleotide analogs, modified nucleotides, or any combination of two or three of the preceding. The term "naturally occurring nucleotides" includes deoxyribonucleotides and ribonucleotides. The term "modified nucleotides" includes nucleotides with modified or substituted sugar groups and/or having a modified backbone. In some embodiments, all of the nucleotides of the ASO are modified nucleotides. Chemical modifications of ASOs or components of ASOs that are compatible with the methods and compositions described herein will be evident to one of skill in the art and can be found, for example, in U.S. Patent No. 8,258,109 B2, U.S. Patent No. 5,656,612, U.S. Patent Publication No. 2012/0190728, and Dias and Stein, Mol. Cancer Ther. 2002, 1 , 347-355, herein incorporated by reference in their entirety.

The nucleobase of an ASO may be any naturally occurring, unmodified nucleobase such as adenine, guanine, cytosine, thymine and uracil, or any synthetic or modified nucleobase that is sufficiently similar to an unmodified nucleobase such that it is capable of hydrogen bonding with a nucleobase present on a target pre-mRNA. Examples of modified nucleobases include, without limitation, hypoxanthine, xanthine, 7-methylguanine, 5,6-dihydrouracil, 5-methylcytosine, and 5-hydroxymethoylcytosine.

The ASOs described herein also comprise a backbone structure that connects the components of an oligomer. The term "backbone structure" and "oligomer linkages" may be used interchangeably and refer to the connection between monomers of the ASO. In naturally occurring oligonucleotides, the backbone comprises a 3'-5' phosphodiester linkage connecting sugar moieties of the oligomer. The backbone structure or oligomer linkages of the ASOs described herein may include (but are not limited to) phosphorothioate, phosphorodithioate, phosphoroselenoate, phosphorodiselenoate, phosphoroanilothioate, phosphoraniladate, phosphoramidate, and the like. See e.g., LaPlanche et al., Nucleic Acids Res. 14:9081 (1986); Stec et al., J. Am. Chem. Soc. 106:6077 (1984), Stein et al., Nucleic Acids Res. 16:3209 (1988), Zon et al., Anti Cancer Drug Design 6:539 (1991); Zon et al., Oligonucleotides and Analogues: A Practical Approach, pp. 87-108 (F. Eckstein, Ed., Oxford University Press, Oxford England (1991)); Stec et al., U.S. Pat. No. 5,151,510; Uhlmann and Peyman, Chemical Reviews 90:543 (1990). In some embodiments, the backbone structure of the ASO does not contain phosphorous but rather contains peptide bonds, for example in a peptide nucleic acid (PNA), or linking groups including carbamate, amides, and linear and cyclic hydrocarbon groups. In some embodiments, the backbone modification is a phosphorothioate linkage. In some embodiments, the backbone modification is a phosphoramidate linkage.

In embodiments, the stereochemistry at each of the phosphorus internucleotide linkages of the ASO backbone is random. In embodiments, the stereochemistry at each of the phosphorus internucleotide linkages of the ASO backbone is controlled and is not random. For example, U.S. Pat. App. Pub. No. 2014/0194610, "Methods for the Synthesis of Functionalized Nucleic Acids," incorporated herein by reference, describes methods for independently selecting the handedness of chirality at each phosphorous atom in a nucleic acid oligomer. In embodiments, an ASO used in the methods of the invention comprises an ASO having phosphorus internucleotide linkages that are not random. In embodiments, a composition used in the methods of the invention comprises a pure diastereomeric ASO. In embodiments, a composition used in the methods of the invention comprises an ASO that has diastereomeric purity of at least about 90%, at least about 91%, at least about 92%, at least about 93%, at least about 94%, at least about 95%, at least about 96%, at least about 97%, at least about 98%, at least about 99%, about 100%, about 90% to about 100%, about 91% to about 100%, about 92% to about 100%, about 93% to about 100%, about 94% to about 100%, about 95% to about 100%, about 96% to about 100%, about 97% to about 100%, about 98% to about 100% , or about 99% to about 100%.

In embodiments, the ASO has a nonrandom mixture of Rp and Sp configurations at its phosphorus internucleotide linkages. For example, it has been suggested that a mix of Rp and Sp is required in antisense oligonucleotides to achieve a balance between good activity and nuclease stability (Wan, et al., 2014, "Synthesis, biophysical properties and biological activity of second generation antisense oligonucleotides containing chiral phosphorothioate linkages," Nucleic Acids Research 42(22): 13456-13468, incorporated herein by reference). In embodiments, an ASO used in the methods of the invention comprises about 5-100% Rp, at least about 5% Rp, at least about 10% Rp, at least about 15% Rp, at least about 20% Rp, at least about 25% Rp, at least about 30% Rp, at least about 35% Rp, at least about 40% Rp, at least about 45% Rp, at least about 50% Rp, at least about 55% Rp, at least about 60% Rp, at least about 65% Rp, at least about 70% Rp, at least about 75% Rp, at least about 80% Rp, at least about 85% Rp, at least about 90% Rp, or at least about 95% Rp, with the remainder Sp, or about 100% Rp. In embodiments, an ASO used in the methods of the invention comprises about 10% to about 100% Rp, about 15% to about 100% Rp, about 20% to about 100% Rp, about 25% to about 100% Rp, about 30% to about 100% Rp, about 35% to about 100% Rp, about 40% to about 100% Rp, about 45% to about 100% Rp, about 50% to about 100% Rp, about 55% to about 100% Rp, about 60% to about 100% Rp, about 65% to about 100% Rp, about 70% to about 100% Rp, about 75% to about 100% Rp, about 80% to about 100% Rp, about 85% to about 100% Rp, about 90% to about 100% Rp, or about 95% to about 100% Rp, about 20% to about 80% Rp, about 25% to about 75% Rp, about 30% to about 70% Rp, about 40% to about 60% Rp, or about 45% to about 55% Rp, with the remainder Sp.

In embodiments, an ASO used in the methods of the invention comprises about 5-100% Sp, at least about 5% Sp, at least about 10% Sp, at least about 15% Sp, at least about 20% Sp, at least about 25% Sp, at least about 30% Sp, at least about 35% Sp, at least about 40% Sp, at least about 45% Sp, at least about 50% Sp, at least about 55% Sp, at least about 60% Sp, at least about 65% Sp, at least about 70% Sp, at least about 75% Sp, at least about 80% Sp, at least about 85% Sp, at least about 90% Sp, or at least about 95% Sp, with the remainder Rp, or about 100% Sp. In embodiments, an ASO used in the methods of the invention comprises about 10% to about 100% Sp, about 15% to about 100% Sp, about 20% to about 100% Sp, about 25% to about 100% Sp, about 30% to about 100% Sp, about 35% to about 100% Sp, about 40% to about 100% Sp, about 45% to about 100% Sp, about 50% to about 100% Sp, about 55% to about 100% Sp, about 60% to about 100% Sp, about 65% to about 100% Sp, about 70% to about 100% Sp, about 75% to about 100% Sp, about 80% to about 100% Sp, about 85% to about 100% Sp, about 90% to about 100% Sp, or about 95% to about 100% Sp, about 20% to about 80% Sp, about 25% to about 75% Sp, about 30% to about 70% Sp, about 40% to about 60% Sp, or about 45% to about 55% Sp, with the remainder Rp.

Any of the ASOs described herein may contain a sugar moiety that comprises ribose or deoxyribose, as present in naturally occurring nucleotides, or a modified sugar moiety or sugar analog, including a morpholine ring. Non-limiting examples of modified sugar moieties include 2' substitutions such as 2'-O-methyl (2'-O-Me), 2'-O-methoxyethyl (2'MOE), 2'-O-aminoethyl, 2'F; N3'->P5' phosphoramidate, 2'dimethylaminooxyethoxy, 2'dimethylaminoethoxyethoxy, 2'-guanidinidium, 2'-O-guanidinium ethyl, carbamate modified sugars, and bicyclic modified sugars. In some embodiments, the sugar moiety modification is selected from 2'-O-Me, 2'F, and 2'MOE. In some embodiments, the sugar moiety modification is an extra bridge bond, such as in a locked nucleic acid (LNA). In some embodiments the sugar analog contains a morpholine ring, such as phosphorodiamidate morpholino (PMO). In some embodiments, the sugar moiety comprises a ribofuransyl or 2'deoxyribofuransyl modification. In some embodiments, the sugar moiety comprises 2'4'-constrained 2'O-methyloxyethyl (cMOE) modifications. In some embodiments, the sugar moiety comprises cEt 2', 4' constrained 2'-O ethyl BNA modifications. In some embodiments, the sugar moiety comprises tricycloDNA (tcDNA) modifications. In some embodiments, the sugar moiety comprises ethylene nucleic acid (ENA) modifications. In some embodiments, the sugar moiety comprises MCE modifications. Modifications are known in the art and described in the literature, e.g., by Jarver, et al., 2014, "A Chemical View of Oligonucleotides for Exon Skipping and Related Drug Applications," Nucleic Acid Therapeutics 24(1): 37-47, incorporated by reference for this purpose herein.

In some examples, each monomer of the ASO is modified in the same way, for example each linkage of the backbone of the ASO comprises a phosphorothioate linkage or each ribose sugar moiety comprises a 2'O-methyl modification. Such modifications that are present on each of the monomer components of an ASO are referred to as "uniform modifications." In some examples, a combination of different modifications may be desired, for example, an ASO may comprise a combination of phosphorodiamidate linkages and sugar moieties comprising morpholine rings (morpholinos). Combinations of different modifications to an ASO are referred to as "mixed modifications" or "mixed chemistries."

In some embodiments, the ASO comprises one or more backbone modification. In some embodiments, the ASO comprises one or more sugar moiety modification. In some embodiments, the ASO comprises one or more backbone modification and one or more sugar moiety modification. In some embodiments, the ASO comprises 2'MOE modifications and a phosphorothioate backbone. In some embodiments, the ASO comprises a phosphorodiamidate morpholino (PMO). In some embodiments, the ASO comprises a peptide nucleic acid (PNA). Any of the ASOs or any component of an ASO *(e.g.,* a nucleobase, sugar moiety, backbone) described herein may be modified in order to achieve desired properties or activities of the ASO or reduce undesired properties or activities of the ASO. For example, an ASO or one or more component of any ASO may be modified to enhance binding affinity to a target sequence on a pre-mRNA transcript; reduce binding to any non-target sequence; reduce degradation by cellular nucleases (i.e., RNase H); improve uptake of the ASO into a cell and/or into the nucleus of a cell; alter the pharmacokinetics or pharmacodynamics of the ASO; and modulate the half-life of the ASO.

In some embodiments, the ASOs are comprised of 2'-O-(2-methoxyethyl) (MOE) phosphorothioate-modified nucleotides. ASOs comprised of such nucleotides are especially well-suited to the methods disclosed herein; oligomers having such modifications have been shown to have significantly enhanced resistance to nuclease degradation and increased bioavailability, making them suitable, for example, for oral delivery in some embodiments described herein. See *e.g.,* Geary et al., J Pharmacol Exp Ther. 2001; 296(3):890-7; Geary et al., J Pharmacol Exp Ther. 2001; 296(3):898-904.

Methods of synthesizing ASOs will be known to one of skill in the art. Alternatively or in addition, ASOs may be obtained from a commercial source.

Unless specified otherwise, the left-hand end of single-stranded nucleic acid *(e.g.,* pre-mRNA transcript, oligonucleotide, ASO, etc.) sequences is the 5' end and the left-hand direction of single or double-stranded nucleic acid sequences is referred to as the 5' direction. Similarly, the right-hand end or direction of a nucleic acid sequence (single or double stranded) is the 3' end or direction. Generally, a region or sequence that is 5' to a reference point in a nucleic acid is referred to as "upstream," and a region or sequence that is 3' to a reference point in a nucleic acid is referred to as "downstream." Generally, the 5' direction or end of an mRNA is where the initiation or start codon is located, while the 3' end or direction is where the termination codon is located. In some aspects, nucleotides that are upstream of a reference point in a nucleic acid may be designated by a negative number, while nucleotides that are downstream of a reference point may be designated by a positive number. For example, a reference point *(e.g.,* an exon-exon junction in mRNA) may be designated as the "zero" site, and a nucleotide that is directly adjacent and upstream of the reference point is designated "minus one," *e.g.,* "-1," while a nucleotide that is directly adjacent and downstream of the reference point is designated "plus one," e.g., "+1."

In other embodiments, the ASOs are complementary to (and bind to) a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is downstream (in the 3' direction) of the 5' splice site of the retained intron in a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA (e.g., the direction designated by positive numbers relative to the 5' splice site) (FIG. 1). In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region +6 to +4000, +6 to +3500, +6 to +3000, +6 to +2500, +6 to +2000, +6 to +1500, +6 to +1000, +6 to +500, +6 to +400, +6 to +300, +6 to +200, or +6 to +100 relative to the 5' splice site of the retained intron. In some embodiments, the ASO is not complementary to nucleotides +1 to +5 relative to the 5' splice site (the first five nucleotides located downstream of the 5' splice site). In some embodiments, the ASOs may be complementary to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region between nucleotides +6 and +50 relative to the 5' splice site of the retained intron. In some aspects, the ASOs are complementary to a targeted portion that is within the region +6 to +90, +6 to +80, +6 to +70, +6 to +60, +6 to +50, +6 to +40, +6 to +30, or +6 to +20 relative to 5' splice site of the retained intron.

In some embodiments, the ASOs are complementary to a targeted region of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is upstream (5' relative) of the 3' splice site of the retained intron in a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA *(e.g.,* in the direction designated by negative numbers) (FIG. 1). In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region -16 to -4000, -16 to -3000, -16 to -2000, -16 to -1000, -16 to -500, -16 to -400, -16 to -300, -6 to -200, or -16 to -100 relative to the 3' splice site of the retained intron. In some embodiments, the ASO is not complementary to nucleotides -1 to -15 relative to the 3' splice site (the first 15 nucleotides located upstream of the 3' splice site). In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region -16 to -50 relative to the 3' splice site of the retained intron. In some aspects, the ASOs are complementary to a targeted portion that is within the region -16 to -90, -16 to -80, -16 to -70, - 16 to -60, -16 to -50, -16 to -40, or -16 to -30 relative to 3' splice site of the retained intron.

In embodiments, the targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA is within the region +100 relative to the 5' splice site of the retained intron to -100 relative to the 3' splice site of the retained intron.

In some embodiments, the ASOs are complementary to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the exon flanking the 5' splice site (upstream) of the retained intron (FIG. 1). In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region +2e to -4e in the exon flanking the 5' splice site of the retained intron. In some embodiments, the ASOs are not complementary to nucleotides -1e to -3e relative to the 5' splice site of the retained intron. In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region -4e to-100e, -4e to -90e, -4e to -80e, -4e to -70e, -4e to -60e, -4e to -50e, -4 to -40e, -4e to -30e, or - 4e to -20e relative to the 5' splice site of the retained intron.

In some embodiments, the ASOs are complementary to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the exon flanking the 3' splice site (downstream) of the retained intron (FIG. 1). In some embodiments, the ASOs are complementary to a targeted portion to the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region +2e to -4e in the exon flanking the 3' splice site of the retained intron. In some embodiments, the ASOs are not complementary to nucleotide +1e relative to the 3' splice site of the retained intron. In some embodiments, the ASOs are complementary to a targeted portion of the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA that is within the region+2e to +100e, +2e to +90e, +2e to +80e, +2e to +70e, +2e to +60e, +2e to +50e, +2e to +40e, +2e to +30e, or +2 to +20e relative to the 3' splice site of the retained intron. The ASOs may be of any length suitable for specific binding and effective enhancement of splicing. In some embodiments, the ASOs consist of 8 to 50 nucleobases. For example, the ASO may be 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, or 50 nucleobases in length. In some embodiments, the ASOs consist of more than 50 nucleobases. In some embodiments, the ASO is from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, 12 to 15 nucleobases, 13 to 50 nucleobases, 13 to 40 nucleobases, 13 to 35 nucleobases, 13 to 30 nucleobases, 13 to 25 nucleobases, 13 to 20 nucleobases, 14 to 50 nucleobases, 14 to 40 nucleobases, 14 to 35 nucleobases, 14 to 30 nucleobases, 14 to 25 nucleobases, 14 to 20 nucleobases, 15 to 50 nucleobases, 15 to 40 nucleobases, 15 to 35 nucleobases, 15 to 30 nucleobases, 15 to 25 nucleobases, 15 to 20 nucleobases, 20 to 50 nucleobases, 20 to 40 nucleobases, 20 to 35 nucleobases, 20 to 30 nucleobases, 20 to 25 nucleobases, 25 to 50 nucleobases, 25 to 40 nucleobases, 25 to 35 nucleobases, or 25 to 30 nucleobases in length. In some embodiments, the ASOs are 30 nucleotides in length. In some embodiments, the ASOs are 29 nucleotides in length. In some embodiments, the ASOs are 28 nucleotides in length. In some embodiments, the ASOs are 27 nucleotides in length. In some embodiments, the ASOs are 26 nucleotides in length. In some embodiments, the ASOs are 25 nucleotides in length. In some embodiments, the ASOs are 24 nucleotides in length. In some embodiments, the ASOs are 23 nucleotides in length. In some embodiments, the ASOs are 22 nucleotides in length. In some embodiments, the ASOs are 21 nucleotides in length. In some embodiments, the ASOs are 20 nucleotides in length. In some embodiments, the ASOs are 19 nucleotides in length. In some embodiments, the ASOs are 18 nucleotides in length. In some embodiments, the ASOs are 17 nucleotides in length. In some embodiments, the ASOs are 16 nucleotides in length. In some embodiments, the ASOs are 15 nucleotides in length. In some embodiments, the ASOs are 14 nucleotides in length.-In some embodiments, the ASOs are 13 nucleotides in length. In some embodiments, the ASOs are 12 nucleotides in length. In some embodiments, the ASOs are 11 nucleotides in length. In some embodiments, the ASOs are 10 nucleotides in length.

In some embodiments, two or more ASOs with different chemistries but complementary to the same targeted portion of the RIC pre-mRNA are used. In some embodiments, two or more ASOs that are complementary to different targeted portions of the RIC pre-mRNA are used.

In embodiments, the antisense oligonucleotides of the invention are chemically linked to one or more moieties or conjugates, e.g., a targeting moiety or other conjugate that enhances the activity or cellular uptake of the oligonucleotide. Such moieties include, but are not limited to, a lipid moiety, *e.g.,* as a cholesterol moiety, a cholesteryl moiety, an aliphatic chain, *e.g*., dodecandiol or undecyl residues, a polyamine or a polyethylene glycol chain, or adamantane acetic acid. Oligonucleotides comprising lipophilic moieties, and preparation methods have been described in the published literature. In embodiments, the antisense oligonucleotide is conjugated with a moiety including, but not limited to, an abasic nucleotide, a polyether, a polyamine, a polyamide, a peptides, a carbohydrate, e.g., N-acetylgalactosamine (GalNAc), N-Ac-Glucosamine (GluNAc), or mannose (e.g., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, *e.g*., using a linker. Linkers can include a bivalent or trivalent branched linker. In embodiments, the conjugate is attached to the 3' end of the antisense oligonucleotide. Methods of preparing oligonucleotide conjugates are described, *e.g*., in U.S. Pat. No. 8,450,467, "Carbohydrate conjugates as delivery agents for oligonucleotides," incorporated by reference herein.

In some embodiments, the nucleic acid to be targeted by an ASO is a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA expressed in a cell, such as a eukaryotic cell. In some embodiments, the term "cell" may refer to a population of cells. In some embodiments, the cell is in a subject. In some embodiments, the cell is isolated from a subject. In some embodiments, the cell is *ex vivo.* In some embodiments, the cell is a condition or disease-relevant cell or a cell line. In some embodiments, the cell is *in vitro* (*e.g*., in cell culture).

### Pharmaceutical Compositions

Pharmaceutical compositions or formulations comprising the antisense oligonucleotide of the described compositions and for use in any of the described methods can be prepared according to conventional techniques well known in the pharmaceutical industry and described in the published literature. In embodiments, a pharmaceutical composition or formulation for treating a subject comprises an effective amount of any antisense oligomer as described above, or a pharmaceutically acceptable salt, solvate, hydrate or ester thereof, and a pharmaceutically acceptable diluent. The antisense oligomer of a pharmaceutical formulation may further comprise a pharmaceutically acceptable excipient, diluent or carrier.

Pharmaceutically acceptable salts are suitable for use in contact with the tissues of humans and lower animals without undue toxicity, irritation, allergic response, etc., and are commensurate with a reasonable benefit/risk ratio. (See, e.g., S. M. Berge, et al., J. Pharmaceutical Sciences, 66: 1-19 (1977), incorporated herein by reference for this purpose. The salts can be prepared in situ during the final isolation and purification of the compounds, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other documented methodologies such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

In embodiments, the compositions are formulated into any of many possible dosage forms such as, but not limited to, tablets, capsules, gel capsules, liquid syrups, soft gels, suppositories, and enemas. In embodiments, the compositions are formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers. In embodiments, a pharmaceutical formulation or composition of the present invention includes, but is not limited to, a solution, emulsion, microemulsion, foam or liposome-containing formulation (e.g., cationic or noncationic liposomes).

The pharmaceutical composition or formulation of the present invention may comprise one or more penetration enhancer, carrier, excipients or other active or inactive ingredients as appropriate and well known to those of skill in the art or described in the published literature. In embodiments, liposomes also include sterically stabilized liposomes, e.g., liposomes comprising one or more specialized lipids. These specialized lipids result in liposomes with enhanced circulation lifetimes. In embodiments, a sterically stabilized liposome comprises one or more glycolipids or is derivatized with one or more hydrophilic polymers, such as a polyethylene glycol (PEG) moiety. In embodiments, a surfactant is included in the pharmaceutical formulation or compositions. The use of surfactants in drug products, formulations and emulsions is well known in the art. In embodiments, the present invention employs a penetration enhancer to effect the efficient delivery of the antisense oligonucleotide, e.g., to aid diffusion across cell membranes and /or enhance the permeability of a lipophilic drug. In embodiments, the penetration enhancers is a surfactant, fatty acid, bile salt, chelating agent, or non-chelating nonsurfactant.

In embodiments, the pharmaceutical formulation comprises multiple antisense oligonucleotides. In embodiments, the antisense oligonucleotide is administered in combination with another drug or therapeutic agent. In embodiments, the antisense oligonucleotide is administered with one or more agents capable of promoting penetration of the subject antisense oligonucleotide across the blood-brain barrier by any method known in the art. For example, delivery of agents by administration of an adenovirus vector to motor neurons in muscle tissue is described in U.S. Pat. No. 6,632,427, "Adenoviral-vector-mediated gene transfer into medullary motor neurons," incorporated herein by reference. Delivery of vectors directly to the brain, e.g., the striatum, the thalamus, the hippocampus, or the substantia nigra, is described, e.g., in U.S. Pat. No. 6,756,523, "Adenovirus vectors for the transfer of foreign genes into cells of the central nervous system particularly in brain," incorporated herein by reference.

In embodiments, the antisense oligonucleotides are linked or conjugated with agents that provide desirable pharmaceutical or pharmacodynamic properties. In embodiments, the antisense oligonucleotide is coupled to a substance, known in the art to promote penetration or transport across the blood-brain barrier, e.g., an antibody to the transferrin receptor. In embodiments, the antisense oligonucleotide is linked with a viral vector, e.g., to render the antisense compound more effective or increase transport across the blood-brain barrier. In embodiments, osmotic blood brain barrier disruption is assisted by infusion of sugars, e.g., meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids, e.g., glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U.S. Pat. No. 4,866,042, "Method for the delivery of genetic material across the blood brain barrier," U.S. Pat. No. 6,294,520, "Material for passage through the blood-brain barrier," and U.S. Pat. No. 6,936,589, "Parenteral delivery systems," each incorporated herein by reference.

In embodiments, the antisense oligonucleotides of the invention are chemically linked to one or more moieties or conjugates, e.g., a targeting moiety or other conjugate that enhances the activity or cellular uptake of the oligonucleotide. Such moieties include, but are not limited to, a lipid moiety, e.g., as a cholesterol moiety, a cholesteryl moiety, an aliphatic chain, e.g., dodecandiol or undecyl residues, a polyamine or a polyethylene glycol chain, or adamantane acetic acid. Oligonucleotides comprising lipophilic moieties, and preparation methods have been described in the published literature. In embodiments, the antisense oligonucleotide is conjugated with a moiety including, but not limited to, an abasic nucleotide, a polyether, a polyamine, a polyamide, a peptides, a carbohydrate, e.g., N-acetylgalactosamine (GalNAc), N-Ac-Glucosamine (GluNAc), or mannose (e.g., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, e.g., using a linker. Linkers can include a bivalent or trivalent branched linker. In embodiments, the conjugate is attached to the 3' end of the antisense oligonucleotide. Methods of preparing oligonucleotide conjugates are described, e.g., in U.S. Pat. No. 8,450,467, "Carbohydrate conjugates as delivery agents for oligonucleotides," incorporated by reference herein.

### Treatment of Subjects

Any of the compositions provided herein may be administered to an individual. "Individual" may be used interchangeably with "subject" or "patient." An individual may be a mammal, for example a human or animal such as a non-human primate, a rodent, a rabbit, a rat, a mouse, a horse, a donkey, a goat, a cat, a dog, a cow, a pig, or a sheep. In embodiments, the individual is a human. In embodiments, the individual is a fetus, an embryo, or a child. In other embodiments, the individual may be another eukaryotic organism, such as a plant. In some embodiments, the compositions provided herein are administered to a cell *ex vivo.*

In some embodiments, the compositions provided herein are administered to an individual as a method of treating a disease or disorder. In some embodiments, the individual has a genetic disease, such as any of the diseases described herein. In some embodiments, the individual is at risk of having the disease, such as any of the diseases described herein. In some embodiments, the individual is at increased risk of having a disease or disorder caused by insufficient amount of a protein or insufficient activity of a protein. If an individual is "at an increased risk" of having a disease or disorder caused insufficient amount of a protein or insufficient activity of a protein, the method involves preventative or prophylactic treatment. For example, an individual may be at an increased risk of having such a disease or disorder because of family history of the disease. Typically, individuals at an increased risk of having such a disease or disorder benefit from prophylactic treatment (e.g., by preventing or delaying the onset or progression of the disease or disorder).

Suitable routes for administration of ASOs of the present invention may vary depending on cell type to which delivery of the ASOs is desired. The ASOs of the present invention may be administered to patients parenterally, for example, by intraperitoneal injection, intramuscular injection, subcutaneous injection, intrathecal injection, intracerebroventricular injection, intraperitoneal injection, or intravenous injection. In embodiments, delivery is to the CNS. In embodiments, a fetus is treated in utero, e.g., by administering the ASO composition to the fetus directly or indirectly (e.g., via the mother).

The compositions of the present invention may be provided to muscle cells by any suitable means, including direct administration (e.g., locally by injection or topical administration at a treatment site) or systemically (e.g., parenterally or orally), intranasally, orally, or by intrathecal, intracerebroventricular, inhalational, enteral, topical, intrauterine, vaginal, sublingual, rectal, intramuscular, intrapleural, intraventricular, intraperitoneal, ophthalmic, intravenous, or subcutaneous means.

### Methods of identifying additional ASOs that enhance splicing

Also within the scope of the present invention are methods for identifying (determining) additional ASOs that enhance splicing of a ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B RIC pre-mRNA, specifically at the target intron. ASOs that specifically hybridize to different nucleotides within the target region of the pre-mRNA may be screened to identify (determine) ASOs that improve the rate and/or extent of splicing of the target intron. In some embodiments, the ASO may block or interfere with the binding site(s) of a splicing repressor(s)/silencer. Any method known in the art may be used to identify (determine) an ASO that when hybridized to the target region of the intron results in the desired effect (e.g., enhanced splicing, protein or functional RNA production). These methods also can be used for identifying ASOs that enhance splicing of the retained intron by binding to a targeted region in an exon flanking the retained intron, or in a non-retained intron. An example of a method that may be used is provided below.

A round of screening, referred to as an ASO "walk" may be performed using ASOs that have been designed to hybridize to a target region of a pre-mRNA. For example, the ASOs used in the ASO walk can be tiled every 5 nucleotides from approximately 100 nucleotides upstream of the 5' splice site of the retained intron *(e.g.,* a portion of sequence of the exon located upstream of the target/retained intron) to approximately 100 nucleotides downstream of the 5' splice site of the target/retained intron and/or from approximately 100 nucleotides upstream of the 3' splice site of the retained intron to approximately 100 nucleotides downstream of the 3' splice site of the target/retained intron *(e.g.,* a portion of sequence of the exon located downstream of the target/retained intron). For example, a first ASO of 15 nucleotides in length may be designed to specifically hybridize to nucleotides +6 to +20 relative to the 5' splice site of the target/retained intron. A second ASO is designed to specifically hybridize to nucleotides +11 to +25 relative to the 5' splice site of the target/retained intron. ASOs are designed as such spanning the target region of the pre-mRNA. In embodiments, the ASOs can be tiled more closely, e.g., every 1, 2, 3, or 4 nucleotides. Further, the ASOs can be tiled from 100 nucleotides downstream of the 5' splice site, to 100 nucleotides upstream of the 3' splice site.

One or more ASOs, or a control ASO (an ASO with a scrambled sequence, sequence that is not expected to hybridize to the target region) are delivered, for example by transfection, into a disease-relevant cell line that expresses the target pre-mRNA (e.g., the RIC pre-mRNA described elsewhere herein). The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some embodiments, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (e.g., enhanced protein production). Any method known in the art for assessing and/or quantifying protein production, such as Western blotting, flow cytometry, immunofluorescence microscopy, and ELISA, can be used.

A second round of screening, referred to as an ASO "micro-walk" may be performed using ASOs that have been designed to hybridize to a target region of a pre-mRNA. The ASOs used in the ASO micro-walk are tiled every 1 nucleotide to further refine the nucleotide acid sequence of the pre-mRNA that when hybridized with an ASO results in enhanced splicing.

Regions defined by ASOs that promote splicing of the target intron are explored in greater detail by means of an ASO "micro-walk", involving ASOs spaced in 1-nt steps, as well as longer ASOs, typically 18-25 nt.

As described for the ASO walk above, the ASO micro-walk can be perform by delivering one or more ASOs, or a control ASO (an ASO with a scrambled sequence, sequence that is not expected to hybridize to the target region), for example by transfection, into a disease-relevant cell line that expresses the target pre-mRNA. The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some embodiments, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g.*, enhanced protein production). Any method known in the art for assessing and/or quantifying protein production, such as Western blotting, flow cytometry, immunofluorescence microscopy, and ELISA, can be used.

ASOs that when hybridized to a region of a pre-mRNA result in enhanced splicing and increased protein production may be tested *in vivo* using animal models, for example transgenic mouse models in which the full-length human gene has been knocked-in or in humanized mouse models of disease. Suitable routes for administration of ASOs may vary depending on the disease and/or the cell types to which delivery of the ASOs is desired. ASOs may be administered, for example, by intraperitoneal injection, intramuscular injection, subcutaneous injection, or intravenous injection. Following administration, the cells, tissues, and/or organs of the model animals may be assessed to determine the effect of the ASO treatment by for example evaluating splicing (efficiency, rate, extent) and protein production by methods known in the art and described herein. The animal models may also be any phenotypic or behavioral indication of the disease or disease severity.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EXAMPLES

The present invention will be more specifically illustrated by the following Examples. However, it should be understood that the present invention is not limited by these examples in any manner.

### Example 1: Identification of intron retention events in ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, and STX1B transcripts by RNAseq using next generation sequencing

Whole transcriptome shotgun sequencing was carried out using next generation sequencing to reveal a snapshot of transcripts produced by the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, and STX1B gene to identify intron-retention events. For this purpose, polyA+ RNA from nuclear and cytoplasmic fractions of cells was isolated and cDNA libraries constructed using Illumina's TruSeq Stranded mRNA library Prep Kit. The libraries were pair-end sequenced resulting in 100-nucleotide reads that were mapped to the human genome (Feb. 2009, GRCh37/hg19 assembly. The mapped reads were visualized using the UCSC genome browser (operated by the UCSC Genome Informatics Group (Center for Biomolecular Science & Engineering, University of California, Santa Cruz, 1156 High Street, Santa Cruz, CA 95064) and described by, e.g., Rosenbloom, et al., 2015, "The UCSC Genome Browser database: 2015 update," Nucleic Acids Research 43, Database Issue, doi: 10.1093/nar/gku1177) and the coverage and number of reads were inferred by the peak signals. The height of the peaks indicates the level of expression given by the density of the reads in a particular region. A schematic representation of the gene was provided by the UCSC genome browser so that peaks could be matched to the exonic and intronic regions. Based on this display, we identified introns that have high read density in the nuclear fraction of the cells, but have very low to no reads in the cytoplasmic fraction. This indicated that these introns were retained and that the intron-containing transcripts remain in the nucleus, and suggested that these retained RIC pre-mRNAs are non-productive, as they were not exported out to the cytoplasm.

### Example 2: Design of ASO-walk targeting

An ASO walk was designed to target introns using the method described herein. A region immediately downstream of an intron 5' splice site spanning nucleotides, e.g., +6 to +69 and a region immediately upstream of the intron's 3' splice site spanning nucleotides, e.g., -16 to -79 of the intron were targeted with 2'-O-Me RNA, PS backbone, 18-mer ASOs shifted by 5-nucleotide intervals.

### Example 3: Improved splicing efficiency via ASO-targeting

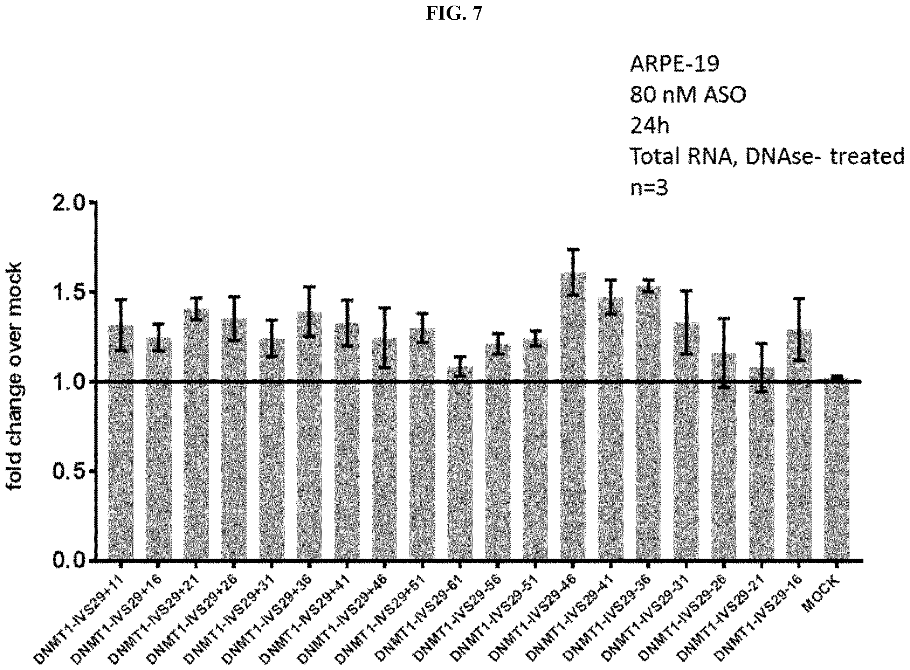

To determine whether an increase in expression of ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B could be achieved by improving splicing efficiency of a retained intron in ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B using ASOs, ARPE-19 or SK-N-AS cells were mock-transfected, or transfected with the targeting ASOs described in FIG. 7, FIG. 9, FIG. 11, FIG. 13, FIG. 15, FIG. 17, FIG. 19, FIG. 22, FIG. 24, FIG. 26, FIG. 37, FIG. 39, FIG. 41, and Table 1. Cells were transfected using Lipofectamine RNAiMax transfection reagent (Thermo Fisher) according to vendor's specifications. Briefly, ASOs were plated in 96-well tissue culture plates and combined with RNAiMax diluted in Opti-MEM. Cells were detached using trypsin and resuspended in full medium, and approximately 25,000 cells were added the ASO-transfection mixture. Transfection experiments were carried out in triplicate plate replicates. Final ASO concentration was 80 nM. Media was changed 6h post-transfection, and cells harvested at 24h, using the Cells-to-Ct lysis reagent, supplemented with DNAse (Thermo Fisher), according to vendor's specifications. cDNA was generated with Cells-to-Ct RT reagents (Thermo Fisher) according to vendor's specifications. To quantify the amount of splicing at the intron of interest, quantitative PCR was carried out using Taqman assays with probes spanning the corresponding exon-exon junction (Thermo Fisher). Taqman assays were carried out according to vendor's specifications, on a QuantStudio 7 Flex Real-Time PCR system (Thermo Fisher). ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B assay values were normalized to RPL32 (deltaCt) and plate-matched mock transfected samples (delta-delta Ct), generating fold-change over mock quantitation (2^-(delta-deltaCt). Average fold-change over mock of the three plate replicates were plotted These results were used to confirm that inducing splicing of a retained intron in the gene using ASOs leads to an increase in gene expression.

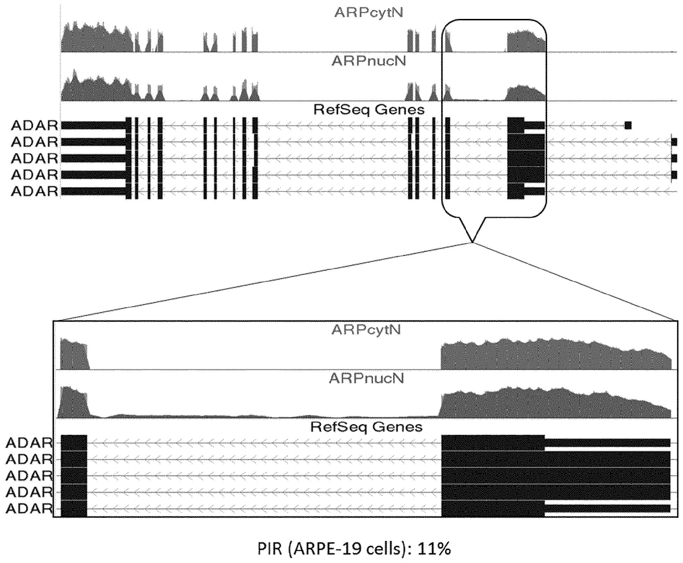

**FIG. 3** depicts a schematic of the ReSeq Genes for *ADAR* intron 2 corresponding to NM_001111. The Percent Intron Retention (PIR) of the circled intron is shown.

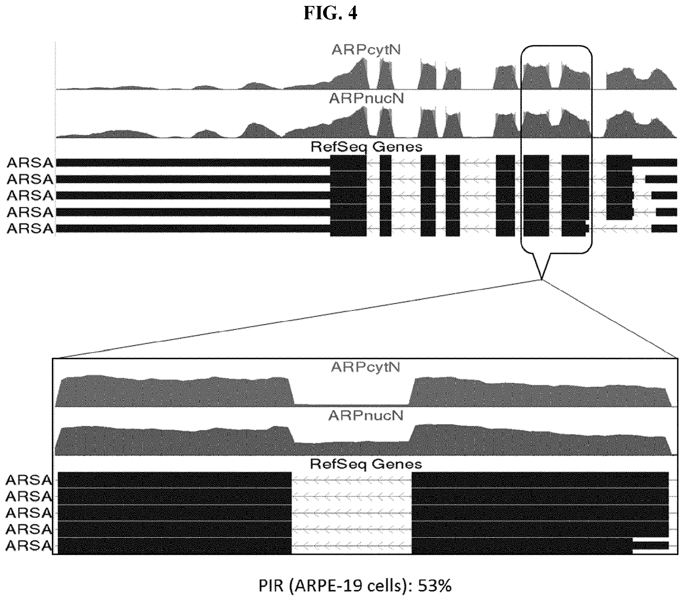

**FIG. 4** depicts a schematic of the ReSeq Genes for *ARSA* intron 3 corresponding to NM_001085425. The Percent Intron Retention (PIR) of the circled intron is shown.

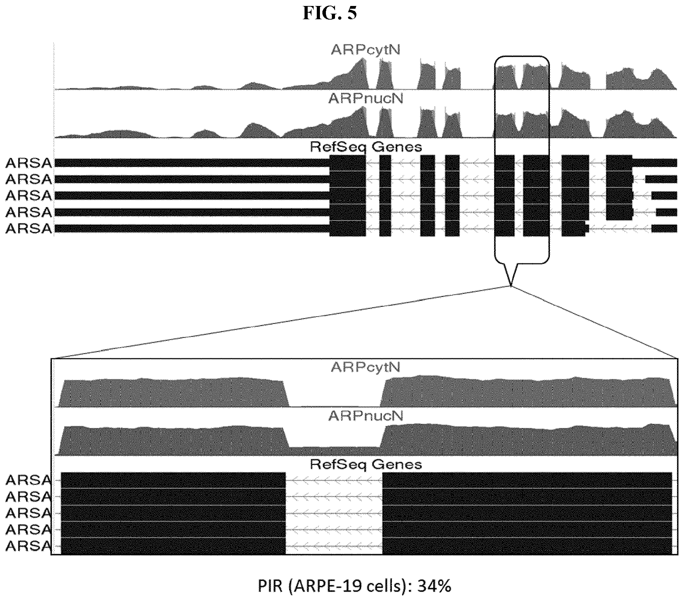

**FIG. 5** depicts a schematic of the ReSeq Genes for *ARSA* intron 4 corresponding to NM_001085425. The Percent Intron Retention (PIR) of the circled intron is shown.

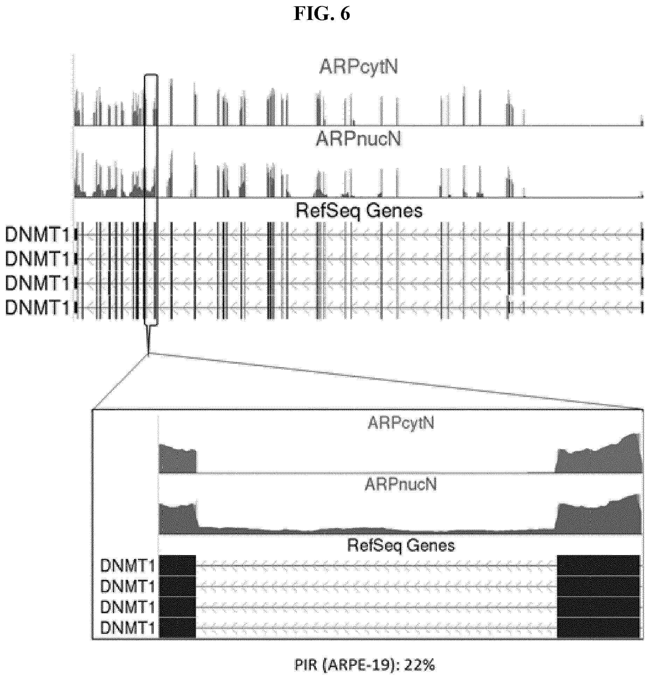

**FIG. 6** depicts a schematic of the ReSeq Genes for *DNMT1* intron 30 corresponding to NM_001130823. The Percent Intron Retention (PIR) of the circled intron is shown.

**FIG. 7** depicts an exemplary graph showing the average (n=3) fold change in expression levels of *DNMT1* mRNA without intron 30 in ARPE-19 cells treated for 24 hrs with 80 nM of the indicated ASOs over mock treated cells. Data is normalized to RPL32 expression.

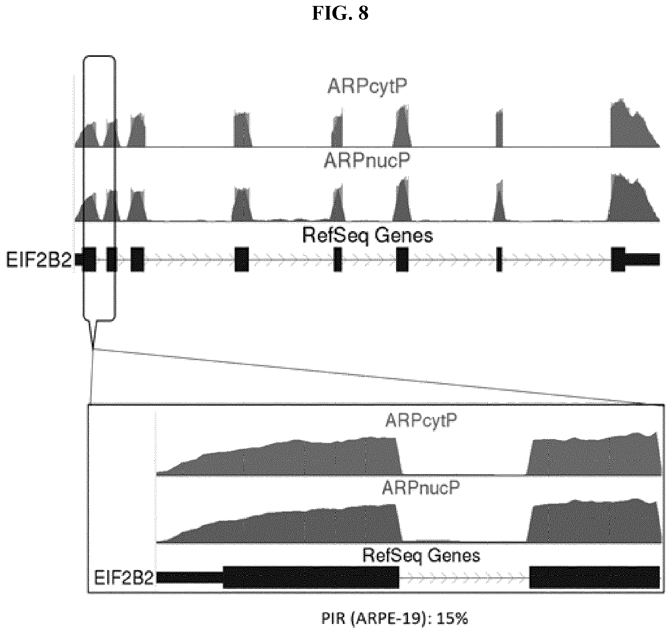

**FIG. 8** depicts a schematic of the ReSeq Genes for *EIF2B2* intron 1 corresponding to NM_014239. The Percent Intron Retention (PIR) of the circled intron is shown.

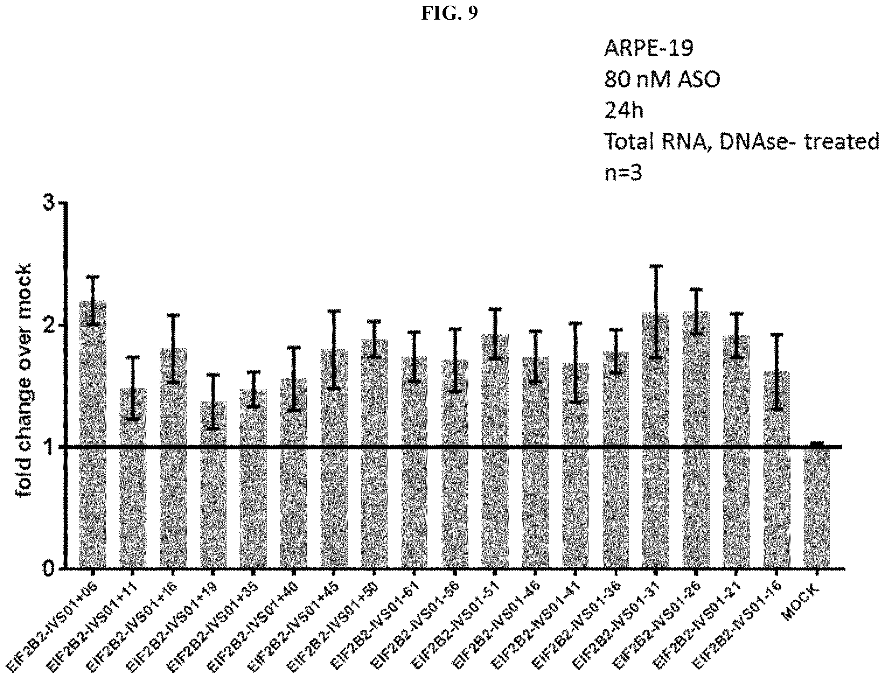

**FIG. 9** depicts an exemplary graph showing the average (n=3) fold change in expression levels of *EIF2B2* mRNA without intron 1 in ARPE-19 cells treated for 24 hrs with 80 nM of the indicated ASOs over mock treated cells. Data is normalized to RPL32 expression.

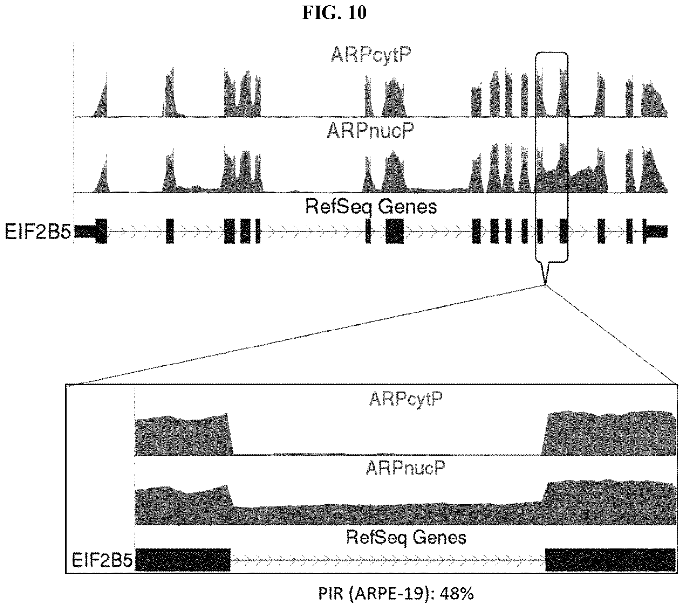

**FIG. 10** depicts a schematic of the ReSeq Genes for *EIF2B5* intron 12 corresponding to NM_003907. The Percent Intron Retention (PIR) of the circled intron is shown.

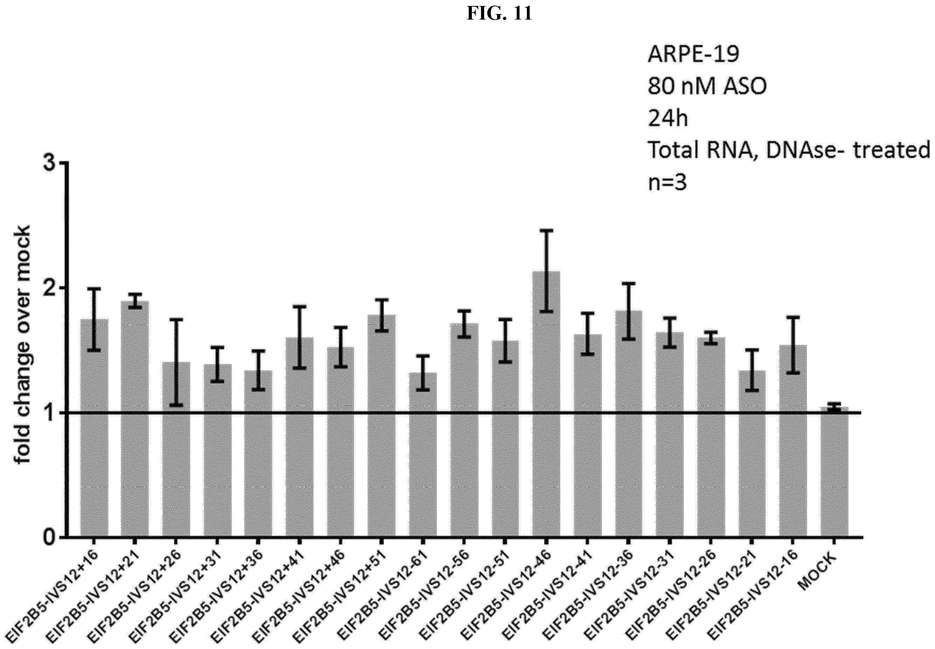

**FIG. 11** depicts an exemplary graph showing the average (n=3) fold change in expression levels of *EIF2B5* mRNA without intron 12 in ARPE-19 cells treated for 24 hrs with 80 nM of the indicated ASOs over mock treated cells. Data is normalized to RPL32 expression.

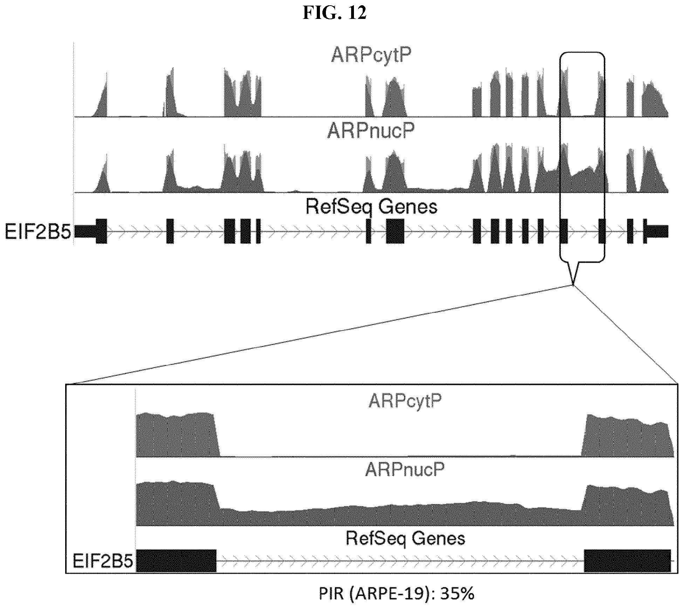

**FIG. 12** depicts a schematic of the ReSeq Genes for *EIF2B5* intron 13 corresponding to NM_003907. The Percent Intron Retention (PIR) of the circled intron is shown.

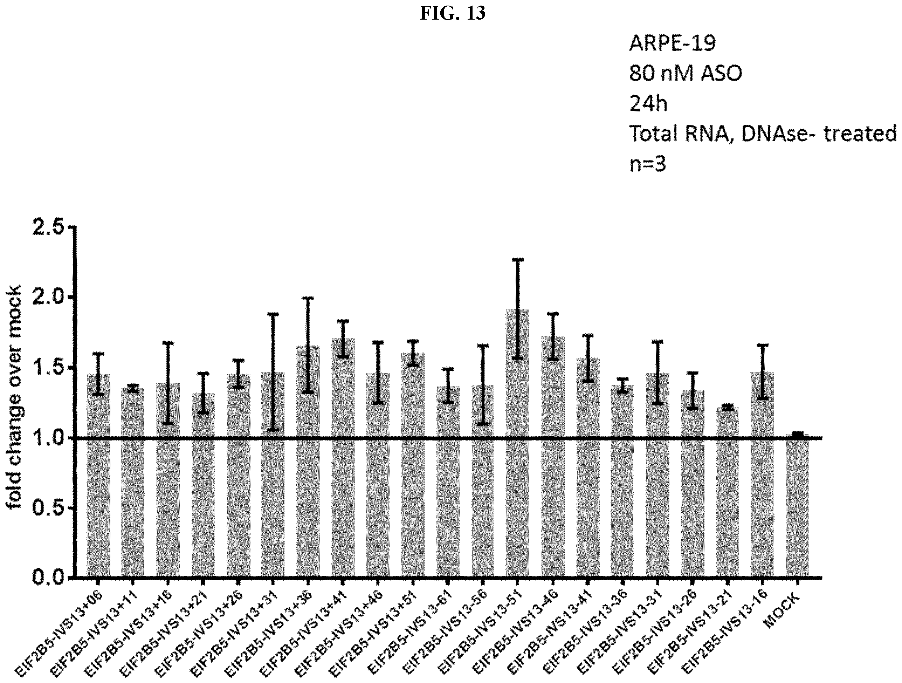

**FIG. 13** depicts an exemplary graph showing the average (n=3) fold change in expression levels of *EIF2B5* mRNA without intron 13 in ARPE-19 cells treated for 24 hrs with 80 nM of the indicated ASOs over mock treated cells. Data is normalized to RPL32 expression.

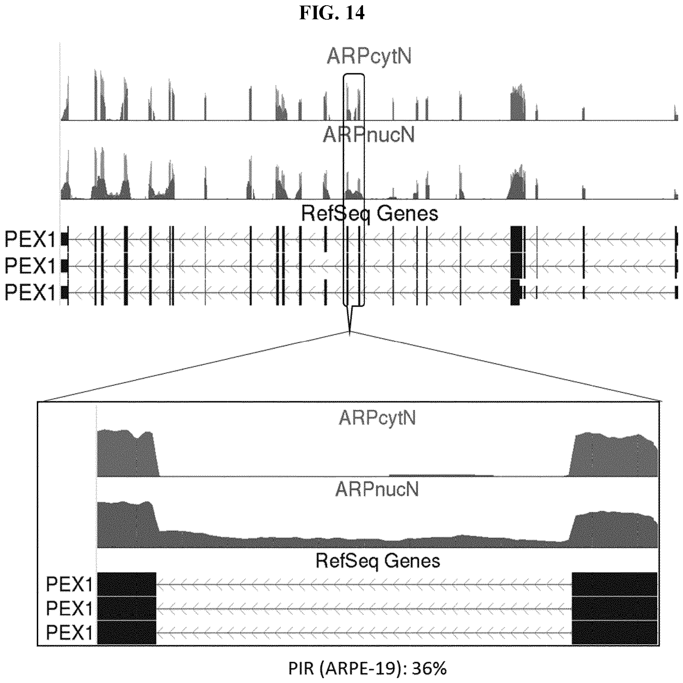

**FIG. 14** depicts a schematic of the ReSeq Genes for *PEX1* intron 10 corresponding to NM_000466. The Percent Intron Retention (PIR) of the circled intron is shown.

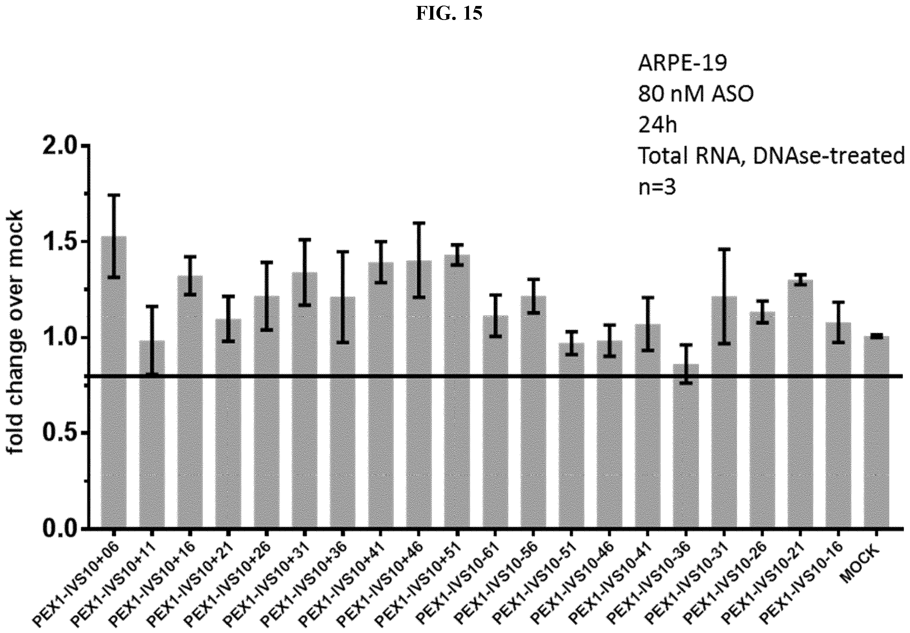

**FIG. 15** depicts an exemplary graph showing the average (n=3) fold change in expression levels of *PEX1* mRNA without intron 10 in ARPE-19 cells treated for 24 hrs with 80 nM of the indicated ASOs over mock treated cells. Data is normalized to RPL32 expression.

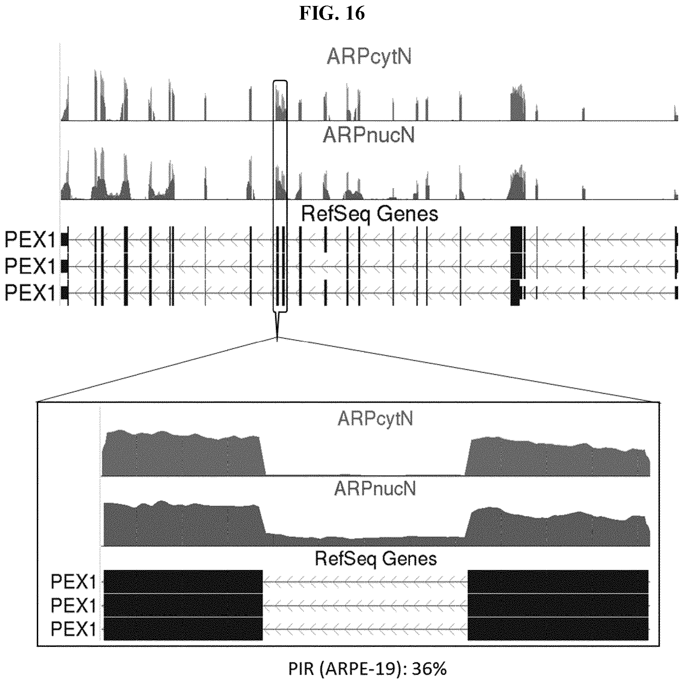

**FIG. 16** depicts a schematic of the ReSeq Genes for *PEX1* intron 14 corresponding to NM_000466. The Percent Intron Retention (PIR) of the circled intron is shown.

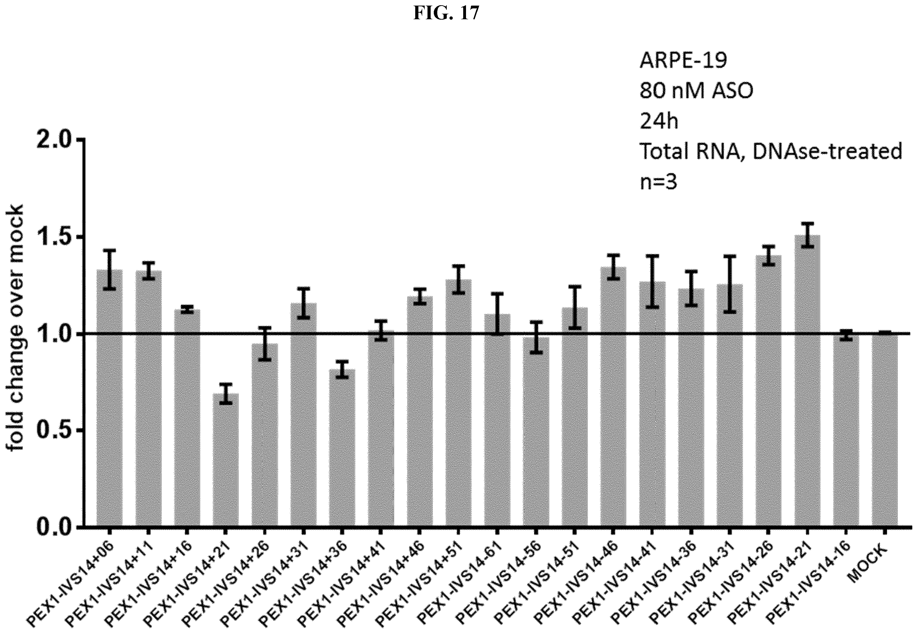

**FIG. 17** depicts an exemplary graph showing the average (n=3) fold change in expression levels of *PEX1* mRNA without intron 14 in ARPE-19 cells treated for 24 hrs with 80 nM of the indicated ASOs over mock treated cells. Data is normalized to RPL32 expression.

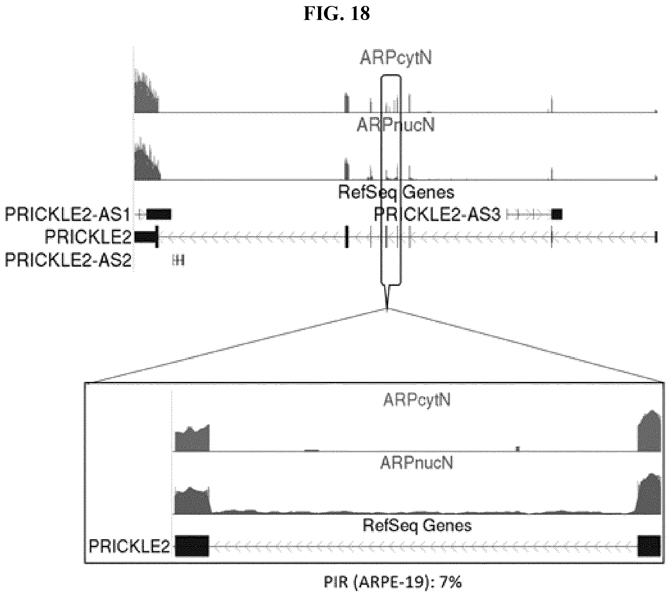

**FIG. 18** depicts a schematic of the ReSeq Genes for *PRICKLE2* intron 4 corresponding to NM_198859. The Percent Intron Retention (PIR) of the circled intron is shown.

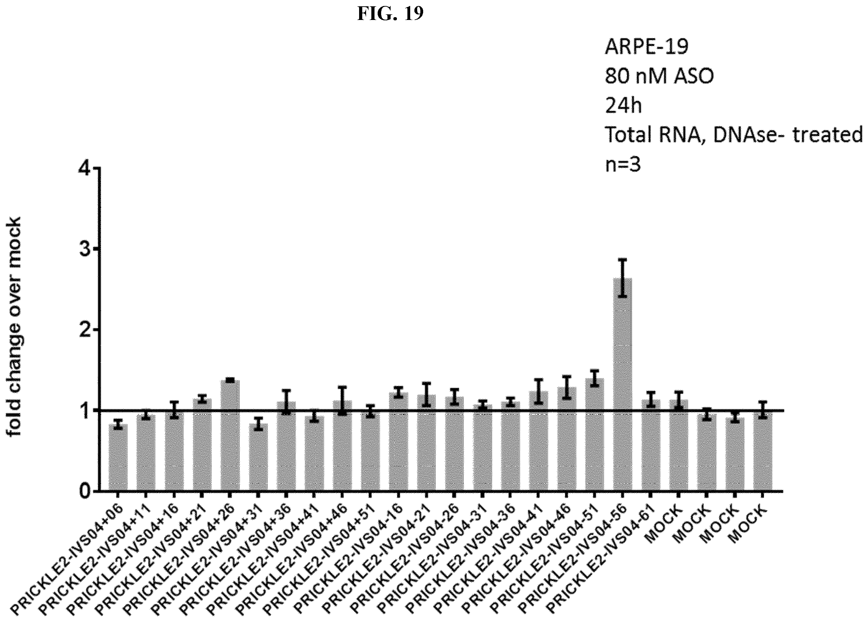

**FIG. 19** depicts an exemplary graph showing the average (n=3) fold change in expression levels of *PRICKLE2* mRNA without intron 4 in ARPE-19 cells treated for 24 hrs with 80 nM of the indicated ASOs over mock treated cells. Data is normalized to RPL32 expression.

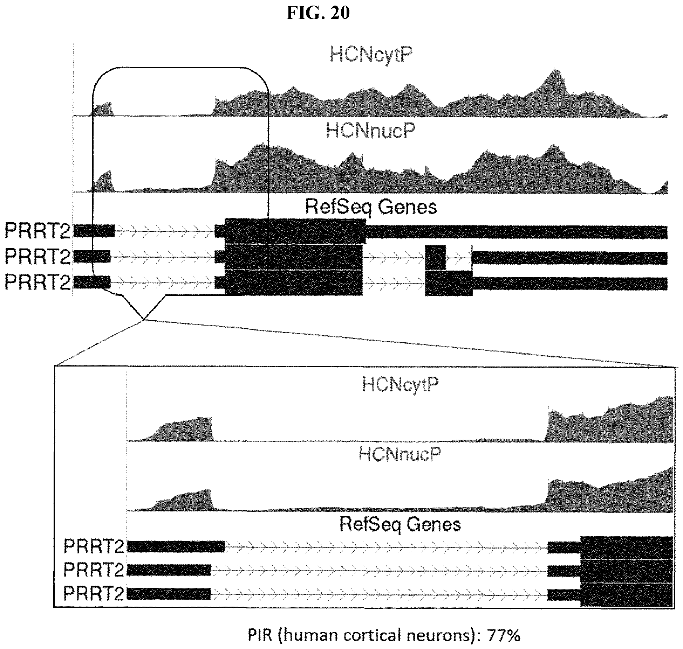

**FIG. 20** depicts a schematic of the ReSeq Genes for *PRRT2* intron 1 corresponding to NM_145239. The Percent Intron Retention (PIR) of the circled intron is shown.

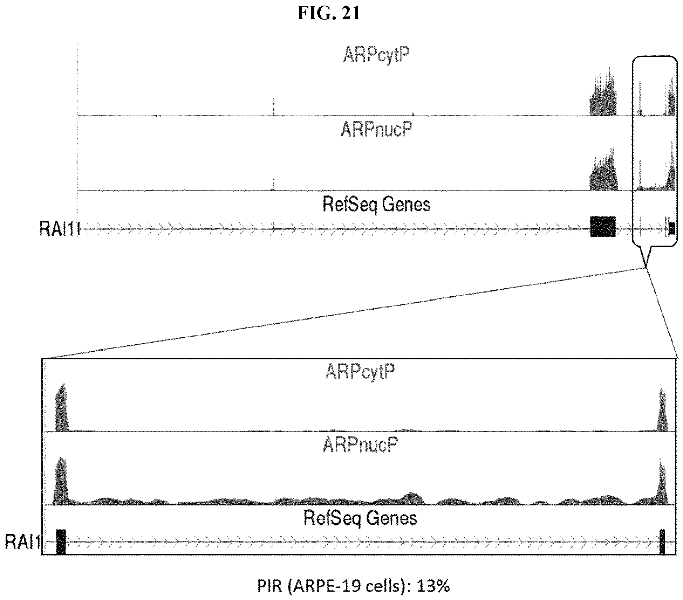

**FIG. 21** depicts a schematic of the ReSeq Genes for *RAI1* intron 4 corresponding to NM_030665. The Percent Intron Retention (PIR) of the circled intron is shown.

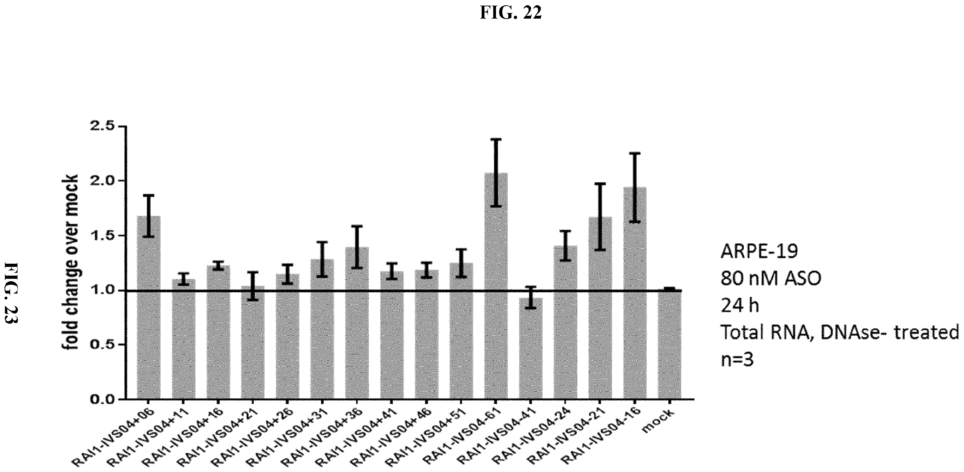

**FIG. 22** depicts an exemplary graph showing the average (n=3) fold change in expression levels of *RAI1* mRNA without intron 4 in ARPE-19 cells treated for 24 hrs with 80 nM of the indicated ASOs over mock treated cells. Data is normalized to RPL32 expression.

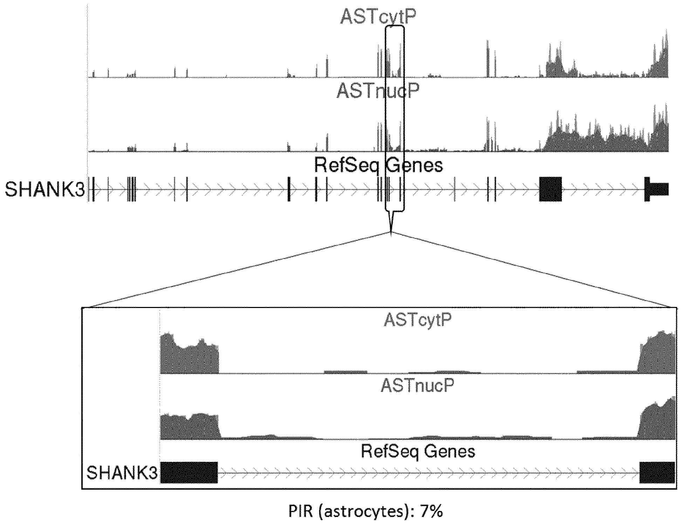

**FIG. 23** depicts a schematic of the ReSeq Genes for *SHANK3* intron 16 corresponding to NM_033517. The Percent Intron Retention (PIR) of the circled intron is shown.

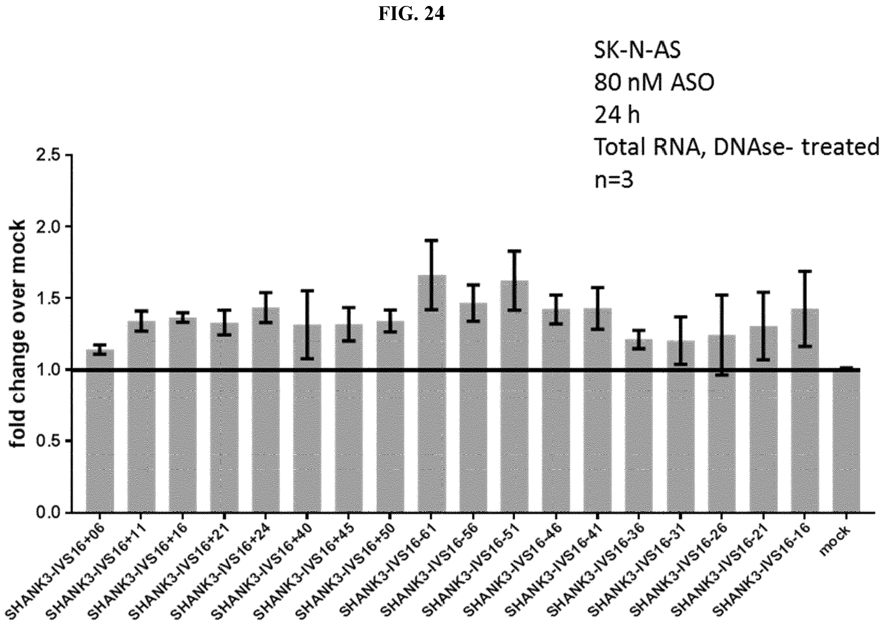

**FIG. 24** depicts an exemplary graph showing the average (n=3) fold change in expression levels of *SHANK3* mRNA without intron 16 in ARPE-19 cells treated for 24 hrs with 80 nM of the indicated ASOs over mock treated cells. Data is normalized to RPL32 expression.

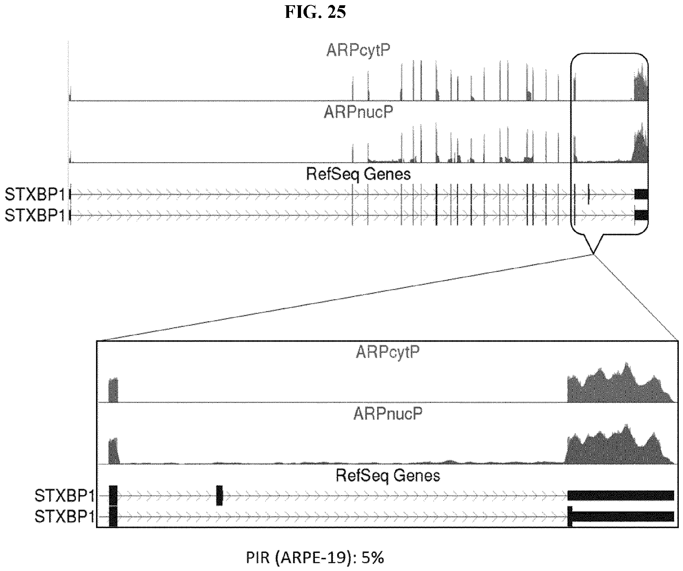

**FIG. 25** depicts a schematic of the ReSeq Genes for *STXBP1* intron 18 corresponding to NM_001032221. The Percent Intron Retention (PIR) of the circled intron is shown.

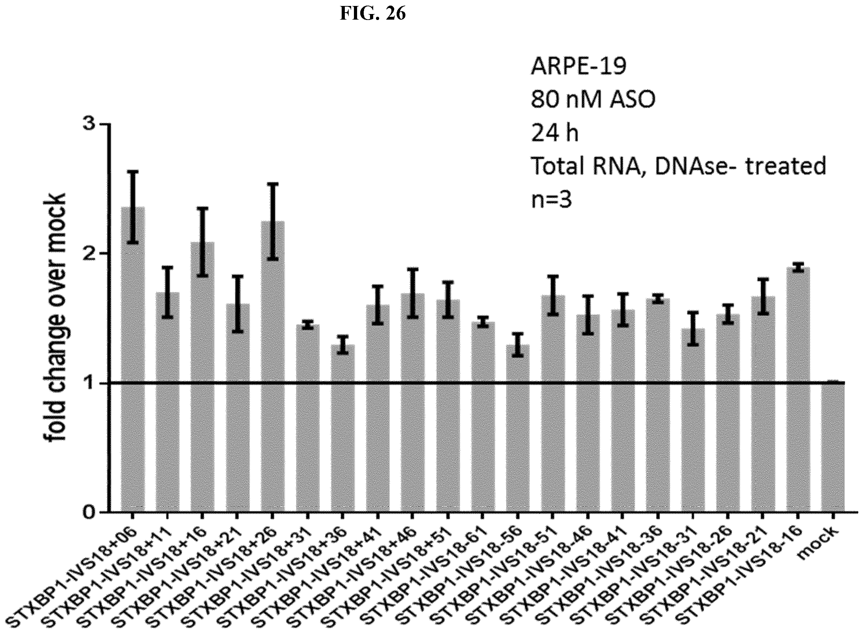

**FIG. 26** depicts an exemplary graph showing the average (n=3) fold change in expression levels of *STXBP1* mRNA without intron 18 in ARPE-19 cells treated for 24 hrs with 80 nM of the indicated ASOs over mock treated cells. Data is normalized to RPL32 expression.

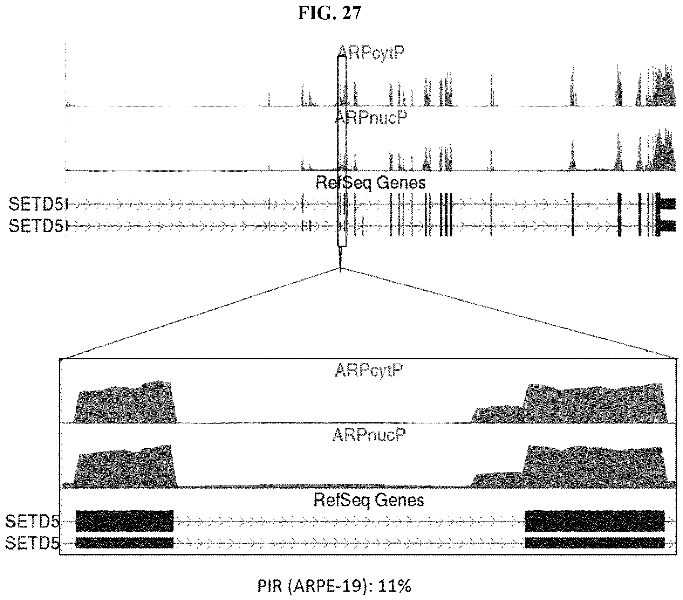

**FIG. 27** depicts a schematic of the ReSeq Genes for *SETD5* intron 4 corresponding to NM_001080517. The Percent Intron Retention (PIR) of the circled intron is shown.

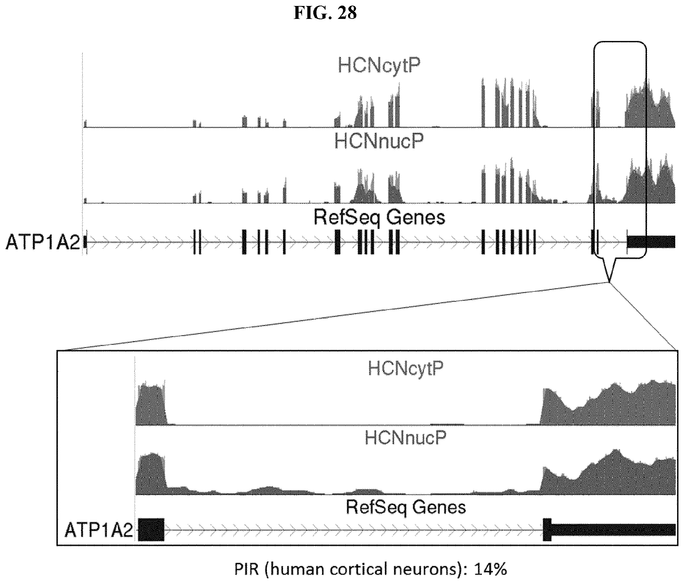

**FIG. 28** depicts a schematic of the ReSeq Genes for *ATP1A2* intron 22 corresponding to NM_000702. The Percent Intron Retention (PIR) of the circled intron is shown.

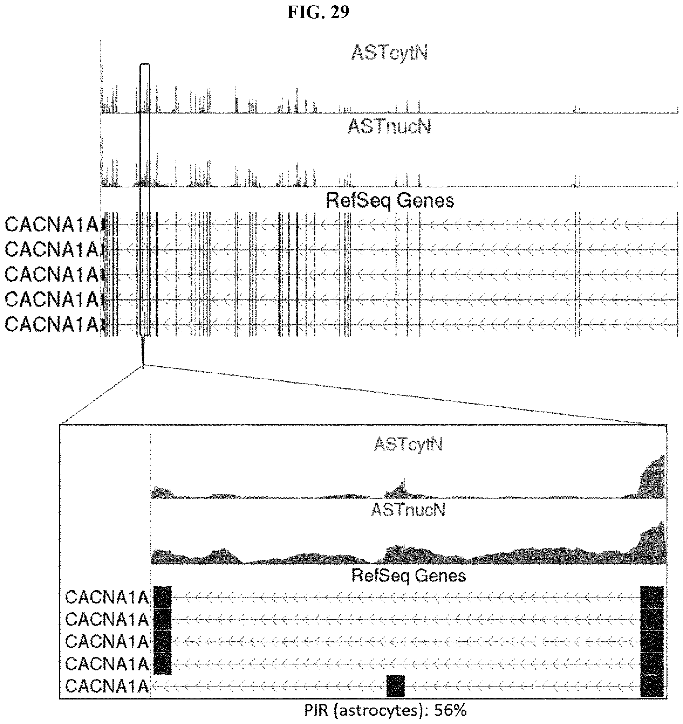

**FIG. 29** depicts a schematic of the ReSeq Genes for *CACNA1A* intron 36 corresponding to NM_023035. The Percent Intron Retention (PIR) of the circled intron is shown.

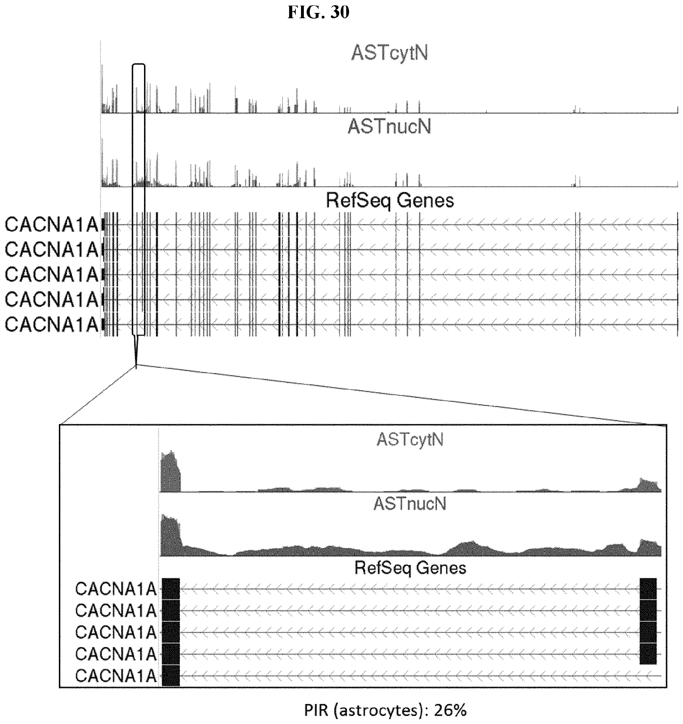

**FIG. 30** depicts a schematic of the ReSeq Genes for *CACNA1A* intron 37 corresponding to NM_023035. The Percent Intron Retention (PIR) of the circled intron is shown.

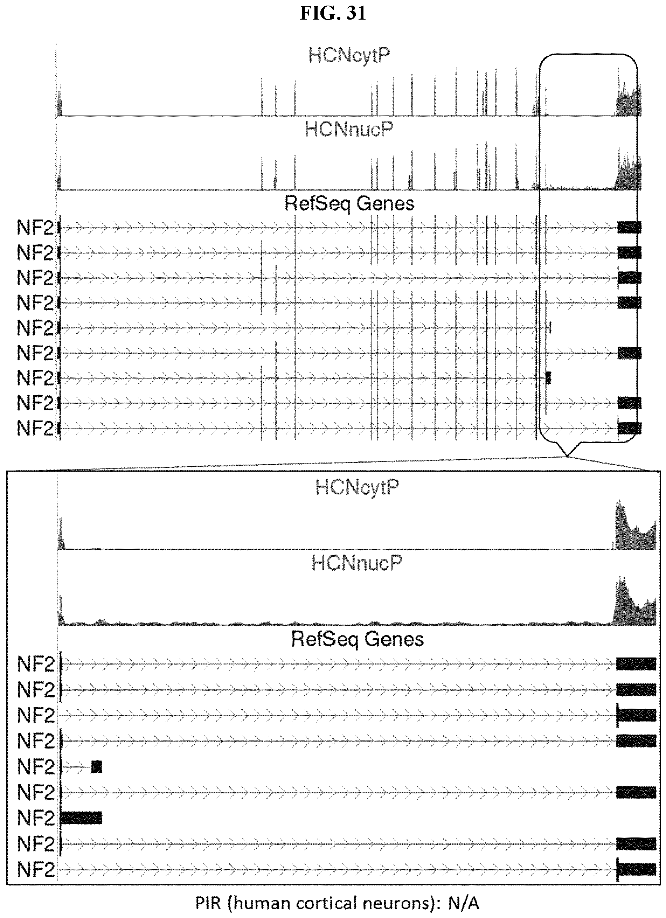

**FIG. 31** depicts a schematic of the ReSeq Genes for *NF2* intron 16 corresponding to NM_181832. The Percent Intron Retention (PIR) of the circled intron is shown.

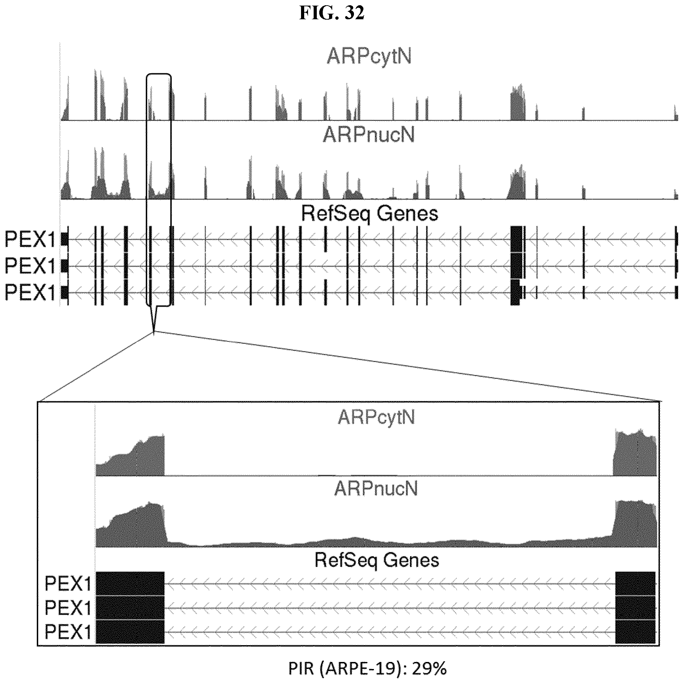

**FIG. 32** depicts a schematic of the ReSeq Genes for *PEX1* intron 19 corresponding to NM_000466. The Percent Intron Retention (PIR) of the circled intron is shown.

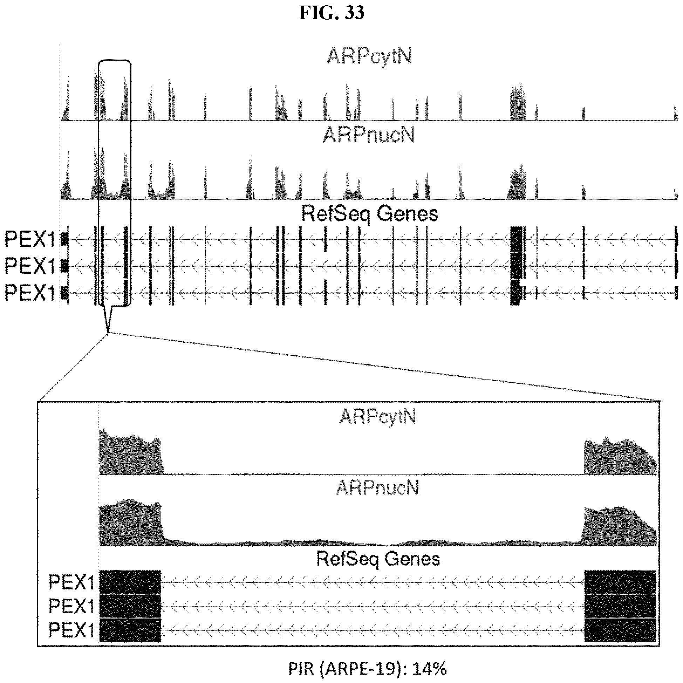

**FIG. 33** depicts a schematic of the ReSeq Genes for *PEX1* intron 21 corresponding to NM_000466. The Percent Intron Retention (PIR) of the circled intron is shown.

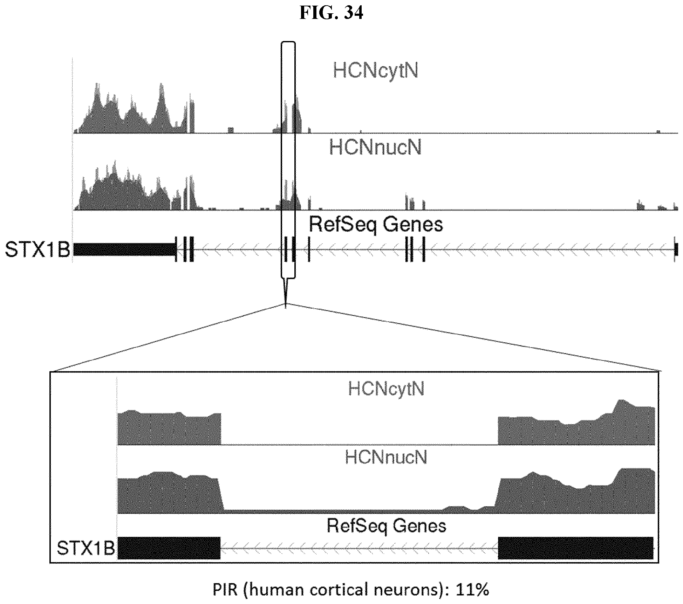

**FIG. 34** depicts a schematic of the ReSeq Genes for *STX1B* intron 6 corresponding to NM_052874. The Percent Intron Retention (PIR) of the circled intron is shown.

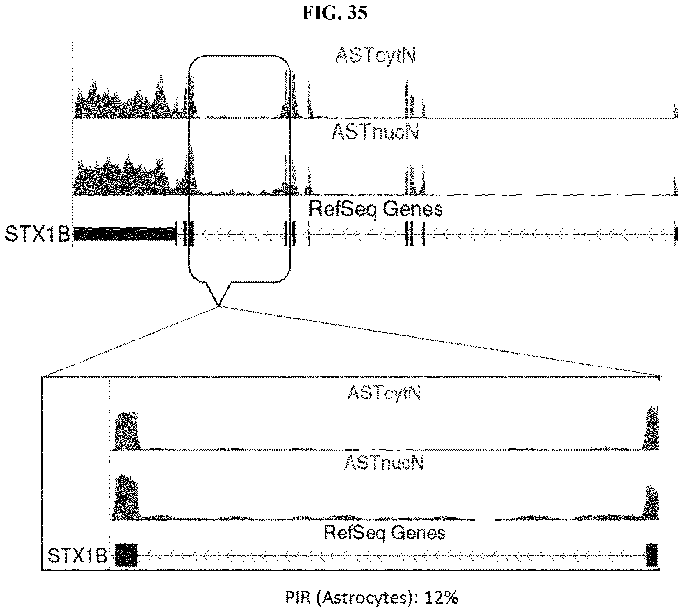

**FIG. 35** depicts a schematic of the ReSeq Genes for *STX1B* intron 7 corresponding to NM_052874. The Percent Intron Retention (PIR) of the circled intron is shown.

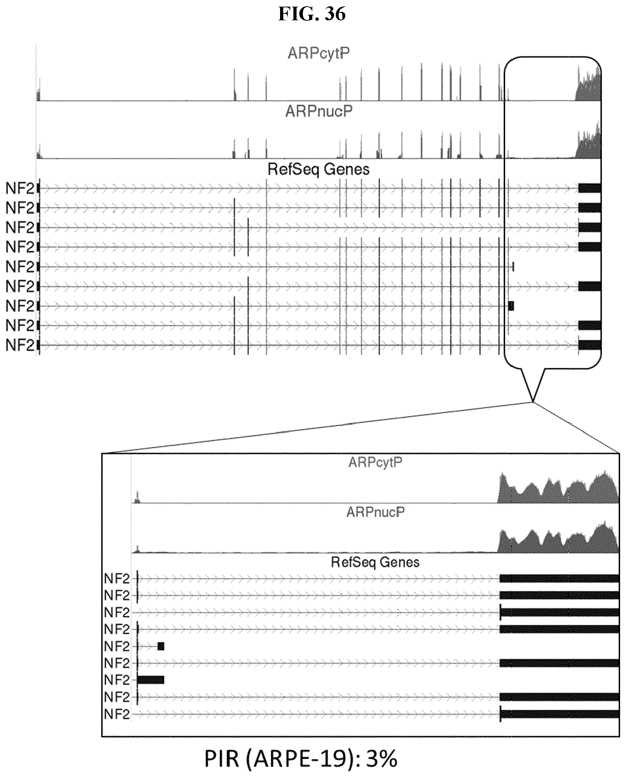

**FIG. 36** depicts a schematic of the ReSeq Genes for *NF2* intron 16 corresponding to NM_181832. The Percent Intron Retention (PIR) of the circled intron is shown.

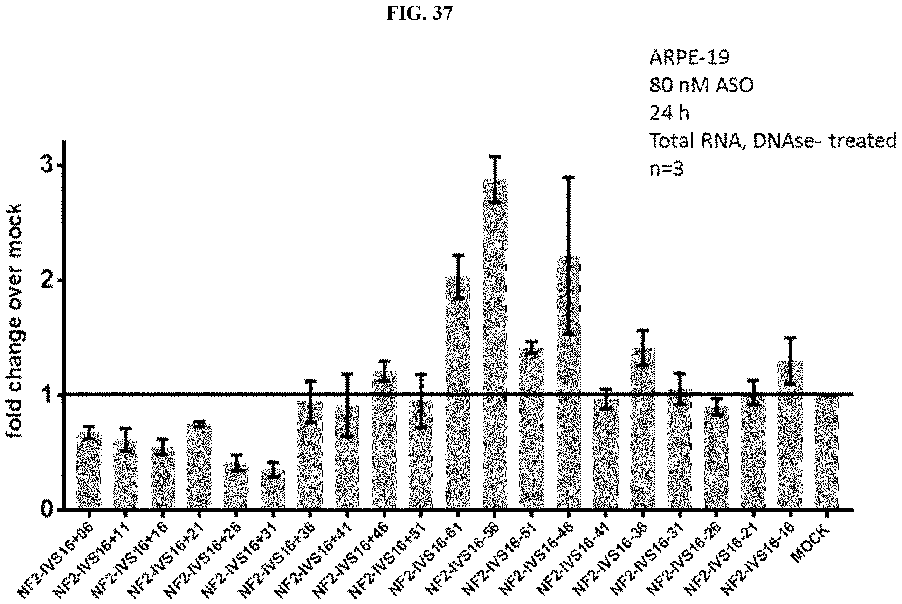

**FIG. 37** depicts an exemplary graph showing the average (n=3) fold change in expression levels of *NF2* mRNA without intron 16 in ARPE-19 cells treated for 24 hrs with 80 nM of the indicated ASOs over mock treated cells. Data is normalized to RPL32 expression.

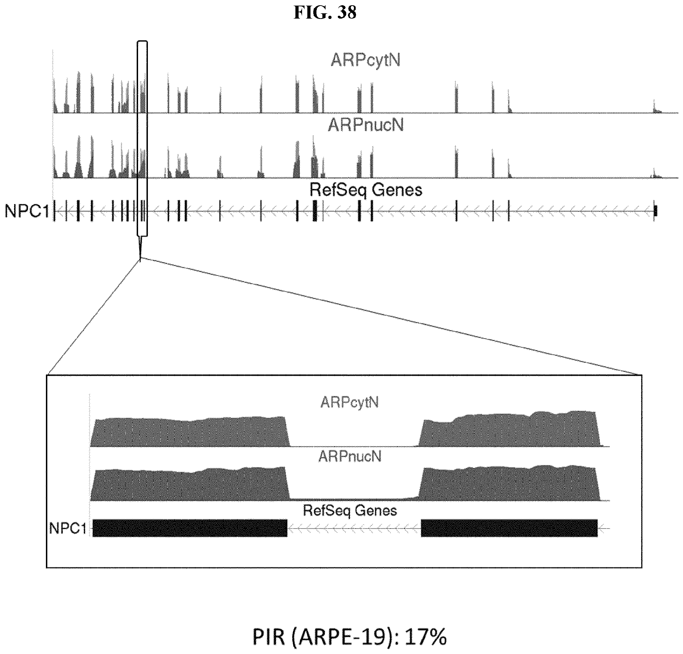

**FIG. 38** depicts a schematic of the ReSeq Genes for *NPC1* intron 15 corresponding to NM_000271. The Percent Intron Retention (PIR) of the circled intron is shown.

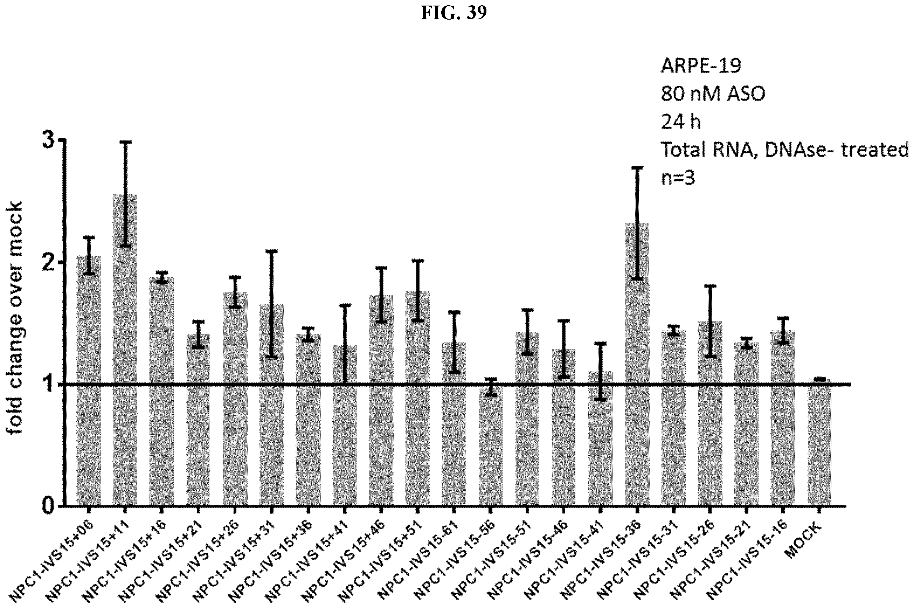

**FIG. 39** depicts an exemplary graph showing the average (n=3) fold change in expression levels of *NPC1* mRNA without intron 15 in ARPE-19 cells treated for 24 hrs with 80 nM of the indicated ASOs over mock treated cells. Data is normalized to RPL32 expression.

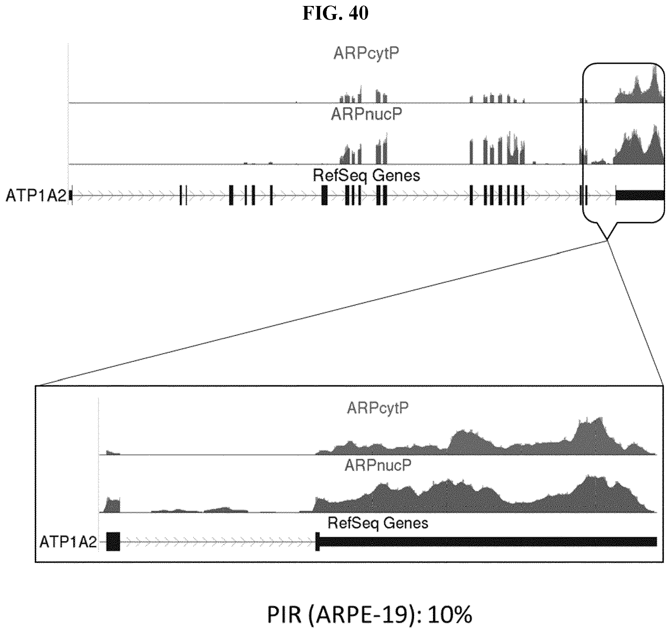

**FIG. 40** depicts a schematic of the ReSeq Genes for *ATP1A2* intron 22 corresponding to NM_000702. The Percent Intron Retention (PIR) of the circled intron is shown.

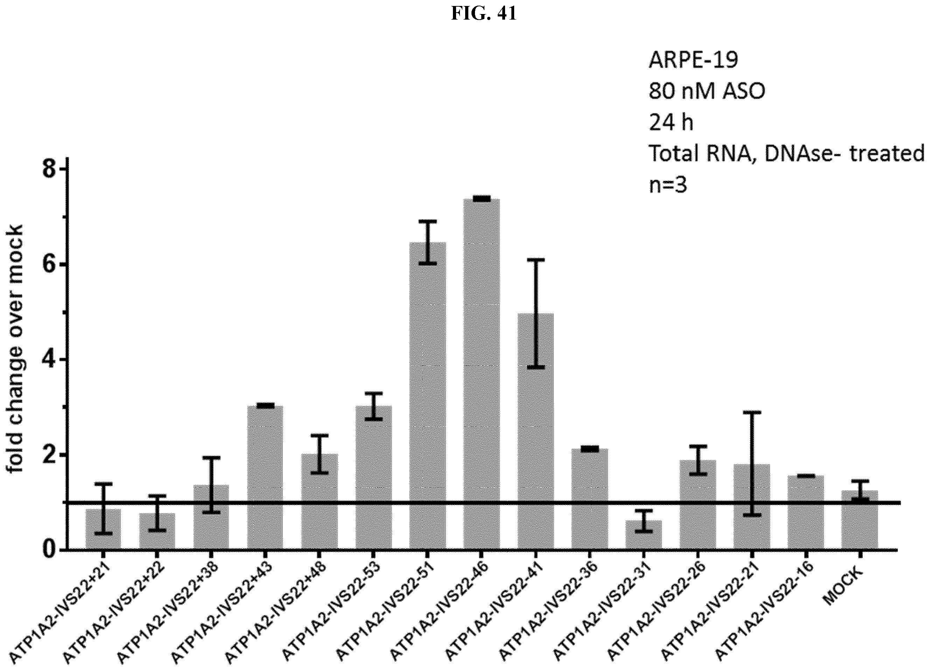

**FIG. 41** depicts an exemplary graph showing the average (n=3) fold change in expression levels of *ATP1A2* mRNA without intron 22 in ARPE-19 cells treated for 24 hrs with 80 nM of the indicated ASOs over mock treated cells. Data is normalized to RPL32 expression.

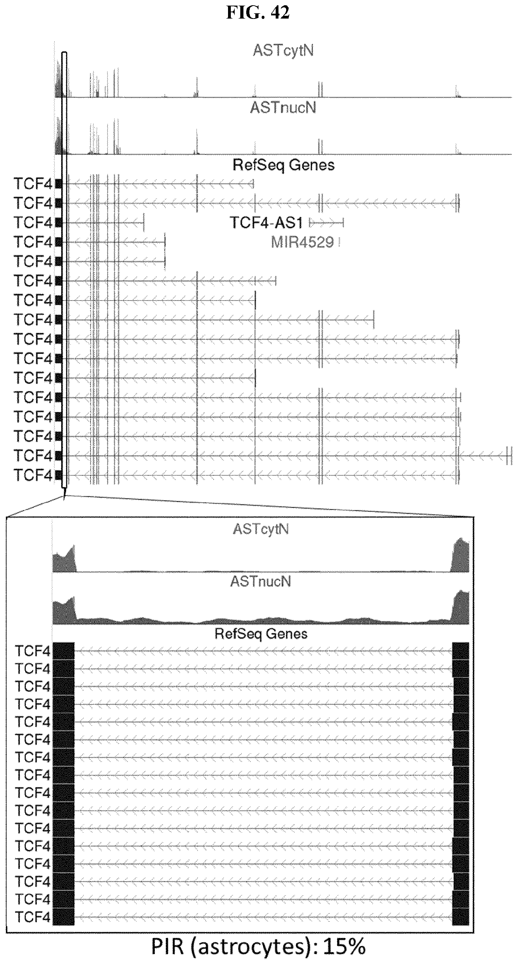

**FIG. 42** depicts a schematic of the ReSeq Genes for *TCF4* intron 18 corresponding to NM_001243226. The Percent Intron Retention (PIR) of the circled intron is shown.

Table 2 illustrates percentage of retained intron with respect to the genes of interest.

**Table 2: Retained Introns in Genes of Interest**

| **Gene Symbol** | **Gene ID** | **RNA Accession Number** | **Retained Intron (Percent Intron Retention)** | **Cells** |
|---|---|---|---|---|
| ATP1A2 | 477 | NM_000702 | Intron 22 (14%) | HCN |
| CACNA1A | 773 | NM_023035 | Intron 37 (26%) Intron 36 (56%) | AST |
| CACNA1A | 773 | NM_001127221 | Intron 36 (55%) | AST |
| SETD5 | 55209 | NM_001080517 | Intron 4 (42%) | HCN |
| SHANK3 | 85358 | NM_033517 | Intron 16 (7%) | AST |
| NF2 | 4771 | NM_181832 | Intron 16 (N/A) | HCN |
| DNMT1 | 1786 | NM_001379 | Intron 29 (13%) | HCN |
| TCF4 | 6925 | NM_003199 | Intron 17 (15%) | AST |
| TCF4 | 6925 | NM_001243228 | Intron 17 (29%) | AST |
| RAI1 | 10743 | NM_030665 | Intron 4 (13%) | AST |
| PEX1 | 5189 | NM_000466 | Intron 10 (25%) Intron 14 (19%) Intron 19 (18%) Intron 21 (14%) | HCN |
| ARSA | 410 | NM_001085425 | Intron 3 (N/A) Intron 4 (N/A) | HCN |
| EIF2B5 | 8893 | NM_003907 | Intron 12 (42%) Intron 13 (29%) | HCN |
| EIF2B 1 | 1967 | NM_001414 | Intron 6 (27%) | HCN |
| EIF2B2 | 8892 | NM_014239 | Intron 1 (9%) | HCN |
| NPC1 | 4864 | NM_000271 | Intron 15 (11%) | HCN |
| ADAR | 103 | NM_001111 | Intron 2 (12%) | AST |
| MFSD8 | 256471 | NM_152778 | Intron 12 (42%) | HCN |
| STXBP1 | 6812 | NM_001032221 | Intron 18 (N/A) | AST |
| PRICKLE2 | 166336 | NM_198859 | Intron 4 (19%) | AST |
| PRRT2 | 112476 | NM_145239 | Intron 1 (77%) | HCN |
| STX1B | 112755 | NM_052874 | Intron 6 (11%) Intron 7 (12%) | HCN, AST |
| IDUA | 607015 | NM_000203 | Intron 3 (25%) Intron 4 (63%) Intron 5 (16%) Intron 6 (N/A) Intron 7 (N/A) | HCN |

The invention includes the following aspects:
1. A method of treating a CNS disease in a subject in need thereof by increasing the expression of a target protein or functional RNA by cells of the subject, wherein the cells have a retained-intron-containing pre-mRNA (RIC pre-mRNA), the RIC pre-mRNA comprising a retained intron, an exon flanking the 5' splice site, an exon flanking the 3' splice site, and wherein the RIC pre-mRNA encodes the target protein or functional RNA, the method comprising contacting the cells of the subject with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of the target protein or functional RNA in the cells of the subject.
2. The method of aspect 1, wherein the CNS disease is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.
3. A method of increasing expression of a target protein, wherein the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B, by cells having a retained-intron-containing pre-mRNA (RIC pre-mRNA), the RIC pre-mRNA comprising a retained intron, an exon flanking the 5' splice site of the retained intron, an exon flanking the 3' splice site of the retained intron, and wherein the RIC pre-mRNA encodes ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, the method comprising contacting the cells with an antisense oligomer (ASO) complementary to a targeted portion of the RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, whereby the retained intron is constitutively spliced from the RIC pre-mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, thereby increasing the level of mRNA encoding ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein, and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B expression by improving splicing efficiency of ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, MFSD8, IDUA, or STX1B protein in the cells.
4. The method of aspect 1 or 2, wherein the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B.
5. The method of aspect 1 or 2, wherein the target protein is ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B.
6. The method of aspect 1 or 2, wherein the target protein or the functional RNA is a compensating protein or a compensating functional RNA that functionally augments or replaces a target protein or functional RNA that is deficient in amount or activity in the subject.
7. The method of aspect 3, wherein the cells are in or from a subject having a condition caused by a deficient amount or activity of ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B protein.
8. The method of aspect 3, wherein the cells are in or from a subject having a condition caused by a deficient amount or activity of ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B protein.
9. The method of any one of aspects 1 to 8, wherein the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a RIC pre-mRNA transcribed from the first allele.
10. The method of any one of aspects 1 to 8, wherein the subject has a condition caused by a disorder resulting from a deficiency in the amount or function of the target protein, wherein the subject has
   (a) a first mutant allele from which
      (i) the target protein is produced at a reduced level compared to production from a wild-type allele,
      (ii) the target protein is produced in a form having reduced function compared to an equivalent wild-type protein, or
      (iii) the target protein is not produced, and
   (b) a second mutant allele from which
      (i) the target protein is produced at a reduced level compared to production from a wild-type allele,
      (ii) the target protein is produced in a form having reduced function compared to an equivalent wild-type protein, or
      (iii) the target protein is not produced, and
      wherein when the subject has a first mutant allele a(iii), the second mutant allele is b(i) or b(ii), and wherein when the subject has a second mutant allele b(iii), the first mutant allele is a(i) or a(ii), and wherein the RIC pre-mRNA is transcribed from either the first mutant allele that is a(i) or a(ii), and/or the second allele that is b(i) or b(ii).
11. The method of aspect 10, wherein the target protein is produced in a form having reduced function compared to the equivalent wild-type protein.
12. The method of aspect 10, wherein the target protein is produced in a form that is fully-functional compared to the equivalent wild-type protein.
13. The method of any one of aspects 1 to 12, wherein the targeted portion of the RIC pre-mRNA is in the retained intron within the region +6 relative to the 5' splice site of the retained intron to -16 relative to the 3' splice site of the retained intron.
14. The method of any one of aspects 1 to 12, wherein the targeted portion of the RIC pre-mRNA is in the retained intron within:
   (a) the region +6 to +4000, +6 to + 3000, +6 to +2000 or +6 to +100 relative to the 5' splice site of the retained intron; or
   (b) the region -16 to -4000, -16 to -2000, -16 to -1000, -16 to -500 or -16 to -100 relative to the 3' splice site of the retained intron.
15. The method of any one of aspects 1 to 12, wherein the targeted portion of the RIC pre-mRNA is within:
   (a) the region +2e to -4e in the exon flanking the 5' splice site of the retained intron; or
   (b) the region +2e to -4e in the exon flanking the 3' splice site of the retained intron.
16. The method of any one of aspects 1 to 15, wherein the RIC pre-mRNA is encoded by a genetic sequence with at least about 80%, 85%, 90%, 95%, 96%, 97%, 98%, 99% or 100% sequence identity to SEQ ID NOs: 1-19.
17. The method of any one of aspects 1 to 16, wherein the RIC pre-mRNA comprises a sequence with at least about 80%, 85%, 90%, 95%, 96%, 97%, 98%, 99% or 100% sequence identity to any one of SEQ ID NOs: 20-80.
18. The method of any one of aspects 1 to 17, wherein the antisense oligomer does not increase the amount of the target protein or the functional RNA by modulating alternative splicing of pre-mRNA transcribed from a gene encoding the functional RNA or target protein.
19. The method of any one of aspects 1 to 18, wherein the antisense oligomer does not increase the amount of the target protein or the functional RNA by modulating aberrant splicing resulting from mutation of the gene encoding the target protein or the functional RNA.
20. The method of any one of aspects 1 to 19, wherein the RIC pre-mRNA was produced by partial splicing of a full-length pre-mRNA or partial splicing of a wild-type pre-mRNA.
21. The method of any one of aspects 1 to 20, wherein the mRNA encoding the target protein or functional RNA is a full-length mature mRNA, or a wild-type mature mRNA.
22. The method of any one of aspects 1 to 21, wherein the target protein produced is full-length protein, or wild-type protein.
23. The method of any one of aspects 1 to 22, wherein the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell.
24. The method of any one of aspects 1 to 23, wherein the total amount of target protein produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of target protein produced by a control cell.
25. The method of any one of aspects 1 to 24, wherein the antisense oligomer comprises a backbone modification comprising a phosphorothioate linkage or a phosphorodiamidate linkage.
26. The method of any one of aspects 1 to 25, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
27. The method of any one of aspects 1 to 26, wherein the antisense oligomer comprises at least one modified sugar moiety.
28. The method of aspect 27, wherein each sugar moiety is a modified sugar moiety.
29. The method of any one of aspects 1 to 28, wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
30. The method of any one of aspects 1 to 29, wherein the antisense oligomer is at least 80%, at least 85%, at least 90%, at least 95%, at least 98%, at least 99%, or 100%, complementary to the targeted portion of the RIC pre-mRNA encoding the protein.
31. The method of any one of aspects 1 to 30, wherein the targeted portion of the RIC pre-mRNA is within a sequence selected from SEQ ID NOs: 24351-24354, 24356-24378, 24380-24386, or 28515-28516.
32. The method of any one of aspects 1 to 31, wherein the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
33. The method of any one of aspects 1 to 31, wherein the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
34. The method of any one of aspects 1 to 33, wherein the cell comprises a population of RIC pre-mRNAs transcribed from the gene encoding the target protein or functional RNA, wherein the population of RIC pre-mRNAs comprises two or more retained introns, and wherein the antisense oligomer binds to the most abundant retained intron in the population of RIC pre-mRNAs.
35. The method of aspect 34, whereby the binding of the antisense oligomer to the most abundant retained intron induces splicing out of the two or more retained introns from the population of RIC pre-mRNAs to produce mRNA encoding the target protein or functional RNA.
36. The method of any one of aspects 1 to 33, wherein the cell comprises a population of RIC pre-mRNAs transcribed from the gene encoding the target protein or functional RNA, wherein the population of RIC pre-mRNAs comprises two or more retained introns, and wherein the antisense oligomer binds to the second most abundant retained intron in the population of RIC pre-mRNAs.
37. The method of aspect 36, whereby the binding of the antisense oligomer to the second most abundant retained intron induces splicing out of the two or more retained introns from the population of RIC pre-mRNAs to produce mRNA encoding the target protein or functional RNA.
38. The method of any one of aspects 7 to 37, wherein the condition is a disease or disorder.
39. The method of aspect 38, wherein the disease or disorder is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.
40. The method of aspect 39, wherein the target protein and the RIC pre-mRNA are encoded by the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene.
41. The method of any one of aspects 1 to 40, wherein the method further comprises assessing protein expression.
42. The method of any one of aspects 1 to 41, wherein the anti sense oligomer binds to a targeted portion of a ATP1A2, CACNA1A, SETD5, SHANK3, NF2, DNMT1, TCF4, RAI1, PEX1, ARSA, EIF2B5, , EIF2B2, NPC1, ADAR, STXBP1, PRICKLE2, PRRT2, or STX1B RIC pre-mRNA.
43. The method of any one of aspects 1 to 42, wherein the subject is a human.
44. The method of any one of aspects 1 to 42, wherein the subject is a non-human animal.
45. The method of any one of aspects 1 to 43, wherein the subject is a fetus, an embryo, or a child.
46. The method of any one of aspects 1 to 44, wherein the cells are *ex vivo.*
47. The method of any one of aspects 1 to 44, wherein the antisense oligomer is administered by intrathecal injection, intracerebroventricular injection, intraperitoneal injection, intramuscular injection, subcutaneous injection, or intravenous injection of the subject.
48. The method of any one of aspects 1 to 47, wherein the 9 nucleotides at -3e to -1e of the exon flanking the 5' splice site and +1 to +6 of the retained intron, are identical to the corresponding wild-type sequence.
49. The method of any one of aspects 1 to 48, wherein the 16 nucleotides at -15 to -1 of the retained intron and +1e of the exon flanking the 3' splice site are identical to the corresponding wild-type sequence.
50. An antisense oligomer as used in a method of any one of aspects 1 to 39.
51. An antisense oligomer comprising a sequence with at least about 80%, 85%, 90%, 95%, 97%, or 100% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
52. A pharmaceutical composition comprising the antisense oligomer of aspect 50 or 51 and an excipient.
53. A method of treating a subject in need thereof by administering the pharmaceutical composition of aspect 52 by intrathecal injection, intracerebroventricular injection, intraperitoneal injection, intramuscular injection, subcutaneous injection, or intravenous injection.
54. A composition comprising an antisense oligomer for use in a method of increasing expression of a target protein or a functional RNA by cells to treat Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smith-magenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9 in a subject in need thereof associated with a deficient protein or deficient functional RNA, wherein the deficient protein or deficient functional RNA is deficient in amount or activity in the subject, wherein the antisense oligomer enhances constitutive splicing of a retained intron-containing pre-mRNA (RIC pre-mRNA) encoding the target protein or the functional RNA, wherein the target protein is:
   (a) the deficient protein; or
   (b) a compensating protein which functionally augments or replaces the deficient protein or in the subject; and
   wherein the functional RNA is:
   (a) the deficient RNA; or
   (b) a compensating functional RNA which functionally augments or replaces the
      deficient functional RNA in the subject;
   wherein the RIC pre-mRNA comprises a retained intron, an exon flanking the 5' splice site and an exon flanking the 3' splice site, and wherein the retained intron is spliced from the RIC pre-mRNA encoding the target protein or the functional RNA, thereby increasing production or activity of the target protein or the functional RNA in the subject.
55. A composition comprising an antisense oligomer for use in a method of treating a condition associated with ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in a subject in need thereof, the method comprising the step of increasing expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein by cells of the subject, wherein the cells have a retained-intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, the method comprising contacting the cells with the antisense oligomer, whereby the retained intron is constitutively spliced from the RIC pre-mRNA transcripts encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, thereby increasing the level of mRNA encoding the target protein or functional RNA, and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, in the cells of the subject.
56. The composition of aspect 55, wherein the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B.
57. The composition of aspect 55, wherein the target protein is ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B.
58. The composition of aspect 55 or 56, wherein the condition is a disease or disorder.
59. The composition of aspect 58, wherein the disease or disorder is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smithmagenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.
60. The composition of aspect 59, wherein the target protein and RIC pre-mRNA are encoded by the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B gene.
61. The composition of aspect 59, wherein the target protein and RIC pre-mRNA are encoded by the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene.
62. The composition of any one of aspects 54 to 60, wherein the antisense oligomer targets a portion of the RIC pre-mRNA that is in the retained intron within the region +6 relative to the 5' splice site of the retained intron to -16 relative to the 3' splice site of the retained intron.
63. The composition of any one of aspects 54 to 62, wherein the antisense oligomer targets a portion of the RIC pre-mRNA that is in the retained intron within:
   (a) the region +6 to +100 relative to the 5' splice site of the retained intron; or
   (b) the region -16 to -100 relative to the 3' splice site of the retained intron.
64. The composition of any one of aspects 54 to 60, wherein the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron.
65. The composition of any one of aspects 54 to 60, wherein the targeted portion of the RIC pre-mRNA is within:
   (a) the region +2e to -4e in the exon flanking the 5' splice site of the retained intron; or
   (b) the region +2e to -4e in the exon flanking the 3' splice site of the retained intron.
66. The composition of any one of aspects 54 to 65, wherein the RIC pre-mRNA is encoded by a genetic sequence with at least about 80%, 85%, 90%, 95%, 96%, 97%, 98%, 99% or 100% sequence identity to SEQ ID NOs: 1-19.
67. The composition of any one of aspects 54 to 66, wherein the RIC pre-mRNA comprises a sequence with at least about 80%, 85%, 90%, 95%, 96%, 97%, 98%, 99% or 100% sequence identity to any one of SEQ ID NOs: 20-80.
68. The composition of any one of aspects 54 to 67, wherein the antisense oligomer does not increase the amount of target protein or functional RNA by modulating alternative splicing of the pre-mRNA transcribed from a gene encoding the target protein or functional RNA.
69. The composition of any one of aspects 54 to 68, wherein the antisense oligomer does not increase the amount of the functional RNA or functional protein by modulating aberrant splicing resulting from mutation of the gene encoding the target protein or faunctional RNA.
70. The composition of any one of aspects 54 to 69, wherein the RIC pre-mRNA was produced by partial splicing from a full-length pre-mRNA or a wild-type pre-mRNA.
71. The composition of any one of aspects 54 to 70, wherein the mRNA encoding the target protein or functional RNA is a full-length mature mRNA, or a wild-type mature mRNA.
72. The composition of any one of aspects 54 to 71, wherein the target protein produced is full-length protein, or wild-type protein.
73. The composition of any one of aspects 54 to 72, wherein the retained intron is a rate-limiting intron.
74. The composition of any one of aspects 54 to 73 wherein said retained intron is the most abundant retained intron in said RIC pre-mRNA.
75. The composition of any one of aspects 54 to 73, wherein the retained intron is the second most abundant retained intron in said RIC pre-mRNA.
76. The composition of any one of aspects 54 to 75, wherein the antisense oligomer comprises a backbone modification comprising a phosphorothioate linkage or a phosphorodiamidate linkage.
77. The composition of any one of aspects 54 to 76 wherein said antisense oligomer is an antisense oligonucleotide.
78. The composition of any one of aspects 54 to 77, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
79. The composition of any one of aspects 54 to 78, wherein the antisense oligomer comprises at least one modified sugar moiety.
80. The composition of aspect 79, wherein each sugar moiety is a modified sugar moiety.
81. The composition of any one of aspects 54 to 80, wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
82. The composition of any one of aspects 54 to 81, wherein the antisense oligomer is at least 80%, at least 85%, at least 90%, at least 95%, at least 98%, at least 99%, or is 100% complementary to the targeted portion of the RIC pre-mRNA encoding the protein.
83. The composition of any one of aspects 54 to 82, wherein the antisense oligomer binds to a targeted portion of a tuberin RIC pre-mRNA, wherein the targeted portion of the RIC pre-mRNA is within a sequence selected from SEQ ID NOs: 24351-24354, 24356-24378, 24380-24386, or 28515-28516.
84. The composition of any one of aspects 54 to 83, wherein the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
85. The composition of any one of aspects 54 to 83, wherein the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
86. The composition of any one of aspects 54 to 85, wherein the antisense oligomer binds to a targeted portion of a ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B RIC pre-mRNA.
87. The composition of any one of aspects 54 to 85, wherein the antisense oligomer binds to a targeted portion of a ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B RIC pre-mRNA.
88. A pharmaceutical composition comprising the antisense oligomer of any of the compositions of aspects 54 to 86, and an excipient.
89. A method of treating a subject in need thereof by administering the pharmaceutical composition of aspect 88 by intrathecal injection, intracerebroventricular injection, intraperitoneal injection, intramuscular injection, subcutaneous injection, or intravenous injection.
90. A pharmaceutical composition comprising:
   an antisense oligomer that hybridizes to a target sequence of a deficient *ATP1A2*, *CACNA1A*, *SETD5, SHANK3*, *NF2, DNMT1*, *TCF4, RAI1*, *PEX1*, *ARSA, EIF2B5, EIF2B1*, *EIF2B2, NPC1*, *ADAR, STXBP1*, *PRICKLE2*, *PRRT2*, *MFSD8, IDUA,* or *STX1B* mRNA transcript, wherein the deficient *ATP1A2*, *CACNA1A*, *SETD5*, *SHANK3*, *NF2*, *DNMT1*, *TCF4, RAI1*, *PEX1*, *ARSA*, *EIF2B5, EIF2B1*, *EIF2B2, NPC1*, *ADAR, STXBP1*, *PRICKLE2*, *PRRT2*, *MFSD8, IDUA,* or *STX1B* mRNA transcript comprises a retained intron, wherein the antisense oligomer induces splicing out of the retained intron from the deficient *ATP1A2*, *CACNA1A*, *SETD5*, *SHANK3*, *NF2*, *DNMT1*, *TCF4, RAI1*, *PEX1*, *ARSA*, *EIF2B5*, *EIF2B1*, *EIF2B2, NPC1*, *ADAR, STXBP1*, *PRICKLE2*, *PRRT2*, *MFSD8, IDUA,* or *STX1B* mRNA transcript; and a pharmaceutical acceptable excipient.
91. The pharmaceutical composition of aspect 90, wherein the antisense oligomer hybridizes to a target sequence of a deficient *ATP1A2*, *CACNA1A*, *SETD5, SHANK3*, *NF2*, *DNMT1*, *TCF4, RAI1*, *PEX1*, *ARSA*, *EIF2B5, EIF2B1*, *EIF2B2, NPC1*, *ADAR, STXBP1*, *PRICKLE2*, *PRRT2*, or *STX1B* mRNA transcript.
92. The pharmaceutical composition of aspect 91, wherein the deficient *ATP1A2*, *CACNA1A*, *SETD5*, *SHANK3*, *NF2*, *DNMT1*, *TCF4, RAI1*, *PEX1*, *ARSA*, *EIF2B5, EIF2B1*, *EIF2B2, NPC1*, *ADAR, STXBP1*, *PRICKLE2*, *PRRT2*, or *STX1B* mRNA transcript is a *ATP1A2*, *CACNA1A*, *SETD5*, *SHANK3*, *NF2*, *DNMT1*, *TCF4, RAI1*, *PEX1*, *ARSA*, *EIF2B5, EIF2B1*, *EIF2B2, NPC1*, *ADAR, STXBP1*, *PRICKLE2*, *PRRT2*, or *STX1B* RIC pre-mRNA transcript.
93. The pharmaceutical composition of aspect 90, wherein the antisense oligomer hybridizes to a target sequence of a deficient *ATP1A2*, *CACNA1A*, *SETD5, SHANK3*, *NF2*, *DNMT1*, *TCF4, RAI1*, *PEX1*, *ARSA*, *EIF2B5, EIF2B2, NPC1*, *ADAR, STXBP1*, *PRICKLE2*, *PRRT2*, or *STX1B* mRNA transcript.
94. The pharmaceutical composition of aspect 93, wherein the deficient *ATP1A2*, *CACNA1A*, *SETD5*, *SHANK3*, *NF2*, *DNMT1*, *TCF4, RAI1*, *PEX1*, *ARSA*, *EIF2B5, EIF2B2, NPC1*, *ADAR, STXBP1*, *PRICKLE2*, *PRRT2*, or *STX1B* mRNA transcript is a *ATP1A2*, *CACNA1A*, *SETD5*, *SHANK3*, *NF2*, *DNMT1*, *TCF4, RAI1*, *PEX1*, *ARSA*, *EIF2B5, EIF2B2*, *NPC1*, *ADAR, STXBP1*, *PRICKLE2*, *PRRT2,* or *STX1B* RIC pre-mRNA transcript.
95. The pharmaceutical composition of any one of aspects 90-94, wherein the targeted portion of the RIC pre-mRNA transcript is in the retained intron within the region +4000 relative to the 5' splice site of the retained intron to -2000 relative to the 3' spliced site of the retained intron.
96. The pharmaceutical composition of any one of aspects 90-95, wherein the RIC pre-mRNA transcript is encoded by a genetic sequence with at least about 80%, 85%, 90%, 95%, 96%, 97%, 98%, 99% or 100% sequence identity to SEQ ID NOs: 1-19.
97. The pharmaceutical composition of any one of aspects 90-96, wherein the RIC pre-mRNA transcript comprises a sequence with at least about 80%, 85%, 90%, 95%, 96%, 97%, 98%, 99% or 100% sequence identity to any one of SEQ ID NOs: 20-80.
98. The pharmaceutical composition of any one of aspects 90-97, wherein the antisense oligomer comprises a backbone modification comprising a phosphorothioate linkage or a phosphorodiamidate linkage.
99. The pharmaceutical composition of any one of aspects 90-98, wherein the antisense oligomer is an antisense oligonucleotide.
100. The pharmaceutical composition of any one of aspects 90-99, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
101. The pharmaceutical composition of any one of aspects 90-100, wherein the antisense oligomer comprises at least one modified sugar moiety.
102. The pharmaceutical composition of any one of aspects 90-101, wherein the antisense oligomer comprises from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
103. The pharmaceutical composition of any one of aspects 90-102, wherein the antisense oligomer is at least 80%, at least 85%, at least 90%, at least 95%, at least 98%, at least 99%, or is 100% complementary to a targeted portion of the RIC pre-mRNA transcript.
104. The pharmaceutical composition of any one of aspects 90-103, wherein the targeted portion of the RIC pre-mRNA transcript is within a sequence selected from SEQ ID NOs: 24351-24354, 24356-24378, 24380-24386, or 28515-28516.
105. The pharmaceutical composition of any one of aspects 90-104, wherein the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
106. The pharmaceutical composition of any one of aspects 90-104, wherein the antisense oligomer comprises a nucleotide sequence selected from SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.
107. The pharmaceutical composition of any one of the aspects 90-105, wherein the pharmaceutical composition is formulated for intrathecal injection, intracerebroventricular injection, intraperitoneal injection, intramuscular injection, subcutaneous injection, or intravenous injection.
108. A method of inducing processing of a mRNA transcript to facilitate removal of a retained intron to produce a fully processed mRNA transcript that encodes a functional form of a target protein, the method comprising:
   (a) contacting an antisense oligomer to a target cell of a subject;
   (b) hybridizing the antisense oligomer to the mRNA transcript, wherein the mRNA transcript is capable of encoding the functional form of the target protein and comprises at least one retained intron;
   (c) removing the at least one retained intron from the mRNA transcript to produce the fully processed mRNA transcript that encodes the functional form of the target protein; and
   (d) translating the functional form of the target protein from the fully processed mRNA transcript;
      wherein the mRNA transcript is selected from *ATP1A2*, *CACNA1A*, *SETD5, SHANK3*, *NF2, DNMT1*, *TCF4, RAI1*, *PEX1*, *ARSA, EIF2B5, EIF2B1*, *EIF2B2, NPC1*, *ADAR, STXBP1*, *PRICKLE2*, *PRRT2, MFSD8, IDUA,* or *STX1B* mRNA transcript.
109. The method of aspect 108, wherein the retained intron is an entire retained intron.
110. The method of aspect 108 or 109, wherein the mRNA transcript is a RIC pre-mRNA transcript.
111. The method of any one of aspects 108-110, wherein the mRNA transcript is selected from *ATP1A2*, *CACNA1A*, *SETD5, SHANK3*, *NF2*, *DNMT1*, *TCF4, RAI1*, *PEX1*, *ARSA*, *EIF2B5, EIF2B1*, *EIF2B2, NPC1*, *ADAR, STXBP1*, *PRICKLE2*, *PRRT2*, or *STX1B* mRNA transcript.
112. The method of any one of aspects 108-110, wherein the mRNA transcript is selected from *ATP1A2*, *CACNA1A*, *SETD5*, *SHANK3*, *NF2*, *DNMT1*, *TCF4, RAI1*, *PEX1*, *ARSA*, *EIF2B5, EIF2B2, NPC1*, *ADAR*, *STXBP1*, *PRICKLE2*, *PRRT2*, or *STX1B* mRNA transcript.
113. A method of treating a subject having a condition caused by a deficient amount or activity of a target protein comprising: administering to the subject an antisense oligomer comprising a nucleotide sequence with at least about 80%, 85%, 90%, 91%, 92%, 93%, 94%, 95%, 96%, 97%, 98%, or 99% sequence identity to any one of SEQ ID NOs: 81-2675, 2676-3302, 3303-3552, 3553-3794, 3795-4032, 4033-6411, 6412-7753, 7754-7964, 7965-8053, 8054-9332, 9333-9600, 9601-9803, 24387-28514, 13692-13760, 13761-15914, 15915-16930, 16931-23347, 23348-23535, or 23536-24350.

## Claims

1. A composition comprising an antisense oligomer for use in a method of treating a condition associated with ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein in a subject in need thereof, the method comprising the step of increasing expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein by cells of the subject, wherein the cells have a retained intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, the method comprising contacting the cells with the antisense oligomer, whereby the retained intron is constitutively spliced from the RIC pre-mRNA transcripts encoding ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, thereby increasing the level of mRNA encoding the target protein or functional RNA, and increasing the expression of ATP1A2, CACNA1A, SETD5, SHANK3, NF2, DNMT1, TCF4, RAI1, PEX1, ARSA, EIF2B5, EIF2B1, EIF2B2, NPC1, ADAR, MFSD8, STXBP1, PRICKLE2, PRRT2, IDUA, or STX1B protein, in the cells of the subject.

2. The composition for use of claim 1, wherein the target protein is ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B.

3. The composition for use of claim 1, wherein the condition is a disease or disorder.

4. The composition for use of claim 3, wherein the disease or disorder is Familial hemiplegic migraine-2, Familial Basilar migraine, Alternating hemiplegia of childhood, Episodic ataxia type 2, Familial hemiplegic migraine, Spinocerebellar ataxia type 6 , Mental retardation-23, 3p25 microdeletion syndrome, Phelan-McDermid syndrome, Schizophrenia-15, Neurofibromatosis, type 2, Meningioma, NF2-related, Schwannomatosis 1, Hereditary sensory neuropathy type IE, Autosomal dominant cerebellar ataxia, deafness, and narcolepsy, Pitt-hopkins syndrome, Smithmagenis syndrome, peroxisome biogenesis disorder 1a, Heimler syndrome-1, Metachromatic Leukodystrophy, Leukoencephalopathy with vanishing white matter, Niemann-Pick disease type C1 and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.

5. The composition for use of claim 4, wherein the target protein and RIC pre-mRNA are encoded by the ATP1A2, CACNA1A, SETD5, SHANK3, NF2, DNMT1, TCF4, RAI1, PEX1, ARSA, EIF2B5, EIF2B1, EIF2B2, NPC1, ADAR, STXBP1, PRICKLE2, PRRT2, or STX1B gene.

6. The composition for use of claim 4, wherein the target protein and RIC pre-mRNA are encoded by the ATP1A2, CACNA1A, SETD5, SHANK3, NF2, DNMT1, TCF4, RAI1, PEX1, ARSA, EIF2B5, EIF2B2, NPC1, ADAR, STXBP1, PRICKLE2, PRRT2, or STX1B gene.

7. The composition for use of any one of claims 1 to 5, wherein the antisense oligomer targets a portion of the RIC pre-mRNA that is in the retained intron within the region +6 relative to the 5' splice site of the retained intron to -16 relative to the 3' splice site of the retained intron.

8. The composition for use of any one of claims 1 to 7, wherein the antisense oligomer targets a portion of the RIC pre-mRNA that is in the retained intron within:
(a) the region +6 to +100 relative to the 5' splice site of the retained intron; or
(b) the region -16 to -100 relative to the 3' splice site of the retained intron.

9. The composition for use of any one of claims 1 to 5, wherein the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron.
